# EUROPEAN PATENT SPECIFICATION

(11) **EP 2 839 005 B1**
(45) Date of publication and mention of the grant of the patent: **06.01.2021**
(21) Application number: 13777669.6
(22) Date of filing: 22.04.2013
(51) Int. Cl.: C12N 15/113, A61K 48/00, C07H 21/02

(54) **MIRNA MODULATORS OF THERMOGENESIS**
MIRNA-MODULATOREN VON THERMOGENESE
MODULATEURS DE MICROARN DE LA THERMOGENÈSE

(30) Priority: 20.04.2012 US 201261636059 P; 10.08.2012 US 201261681750 P; 14.03.2013 US 201361782838 P
(43) Date of publication of application: 25.02.2015
(73) Proprietor: Aptamir Therapeutics, Inc., Austin, TX 78759 (US)
(72) Inventor: THIBONNIER, Marc, Naples, Florida 34119 (US)
(74) Representative: Cabinet Beau de Loménie
(86) International application number: PCT/US2013/037579
(87) International publication number: WO 2013/159091

(56) References cited:
- WO-A1-2008/116267
- WO-A1-2011/138457
- WO-A2-2010/108126
- WO-A2-2011/079263
- WO-A2-2011/153542
- WO-A2-2012/007725
- US-A1- 2011 224 286
- LIANG-LIANG SUN ET AL: "MicroRNA-15a positively regulates insulin synthesis by inhibiting uncoupling protein-2 expression", DIABETES RESEARCH AND CLINICAL PRACTICE, AMSTERDAM, NL, vol. 91, no. 1, 4 November 2010 (2010-11-04), pages 94-100, XP028359383, ISSN: 0168-8227, DOI: 10.1016/J.DIABRES.2010.11.006 [retrieved on 2010-11-10]
- PHILIP WING-LOK HO ET AL: "Mitochondrial Uncoupling Protein-2 (UCP2) Mediates Leptin Protection Against MPP+ Toxicity in Neuronal Cells", NEUROTOXICITY RESEARCH, vol. 17, no. 4, 10 September 2009 (2009-09-10), pages 332-343, XP055263959, CH ISSN: 1029-8428, DOI: 10.1007/s12640-009-9109-y
- LEI CAO ET AL: "White to Brown Fat Phenotypic Switch Induced by Genetic and Environmental Activation of a Hypothalamic-Adipocyte Axis", CELL METABOLISM, CELL PRESS, UNITED STATES, vol. 14, no. 3, 21 June 2011 (2011-06-21), pages 324-338, XP028388615, ISSN: 1550-4131, DOI: 10.1016/J.CMET.2011.06.020 [retrieved on 2011-08-12]
- CHEN, X ET AL.: 'RNA-Mediated Regulation And Noncoding RNAS: In Vitro Evidence Suggests That miR-133a-mediated Regulation Of Uncoupling Protein 2 (UCP2) Is An Indispensable Step In Myogenic Differentiation.' THE JOURNAL OF BIOLOGICAL CHEMISTRY . vol. 284, 2009, pages 5362 - 5369, XP055082654
- ALEXANDER, R ET AL.: 'MicroRNAs In Adipogenesis And As Therapeutic Targets For Obesity.' EXPERT OPINION IN THERAPEUTIC TARGETS. vol. 15, no. 5, May 2011, pages 623 - 636, XP008176641
- SUN, T ET AL.: 'MicroRNA let-7 Regulates 3T3-L1 Adipogenesis.' MOLECULAR ENDOCRINOLOGY vol. 23, 2009, pages 925 - 931, XP009134658
- ZARAGOSI, LE ET AL.: 'Small RNA Sequencing Reveals miR-642-3p As A Novel Adipoctye-Specific microRNA And miR-30 As A Key Regulator Of Human Adipogenesis.' GENOME BIOLOGY. vol. 12, 2011, pages 1 - 13, XP055194791
- RANTALAINEN, M ET AL.: 'MicroRNA Expression In Abdominal And Gluteal Adipose Tissue Is Associated With mRNA Expression Levels And Partly Genetically Driven' PLOS ONE vol. 6, no. 11, 2011, pages 1 - 12, XP055194792

## Description

### BACKGROUND OF THE INVENTION

Obesity has reached pandemic proportions, affecting all ages and socioeconomic groups. The World Health Organization estimated that in 2008, 1.5 billion adults aged 20 years and older were overweight and over 200 million men and 300 million women were obese. These figures are estimated to increase to 2.16 billion overweight and 1.12 billion obese individuals by 2030. Obesity is the source of lost earnings, restricted activity days, absenteeism, lower productivity at work (presenteeism), reduced quality of life, permanent disability, significant morbidity and mortality, and shortened lifespan. Indeed, the total annual economic cost of overweight and obesity in the United States and Canada caused by medical costs, excess mortality and disability was estimated to be about $300 billion in 2009. International studies on the economic costs of obesity have shown that they account for between 2% and 10% of total health care costs.

Obesity is the result of a chronic imbalance between energy intake and expenditure. This leads to storage of excess energy into adipocytes, which typically exhibit both hypertrophy (increase in cell size) and hyperplasia (increase in cell number or adipogenesis). The recent worsening of obesity is due to the combination of excessive consumption of energy-dense foods high in saturated fats and sugars, and reduced physical activity.

The current symptomatic medical treatments of obesity fail to achieve their long-term therapeutic goals, largely due to limited drug efficacy and patients' poor adherence with lifestyle changes and therapies. Several obesity drugs have been removed from the market for safety reasons and small molecules currently in development are struggling to gain regulatory approval because of their modest short-term efficacy and unknown safety profile. Presently, only restrictive and malabsorptive bariatric surgery can achieve significant long-term reduction of weight excess with some favorable cardiovascular benefits.

Accordingly, there is a need in the art for novel treatments for obesity.

### SUMMARY OF THE INVENTION

The present invention is further described in the claims.
In particular, the present invention relates to an antagomir of hsa-miR-22-3p for use in a method of treating diabetes or obesity in a human subject in need of treatment thereof. The present invention also relates to an *in vitro* or *ex vivo* method of:
- modulating respiratory chain uncoupling in a cell;
- modulating thermogenesis in a tissue; or
- increasing expression or activity of at least one thermogenic regulator in a cell, preferably of UCP1;
comprising contacting said cell or tissue with an antagomir of hsa-miR-22-3p.

In certain embodiments of the *in vitro* or *ex vivo* method, the cell is a pre-adipocyte, adipocyte, adipose tissue derived mesenchymal stem cell, hepatocyte, myocyte, or a precursor thereof. Optionally, adipocytes can be white fat or brown fat adipocytes.
Obesity is the consequence of a chronic imbalance of energy intake over expenditure, leading to the storage of excess energy inside white adipocytes. This disclosure features a novel treatment for obesity targeting peripheral adipocytes, including energy-storing lipid-filled white adipocytes (WAT), and energy-expending mitochondria-rich brown adipocytes (BAT). In addition, the disclosure provides methods for the modulation of thermogenesis (the process of heat production in organisms) using microRNA (miRNAs) agents. The methods described herein generally involve the direct and/or indirect modulation of at least one thermogenic regulator (e.g., a mitochondrial uncoupler, such as Uncoupling Protein 1 (UCP1 also known as Thermogenin) or Uncoupling Protein 2 (UCP2)) in a cell, tissue and/or subject using an isolated miRNA agent. UCPs uncouple oxidative phosphorylation from ATP synthesis. In certain instances, this uncoupling reaction results in energy dissipated as heat. Such methods are particularly advantageous in that they allow for the reduction of body fat in a subject without the subject having to adjust their caloric intake through dieting, modify their physical activity or undergo bariatric surgery. Accordingly, the antagomir of the invention is particularly useful for treating obesity.

The disclosure also provides novel miRNA agent compositions (e.g., miRNA, agomirs, and antagomirs) that can modulate the activity of thermogenic regulators. Yet further, the invention provides methods of screening for novel miRNA agents that modulate the activity of thermogenic regulators. Further still, the disclosure provides novel agent compositions (e.g. aptamer-miRNA complexes or "aptamirs") that provide cell/tissue-specific delivery of the miRNA agents.

The disclosure also provides a method of modulating respiratory chain uncoupling in a cell, the method comprising contacting the cell with an isolated miRNA agent that modulates the expression level and/or activity of at least one mitochondrial uncoupler. According to the disclosure, the method may further comprise the step of selecting a subject in need of modulating respiratory chain uncoupling (e.g., an obese patient). In one embodiment, the miRNA agent increases the expression level and/or activity of the at least one mitochondrial uncoupler. According to the disclosure, the mitochondrial uncoupler is UCP1 or UCP2. According to the disclosure, the method may further comprise determining the level of expression (mRNA or protein) or activity of the mitochondrial uncoupler.

The invention provides a method of modulating thermogenesis in a tissue, the method comprising contacting the tissue with an isolated miRNA agent that modulates the expression level and/or activity of at least one mitochondrial uncoupler, wherein the miRNA agent is the antagomir of has-miR-22-3p. In some embodiments, the method further comprises the step of selecting a subject in need of modulating thermogenesis (e.g., an obese patient). In one embodiment, the miRNA agent increases the expression level and/or activity of the at least one mitochondrial uncoupler. In certain embodiments, the mitochondrial uncoupler is UCP1 or UCP2. In certain embodiments, the method involves increasing thermogenesis. In certain embodiments, the method further comprises determining the level of expression (mRNA or protein) or activity of the mitochondrial uncoupler. In certain embodiments, the tissue is brown fat, white fat, subcutaneous adipose tissue, liver or muscle. In certain embodiments, the tissue is contacted with the miRNA agent *ex vivo.*

The disclosure also provides a method of treating obesity in human subject in need of treatment thereof, the method generally comprising administering to the human subject an effective amount of a miRNA agent that modulates activity of at least one mitochondrial uncoupler. In certain embodiments, the human subject selected for treatment has a genetic or epigenetic predisposition to obesity. In certain embodiments, the mitochondrial uncoupler is UCP1, UCP2 or UCP3.

According to the disclosure, the miRNA agent is an isolated miRNA selected from the group consisting of hsa-miR-1-1, hsa-miR-1-2, miR-19a-b, hsa-miR-105, hsa-miR-1283, hsa-mir-129, hsa-miR-133a-1, hsa-miR-133a-2, hsa-miR-143, hsa-mir-143-5p, hsa-mir-147, hsa-mir-149, hsa-mir-199a, hsa-mir-199b, hsa-mir-200c, hsa-mir-204, hsa-mir-205, hsa-miR-206, hsa-mir-21, hsa-mir-211, hsa-mir-218, hsa-mir-218-1, hsa-mir-218-2, hsa-mir-219-2, hsa-mir-219-2-3p, hsa-mir-22, hsa-mir-22-3p, hsa-mir-22-5p, hsa-mir-24-2, hsa-miR-30a-e, hsa-miR-3177-5p, hsa-mir-325, hsa-mir-331, hsa-mir-331-5p, hsa-miR-3613-3p, hsa-mir-362, hsa-mir-362-5p, hsa-miR-3658, hsa-mir-367, hsa-mir-371, hsa-mir-371-5p, hsa-mir-377, hsa-mir-378, hsa-mir-378a-5p, hsa-mir-382, hsa-mir-383, hsa-mir-422a, hsa-mir-425, hsa-miR-455-3p, hsa-miR-455-5p, hsa-miR-491, hsa-mir-508, hsa-mir-508-5p, hsa-mir-512-1, hsa-mir-512-2, hsa-miR-515-3p, hsa-mir-519e, hsa-miR-520a, hsa-mir-543, hsa-mir-545, hsa-mir-549, hsa-mir-556, and hsa-miR-568, hsa-mir-620, hsa-mir-643, hsa-mir-654-3p, hsa-miR-7a-g, hsa-mir-765, hsa-mir-871, hsa-mir-888, hsa-mir-888-3p, hsa-mir-92b, hsa-mir-93, hsa-mir-96, and hsa-mir-99a. In certain embodiments of all of the above aspects, the miRNA agent is an isolated miRNA that is 80%, 85%, 90%, 91%, 92%, 93%, 94%, 95%, 96%, 97%, 98%, or 99% identical to the sequence of a miRNA listed above. According to the disclosure, the miRNA agent is a seed sequence of a miRNA listed above.

Sun et al., 2011 (Diabetes Research and Clinical Practice Vol.91, pp 94-100) investigate the molecular mechanism of miR-15a promoting insulin biosynthesis. The increased expression of miR15a contributed to intracellular accumulation of insulin by repressing UCP-2 expression.
- Wing-Lok Ho et al., 2009 (Neurotoxicity Research, vol. 17, no. 4 pp 332-343) describes the critical role of uncoupling protein 2 (UCP2) in neuroprotective effects of leptin against MPP⁺ toxicity (Mitochondrial Membrane Potential) in a cell model in which UCP2 expression was knocked down by transfection of a plasmid expressing miRNA against human UCP2.
- Cao et al., 2011 (Cell Metabolism Vol. 14 no. 3 pp 324-338) investigated whether brain-derived neutrophic factor (BDNF) mediated the Enriched environment (EE)-associated molecular browning of White Adipose Tissue (WAT) by using micro-RNA (miR-Bdnf and miR-scr) to block EE-induced BDNF upregulation in hypothalamus.
- WO2011/153542 relates to the treatment and prevention of cardiac and metabolic disorders (such as cardiovascular diseases, obesity or diabetes) by inhibiting the expression or activity of miR-378.
- WO 2011/079263 describes a method of modulating function and/or expression of an UCP2 polynucleotide in patient cells or tissues *in vivo* or *in vitro* using miRNA.
- Chen et al., 2009 (The Journal of Biochemical Chemistry, Vol. 284, no. 8, pp5362-5369) describe for the first time, that the repression of UCP2 expression in cardiac and skeletal muscle resulted from its targeting by a muscle-specific microRNA, miR-133a.
- Alexander et al., 2011 (Expert Opinion in Therapeutic Targets, Vol. 15 no. 5 pages 623-636) provides a review of miRNAs involved in adipogenesis, from mesenchymal stem cell lineage determination through terminal adipocyte differentiation. They discuss the differential expression of miRNAs among adipose depots and the dysregulation of miRNAs in other metabolic tissues during metabolic pathophysiology and the therapeutic potential of targeting miRNAs in obesity.
- WO2011/138457 relates to microRNAs, such as miR-26a, that are capable of inducing and/or up-regulating the expression of UCP1 ("uncoupling protein 1", also known as thermogenin) for use in treating or preventing a disorder of the energy homeostasis such as obesity, overweight, adiposity, metabolic syndrome, or diseases or disorders related to energy homeostasis disorders such as diabetes (e.g., diabetes type 2), hypercholesterolemia or hypertension.

None of the above cited documents describe nor suggest the use of an antagomir of hsa-mir-22-3p to treat diabetes or obesity, to modulate respiratory chain uncoupling or to modulate thermogenesis.
According to the disclosure, the miRNA agent is an isolated miRNA selected from the group consisting of the 536 miRNAs set forth in Table 1.
According to the invention, the miRNA agent is an antagomir of hsa-miR-22-3p.
According to the disclosure, the miRNA agent is an isolated miRNA that is 80%, 85%, 90%, 91%, 92%, 93%, 94%, 95%, 96%, 97%, 98%, or 99% identical to the sequence of a miRNA listed in Table 1. According to the disclosure, the miRNA agent is a seed sequence of a miRNA listed in Table 1.

**Table 1.**

| **Adipocyte miRNAs listed in ascending order (miRBase 19 nomenclature):** | | | |
|---|---|---|---|
| hsa-let-7a-3p | hsa-miR-185-5p | hsa-miR-329 | hsa-miR-509-3p |
| hsa-let-7a-5p | hsa-miR-186-3p | hsa-miR-330-3p | hsa-miR-511 |
| hsa-let-7b-3p | hsa-miR-186-5p | hsa-miR-330-5p | hsa-miR-513a-3p |
| hsa-let-7b-5p | hsa-miR-187-3p | hsa-miR-331-3p | hsa-miR-513a-5p |
| hsa-let-7c | hsa-miR-188-5p | hsa-miR-331-5p | hsa-miR-513b |
| hsa-let-7d-3p | hsa-miR-18a-3p | hsa-miR-335-3p | hsa-miR-514a-3p |
| hsa-let-7d-5p | hsa-miR-18a-5p | hsa-miR-335-5p | hsa-miR-515-3p |
| hsa-let-7e-5p | hsa-miR-18b-5p | hsa-miR-337-3p | hsa-miR-516b-3p |
| hsa-let-7f-1-3p | hsa-miR-1909-3p | hsa-miR-337-5p | hsa-miR-516b-5p |
| hsa-let-7f-5p | hsa-miR-190a | hsa-miR-338-3p | hsa-miR-518b |
| hsa-let-7g-3p | hsa-miR-190b | hsa-miR-338-5p | hsa-miR-518e-3p |
| hsa-let-7g-5p | hsa-miR-191-3p | hsa-miR-339-3p | hsa-miR-518e-5p |
| hsa-let-7i-3p | hsa-miR-191-5p | hsa-miR-339-5p | hsa-miR-518f-3p |
| hsa-let-7i-5p | hsa-miR-192-5p | hsa-miR-33a-5p | hsa-miR-519a-5p |
| hsa-miR-1 | hsa-miR-193a-3p | hsa-miR-33b-5p | hsa-miR-519b-5p |
| hsa-miR-100-5p | hsa-miR-193a-5p | hsa-miR-340-3p | hsa-miR-519c-3p |
| hsa-miR-101-3p | hsa-miR-193b-3p | hsa-miR-340-5p | hsa-miR-519c-5p |
| hsa-miR-101-5p | hsa-miR-193b-5p | hsa-miR-342-3p | hsa-miR-519d |
| hsa-miR-103a-2-5p | hsa-miR-194-5p | hsa-miR-342-5p | hsa-miR-520c-3p |
| hsa-miR-103a-3p | hsa-miR-195-3p | hsa-miR-345-5p | hsa-miR-520e |
| hsa-miR-103b | hsa-miR-195-5p | hsa-miR-346 | hsa-miR-520f |
| hsa-miR-105-5p | hsa-miR-196a-5p | hsa-miR-34a-5p | hsa-miR-520g |
| hsa-miR-106a-5p | hsa-miR-196b-5p | hsa-miR-34b-3p | hsa-miR-520h |
| hsa-miR-106b-3p | hsa-miR-197-3p | hsa-miR-34b-5p | hsa-miR-521 |
| hsa-miR-106b-5p | hsa-miR-198 | hsa-miR-34c-5p | hsa-miR-522-5p |
| hsa-miR-107 | hsa-miR-199a-3p | hsa-miR-3545-5p | hsa-miR-523-5p |
| hsa-miR-10a-3p | hsa-miR-199a-5p | hsa-miR-3591-3p | hsa-miR-525-3p |
| hsa-miR-10a-5p | hsa-miR-199b-3p | hsa-miR-361-3p | hsa-miR-532-3p |
| hsa-miR-10b-3p | hsa-miR-199b-5p | hsa-miR-361-5p | hsa-miR-532-5p |
| hsa-miR-10b-5p | hsa-miR-19a-3p | hsa-miR-3613-5p | hsa-miR-539-5p |
| hsa-miR-1179 | hsa-miR-19b-3p | hsa-miR-3615 | hsa-miR-542-3p |
| hsa-miR-1185-5p | hsa-miR-200a-3p | hsa-miR-362-3p | hsa-miR-542-5p |
| hsa-miR-1208 | hsa-miR-200a-5p | hsa-miR-362-5p | hsa-miR-545-3p |
| hsa-miR-122-5p | hsa-miR-200b-3p | hsa-miR-363-3p | hsa-miR-545-5p |
| hsa-miR-1227-3p | hsa-miR-200c-3p | hsa-miR-363-5p | hsa-miR-548d-3p |
| hsa-miR-1228-5p | hsa-miR-202-3p | hsa-mir-365a-3p | hsa-miR-548e |
| hsa-miR-1229-3p | hsa-miR-203a | hsa-mir-3653 | hsa-miR-548i |
| hsa-miR-124-3p | hsa-miR-204-5p | hsa-miR-3656 | hsa-miR-548m |
| hsa-miR-125a-3p | hsa-miR-205-5p | hsa-miR-365a-3p | hsa-miR-550a-5p |
| hsa-miR-125a-5p | hsa-miR-206 | hsa-miR-365a-5p | hsa-miR-551b-3p |
| hsa-miR-125b-1-3p | hsa-miR-20a-3p | hsa-miR-367-3p | hsa-miR-552 |
| hsa-miR-125b-2-3p | hsa-miR-20a-5p | hsa-mir-3676-3p | hsa-miR-553 |
| hsa-miR-125b-5p | hsa-miR-20b-5p | hsa-miR-369-3p | hsa-miR-554 |
| hsa-miR-126-3p | hsa-miR-21-3p | hsa-miR-369-5p | hsa-miR-557 |
| hsa-miR-126-5p | hsa-miR-21-5p | hsa-miR-370 | hsa-miR-563 |
| hsa-miR-1260a | hsa-miR-210 | hsa-miR-371a-3p | hsa-miR-564 |
| hsa-miR-1260b | hsa-miR-211-5p | hsa-miR-373-3p | hsa-miR-567 |
| hsa-miR-1268a | hsa-miR-2110 | hsa-miR-373-5p | hsa-miR-569 |
| hsa-miR-127-3p | hsa-miR-212-3p | hsa-miR-374a-3p | hsa-miR-570-3p |
| hsa-miR-127-5p | hsa-miR-214-3p | hsa-miR-374a-5p | hsa-miR-572 |
| hsa-miR-1271-5p | hsa-miR-214-5p | hsa-miR-374b-3p | hsa-miR-574-3p |
| hsa-miR-1273a | hsa-miR-215 | hsa-miR-374b-5p | hsa-miR-574-5p |
| hsa-miR-1277-3p | hsa-miR-216a-5p | hsa-miR-375 | hsa-miR-575 |
| hsa-miR-128 | hsa-miR-217 | hsa-mir-376a-2-5p | hsa-miR-576-3p |
| hsa-miR-128-2 | hsa-miR-218-5p | hsa-miR-376a-3p | hsa-miR-576-5p |
| hsa-miR-1285-3p | hsa-miR-219-1-3p | hsa-miR-376a-5p | hsa-miR-582-3p |
| hsa-miR-1287 | hsa-miR-219-5p | hsa-miR-376b-3p | hsa-miR-582-5p |
| hsa-miR-1288 | hsa-miR-22-3p | hsa-miR-376c-3p | hsa-miR-583 |
| hsa-miR-129-5p | hsa-miR-22-5p | hsa-miR-377-3p | hsa-miR-584-5p |
| hsa-miR-1290 | hsa-miR-221-3p | hsa-miR-378a-3p | hsa-miR-585 |
| hsa-miR-1292-5p | hsa-miR-221-5p | hsa-miR-378a-5p | hsa-miR-586 |
| hsa-miR-1301 | hsa-miR-222-3p | hsa-miR-378c | hsa-miR-589-5p |
| hsa-miR-1305 | hsa-miR-222-5p | hsa-miR-378d | hsa-miR-590-3p |
| hsa-mir-1307-3p | hsa-miR-223-3p | hsa-miR-379-5p | hsa-miR-590-5p |
| hsa-miR-130a-3p | hsa-miR-223-5p | hsa-miR-380-3p | hsa-miR-595 |
| hsa-miR-130b-3p | hsa-miR-224-3p | hsa-miR-381-3p | hsa-miR-598 |
| hsa-miR-130b-5p | hsa-miR-224-5p | hsa-miR-382-5p | hsa-miR-601 |
| hsa-miR-132-3p | hsa-miR-2355-3p | hsa-miR-3 83 | hsa-miR-602 |
| hsa-miR-132-5p | hsa-miR-23a-3p | hsa-miR-3 84 | hsa-miR-603 |
| hsa-miR-1323 | hsa-miR-23b-3p | hsa-miR-3912 | hsa-miR-605 |
| hsa-miR-133a | hsa-miR-23b-5p | hsa-miR-3928 | hsa-miR-606 |
| hsa-miR-133b | hsa-miR-24-1-5p | hsa-miR-409-3p | hsa-miR-609 |
| hsa-miR-134 | hsa-miR-24-2-5p | hsa-miR-409-5p | hsa-miR-611 |
| hsa-miR-135a-5p | hsa-miR-24-3p | hsa-miR-410 | hsa-miR-615-3p |
| hsa-miR-135b-5p | hsa-miR-25-3p | hsa-miR-411-5p | hsa-miR-619 |
| hsa-miR-136-3p | hsa-miR-26a-2-3p | hsa-miR-421 | hsa-miR-625-5p |
| hsa-miR-136-5p | hsa-miR-26a-5p | hsa-miR-422a | hsa-miR-627 |
| hsa-miR-137 | hsa-miR-26b-3p | hsa-miR-422b | hsa-miR-628-3p |
| hsa-miR-138-1-3p | hsa-miR-26b-5p | hsa-miR-423-3p | hsa-miR-628-5p |
| hsa-miR-138-5p | hsa-miR-27a-3p | hsa-miR-423-5p | hsa-miR-629-3p |
| hsa-miR-139-3p | hsa-miR-27a-5p | hsa-miR-424-3p | hsa-miR-629-5p |
| hsa-miR-139-5p | hsa-miR-27b-3p | hsa-miR-424-5p | hsa-miR-630 |
| hsa-miR-140-3p | hsa-miR-27b-5p | hsa-miR-425-3p | hsa-miR-636 |
| hsa-miR-140-5p | hsa-miR-28-3p | hsa-miR-425-5p | hsa-miR-63 8 |
| hsa-miR-141-3p | hsa-miR-28-5p | hsa-miR-429 | hsa-miR-639 |
| hsa-miR-142-3p | hsa-miR-296-5p | hsa-miR-431-5p | hsa-miR-641 |
| hsa-miR-142-5p | hsa-miR-297 | hsa-miR-432-5p | hsa-miR-642a-3p |
| hsa-miR-143-3p | hsa-miR-298 | hsa-miR-433 | hsa-miR-642a-5p |
| hsa-miR-143-5p | hsa-miR-299-3p | hsa-miR-4421 | hsa-miR-646 |
| hsa-miR-144-3p | hsa-miR-299-5p | hsa-miR-449a | hsa-miR-649 |
| hsa-miR-144-5p | hsa-miR-29a-3p | hsa-miR-450a-5p | hsa-miR-651 |
| hsa-miR-145-3p | hsa-miR-29a-5p | hsa-miR-450b-3p | hsa-miR-652-3p |
| hsa-miR-145-5p | hsa-miR-29b-1-5p | hsa-miR-450b-5p | hsa-miR-653 |
| hsa-miR-1468 | hsa-miR-29b-2-5p | hsa-miR-4510 | hsa-miR-654-3p |
| hsa-miR-146a-5p | hsa-miR-29b-3p | hsa-miR-4516 | hsa-miR-659-3p |
| hsa-miR-146b-3p | hsa-miR-29c-3p | hsa-miR-451a | hsa-miR-660-5p |
| hsa-miR-146b-5p | hsa-miR-29c-5p | hsa-miR-452-3p | hsa-miR-663a |
| hsa-miR-147a | hsa-miR-301a-3p | hsa-miR-452-5p | hsa-miR-664a-3p |
| hsa-miR-148a-3p | hsa-miR-301b | hsa-miR-454-3p | hsa-miR-664a-5p |
| hsa-miR-148a-5p | hsa-miR-302a-5p | hsa-miR-454-5p | hsa-miR-668 |
| hsa-miR-148b-3p | hsa-miR-302b-5p | hsa-miR-455-3p | hsa-miR-671-5p |
| hsa-miR-148b-5p | hsa-miR-302c-5p | hsa-miR-455-5p | hsa-miR-675-3p |
| hsa-miR-149-5p | hsa-miR-302d-3p | hsa-miR-4634 | hsa-miR-675-5p |
| hsa-miR-150-3p | hsa-miR-3065-3p | hsa-miR-4732-5p | hsa-miR-7-2-3p |
| hsa-miR-150-5p | hsa-miR-3065-5p | hsa-miR-4792 | hsa-miR-7-5p |
| hsa-miR-151a-3p | hsa-miR-3074-3p | hsa-miR-483-3p | hsa-miR-708-3p |
| hsa-miR-151a-5p | hsa-miR-3074-5p | hsa-miR-483-5p | hsa-miR-708-5p |
| hsa-miR-151b | hsa-miR-30a-3p | hsa-miR-484 | hsa-miR-718 |
| hsa-miR-152 | hsa-miR-30a-5p | hsa-miR-485-5p | hsa-miR-744-5p |
| hsa-miR-153 | hsa-miR-30b-3p | hsa-miR-486-3p | hsa-miR-765 |
| hsa-miR-1539 | hsa-miR-30b-5p | hsa-miR-486-5p | hsa-miR-769-5p |
| hsa-miR-154-3p | hsa-miR-30c-1-3p | hsa-miR-487b | hsa-miR-770-5p |
| hsa-miR-154-5p | hsa-miR-30c-2-3p | hsa-miR-488-3p | hsa-miR-874 |
| hsa-miR-155-5p | hsa-miR-30c-5p | hsa-miR-489 | hsa-miR-885-3p |
| hsa-miR-15a-3p | hsa-miR-30d-3p | hsa-miR-491-3p | hsa-miR-887 |
| hsa-miR-15a-5p | hsa-miR-30d-5p | hsa-miR-491-5p | hsa-miR-889 |
| hsa-miR-15b-3p | hsa-miR-30e-3p | hsa-miR-492 | hsa-miR-890 |
| hsa-miR-15b-5p | hsa-miR-30e-5p | hsa-miR-493-3p | hsa-miR-891a |
| hsa-miR-16-1-3p | hsa-miR-31-3p | hsa-miR-493-5p | hsa-miR-891b |
| hsa-miR-16-2-3p | hsa-miR-31-5p | hsa-miR-494 | hsa-miR-9-5p |
| hsa-miR-16-5p | hsa-miR-3120-3p | hsa-miR-495-3p | hsa-miR-92a-3p |
| hsa-miR-17-3p | hsa-miR-3120-5p | hsa-miR-497-5p | hsa-miR-92b-3p |
| hsa-miR-17-5p | hsa-miR-3184-5p | hsa-miR-498 | hsa-miR-93-3p |
| hsa-miR-181a-2-3p | hsa-miR-32-3p | hsa-miR-499a-5p | hsa-miR-93-5p |
| hsa-miR-181a-3p | hsa-miR-32-5p | hsa-miR-500a-3p | hsa-miR-93 5 |
| hsa-miR-181a-5p | hsa-miR-320a | hsa-miR-501-3p | hsa-miR-942 |
| hsa-miR-181b-5p | hsa-miR-320b | hsa-miR-501-5p | hsa-miR-95 |
| hsa-miR-181c-3p | hsa-miR-320c | hsa-miR-502-3p | hsa-miR-96-3p |
| hsa-miR-181c-5p | hsa-miR-323a-3p | hsa-miR-502-5p | hsa-miR-96-5p |
| hsa-miR-181d | hsa-miR-324-3p | hsa-miR-503-5p | hsa-miR-98-5p |
| hsa-miR-182-5p | hsa-miR-324-5p | hsa-miR-504 | hsa-miR-99a-3p |
| hsa-miR-183-5p | hsa-miR-325 | hsa-miR-505-3p | hsa-miR-99a-5p |
| hsa-miR-184 | hsa-miR-326 | hsa-miR-505-5p | hsa-miR-99b-3p |
| hsa-miR-185-3p | hsa-miR-328 | hsa-miR-506-3p | hsa-miR-99b-5p |

According to the disclosure, the miRNA agent is a miRNA selected from the group consisting of the isolated miRNAs set forth in Tables 11, 13 and 14. According to the disclosure, the miRNA agent is an isolated miRNA that is 80%, 85%, 90%, 91%, 92%, 93%, 94%, 95%, 96%, 97%, 98%, or 99% identical to the sequence of a miRNA listed in Tables 1, 11, 13 and 14. According to the disclosure, the miRNA agent is a seed sequence of a miRNA listed in Tables 11, 13 and 14.

According to the disclosure, the miRNA agent is an agomir or antagomir of a miRNA selected from the group consisting of the miRNAs set forth in Table 1.

According to the disclosure, the miRNA agent is an agomir or antagomir of a miRNA selected from the group consisting of hsa-miR-1-1, hsa-miR-1-2, miR-19a-b, hsa-miR-105, hsa-miR-1283, hsa-mir-129, hsa-miR-133a-1, hsa-miR-133a-2, hsa-miR-143, hsa-mir-143-5p, hsa-mir-147, hsa-mir-149, hsa-mir-199a, hsa-mir-199b, hsa-mir-200c, hsa-mir-204, hsa-mir-205, hsa-miR-206, hsa-mir-21, hsa-mir-211, hsa-mir-218, hsa-mir-218-1, hsa-mir-218-2, hsa-mir-219-2, hsa-mir-219-2-3p, hsa-mir-22, hsa-mir-22-3p, hsa-mir-22-5p, hsa-mir-24-2, hsa-miR-30a-e, hsa-miR-3177-5p, hsa-mir-325, hsa-mir-331, hsa-mir-331-5p, hsa-miR-3613-3p, hsa-mir-362, hsa-mir-362-5p, hsa-miR-3658, hsa-mir-367, hsa-mir-371, hsa-mir-371-5p, hsa-mir-377, hsa-mir-378, hsa-mir-378a-5p, hsa-mir-382, hsa-mir-383, hsa-mir-422a, hsa-mir-425, hsa-miR-455-3p, hsa-miR-455-5p, hsa-miR-491, hsa-mir-508, hsa-mir-508-5p, hsa-mir-512-1, hsa-mir-512-2, hsa-miR-515-3p, hsa-mir-519e, hsa-miR-520a, hsa-mir-543, hsa-mir-545, hsa-mir-549, hsa-mir-556, and hsa-miR-568, hsa-mir-620, hsa-mir-643, hsa-mir-654-3p, hsa-miR-7a-g, hsa-mir-765, hsa-mir-871, hsa-mir-888, hsa-mir-888-3p, hsa-mir-92b, hsa-mir-93, hsa-mir-96, and hsa-mir-99a.

According to the disclosure, the miRNA agent is an agomir or antagomir of a miRNA selected from the group consisting of the miRNAs set forth in Table 11.

According to the disclosure, the miRNA agent is an antagomir of a miRNA selected from the group consisting of hsa-miR-19b-2-5p, hsa-miR-21-5p, hsa-miR-130b-5p, hsa-miR-211, hsa-miR-325, hsa-miR-382-3p/5p, hsa-miR-543, hsa-miR-515-3p, and hsa-miR-545.

According to the disclosure, the miRNA agent is an antagomir of a miRNA selected from the group consisting of hsa-miR-331-5p, hsa-miR-552, hsa-miR-620, and hsa-miR-1179.

In certain embodiments of all of the above aspects, the miRNA agent is linked to a targeting moiety (e.g., an aptamer). In one embodiment, the targeting moiety delivers the miRNA agent to a specific cell type or tissue.

In certain embodiments of all of the above aspects, the miRNA agent directly binds to the mRNA or promoter region of at least one mitochondrial uncoupler.

In certain embodiments of all of the above aspects, the miRNA agent directly binds to the 5'UTR or coding sequence of the mRNA of at least one mitochondrial uncoupler.

In certain embodiments of all of the above aspects, the miRNA agent directly binds to the 3'UTR of the mRNA of at least one mitochondrial uncoupler.

In certain embodiments of all of the above aspects, the miRNA agent modulates the activity of an activator or repressor of a mitochondrial uncoupling protein. In one embodiment, the miRNA agent directly binds to the mRNA or promoter region of the activator or repressor. In one embodiment, the miRNA agent directly binds to the 5'UTR or coding sequence of the mRNA of the activator or repressor. In one embodiment, the miRNA agent directly binds to the 3'UTR of the mRNA of the activator or repressor. In one embodiment, the activator or repressor is selected from the group listed in Table 2.

In certain embodiments of all of the above aspects, the mRNA or protein expression of the mitochondrial uncoupling protein is upregulated.

In certain embodiments of all of the above aspects, the mitochondrial uncoupling activity of the mitochondrial uncoupling protein is upregulated.

The disclosure also provides a method of screening for a miRNA agent that modulates thermogenesis, the method generally comprising: providing an indicator cell; contacting the indicator cell with a test miRNA agent; and determining the cellular activity of at least one thermogenic regulator in the indicator cell in the presence and absence of the miRNA agent, wherein a change in the activity of the thermogenic regulator in the presence of the test miRNA agent identifies the test miRNA agent as a miRNA agent that modulates thermogenesis. The indicator cell can be a mammalian cell. According to the disclosure, the indicator cell is a human cell comprising at least a portion of a human genome.

In certain embodiments, the cell is a pre-adipocyte, adipocyte, adipose tissue derived mesenchymal stem cell, hepatocyte, myocyte, or a precursor thereof.
According to the disclosure, the cellular activity of the thermogenic regulator determined in the method is the mRNA expression level, protein expression level or mitochondrial uncoupling activity of the thermogenic regulator.

According to the disclosure, the test miRNA agent increases the activity of the thermogenic regulator compared to the level of activity of the thermogenic regulator in the absence of the test miRNA agent.

In certain embodiments, the thermogenic regulator is UCP1 or UCP2.

The disclosure provides an agomir or antagomir that modulates the activity of at least one thermogenic regulator in a cell.

According to the disclosure, the agomir or antagomir is an agomir or antagomir of a miRNA selected from the group consisting of the miRNAs set forth in Tables 11, 13 and 14.

According to the disclosure, the agomir or antagomir is an agomir or antagomir of a miRNA selected from the group consisting of the miRNAs set forth in Table 1.

According to the disclosure, the agomir or antagomir is an agomir or antagomir of a miRNA selected from the group consisting of hsa-miR-1-1, hsa-miR-1-2, miR-19a-b, hsa-miR-105, hsa-miR-1283, hsa-mir-129, hsa-miR-133a-1, hsa-miR-133a-2, hsa-miR-143, hsa-mir-143-5p, hsa-mir-147, hsa-mir-149, hsa-mir-199a, hsa-mir-199b, hsa-mir-200c, hsa-mir-204, hsa-mir-205, hsa-miR-206, hsa-mir-21, hsa-mir-211, hsa-mir-218, hsa-mir-218-1, hsa-mir-218-2, hsa-mir-219-2, hsa-mir-219-2-3p, hsa-mir-22, hsa-mir-22-3p, hsa-mir-22-5p, hsa-mir-24-2, hsa-miR-30a-e, hsa-miR-3177-5p, hsa-mir-325, hsa-mir-331, hsa-mir-331-5p, hsa-miR-3613-3p, hsa-mir-362, hsa-mir-362-5p, hsa-miR-3658, hsa-mir-367, hsa-mir-371, hsa-mir-371-5p, hsa-mir-377, hsa-mir-378, hsa-mir-378a-5p, hsa-mir-382, hsa-mir-383, hsa-mir-422a, hsa-mir-425, hsa-miR-455-3p, hsa-miR-455-5p, hsa-miR-491, hsa-mir-508, hsa-mir-508-5p, hsa-mir-512-1, hsa-mir-512-2, hsa-miR-515-3p, hsa-mir-519e, hsa-miR-520a, hsa-mir-543, hsa-mir-545, hsa-mir-549, hsa-mir-556, and hsa-miR-568, hsa-mir-620, hsa-mir-643, hsa-mir-654-3p, hsa-miR-7a-g, hsa-mir-765, hsa-mir-871, hsa-mir-888, hsa-mir-888-3p, hsa-mir-92b, hsa-mir-93, hsa-mir-96, and hsa-mir-99a.

According to the disclosure, the agomir or antagomir is an antagomir of a miRNA selected from the group consisting of hsa-miR-19b-2-5p, ha-miR-21-5p, hsa-miR-130b-5p, hsa-miR-211, hsa-miR-325, hsa-miR-382-3p/5p, hsa-miR-543, hsa-miR-515-3p, and hsa-miR-545.

According to the disclosure, the agomir or antagomir is an antagomir of a miRNA selected from the group consisting of hsa-miR-331-5p, hsa-miR-552, hsa-miR-620, and hsa-miR-1179.

In certain embodiments, the agomir or antagomir is linked to a targeting moiety.

In certain embodiments, the targeting moiety is an aptamer.

In certain embodiments, the targeting moiety delivers the agomir or antagomir to a specific cell type or tissue.

In certain embodiments, the agomir or antagomir directly binds to the mRNA or promoter region of at least one mitochondrial uncoupler.

In certain embodiments, the agomir or antagomir directly binds to the 5'UTR or coding sequence of the mRNA of at least one mitochondrial uncoupler.

In certain embodiments, the agomir or antagomir directly binds to the 3'UTR of the mRNA of at least one mitochondrial uncoupler.

In certain embodiments, the agomir or antagomir modulates the activity of an activator or repressor of a mitochondrial uncoupling protein.

In certain embodiments, the activator or repressor is selected from the group listed in Table 2.

In certain embodiments, the agomir or antagomir directly binds to the mRNA or promoter region of the activator or repressor.

In certain embodiments, the agomir or antagomir directly binds to the 5'UTR or coding sequence of the mRNA of the activator or repressor. In other embodiments, the agomir or antagomir directly binds to the 3'UTR of the mRNA of the activator or repressor.

The disclosure also provides a pharmaceutical composition comprising two or more miRNAs selected from hsa-let-7a agomir, hsa-let-7a antagomir, hsa-miR-1 agomir, hsa-miR-1 antagomir, hsa-miR-19b agomir, hsa-miR-19b antagomir, hsa-miR-30b agomir and hsa-miR-30b antagomir. In certain embodiments the pharmaceutical composition also includes a pharmaceutically acceptable excipient. In certain embodiments, the two or more miRNAs are expressed from a recombinant vector. The recombinant vector can be selected from DNA plasmids, viral vectors and DNA minicircles.

The disclosure also provides a method of inducing pre-adipocytes to differentiate initially into white adipocytes and subsequently into brown adipocytes comprising administering to a population of pre-adipocytes one or more miRNAs selected from hsa-let-7a agomir, hsa-let-7a antagomir, hsa-miR-1 agomir, hsa-miR-1 antagomir, hsa-miR-19b agomir, hsa-miR-19b antagomir, hsa-miR-30b agomir and hsa-miR-30b antagomir. The one or more miRNAs can also be selected from hsa-let-7a agomir, hsa-let-7a antagomir, hsa-miR-1 agomir, hsa-miR-1 antagomir, hsa-miR-19b agomir, hsa-miR-19b antagomir, hsa-miR-30b agomir and hsa-miR-30b antagomir. In certain embodiments, the induction of pre-adipocytes to differentiate into adipocytes is greater than the differentiation of pre-adipocytes to adipocytes when pre-adipocytes are exposed to 100 nM rosiglitazone for two days followed by maintenance medium. In certain embodiments, the adipocytes are brown adipocytes. In other embodiments, the adipocytes are white adipocytes. Additional criteria for differentiation can be found in the Examples, below.

The disclosure also provides a method for decreasing the lipid content of adipocytes comprising administering to a population of adipocytes one or more miRNAs selected from the group consisting of hsa-let-7a agomir, hsa-let-7a antagomir, hsa-miR-1 agomir, hsa-miR-1 antagomir, hsa-miR-19b agomir, hsa-miR-19b antagomir, hsa-miR-30b agomir and hsa-miR-30b antagomir. In certain embodiments, the lipid content of the adipocytes is less than the lipid content of adipocytes exposed to 100 nM rosiglitazone for two days followed by maintenance medium or less than the fat content of adipocytes exposed to 100 nM rosiglitazone for the duration of culture. The duration of culture can be 8-16, 10-14 or 14 days. The duration of culture can also be 8, 9, 10, 11, 12, 13, 14, 15, 16, 17, 18, 19 or 20 days. Additional criteria for lipid content of adipocytes can be found in the Examples, below.

The disclosure also provides a method for increasing insulin sensitivity in a subject in need thereof comprising administering the subject one or more miRNAs selected from the group consisting of hsa-let-7a agomir, hsa-let-7a antagomir, hsa-miR-1 agomir, hsa-miR-1 antagomir, hsa-miR-19b agomir, hsa-miR-19b antagomir, hsa-miR-30b agomir and hsa-miR-30b antagomir.

In certain embodiments, the subject is a mammal.

The disclosure also provides a method of increasing expression or activity of one or more uncoupling proteins in a cell comprising administering to the cell one or more, two or more, or three or more miRNAs selected from the group consisting of hsa-let-7a antagomir, hsa-miR-1 agomir, hsa-miR-19b agomir and hsa-miR-30b agomir. In certain embodiments, the cell is selected from the group consisting of a brown adipocyte, a white adipocyte, a subcutaneous adipocyte, a liver cell or a muscle cell. In other embodiments, the one or more uncoupling proteins include UCP1 or UCP2. In certain embodiments, the method is an ex vivo method. In other embodiments, the method is an in vivo method. In certain embodiments, the method involves selecting a subject (e.g., a human) in need of increasing the level of expression or activity of one or more uncoupling proteins (e.g., UCP1, UCP2). In some embodiments, the subject has, or is at risk of developing, obesity. In certain embodiments, the subject has, or is at risk of developing, diabetes. In certain embodiments, the method further comprises determining the expression level (mRNA or protein) or activity of the one or more uncoupling proteins.

The disclosure also provides a method of causing fat loss in a subject in need thereof comprising administering the subject one or more miRNAs selected from the group consisting of hsa-let-7a antagomir, hsa-miR-1 agomir, hsa-miR-19b agomir and hsa-miR-30b agomir. In certain embodiments, the subject is a mammal. In other embodiments, the mammal is a human.

### BRIEF DESCRIPTION OF THE DRAWINGS

Figures 1A and 1B are schematic representations of the interactions of 83 thermogenic regulators determined using the STRING 9.0 database at two different levels of stringency.
Figure 2A is a schematic representation of the interaction of 83 thermogenic regulators determined using the Ingenuity Pathway Analysis Software program.
Figure 2B is a schematic representation of the interaction of 83 thermogenic regulators determined using the Reactome Functional Interaction Network program.
Figure 3 is a schematic representation of the overlap of results from multiple miRNA prediction programs predicting miRNA binding sites in the 5'UTR, promoter region, coding sequence and 3'UTR of the human UCP1 gene.
Figure 4 is a schematic representation of the overlap of results from multiple miRNA prediction programs predicting miRNA binding sites in the 5'UTR, promoter region, coding sequence and 3'UTR of the genes of 83 thermogenic regulators.
Figure 5 is a schematic representation of oxidative phosphorylation in mitochondria, illustrating the uncoupling of oxidative phosphorylation from ATP synthesis by UCP1 to generate heat.
Figure 6 depicts the transcriptional control of UCP1 by other exemplary thermogenic regulators.
Figure 7 depicts exemplary positive (a) and negative (b) transcriptional regulators of UCP1 gene transcription.
Figure 8A depicts the location of various regulatory elements in reference to the transcription start site (position 5,001) in the 15,910 base pair (bp) sequence of the human UCP1 gene (NCBI Reference Sequence: gi|237858805|ref|NG_012139.1| Homo sapiens uncoupling protein 1 (mitochondrial, proton carrier) (UCP1), RefSeqGene on chromosome 4).
Figure 8B depicts the location of various regulatory elements in reference to the transcription start site (position 5,001) in the 15,174 bp sequence of the human UCP2 gene (ENSG00000175567), including 5,000 bp 5'UTR and 2,000 bp 3'UTR on chromosome 11.
Figure 9 is a bar graph showing relative fluorescence in pre-adipocytes either unlabeled or transfected with a Dy547-labeled non-targeting miRNA mimic or hairpin inhibitor.
Figure 10A is a bar graph showing the reduction of GAPDH gene expression in pre-adipocytes transfected with siRNA control and a GAPDH siRNA 4 days after transfection.
Figure 10B is a bar graph showing the reduction of GAPDH gene expression in pre-adipocytes transfected with siRNA control and a GAPDH siRNA 12 days after transfection.
Figure 11A is a light micrograph of pre-adipocytes stained with Oil Red O cultured for 2 weeks in maintenance medium alone.
Figure 11B is a light micrograph of pre-adipocytes stained with Oil Red O cultured in the presence of insulin, tri-iodothyronine, dexamethasone, isobutyl-methylxanthine and rosiglitazone for two days followed by maintenance medium for 12 days.
Figure 11C is a light micrograph of pre-adipocytes stained with Oil Red O cultured in the presence of insulin, tri-iodothyronine, dexamethasone, isobutyl-methylxanthine and rosiglitazone throughout the experiment.
Figure 11D is a light micrograph of pre-adipocytes stained with Oil Red O cultured in the presence of hsa-miR-30b mimic.
Figure 11E is a light micrograph of pre-adipocytes stained with Oil Red O cultured in the presence of a non-targeting miRNA mimic.
Figure 11F is a light micrograph of pre-adipocytes stained with Oil Red O cultured in the presence of a non-targeting miRNA inhibitor.
Figure 12A is a bar graph showing mRNA expression of thermogenesis targets in the presence of rosiglitazone.
Figure 12B is a bar graph showing mRNA expression of thermogenesis targets in the presence of hsa-let-7a inhibitor.
Figure 12C is a bar graph showing mRNA expression of thermogenesis targets in the presence of hsa-miR-1 mimic.
Figure 12D is a bar graph showing mRNA expression of thermogenesis targets in the presence of hsa-miR-19b mimic.
Figure 12E is a bar graph showing mRNA expression of thermogenesis targets in the presence of and hsa-miR-30b mimic.
Figure 12F is a bar graph showing mRNA expression of thermogenesis targets in untreated pre-adipocytes.
Figure 13 is an M-A plot showing the mean gene expression on the x-axis and the difference between pairs in logarithmic scale on the y-axis.
Figure 14 is a schematic showing a Venn Diagram showing that the numbers of genes significantly upregulated in the presence of the miRNA analogs hsa-let-7a inhibitor, hsa-miR-1 mimic, hsa-miR-19b mimic and hsa-miR-30b mimic were respectively 305, 247, 255 and 267. A set of 127 genes was commonly upregulated by the listed miRNA analogs.
Figure 15 is a schematic showing a Venn diagram showing that the numbers of genes significantly downregulated in the presence of the miRNA analogs hsa-let-7a inhibitor, hsa-miR-1 mimic, hsa-miR-19b mimic and hsa-miR-30b mimic were respectively 143, 177, 115 and 165. A set of 60 genes was commonly downregulated by the listed miRNA analogs.
Figure 16 is a bar graph showing relative fluorescence in adipocytes either unlabeled or transfected with a Dy547 labeled non-targeting miRIDIAN mimic or hairpin inhibitor.
Figure 17A is a bar graph showing the reduction of GAPDH gene expression in cells transfected with siRNA control and a GAPDH siRNA 4 days after transfection.
Figure 17B is a bar graph showing the reduction of GAPDH gene expression in cells transfected with siRNA control and a GAPDH siRNA 12 days after transfection.
Figure 18A is a light micrograph of mature adipocytes stained with Oil Red O cultured for 2 weeks in maintenance medium alone.
Figure 18B is a light micrograph of mature adipocytes stained with Oil Red O cultured in the presence of rosiglitazone for two weeks.
Figure 18C is a light micrograph of mature adipocytes stained with Oil Red O cultured in the presence of a non-targeting miRNA.
Figure 18D is a light micrograph of mature adipocytes stained with Oil Red O cultured in the presence of hsa-miR-30b mimic.
Figure 19 is a bar graph showing the amount of lipids (Nile Red fluorescent dye) in mature adipocytes exposed to various miRNA analogs or rosiglitazone.
Figure 20 is a bar graph showing the amounts of total RNA extracted from mature adipocytes exposed to various transfecting agents.
Figure 21 is a bar graph showing reduction of GAPDH gene expression in mature adipocytes transfected with a GAPDH-specific miRNA mimic using various transfecting agents.
Figure 22 represents bright field micrographs of mature adipocytes cultured for 2 weeks in maintenance medium alone (control), 50 nM hsa-let-7a inhibitor, 10 µM beta adrenergic receptor agonist CL316,243, 50 nM hsa-miR-1 mimic, 10 nM thyroid hormone tri-iodothyronine, 50 nM hsa-miR-19b mimic, 100 nM Rosiglitazone or 50 nM hsa-miR-30b mimic.
Figure 23 is a schematic representation of the Cell-SELEX process use to isolate aptamers specifically directed against unique targets at the surface of human cells.
Figure 24 depicts the results of a FACS experiment assessing binding of selected fluorescent aptamers to human hepatocytes and adipocytes.

### DETAILED DESCRIPTION OF THE INVENTION

### I. Definitions

Unless otherwise defined, all technical and scientific terms used herein have the same meaning as commonly understood by one of ordinary skill in the art to which this invention belongs. In case of conflict, the present application, including definitions, will control.

As used herein, the term "miRNA agent" refers to an oligonucleotide or oligonucleotide mimetic that directly or indirectly modulates the activity of a thermogenic regulator (e.g., a mitochondrial uncoupler or an activator or repressor thereof). miRNA agents can act on a target gene or on a target miRNA.

As used herein, the term "miRNA" refers to a single-stranded RNA molecule (or a synthetic derivative thereof), which is capable of binding to a target gene (either the mRNA or the DNA) and regulating expression of that gene. In certain embodiments, the miRNA is naturally expressed in an organism.

As used herein, the term "seed sequence" refers to a 6-8 nucleotide (nt) long substring within the first 8 nt at the 5'-end of the miRNA (i.e., seed sequence) that is an important determinant of target specificity.

As used herein, the term "agomir" refers to a synthetic oligonucleotide or oligonucleotide mimetic that functionally mimics a miRNA. An agomir can be an oligonucleotide with the same or similar nucleic acid sequence to a miRNA or a portion of a miRNA. In certain embodiments, the agomir has 1, 2, 3, 4, 5, 6, 7, 8, 9 or 10 nucleotide differences from the miRNA that it mimics. Further, agomirs can have the same length, a longer length or a shorter length than the miRNA that it mimics. In certain embodiments, the agomir has the same sequence as 6-8 nucleotides at the 5' end of the miRNA it mimics. In other embodiments, an agomir can be 5, 6, 7, 8, 9, 10, 11, 12, 13, 14, 15, 16, 17, 18, 19, 20, 21, 22, 23, 24, 25, 27, 28, 29, 30, 31, 32, 33, 34, 35, 36, 37, 38, 39, 40, 41, 42, 43, 44, 45, 46, 47, 48, 49 or 50 nucleotides in length. In other embodiments, an agomir can be 5-10, 6-8, 10-20, 10-15 or 5-500 nucleotides in length. In certain embodiments, agomirs include any of the sequences shown in Tables 1, 11, 13 and 14. These chemically modified synthetic RNA duplexes include a guide strand that is identical or substantially identical to the miRNA of interest to allow efficient loading into the miRISC complex, whereas the passenger strand is chemically modified to prevent its loading to the Argonaute protein in the miRISC complex (Thorsen SB et al., Cancer J., 18(3):275-284 (2012); Broderick JA et al., Gene Ther., 18(12): 1104-1110 (2011)).

As used herein, the term "antagomir" refers to a synthetic oligonucleotide or oligonucleotide mimetic having complementarity to a specific microRNA, and which inhibits the activity of that miRNA. In certain embodiments, the antagomir has 1, 2, 3, 4, 5, 6, 7, 8, 9 or 10 nucleotide differences from the miRNA that it inhibits. Further, antagomirs can have the same length, a longer length or a shorter length than the miRNA that it inhibits. In certain embodiments, the antagomir hybridizes to 6-8 nucleotides at the 5' end of the miRNA it inhibits. In other embodiments, an antagomir can be 5, 6, 7, 8, 9, 10, 11, 12, 13, 14, 15, 16, 17, 18, 19, 20, 21, 22, 23, 24, 25, 27, 28, 29, 30, 31, 32, 33, 34, 35, 36, 37, 38, 39, 40, 41, 42, 43, 44, 45, 46, 47, 48, 49 or 50 nucleotides in length. In other embodiments, an antagomir can be 5-10, 6-8, 10-20, 10-15 or 5-500 nucleotides in length. In certain embodiments, antagomirs include nucleotides that are complementary to any of the sequences shown in Tables 1, 11, 13 and 14. The antagomirs are synthetic reverse complements that tightly bind to and inactivate a specific miRNA. Various chemical modifications are used to improve nuclease resistance and binding affinity. The most commonly used modifications to increase potency include various 2'sugar modifications, such as 2'-O-Me, 2'-O-methoxyethyl (2'-MOE), or 2'-fluoro(2'-F). The nucleic acid structure of the miRNA can also be modified into a locked nucleic acid (LNA) with a methylene bridge between the 2'oxygen and the 4' carbon to lock the ribose in the 3'-endo (North) conformation in the A-type conformation of nucleic acids (Lennox KA et al.. Gene Ther. Dec 2011;18(12):1111-1120; Bader AG et al. Gene Ther. Dec 2011;18(12):1121-1126). This modification significantly increases both target specificity and hybridization properties of the molecules.

As used herein, the term "aptamir" refers to the combination of an aptamer (oligonucleic acid or peptide molecule that bind to a specific target molecule) and an agomir or antagomir as defined above, which allows cell or tissue-specific delivery of the miRNA agents.

As used herein, the term "interfering RNA" refers to any double stranded or single stranded RNA sequence capable of inhibiting or down-regulating gene expression by mediating RNA interference. Interfering RNAs include, but are not limited to, small interfering RNA ("siRNA") and small hairpin RNA ("shRNA"). "RNA interference" refers to the selective degradation of a sequence-compatible messenger RNA transcript.

As used herein, the term "small interfering RNA" or "siRNA" refers to any small RNA molecule capable of inhibiting or down regulating gene expression by mediating RNA interference in a sequence specific manner. The small RNA can be, for example, about 16 to 21 nucleotides long.

As used herein, the term "shRNA" (small hairpin RNA) refers to an RNA molecule comprising an antisense region, a loop portion and a sense region, wherein the sense region has complementary nucleotides that base pair with the antisense region to form a duplex stem. Following post-transcriptional processing, the small hairpin RNA is converted into a small interfering RNA (siRNA) by a cleavage event mediated by the enzyme Dicer, which is a member of the RNase III family.

As used herein, the term "antisense oligonucleotide" refers to a synthetic oligonucleotide or oligonucleotide mimetic that is complementary to a DNA or mRNA sequence (e.g., a miRNA).

As used herein, the term "miR-mask" refers to a single stranded antisense oligonucleotide that is complementary to a miRNA binding site in a target mRNA, and that serves to inhibit the binding of miRNA to the mRNA binding site. See, e.g., Xiao, et al. "Novel approaches for gene-specific interference via manipulating actions of microRNAs: examination on the pacemaker channel genes HCN2 and HCN4," Journal of Cellular Physiology, vol. 212, no. 2, pp. 285-292, 2007.

As used herein, the term "miRNA sponge" refers to a synthetic nucleic acid (e.g. a mRNA transcript) that contains multiple tandem-binding sites for a miRNA of interest, and that serves to titrate out the endogenous miRNA of interest, thus inhibiting the binding of the miRNA of interest to its endogenous targets. See, e.g., Ebert et al., "MicroRNA sponges: competitive inhibitors of small RNAs in mammalian cells," Nature Methods, vol. 4, no. 9, pp. 721-726, 2007.

As used herein, the term "respiratory chain uncoupling" refers to the dissipation of the mitochondrial inner membrane proton gradient, thereby preventing the synthesis of ATP in the mitochondrion by oxidative phosphorylation.

As used herein, the term "mitochondrial uncoupler" refers to a protein (or the encoding nucleic acid) that can dissipate of the mitochondrial inner membrane proton gradient, thereby preventing the synthesis of ATP in the mitochondrion by oxidative phosphorylation. Exemplary mitochondrial uncouplers include UCP1 and UCP2.

As used herein, the terms "activator" or "repressor" of a mitochondrial uncoupler refers to a protein that serves to upregulate or downregulate, respectively, an activity of a mitochondrial uncoupler.

As used herein, the term "thermogenic regulator" refers to a protein (or the encoding nucleic acid) that regulates thermogenesis either directly or indirectly. The term encompasses mitochondrial uncouplers, and also activators and repressors of mitochondrial uncouplers. Exemplary thermogenic regulators are set forth in Table 2 herein.

As used herein, the term "modulate" refers to increasing or decreasing a parameter. For example, to modulate the activity of a protein that protein's activity could be increased or decreased.

As used herein, the term "activity" of mitochondrial uncoupler or thermogenic regulator refers to any measurable biological activity including, without limitation, mRNA expression, protein expression, or respiratory chain uncoupling.

The "effective amount" of the miRNA agent composition is an amount sufficient to be effective in treating or preventing a disorder or to regulate a physiological condition in humans. In certain embodiments, this physiological condition is obesity.

A "subject" is a vertebrate, including any member of the class Mammalia, including humans, domestic and farm animals, zoo, sports or pet animals, such as mouse, rabbit, pig, sheep, goat, cattle and higher primates.

The term "mammal" refers to any species that is a member of the class Mammalia, including rodents, primates, dogs, cats, camelids and ungulates. The term "rodent" refers to any species that is a member of the order rodentia including mice, rats, hamsters, gerbils and rabbits. The term "primate" refers to any species that is a member of the order primates, including monkeys, apes and humans. The term "camelids" refers to any species that is a member of the family camelidae including camels and llamas. The term "ungulates" refers to any species that is a member of the superorder ungulata including cattle, horses and camelids. According to some embodiments, the mammal is a human.

"Treatment", or "treating" as used herein, is defined as the application or administration of a therapeutic agent (e.g., a miRNA agent or vector or transgene encoding same) to a patient, or application or administration of a therapeutic agent to an isolated tissue or cell line from a patient, who has the disease or disorder, a symptom of disease or disorder or a predisposition toward a disease or disorder, with the purpose to cure, heal, alleviate, relieve, alter, remedy, ameliorate, improve or affect the disease or disorder, the symptoms of the disease or disorder, or the predisposition toward disease.

"Pharmacogenomics", as used herein, refers to the application of genomics technologies such as gene sequencing, statistical genetics, and gene expression analysis to drugs in clinical development and on the market. More specifically, the term refers to the study of how a patient's genes determine his or her response to a drug (e.g., a patient's "drug response phenotype", or "drug response genotype").

The "effective amount" of the miRNA agent composition is an amount sufficient to be effective in treating or preventing a disorder or to regulate a physiological condition in humans.

### II. Thermogenesis and Obesity

In certain embodiments, the disclosure provides methods for modulating thermogenesis. These methods generally involve contacting cells or tissue with a miRNA agent that modulates activity of at least one mitochondrial uncoupler (e.g., UCP1 and/or UCP2). Such methods and compositions are particularly useful for treating obesity.

Mammalian adipocytes can be categorized into two major categories based on their functional profiles: 1) energy-storing and releasing, lipid-filled white adipocytes (WAT) and; 2) energy-expending and heat producing, mitochondria-rich brown adipocytes (BAT). Until recently, it was believed that BAT underwent rapid involution in early childhood, leaving only vestigial amounts in adults. However, positron-emission tomography (PET) studies performed in humans with the tracer 18F-fluorodeoxyglucose (18F-FDG) demonstrated that: 1) multiple depots of BAT are still present in the cervical, supraclavicular, axillary and paravertebral regions in adult subjects; 2) BAT in adult humans can be rapidly activated by exposure to cold temperatures; 3) there is an inverse correlation between the activity of BAT and age, body-mass index (BMI), the percentage of body fat, fasting plasma glucose level, beta-blocker use and outdoor temperature; and 4) BAT expansion may drive the weight loss associated with catecholamine-producing phaeochromocytomas, whereas beta3-adrenoreceptor polymorphisms leading to a reduction in receptor function have been linked to weight gain and early onset type 2 diabetes mellitus.

Although WAT and BAT are derived from mesenchymal stem cells, they have distinct lineages, with Myf5 (Myogenic Regulatory Factor 5) (shared with skeletal myocyte progenitors), PGC-1alpha and PRDM16 (PR-domain-containing 16) expression distinguishing the brown from white adipocyte precursors. In addition to the classic brown adipocytes, a different type of brown fat cells can be induced in tissues where WAT predominates. The termed "brite" (brown-in-white) adipocyte has been coined and the appearance of brown-like adipocytes within WAT depots is associated with improved metabolic phenotypes. Increasing BAT mass and/or activity offers a degree of protection from obesity. Heat production by BAT is 300 W/g compared to 1 W/g in all other tissues. Relatively limited amounts of BAT would be required to make significant impact on energy balance, since as little as 50 g of BAT would account for 20% of daily energy expenditure. It has been speculated that the estimated 63 g of BAT found in the supraclavicular/paracervical depot of one subject could combust the energy equivalent of 4.1 kg of WAT over 1 year.

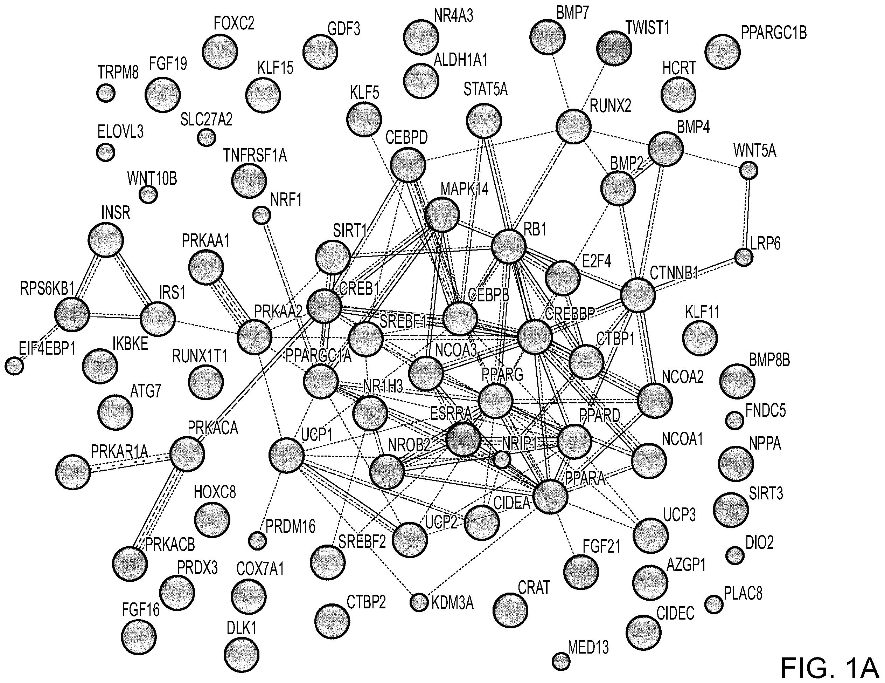

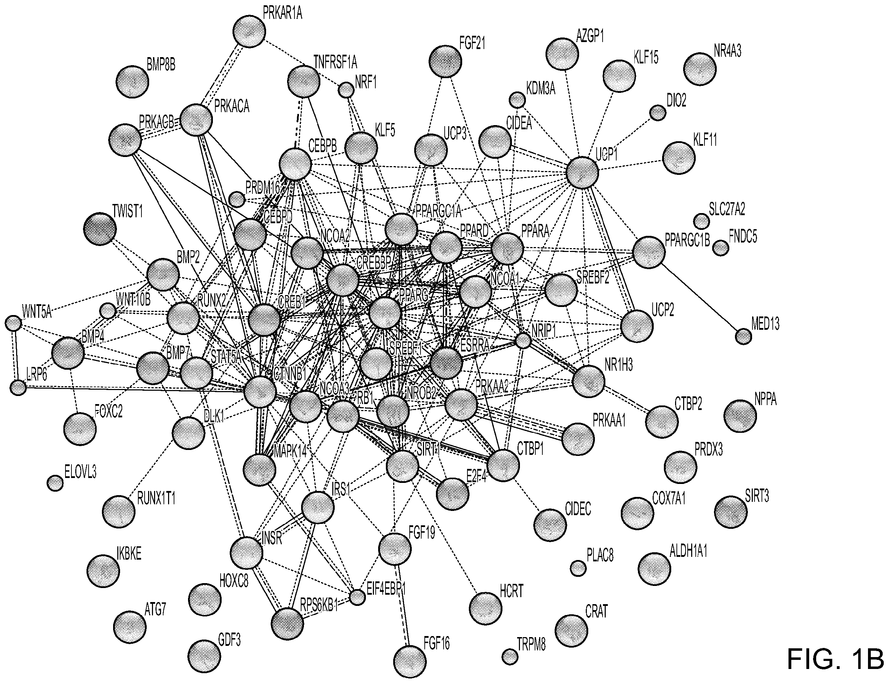

Mitochondrial uncoupling proteins (UCP) are members of the family of mitochondrial anion carrier proteins (MACP). UCPs separate oxidative phosphorylation from ATP synthesis with energy dissipated as heat (also referred to as the "mitochondrial proton leak"). UCPs facilitate the transfer of anions from the inner to the outer mitochondrial membrane and the return transfer of protons from the outer to the inner mitochondrial membrane generating heat in the process. UCPs are the primary proteins responsible for thermogenesis and heat dissipation. Uncoupling Protein 1 (UCP1), also named thermogenin, is a BAT specific protein responsible for thermogenesis and heat dissipation. UCP2 is another Uncoupling Protein also expressed in adipocytes. UCPs are part of network of thermogenic regulator proteins (see Figure 1). Exemplary thermogenic regulators are set forth in Table 2.

Modulation of thermogenic regulators to induce BAT differentiation and/or mitochondrial uncoupling proteins provides a method to induce thermogenesis in a subject and, hence, to treat obesity. However, chemical pharmacologic approaches cannot target these molecules, as they do not belong to the classic 'target classes' (kinases, ion channels, G-protein coupled receptors, etc.) that dominate the 'druggable space' of traditional drug discovery. Accordingly, the invention provides novel methods and compositions for modulating these thermogenic regulators using miRNA agents.

In certain embodiments, miRNA agents are employed to upregulate the activity of a mitochondrial uncoupler (e.g., the mRNA expression level, protein expression level, or mitochondrial uncoupling activity). Upregulation of a mitochondrial uncoupler can be achieved in several ways. In one embodiment, the miRNA agent directly inhibits the activity of a naturally occurring miRNA that is responsible for downregulation of the activity (e.g., the mRNA expression level, protein expression level) of the mitochondrial uncoupler. In another embodiment, the miRNA agent upregulates the activity (e.g., the mRNA expression level or the protein expression level) of an activator of the mitochondrial uncoupler. This upregulation can be achieved, for example, by directly inhibiting the activity of a naturally occurring miRNA that is responsible for downregulation of the expression of the activator. In yet another embodiment, the miRNA agent downregulates the activity (e.g., the mRNA expression level or the protein expression level) of a repressor of the mitochondrial uncoupler. This downregulation can be achieved, for example, by directly inhibiting the expression of a repressor of a mitochondrial uncoupler using a miRNA agent.

In certain embodiments, miRNA agents are employed that are capable of modulating the activity of multiple thermogenic regulators simultaneously (Pathway-specific miRNA agents as opposed to universal miRNA agents). For example, a single miRNA, agomir or antagomir that binds to multiple thermogenic regulators can be used. This approach is particularly advantageous in that it allows for the modulation of multiple members of an entire signaling pathway using a single miRNA agent.

In certain embodiments, multiple inhibitory miRNA agents (e.g., antagomirs or miR-masks) are employed. These inhibitory miRNA agents can have the same or different miRNA targets.

### III. miRNA Agents

In certain embodiments, the disclosure employs miRNA agents for the modulation of thermogenic regulators (e.g., mitochondrial uncouplers, such as UCP1 and/or UCP2). miRNA agents, suitable for use in the methods disclosed herein, included, without limitation, miRNA, agomirs, antagomirs, miR-masks, miRNA-sponges, siRNA (single- or double-stranded), shRNA, antisense oligonucleotides, ribozymes, or other oligonucleotide mimetics which hybridize to at least a portion of a target nucleic acid and modulate its function.

According to the disclosure, the miRNA agents are miRNA molecules or synthetic derivatives thereof (e.g., agomirs). In one particular embodiment, the miRNA agent is a miRNA. miRNAs are a class of small (e.g., 18-24 nucleotides) non-coding RNAs that exist in a variety of organisms, including mammals, and are conserved in evolution. miRNAs are processed from hairpin precursors of about 70 nucleotides which are derived from primary transcripts through sequential cleavage by the RNAse III enzymes drosha and dicer. Many miRNAs can be encoded in intergenic regions, hosted within introns of pre-mRNAs or within ncRNA genes. Many miRNAs also tend to be clustered and transcribed as polycistrons and often have similar spatial temporal expression patterns. In general, miRNAs are post-transcriptional regulators that bind to complementary sequences on a target gene (mRNA or DNA), resulting in gene silencing by, e.g., translational repression or target degradation. One miRNA can target many different genes simultaneously. Exemplary miRNA molecules for use in the disclosed methods include without limitation: hsa-miR-1-1, hsa-miR-1-2, hsa-miR-7a-g, hsa-miR-105, hsa-miR-1283, hsa-mir-129, hsa-miR-133a-1, hsa-miR-133a-2, hsa-miR-143, hsa-mir-143-5p, hsa-mir-147, hsa-mir-149, hsa-miR-19a-b, hsa-mir-199a, hsa-mir-199b, hsa-mir-200c, hsa-mir-204, hsa-mir-205, hsa-miR-206, hsa-mir-21, hsa-mir-211, hsa-mir-218, hsa-mir-218-1, hsa-mir-218-2, hsa-mir-219-2, hsa-mir-219-2-3p, hsa-mir-22, hsa-mir-22-3p, hsa-mir-22-5p, hsa-mir-24-2, hsa-miR-30a-e, hsa-miR-3177-5p, hsa-mir-325, hsa-mir-331, hsa-mir-331-5p, hsa-miR-3613-3p, hsa-mir-362, hsa-mir-362-5p, hsa-mir-367, hsa-mir-371, hsa-mir-371-5p, hsa-mir-377, hsa-mir-378, hsa-mir-378a-5p, hsa-mir-382, hsa-mir-383, hsa-miR-3658, hsa-mir-422a, hsa-mir-425, hsa-miR-455-3p, hsa-miR-455-5p, hsa-miR-491, hsa-mir-508, hsa-mir-508-5p, hsa-mir-512-1, hsa-mir-512-2, hsa-miR-515-3p, hsa-mir-519e, hsa-miR-520a, hsa-mir-543, hsa-mir-545, hsa-mir-549, hsa-mir-556, hsa-miR-568, hsa-mir-620, hsa-mir-643, hsa-mir-654-3p, hsa-mir-765, hsa-mir-871, hsa-mir-888, hsa-mir-888-3p, hsa-mir-92b, hsa-mir-93, hsa-mir-96, hsa-mir-99a. In other embodiments, exemplary miRNA molecules for use in the disclosed methods miRNA disclosed in Tables 1, 11, 13 and 14, herein. In one particular embodiment, the miRNA agent is human miR-22, or a functional derivative thereof.

According to the disclosure, the miRNA agent is an agomir. Agomirs of a particular miRNA can be identified using the screening methods disclosed herein. In one particular embodiment, the agomir is a functional mimetic of human miR-22 (Davidson BL et al., Nat. Rev. Genet., 12(5):329-340 (2011).

According to the disclosure, the miRNA agents are oligonucleotide or oligonucleotide mimetics that inhibit the activity of one or more miRNA. Examples of such molecules include, without limitation, antagomirs, interfering RNA, antisense oligonucleotides, ribozymes, miRNA sponges and miR-masks. According to the invention, the miRNA agent is an antagomir. In general, antagomirs are chemically modified antisense oligonucleotides that bind to a target miRNA and inhibit miRNA function by preventing binding of the miRNA to its cognate gene target. Antagomirs can include any base modification known in the art. In one particular embodiment, the antagomir inhibits the activity of human miR-22 (van Rooij E et al., Circ. Res., 110(3):496-507 (2012); Snead NM et al., Nucleic Acid Ther., 22(3):139-146 (2012); Czech MP et al., Nat. Rev. Endocrinol., 7(8):473-484 (2011).

According to the disclosure, the miRNA agents are 10 to 50 nucleotides in length. One having ordinary skill in the art will appreciate that this embodies oligonucleotides having antisense portions of 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 nucleotides in length, or any range therewithin.

According to the disclosure, the miRNA agents are chimeric oligonucleotides that contain two or more chemically distinct regions, each made up of at least one nucleotide. These oligonucleotides typically contain at least one region of modified nucleotides that confers one or more beneficial properties (such as, for example, increased nuclease resistance, increased uptake into cells, increased binding affinity for the target) and a region that is a substrate for enzymes capable of cleaving RNA:DNA or RNA:RNA hybrids. Chimeric inhibitory nucleic acids of the invention may be formed as composite structures of two or more oligonucleotides, modified oligonucleotides, oligonucleosides and/or oligonucleotide mimetics as described above. Such compounds have also been referred to in the art as hybrids or gapmers. Representative United States patents that teach the preparation of such hybrid structures comprise, but are not limited to, US patent nos: 5,013,830; 5,149,797; 5, 220,007; 5,256,775; 5,366,878; 5,403,711; 5,491,133; 5,565,350; 5,623,065; 5,652,355; 5,652,356; and 5,700,922.

In certain embodiments, the miRNA agents comprise at least one nucleotide modified at the 2' position of the sugar, most preferably a 2'-O-alkyl, 2'-O-alkyl-O-alkyl or 2'-fluoro-modified nucleotide. In other preferred embodiments, RNA modifications include 2'-fluoro, 2'-amino and 2' O-methyl modifications on the ribose of pyrimidines, a basic residue or an inverted base at the 3' end of the RNA. Such modifications are routinely incorporated into oligonucleotides and these oligonucleotides have been shown to have a higher Tm (i.e., higher target binding affinity) than 2'-deoxyoligonucleotides against a given target.

A number of nucleotide and nucleoside modifications have been shown to make an oligonucleotide more resistant to nuclease digestion, thereby prolonging *in vivo* half-life. Specific examples of modified oligonucleotides include those comprising backbones comprising, for example, phosphorothioates, phosphotriesters, methyl phosphonates, short chain alkyl or cycloalkyl intersugar linkages or short chain heteroatomic or heterocyclic intersugar linkages. Most preferred are oligonucleotides with phosphorothioate backbones and those with heteroatom backbones, particularly CH₂ -NH-O-CH₂, CH₂∼N(CH₃)∼O∼CH₂ (known as a methylene(methylimino) or MMI backbone], CH₂ -O-N (CH₃)-CH₂, CH₂ -N (CH₃)-N (CH₃)-CH₂ and O-N (CH₃)-CH₂-CH₂ backbones, wherein the native phosphodiester backbone is represented as O- P- O- CH,); amide backbones (see De Mesmaeker et al. Ace. Chem. Res. 1995, 28:366-374); morpholino backbone structures (see Summerton and Weller, U.S. Pat. No. 5,034,506); peptide nucleic acid (PNA) backbone (wherein the phosphodiester backbone of the oligonucleotide is replaced with a polyamide backbone, the nucleotides being bound directly or indirectly to the aza nitrogen atoms of the polyamide backbone, see Nielsen et al., Science 1991, 254, 1497), each of which is herein incorporated by reference in its entirety. Phosphorus-containing linkages include, but are not limited to, phosphorothioates, chiral phosphorothioates, phosphorodithioates, phosphotriesters, aminoalkylphosphotriesters, methyl and other alkyl phosphonates comprising 3'alkylene phosphonates and chiral phosphonates, phosphinates, phosphoramidates comprising 3 '-amino phosphoramidate and aminoalkylphosphoramidates, thionophosphoramidates, thionoalkylphosphonates, thionoalkylphosphotriesters, and boranophosphates having normal 3 '-5' linkages, 2'-5' linked analogs of these, and those having inverted polarity wherein the adjacent pairs of nucleoside units are linked 3'-5' to 5'-3' or 2'-5' to 5'-2'; see US patent nos. 3,687,808; 4,469,863; 4,476,301; 5,023,243; 5, 177,196; 5,188,897; 5,264,423; 5,276,019; 5,278,302; 5,286,717; 5,321, 131; 5,399,676; 5,405,939; 5,453,496; 5,455, 233; 5,466,677; 5,476,925; 5,519,126; 5,536,821; 5,541,306; 5,550,111; 5,563, 253; 5,571,799; 5,587,361; and 5,625,050. Morpholino-based oligomeric compounds are described in Dwaine A. Braasch and David R. Corey, Biochemistry, 2002, 41(14), 4503-4510); Genesis, volume 30, issue 3, 2001; Heasman, J., Dev. Biol., 2002, 243, 209-214; Nasevicius et al., Nat. Genet., 2000, 26, 216-220; Lacerra et al., Proc. Natl. Acad. Sci., 2000, 97, 9591-9596; and U.S. Pat. No. 5,034,506, issued Jul. 23, 1991. Cyclohexenyl nucleic acid oligonucleotide mimetics are described in Wang et al., J. Am. Chem. Soc., 2000, 122, 8595-8602.

Modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These comprise those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts; see US patent nos. 5,034,506; 5,166,315; 5, 185,444; 5,214,134; 5,216, 141; 5,235,033; 5,264,562; 5, 264,564; 5,405,938; 5,434,257; 5,466,677; 5,470,967; 5,489,677; 5,541,307; 5,561,225; 5,596, 086; 5,602,240; 5,610,289; 5,602,240; 5,608,046; 5,610,289; 5,618,704; 5,623, 070; 5,663,312; 5,633,360; 5,677,437; and 5,677,439.

In certain embodiments, miRNA agents comprise one or more substituted sugar moieties, e.g., one of the following at the 2' position: OH, SH, SCH₃, F, OCN, OCH₃ OCH₃, OCH₃O(CH₂)nCH₃, O(CH₂)nNH₂ or O(CH₂)nCH₃ where n is from 1 to about 10; Ci to CIO lower alkyl, alkoxyalkoxy, substituted lower alkyl, alkaryl or aralkyl; CI; Br; CN; CF3 ; OCF3; O-, S-, or N-alkyl; O-, S-, or N-alkenyl; SOCH₃; SO₂ CH₃; ONO₂; NO₂; N₃; NH₂; heterocycloalkyl; heterocycloalkaryl; aminoalkylamino; polyalkylamino; substituted silyl; an RNA cleaving group; a reporter group; an intercalator; a group for improving the pharmacokinetic properties of an oligonucleotide; or a group for improving the pharmacokinetic/pharmacodynamic properties of an oligonucleotide and other substituents having similar properties. A preferred modification includes 2'-methoxyethoxy [2'-O-CH₂CH₂OCH₃, also known as 2'-O-(2-methoxyethyl)] (Martin et al., Helv. Chim. Acta, 1995, 78, 486). Other preferred modifications include 2'-methoxy (2'-O-CH₃), 2'-propoxy (2'-OCH₂ CH₂CH₃) and 2'-fluoro (2'-F). Similar modifications may also be made at other positions on the oligonucleotide, particularly the 3' position of the sugar on the 3' terminal nucleotide and the 5' position of 5' terminal nucleotide. Oligonucleotides may also have sugar mimetics such as cyclobutyls in place of the pentofuranosyl group.

In certain embodiments, miRNA agents comprise one or more base modifications and/or substitutions. As used herein, "unmodified" or "natural" bases include adenine (A), guanine (G), thymine (T), cytosine (C) and uracil (U). Modified bases include, without limitation, bases found only infrequently or transiently in natural nucleic acids, e.g., hypoxanthine, 6-methyladenine, 5-Me pyrimidines, particularly 5-methylcytosine (also referred to as 5-methyl-2' deoxycytosine and often referred to in the art as 5-Me-C), 5-hydroxymethylcytosine (HMC), glycosyl HMC and gentobiosyl HMC, as well as synthetic bases, e.g., 2-aminoadenine, 2-(methylamino)adenine, 2-(imidazolylalkyl)adenine, 2-(aminoalklyamino)adenine or other heterosubstituted alkyladenines, 2-thiouracil, 2-thiothymine, 5-bromouracil, 5-hydroxymethyluracil, 8-azaguanine, 7-deazaguanine, N6 (6-aminohexyl)adenine and 2,6-diaminopurine. Kornberg, A., DNA Replication, W. H. Freeman & Co., San Francisco, 1980, pp75-77; Gebeyehu, G., et al. Nucl. Acids Res. 1987, 15:4513). A "universal" base known in the art, e.g., inosine, can also be included. 5-Me-C substitutions can also be included. These have been shown to increase nucleic acid duplex stability by 0.6-1.2OC. (Sanghvi, Y. S., in Crooke, S. T. and Lebleu, B., eds., Antisense Research and Applications, CRC Press, Boca Raton, 1993, pp. 276-278). Further suitable modified bases are described in US patent nos. 3,687,808, as well as 4,845,205; 5,130,302; 5,134,066; 5,175, 273; 5, 367,066; 5,432,272; 5,457,187; 5,459,255; 5,484,908; 5,502,177; 5,525,711; 5,552,540; 5,587,469; 5,596,091; 5,614,617; 5,750,692, and 5,681,941.

It is not necessary for all positions in a given oligonucleotide to be uniformly modified, and in fact more than one of the aforementioned modifications may be incorporated in a single oligonucleotide or even at a single nucleoside within an oligonucleotide.

In certain embodiments, both a sugar and an internucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, for example, an aminoethylglycine backbone. The nucleobases are retained and are bound directly or indirectly to aza nitrogen atoms of the amide portion of the backbone. Representative United States patents that teach the preparation of PNA compounds comprise, but are not limited to, US patent nos. 5,539,082; 5,714,331; and 5,719,262. Further teaching of PNA compounds can be found in Nielsen et al., Science, 1991, 254, 1497-1500.

In certain embodiments, the miRNA agent is linked (covalently or non-covalently) to one or more moieties or conjugates that enhance the activity, cellular distribution, or cellular uptake of the oligonucleotide. Such moieties include, without limitation, lipid moieties such as a cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, e.g., hexyl-S- tritylthiol (Manoharan et al., Ann. N. Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, e.g., dodecandiol or undecyl residues (Kabanov et al., FEBS Lett., 1990, 259, 327-330; Svinarchuk et al., Biochimie, 1993, 75, 49-54), a phospholipid, e.g., di-hexadecyl-rac-glycerol or triethylammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Mancharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-t oxycholesterol moiety (Crooke et al., J. Pharmacol. Exp. Ther., 1996, 277, 923-937). See also US patent nos. 4,828,979; 4,948,882; 5,218,105; 5,525,465; 5,541,313; 5,545,730; 5,552, 538; 5,578,717, 5,580,731; 5,580,731; 5,591,584; 5,109,124; 5,118,802; 5,138,045; 5,414,077; 5,486, 603; 5,512,439; 5,578,718; 5,608,046; 4,587,044; 4,605,735; 4,667,025; 4,762, 779; 4,789,737; 4,824,941; 4,835,263; 4,876,335; 4,904,582; 4,958,013; 5,082, 830; 5,112,963; 5,214,136; 5,082,830; 5,112,963; 5,214,136; 5, 245,022; 5,254,469; 5,258,506; 5,262,536; 5,272,250; 5,292,873; 5,317,098; 5,371,241, 5,391, 723; 5,416,203, 5,451,463; 5,510,475; 5,512,667; 5,514,785; 5, 565,552; 5,567,810; 5,574,142; 5,585,481; 5,587,371; 5,595,726; 5,597,696; 5,599,923; 5,599, 928 and 5,688,941.

In one particular embodiment, the miRNA agent is linked to (covalently or non-covalently) to a nucleic acid aptamer. Aptamers are synthetic oligonucleotides or peptide molecules that bind to a specific target molecule. Aptamers appropriate for use with the miRNA agents provided herein are described in U.S. Provisional Patent Application No. 61/695,477 filed 8/31/2012.

Accordingly, in a first aspect, the invention provides an adipocyte-specific miRNA modulator composition comprising: I) a targeting moiety that selectively binds to a cellular surface marker on an adipose target cell in a human and II) a thermogenic miRNA modulator moiety, wherein the targeting moiety facilitates uptake of the miRNA modulatory moiety by the target cell such that the miRNA is capable of targeting a thermogenic pathway and up regulating thermogenesis in the target cell.

In one embodiment, the composition comprises an aptamir comprising an aptamer as the targeting moiety.

In certain embodiments, the aptamers used with the miRNAs disclosed herein specifically bind to cell surface marker proteins on an adipose tissue mesenchymal stem cell (ATMSC), white adipose tissue (WAT) adipocytes and brown adipose tissue (BAT) adipocytes. Cell surface markers for ATMSCs include CD9, CD10, CD13, CD29, CD36, CD44, CD49d, CD54, CD55, CD59, CD73, CD90, CD91, CD105, CD137, CD146, CD166, and HLA-ABC. Cell surface markers for WAT adipocytes include Adiponectin, Caveolin-1, Caveolin-2, CD36 (FAT), CLH-22 (Clathrin Heavy Chain Chr 22), FABP4 (Adipocyte protein 2, aP2), SLC27A1 (FATP1), SLC27A2 (FATP2), GLUT4 (Glucose Transporter 4), Perilipin 2 or Resistin. Cell surface markers for all adipocytes include Neprilysin (CD10), FAT (CD36), Thy-1 (CD90), Low density lipoprotein receptor-related protein 1 (LRP1 or CD91), Caveolin-1, Caveolin-2, Fatty acid binding protein 4 (FABP4), Cell surface glycoprotein MUC18 (CD146), Activated leukocyte cell adhesion molecule (CD166) and Natriuretic peptide receptor A (NPR1). According to other embodiments, the aptamers for use with the miRNAs disclosed herein can also specifically bind to markers of adipose tissue including adiponectin, leptin, resistin, FGF 17, FGF 19, BMP7, PYY, MetAP2, RBP4, endostatin, and angiostatin.

In certain embodiments, the aptamers are selected by the Cell-SELEX technology which uses whole living cells as the target, whereby aptamers that recognize specific molecules in their native conformation in their natural environment on the surface of intact cells are selected by repeated amplification and binding to living cells. In this cell-based selection, specific cell surface molecules or even unknown membrane receptors can be directly targeted within their native environment, allowing a straightforward enrichment of cell-specific aptamers.

In certain exemplary embodiments, the miRNA modulator is combined with an aptamer to create an "AptamiR" composition. There are many different ways to combine an aptamer and miRNA analog(s) to create an aptamir. They include, for example, aptamer-miRNA analog chimeras, aptamer-splice-switching oligonucleotide chimeras, and aptamer conjugated to nanoparticles or liposomes containing the miRNA analog(s). "Escort Aptamers" may be inserted at the surface of functional polymers, liposomes, and nanoparticles, each of which can carry many miRNA analogs. For instance, the size of thioaptamer-conjugated liposomes is about 120 nm. Nanoparticle approaches have several functional advantages, including, for example, cellular uptake, the ability to cross membranes, and triggered nanoparticle disassembly.

In one embodiment, an aptamiR composition comprises an aptamer that is directly linked or fused to a miRNA modulator. Such aptamiRs are entirely chemically synthesized, which provides more control over the composition of the conjugate. For instance, the stoichiometry (ratio of miRNA analog per aptamer) and site of attachment can be precisely defined. The linkage portion of the conjugate presents a plurality (2 or more) of nucleophilic and/or electrophilic moieties that serve as the reactive attachment point for the aptamers and miRNA analogs. In addition, the aptamir may further comprise a linker between the aptamer and the miRNA analog. In some embodiments, the linker is a polyalkylene glycol, particularly a polyethylene glycol. In other embodiments, the linker is a liposome, exosome, dendrimer, or comb polymer. Other linkers can mediate the conjugation between the aptamer and the miRNA analog, including a biotinstreptavidin bridge, or a ribonucleic acid. Exemplary non-covalent linkers include linkers formed by base pairing a single stranded portion or overhang of the miRNA moiety and a complementary single-stranded portion or overhang of the aptamer moiety.

In another particular embodiment, an aptamer is combined with a miRNA analog in the form of a liposome-based aptamiR. Liposomes are spherical nanostructures made of a lipid bilayer that can be loaded with pharmaceuticals, such as miRNAs. Furthermore, the liposome surface can be loaded with different substances, such as polyethylene glycol (extending their systemic half-life) or molecular recognition moieties like aptamers for specific binding to targeted cells. For example, aptamer-modified liposomes have been developed, with each liposome displaying approximately 250 aptamers tethered to its surface to facilitate target binding. In a preferred embodiment, liposomes are created to encapsulate miRNA analog(s) and display at their surface aptamers that specifically bind with high affinity and specificity to molecules (e.g. lipid transporters) highly expressed at the surface of adipocytes and ATMSCs. The fusion of the liposomes with the targeted cells causes the release of the miRNA analog(s) into the cell cytoplasm, which then alter a specific intra-cellular pathway. Alternatively, stable thioaptamers may be inserted at the surface of liposomes to guide delivery of the liposome miRNA analog(s) load to targeted ATMSCs and adipocytes.

In a further particular embodiment, an aptamer is combined with a miRNA analog in the form of a carrier-based aptamiR. Exemplary carriers include nanoparticles, lipsomes or exosomes. Such carrier-based aptamiR compositions have the capability of delivering a cargo of multiple miRNA modulators to the target cell in a single carrier. To accomplish targeting and accumulation, the carriers are formulated to present the targeting moiety on their external surface so they can react/bind with selected cell surface antigens or receptors on the adipose target cell. As an example, carriers may be created to encapsulate miRNA modulators while displaying at their surface aptamers that specifically bind with high affinity and specificity to molecules (e.g. lipid transporters) highly expressed at the surface of adipocytes and ATMSCs. The internalized exosomes release inside the cell cytoplasm their miRNA analog(s) load, which alters a specific intra-cellular pathway.

In one embodiment, the carrier is an exosome. Exosomes, which originate from late endosomes, are naturally occurring nanoparticles that are specifically loaded with proteins, mRNAs, or miRNAs, and are secreted endogenously by cells. Exosomes are released from host cells, are not cytotoxic, and can transfer information to specific cells based on their composition and the substance in/on the exosome. Because exosomes are particles of approximately 20-100 nm in diameter, the exosomes evade clearance by the mononuclear phagocyte system (which clears circulating particles >100 nm in size), and are very efficiently delivered to target tissues.

Moreover, synthetic exosomes may offer several advantages over other carriers. For example, they may deliver their cargo directly into the cytosol, while their inertness avoids attack and clearance in the extracellular environment. The structural constituents of exosomes may include small molecules responsible for processes like signal transduction, membrane transport, antigen presentation, targeting/adhesion, among many others.

The miRNA agents must be sufficiently complementary to the target mRNA, i.e., hybridize sufficiently well and with sufficient specificity, to give the desired effect. "Complementary" refers to the capacity for pairing, through hydrogen bonding, between two sequences comprising naturally or non-naturally occurring bases or analogs thereof. For example, if a base at one position of a miRNA agent is capable of hydrogen bonding with a base at the corresponding position of a target nucleic acid sequence, then the bases are considered to be complementary to each other at that position. In certain embodiments, 100% complementarity is not required. In other embodiments, 100% complementarity is required.

miRNA agents for use in the methods disclosed herein can be designed using routine methods. While the specific sequences of certain exemplary target nucleic acid sequences and miRNA agents are set forth herein, one of skill in the art will recognize that these serve to illustrate and describe particular embodiments within the scope of the present invention. Additional target segments are readily identifiable by one having ordinary skill in the art in view of this disclosure. Target segments of 5, 6, 7, 8, 9, 10 or more nucleotides in length comprising a stretch of at least five (5) consecutive nucleotides within the seed sequence, or immediately adjacent thereto, are considered to be suitable for targeting a gene. In some embodiments, target segments can include sequences that comprise at least the 5 consecutive nucleotides from the 5 '-terminus of one of the seed sequence (the remaining nucleotides being a consecutive stretch of the same RNA beginning immediately upstream of the 5'-terminus of the seed sequence and continuing until the miRNA agent contains about 5 to about 30 nucleotides). In some embodiments, target segments are represented by RNA sequences that comprise at least the 5 consecutive nucleotides from the 3 '-terminus of one of the seed sequence (the remaining nucleotides being a consecutive stretch of the same miRNA beginning immediately downstream of the 3'-terminus of the target segment and continuing until the miRNA agent contains about 5 to about 30 nucleotides). One having skill in the art armed with the sequences provided herein will be able, without undue experimentation, to identify further preferred regions to target using miRNA agents. Once one or more target regions, segments or sites have been identified, inhibitory nucleic acid compounds are chosen that are sufficiently complementary to the target, i.e., that hybridize sufficiently well and with sufficient specificity (i.e., do not substantially bind to other non-target nucleic acid sequences), to give the desired effect.

In certain embodiments, miRNA agents used to practice this invention are expressed from a recombinant vector. Suitable recombinant vectors include, without limitation, DNA plasmids, viral vectors or DNA minicircles. Generation of the vector construct can be accomplished using any suitable genetic engineering techniques well known in the art, including, without limitation, the standard techniques of PCR, oligonucleotide synthesis, restriction endonuclease digestion, ligation, transformation, plasmid purification, and DNA sequencing, for example as described in Sambrook et al. Molecular Cloning: A Laboratory Manual. (1989), Coffin et al. (Retroviruses. (1997) and "RNA Viruses: A Practical Approach" (Alan J. Cann, Ed., Oxford University Press, (2000)). As will be apparent to one of ordinary skill in the art, a variety of suitable vectors are available for transferring nucleic acids of the invention into cells. The selection of an appropriate vector to deliver nucleic acids and optimization of the conditions for insertion of the selected expression vector into the cell, are within the scope of one of ordinary skill in the art without the need for undue experimentation. Viral vectors comprise a nucleotide sequence having sequences for the production of recombinant virus in a packaging cell. Viral vectors expressing nucleic acids of the invention can be constructed based on viral backbones including, but not limited to, a retrovirus, lentivirus, adenovirus, adeno-associated virus, pox virus or alphavirus. The recombinant vectors can be delivered as described herein, and persist in target cells (e.g., stable transformants).

In certain embodiments, miRNA agents used to practice this invention are synthesized *in vitro* using chemical synthesis techniques, as described in, e.g., Adams (1983) J. Am. Chem. Soc. 105:661; Belousov (1997) Nucleic Acids Res. 25:3440-3444; Frenkel (1995) Free Radic. Biol. Med. 19:373-380; Blommers (1994) Biochemistry 33:7886-7896; Narang (1979) Meth. Enzymol. 68:90; Brown (1979) Meth. Enzymol. 68: 109; Beaucage (1981) Tetra. Lett. 22: 1859; U.S. Patent No. 4,458,066.

### IV. Methods of Treatment

In one aspect, the invention provides a method of treating obesity in human subject. According to the disclosure, the method generally comprises administering to the human subject an effective amount of a miRNA agent that modulates activity of at least one thermogenic regulator, (e.g., a mitochondrial uncoupler, such as UCP1 and/or UCP2).

Such methods of treatment may be specifically tailored or modified, based on knowledge obtained from the field of pharmacogenomics. Thus, another aspect of the disclosure provides methods for tailoring an individual's prophylactic or therapeutic treatment with either the target gene molecules of the present invention or target gene modulators according to that individual's drug response genotype. Pharmacogenomics allows a clinician or physician to target prophylactic or therapeutic treatments to patients who will most benefit from the treatment and to avoid treatment of patients who will experience toxic drug-related side effects.

miRNA agents can be tested in an appropriate animal model e.g., an obesity model including ob/ob mice (Lindstrom P., Scientific World Journal, 7:666-685 (2007) and db/db mice (Sharma K et al., Am J Physiol Renal Physiol., 284(6):F1138-1144 (2003)). For example, a miRNA agent (or expression vector or transgene encoding same) as described herein can be used in an animal model to determine the efficacy, toxicity, or side effects of treatment with said agent. Alternatively, a therapeutic agent can be used in an animal model to determine the mechanism of action of such an agent. For example, a miRNA agent can be used in an animal model to determine the efficacy, toxicity, or side effects of treatment with such an agent. Alternatively, an agent can be used in an animal model to determine the mechanism of action of such an agent.

The disclosure also provides a method of inducing pre-adipocytes to differentiate into white adipocytes and white adipocytes into brown adipocytes, comprising administering to a population of pre-adipocytes one or more miRNAs selected from hsa-let-7a agomir, hsa-let-7a antagomir, hsa-miR-1 agomir, hsa-miR-1 antagomir, hsa-miR-19b agomir, hsa-miR-19b antagomir, hsa-miR-30b agomir and hsa-miR-30b antagomir. In certain embodiments, the induction of pre-adipocytes to differentiate into adipocytes is greater than the differentiation of pre-adipocytes to adipocytes than when pre-adipocytes are exposed to 100 nM rosiglitazone for two days followed by maintenance medium. In certain embodiments, the adipocytes are brown adipocytes. In other embodiments, the adipocytes are white adipocytes.

The disclosure also provides a method for increasing insulin sensitivity in a subject in need thereof comprising administering the subject one or more miRNAs selected from the group consisting of hsa-let-7a agomir, hsa-let-7a antagomir, hsa-miR-1 agomir, hsa-miR-1 antagomir, hsa-miR-19b agomir, hsa-miR-19b antagomir, hsa-miR-30b agomir and hsa-miR-30b antagomir. In certain embodiments, the subject is a mammal.

The disclosure also provides a method of causing fat loss in a subject in need thereof comprising administering the subject one or more miRNAs selected from the group consisting of hsa-let-7a antagomir, hsa-miR-1 agomir, hsa-miR-19b agomir and hsa-miR-30b agomir. In certain embodiments, the subject is a mammal. In other embodiments, the mammal is a human.

A miRNA agent modified for enhancing uptake into cells (e.g., adipose cells) can be administered at a unit dose less than about 15 mg per kg of bodyweight, or less than 10, 5, 2, 1, 0.5, 0.1, 0.05, 0.01, 0.005, 0.001, 0.0005, 0.0001, 0.00005 or 0.00001 mg per kg of bodyweight, and less than 200 nmole of miRNA agent (e.g., about 4.4 x 10¹⁶ copies) per kg of bodyweight, or less than 1500, 750, 300, 150, 75, 15, 7.5, 1.5, 0.75, 0.15, 0.075, 0.015, 0.0075, 0.0015, 0.00075, 0.00015 nmole of RNA silencing agent per kg of bodyweight. The unit dose, for example, can be administered by injection (e.g., intravenous or intramuscular), an inhaled dose, or a topical application. Particularly preferred dosages are less than 2, 1, or 0.1 mg/kg of body weight.

Delivery of a miRNA agent directly to an organ or tissue (e.g., directly to adipose tissue) can be at a dosage on the order of about 0.00001 mg to about 3 mg per organ/tissue, or preferably about 0.0001-0.001 mg per organ/tissue, about 0.03-3.0 mg per organ/tissue, about 0.1-3.0 mg per organ/tissue or about 0.3-3.0 mg per organ/tissue. The dosage can be an amount effective to treat or prevent obesity or to increase insulin sensitivity. In one embodiment, the unit dose is administered less frequently than once a day, e.g., less than every 2, 4, 8 or 30 days. In another embodiment, the unit dose is not administered with a frequency (e.g., not a regular frequency). For example, the unit dose may be administered a single time. In one embodiment, the effective dose is administered with other traditional therapeutic modalities.

In certain embodiment, a subject is administered an initial dose, and one or more maintenance doses of a miRNA agent. The maintenance dose or doses are generally lower than the initial dose, e.g., one-half less of the initial dose. A maintenance regimen can include treating the subject with a dose or doses ranging from 0.01 mg/kg to 1.4 mg/kg of body weight per day, e.g., 10, 1, 0.1, 0.01, 0.001, or 0.00001 mg per kg of bodyweight per day. The maintenance doses are preferably administered no more than once every 5, 10, or 30 days. Further, the treatment regimen may last for a period of time which will vary depending upon the nature of the particular disease, its severity and the overall condition of the patient. In preferred embodiments the dosage may be delivered no more than once per day, e.g., no more than once per 24, 36, 48, or more hours, e.g., no more than once every 5 or 8 days. Following treatment, the patient can be monitored for changes in condition, e.g., changes in percentage body fat. The dosage of the compound may either be increased in the event the patient does not respond significantly to current dosage levels, or the dose may be decreased if a decrease in body fat is observed, or if undesired side effects are observed.

The effective dose can be administered in a single dose or in two or more doses, as desired or considered appropriate under the specific circumstances. If desired to facilitate repeated or frequent infusions, implantation of a delivery device, e.g., a pump, semi-permanent stent (e.g., sub-cutaneous, intravenous, intraperitoneal, intracisternal or intracapsular), or reservoir may be advisable. In one embodiment, a pharmaceutical composition includes a plurality of miRNA agent species. In another embodiment, the miRNA agent species has sequences that are non-overlapping and non-adjacent to another species with respect to a naturally occurring target sequence. In another embodiment, the plurality of miRNA agent species is specific for different naturally occurring target genes. In another embodiment, the miRNA agent is allele specific. In another embodiment, the plurality of miRNA agent species target two or more SNP alleles (e.g., two, three, four, five, six, or more SNP alleles).

Following successful treatment, it may be desirable to have the patient undergo maintenance therapy to prevent the recurrence of the disease state, wherein the compound of the invention is administered in maintenance doses, ranging from 0.01 mg per kg to 100 mg per kg of body weight (see U.S. Pat. No. 6,107,094).

The concentration or amount of miRNA agent administered will depend on the parameters determined for the agent and the method of administration, e.g. nasal, buccal, or pulmonary. For example, nasal formulations tend to require much lower concentrations of some ingredients in order to avoid irritation or burning of the nasal passages. It is sometimes desirable to dilute an oral formulation up to 10-100 times in order to provide a suitable nasal formulation.

Certain factors may influence the dosage required to effectively treat a subject, including but not limited to the severity of the disease or disorder, previous treatments, the general health and/or age of the subject, and other diseases present. Moreover, treatment of a subject with a therapeutically effective amount of a miRNA agent can include a single treatment or, preferably, can include a series of treatments. It will also be appreciated that the effective dosage of a miRNA agent for treatment may increase or decrease over the course of a particular treatment. Changes in dosage may result and become apparent from the results of diagnostic assays as described herein. For example, the subject can be monitored after administering a miRNA agent composition. Based on information from the monitoring, an additional amount of the miRNA agent composition can be administered.

Dosing is dependent on severity and responsiveness of the disease condition to be treated, with the course of treatment lasting from several days to several months, or until a cure is effected or a diminution of disease state is achieved. Optimal dosing schedules can be calculated from measurements of drug accumulation in the body of the patient. Persons of ordinary skill can easily determine optimum dosages, dosing methodologies and repetition rates. Optimum dosages may vary depending on the relative potency of individual compounds, and can generally be estimated based on EC50s found to be effective in *in vitro* and *in vivo* animal models. In some embodiments, the animal models include transgenic animals that express a human gene, e.g., a gene that produces a target mRNA (e.g., a thermogenic regulator). The transgenic animal can be deficient for the corresponding endogenous mRNA. In another embodiment, the composition for testing includes a miRNA agent that is complementary, at least in an internal region, to a sequence that is conserved between a nucleic acid sequence in the animal model and the target nucleic acid sequence in a human.

Several studies have reported successful mammalian dosing using miRNA agents. For example, Esau C, et al., Cell Metabolism, 3(2): 87-98 (2006) reported dosing of normal mice with intraperitoneal doses of miR-122 antisense oligonucleotide ranging from 12.5 to 75 mg/kg twice weekly for 4 weeks. The mice appeared healthy and normal at the end of treatment, with no loss of body weight or reduced food intake. Plasma transaminase levels were in the normal range (AST¾ 45, ALT ¾ 35) for all doses with the exception of the 75 mg/kg dose of miR-122 ASO, which showed a very mild increase in ALT and AST levels. They concluded that 50mg/kg was an effective, nontoxic dose. Another study by Krutzfeldt J., et al., Nature, 438, 685-689 (2005), injected antagomirs to silence miR-122 in mice using a total dose of 80, 160 or 240 mg per kg body weight. The highest dose resulted in a complete loss of miR-122 signal. In yet another study, locked nucleic acids ("LNAs") were successfully applied in primates to silence miR-122. Elmen J., et al., (2008) Nature 452, 896-899, report that efficient silencing of miR-122 was achieved in primates by three doses of 10 mg per kg LNA-antimiR, leading to a long-lasting and reversible decrease in total plasma cholesterol without any evidence for LNA-associated toxicities or histopathological changes in the study animals.

In certain embodiments, miRNA agents used to practice this invention are administered through expression from a recombinant vector. Suitable recombinant vectors include, without limitation, DNA plasmids, viral vectors or DNA minicircles. Generation of the vector construct can be accomplished using any suitable genetic engineering techniques well known in the art, including, without limitation, the standard techniques of PCR, oligonucleotide synthesis, restriction endonuclease digestion, ligation, transformation, plasmid purification, and DNA sequencing, for example as described in Sambrook et al. Molecular Cloning: A Laboratory Manual. (1989)), Coffin et al. (Retroviruses. (1997)) and "RNA Viruses: A Practical Approach" (Alan J. Cann, Ed., Oxford University Press, (2000)). As will be apparent to one of ordinary skill in the art, a variety of suitable vectors are available for transferring nucleic acids of the invention into cells. The selection of an appropriate vector to deliver nucleic acids and optimization of the conditions for insertion of the selected expression vector into the cell, are within the scope of one of ordinary skill in the art without the need for undue experimentation. Viral vectors comprise a nucleotide sequence having sequences for the production of recombinant virus in a packaging cell. Viral vectors expressing nucleic acids of the invention can be constructed based on viral backbones including, but not limited to, a retrovirus, lentivirus, adenovirus, adeno-associated virus, pox virus or alphavirus. The recombinant vectors can be delivered as described herein, and persist in target cells (e.g., stable transformants).

miRNA agents may be directly introduced into a cell (e.g., an adipocyte); or introduced extracellularly into a cavity, interstitial space, into the circulation of an organism, introduced orally, or may be introduced by bathing a cell or organism in a solution containing the nucleic acid. Vascular or extravascular circulation, the blood or lymph system, and the cerebrospinal fluid are sites where the nucleic acid may be introduced.

The miRNA agents of the invention can be introduced using nucleic acid delivery methods known in art including injection of a solution containing the nucleic acid, bombardment by particles covered by the nucleic acid, soaking the cell or organism in a solution of the nucleic acid, or electroporation of cell membranes in the presence of the nucleic acid. Other methods known in the art for introducing nucleic acids to cells may be used, such as lipid-mediated carrier transport, chemical-mediated transport, and cationic liposome transfection such as calcium phosphate, and the like. The miRNA agents may be introduced along with other components e.g., compounds that enhance miRNA agent uptake by a cell.

In certain embodiments, the methods described herein include co-administration of miRNA agents with other drugs or pharmaceuticals, e.g., compositions for modulating thermogenesis, compositions for treating diabetes, compositions for treating obesity. Compositions for modulating thermogenesis include beta-3 adrenergic receptor agonists, thyroid hormones, PPARG agonists, leptin, adiponectin, and orexin.

### V. Screening Methods

In another aspect, the disclosure provides a method of screening for a miRNA agent that modulates thermogenesis, decreases obesity, or improves insulin sensitivity. The method generally comprises the steps of: providing an indicator cell; contacting the indicator cell with a test miRNA agent; and determining the expression level and/or cellular activity of at least one thermogenic regulator in the indicator cell in the presence and absence of the miRNA agent, wherein a change in the activity of the thermogenic regulator in the presence of the test miRNA agent identifies the test miRNA agent as a miRNA agent that modulates thermogenesis, decreases obesity, or improves insulin sensitivity. According to the disclosure, the method involves determining an increase the expression level and/or activity of the thermogenic regulator (e.g., UCP1, UCP2). The indicator cell can be a mammalian cell. According to the disclosure, the mammalian cell is a human cell, which comprises at least a portion of a human genome.

Any thermogenic regulator can be assayed in the methods disclosed herein. Exemplary thermogenic regulators are set forth in Table 2. According to the disclosure, the thermogenic regulator is a mitochondrial uncoupling protein e.g., UCP1 and/or UCP2.

Any cell in which the activity of a thermogenic regulator can be measured is suitable for use in the methods disclosed herein. Exemplary cells include pre-adipocytes, adipocytes, adipose tissue derived mesenchymal stem cells, hepatocytes, myocytes, or precursors thereof.

According to the disclosure, any activity of a thermogenic regulator can be assayed, including, without limitation, mRNA expression level, protein expression level or mitochondrial uncoupling activity of the thermogenic regulator. Methods for determining such activities are well known in the art.

According to the disclosure, any miRNA agent can be screened, including, without limitation, miRNA, agomirs, antagomirs, aptamirs, miR-masks, miRNA sponges, siRNA (single- or double-stranded), shRNA, antisense oligonucleotides, ribozymes, or other oligonucleotide mimetics which hybridize to at least a portion of a target nucleic acid and modulate its function.

### VI. Pharmaceutical Compositions

According to the disclosure, the methods disclosed herein can include the administration of pharmaceutical compositions and formulations comprising miRNA agents capable of modulating the activity of at least one thermogenic modulator.

According to the disclosure, the compositions are formulated with a pharmaceutically acceptable carrier. The pharmaceutical compositions and formulations can be administered parenterally, topically, by direct administration into the gastrointestinal tract (e.g., orally or rectally), or by local administration, such as by aerosol or transdermally. The pharmaceutical compositions can be formulated in any way and can be administered in a variety of unit dosage forms depending upon the condition or disease and the degree of illness, the general medical condition of each patient, the resulting preferred method of administration and the like. Details on techniques for formulation and administration of pharmaceuticals are well described in the scientific and patent literature, see, e.g., Remington: The Science and Practice of Pharmacy, 21st ed., 2005.

According to the disclosure, the miRNA agents can be administered alone or as a component of a pharmaceutical formulation (composition). The compounds may be formulated for administration, in any convenient way for use in human or veterinary medicine. Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Formulations of the compositions of the disclosure include those suitable for intradermal, inhalation, oral/ nasal, topical, parenteral, rectal, and/or intravaginal administration. The formulations may conveniently be presented in unit dosage form and may be prepared by any methods well known in the art of pharmacy. The amount of active ingredient (e.g., nucleic acid sequences of this invention) which can be combined with a carrier material to produce a single dosage form will vary depending upon the host being treated, the particular mode of administration, e.g., intradermal or inhalation. The amount of active ingredient which can be combined with a carrier material to produce a single dosage form will generally be that amount of the compound which produces a therapeutic effect, e.g., an antigen specific T cell or humoral response.

Pharmaceutical formulations of the disclosure can be prepared according to any method known to the art for the manufacture of pharmaceuticals. Such drugs can contain sweetening agents, flavoring agents, coloring agents and preserving agents. A formulation can be admixtured with nontoxic pharmaceutically acceptable excipients which are suitable for manufacture. Formulations may comprise one or more diluents, emulsifiers, preservatives, buffers, excipients, etc. and may be provided in such forms as liquids, powders, emulsions, lyophilized powders, sprays, creams, lotions, controlled release formulations, tablets, pills, gels, on patches, in implants, etc.

Pharmaceutical formulations for oral administration can be formulated using pharmaceutically acceptable carriers well known in the art in appropriate and suitable dosages. Such carriers enable the pharmaceuticals to be formulated in unit dosage forms as tablets, pills, powder, dragées, capsules, liquids, lozenges, gels, syrups, slurries, suspensions, etc., suitable for ingestion by the patient. Pharmaceutical preparations for oral use can be formulated as a solid excipient, optionally grinding a resulting mixture, and processing the mixture of granules, after adding suitable additional compounds, if desired, to obtain tablets or dragee cores. Suitable solid excipients are carbohydrate or protein fillers include, e.g., sugars, including lactose, sucrose, mannitol, or sorbitol; starch from corn, wheat, rice, potato, or other plants; cellulose such as methyl cellulose, hydroxypropylmethyl-cellulose, or sodium carboxymethylcellulose; and gums including arabic and tragacanth; and proteins, e.g., gelatin and collagen. Disintegrating or solubilizing agents may be added, such as the cross-linked polyvinyl pyrrolidone, agar, alginic acid, or a salt thereof, such as sodium alginate. Push-fit capsules can contain active agents mixed with a filler or binders such as lactose or starches, lubricants such as talc or magnesium stearate, and, optionally, stabilizers. In soft capsules, the active agents can be dissolved or suspended in suitable liquids, such as fatty oils, liquid paraffin, or liquid polyethylene glycol with or without stabilizers.

Aqueous suspensions can contain an active agent (e.g., nucleic acid sequences of the invention) in admixture with excipients suitable for the manufacture of aqueous suspensions, e.g., for aqueous intradermal injections. Such excipients include a suspending agent, such as sodium carboxymethylcellulose, methylcellulose, hydroxypropylmethylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethylene oxycetanol), a condensation product of ethylene oxide with a partial ester derived from a fatty acid and a hexitol (e.g., polyoxyethylene sorbitol mono-oleate), or a condensation product of ethylene oxide with a partial ester derived from fatty acid and a hexitol anhydride (e.g., polyoxyethylene sorbitan mono-oleate). The aqueous suspension can also contain one or more preservatives such as ethyl or n-propyl p-hydroxybenzoate, one or more coloring agents, one or more flavoring agents and one or more sweetening agents, such as sucrose, aspartame or saccharin. Formulations can be adjusted for osmolarity.

According to the disclosure, oil-based pharmaceuticals are used for administration of the miRNA agents. Oil-based suspensions can be formulated by suspending an active agent in a vegetable oil, such as arachis oil, olive oil, sesame oil or coconut oil, or in a mineral oil such as liquid paraffin; or a mixture of these. See e.g., U.S. Patent No. 5,716,928 describing using essential oils or essential oil components for increasing bioavailability and reducing inter- and intra-individual variability of orally administered hydrophobic pharmaceutical compounds (see also U.S. Patent No. 5,858,401). The oil suspensions can contain a thickening agent, such as beeswax, hard paraffin or cetyl alcohol. Sweetening agents can be added to provide a palatable oral preparation, such as glycerol, sorbitol or sucrose. These formulations can be preserved by the addition of an antioxidant such as ascorbic acid. As an example of an injectable oil vehicle, see Minto (1997) J. Pharmacol. Exp. Ther. 281:93-102.

According to the disclosure, the pharmaceutical compositions and formulations are in the form of oil-in- water emulsions. The oily phase can be a vegetable oil or a mineral oil, described above, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsion can also contain sweetening agents and flavoring agents, as in the formulation of syrups and elixirs. Such formulations can also contain a demulcent, a preservative, or a coloring agent. In alternative embodiments, these injectable oil-in-water emulsions of the invention comprise a paraffin oil, a sorbitan monooleate, an ethoxylated sorbitan monooleate and/or an ethoxylated sorbitan trioleate.

According to the disclosure, the pharmaceutical compositions and formulations are administered by in intranasal, intraocular and intravaginal routes including suppositories, insufflation, powders and aerosol formulations (for examples of steroid inhalants, see e.g., Rohatagi (1995) J. Clin. Pharmacol. 35: 1 187-1193; Tjwa (1995) Ann. Allergy Asthma Immunol. 75: 107-1 11). Suppositories formulations can be prepared by mixing the drug with a suitable non-irritating excipient which is solid at ordinary temperatures but liquid at body temperatures and will therefore melt in the body to release the drug. Such materials are cocoa butter and polyethylene glycols.

According to the disclosure, the pharmaceutical compositions and formulations are delivered transdermally, by a topical route, formulated as applicator sticks, solutions, suspensions, emulsions, gels, creams, ointments, pastes, jellies, paints, powders, and aerosols.

According to the disclosure, the pharmaceutical compositions and formulations are delivered as microspheres for slow release in the body. For example, microspheres can be administered via intradermal injection of drug which slowly release subcutaneously; see Rao (1995) J. Biomater Sci. Polym. Ed. 7:623-645; as biodegradable and injectable gel formulations, see, e.g., Gao (1995) Pharm. Res. 12:857-863 (1995); or, as microspheres for oral administration, see, e.g., Eyles (1997) J. Pharm. Pharmacol. 49:669-674.

According to the disclosure, the pharmaceutical compositions and formulations are parenterally administered, such as by intravenous (IV) administration or administration into a body cavity or lumen of an organ. These formulations can comprise a solution of active agent dissolved in a pharmaceutically acceptable carrier. Acceptable vehicles and solvents that can be employed are water and Ringer's solution, an isotonic sodium chloride. In addition, sterile fixed oils can be employed as a solvent or suspending medium. For this purpose any bland fixed oil can be employed including synthetic mono- or diglycerides. In addition, fatty acids such as oleic acid can likewise be used in the preparation of injectables. These solutions are sterile and generally free of undesirable matter. These formulations may be sterilized by conventional, well-known sterilization techniques. The formulations may contain pharmaceutically acceptable auxiliary substances as required to approximate physiological conditions such as pH adjusting and buffering agents, toxicity adjusting agents, e.g., sodium acetate, sodium chloride, potassium chloride, calcium chloride, sodium lactate and the like. The concentration of active agent in these formulations can vary widely, and will be selected primarily based on fluid volumes, viscosities, body weight, and the like, in accordance with the particular mode of administration selected and the patient's needs. For IV administration, the formulation can be a sterile injectable preparation, such as a sterile injectable aqueous or oleaginous suspension. This suspension can be formulated using those suitable dispersing or wetting agents and suspending agents. The sterile injectable preparation can also be a suspension in a nontoxic parenterally-acceptable diluent or solvent, such as a solution of 1,3-butanediol. The administration can be by bolus or continuous infusion (e.g., substantially uninterrupted introduction into a blood vessel for a specified period of time).

According to the disclosure, the pharmaceutical compounds and formulations are lyophilized. Stable lyophilized formulations comprising an inhibitory nucleic acid can be made by lyophilizing a solution comprising a pharmaceutical of the invention and a bulking agent, e.g., mannitol, trehalose, raffinose, and sucrose or mixtures thereof. A process for preparing a stable lyophilized formulation can include lyophilizing a solution about 2.5 mg/mL nucleic acid, about 15 mg/mL sucrose, about 19 mg/mL NaCl, and a sodium citrate buffer having a pH greater than 5.5 but less than 6.5. See, e.g., U.S. 20040028670.

According to the disclosure, the pharmaceutical compositions and formulations are delivered by the use of liposomes. By using liposomes, particularly where the liposome surface carries ligands specific for target cells, or are otherwise preferentially directed to a specific organ, one can focus the delivery of the active agent into target cells in vivo. See, e.g., U.S. Patent Nos. 6,063,400; 6,007,839; Al-Muhammed (1996) J. Microencapsul. 13 :293-306; Chonn (1995) Curr. Opin. Biotechnol. 6:698-708; Ostro (1989) Am. J. Hosp. Pharm. 46: 1576-1587.

The formulations of the disclosure can be administered for prophylactic and/or therapeutic treatments. In certain embodiments, for therapeutic applications, compositions are administered to a subject who is need of reduced triglyceride levels, or who is at risk of or has a disorder described herein, in an amount sufficient to cure, alleviate or partially arrest the clinical manifestations of the disorder or its complications; this can be called a therapeutically effective amount. For example, in certain embodiments, pharmaceutical compositions of the invention are administered in an amount sufficient to treat obesity in a subject.

The amount of pharmaceutical composition adequate to accomplish this is a therapeutically effective dose. The dosage schedule and amounts effective for this use, i.e., the dosing regimen, will depend upon a variety of factors, including the stage of the disease or condition, the severity of the disease or condition, the general state of the patient's health, the patient's physical status, age and the like. In calculating the dosage regimen for a patient, the mode of administration also is taken into consideration.

The dosage regimen also takes into consideration pharmacokinetics parameters well known in the art, i.e., the active agents' rate of absorption, bioavailability, metabolism, clearance, and the like (see, e.g., Hidalgo-Aragones (1996) J. Steroid Biochem. Mol. Biol. 58:611-617; Groning (1996) Pharmazie 51:337-341; Fotherby (1996) Contraception 54:59-69; Johnson (1995) J. Pharm. Sci. 84: 1 144-1 146; Rohatagi (1995) Pharmazie 50:610-613; Brophy (1983) Eur. J. Clin. Pharmacol. 24: 103-108; Remington: The Science and Practice of Pharmacy, 21st ed., 2005). The state of the art allows the clinician to determine the dosage regimen for each individual patient, active agent and disease or condition treated. Guidelines provided for similar compositions used as pharmaceuticals can be used as guidance to determine the dosage regiment, i.e., dose schedule and dosage levels, administered practicing the methods of the invention are correct and appropriate. Single or multiple administrations of formulations can be given depending on for example: the dosage and frequency as required and tolerated by the patient, the degree and amount of cholesterol homeostasis generated after each administration, and the like. The formulations should provide a sufficient quantity of active agent to effectively treat, prevent or ameliorate conditions, diseases or symptoms, e.g., treat obesity.

According to the disclosure, pharmaceutical formulations for oral administration are in a daily amount of between about 1 to 100 or more mg per kilogram of body weight per day. Lower dosages can be used, in contrast to administration orally, into the blood stream, into a body cavity or into a lumen of an organ. Substantially higher dosages can be used in topical or oral administration or administering by powders, spray or inhalation. Actual methods for preparing parenterally or non-parenterally administrable formulations will be known or apparent to those skilled in the art and are described in more detail in such publications as Remington: The Science and Practice of Pharmacy, 21st ed., 2005.

### VII. Exemplification

The present invention is further illustrated by the following examples which should not be construed as further limiting. The contents of Sequence Listing, figures and all references, patents and published patent applications cited throughout this application are expressly incorporated herein by reference.

Furthermore, in accordance with the present invention there may be employed conventional molecular biology, microbiology, and recombinant DNA techniques within the skill of the art. Such techniques are explained fully in the literature. See, *e.g*., Sambrook, Fritsch & Maniatis, Molecular Cloning: A Laboratory Manual, Second Edition (1989) Cold Spring Harbor Laboratory Press, Cold Spring Harbor, New York (herein "Sambrook et al., 1989"); DNA Cloning: A Practical Approach, Volumes I and II (D.N. Glover ed. 1985); Oligonucleotide Synthesis (M.J. Gait ed. 1984); Nucleic Acid Hybridization [B.D. Hames & S.J. Higgins eds. (1985)]; Transcription And Translation [B.D. Hames & S.J. Higgins, Eds. (1984)]; Animal Cell Culture [R.I. Freshney, ed. (1986)]; Immobilized Cells And Enzymes [IRL Press, (1986)]; B. Perbal, A Practical Guide To Molecular Cloning (1984); F.M. Ausubel et al. (eds.), Current Protocols in Molecular Biology, John Wiley & Sons, Inc. (1994).

### Example 1. In-silico Analysis of Thermogenic Regulators

Eighty-three proteins that are involved in regulation of thermogenesis were selected based upon a critical assessment and review of the available scientific information and our own experimental data. These proteins were categorized as activators or repressors of thermogenesis based upon their functions. These thermogenic regulator proteins are set forth in Table 2.

**Table 2.**

| **Thermogenic regulator proteins:** | | | |
|---|---|---|---|
| **Activators** | | | |
| | **Name** | **Entrez Gene ID** | **Ensembl Gene ID** |
| 1 | ALDH1A1 | 216 | ENSG00000165092 |
| 2 | ANP (NPPA) | 4878 | ENSG00000175206 |
| 3 | AZGP1 | 563 | ENSG00000160862 |
| 4 | BMP7 | 655 | ENSG00000101144 |
| 5 | BMP8B | 656 | ENSG00000116985 |
| 6 | CEBPA | 1050 | ENSG00000245848 |
| 7 | CEBPB | 1051 | ENSG00000172216 |
| 8 | CEBPD | 1052 | ENSG00000221869 |
| 9 | CIDEA | 1149 | ENSG00000176194 |
| 10 | COX7A1 | 1346 | ENSG00000161281 |
| 11 | CRAT | 1384 | ENSG00000095321 |
| 12 | CREB1 | 1385 | ENSG00000118260 |
| 13 | CREBBP | 1387 | ENSG00000005339 |
| 14 | CTBP1 | 1487 | ENSG00000159692 |
| 15 | CTBP2 | 1488 | ENSG00000175029 |
| 16 | DIO2 | 1734 | ENSG00000211448 |
| 17 | ELOVL3 | 83401 | ENSG00000119915 |
| 18 | FGF16 | 8823 | ENSG00000196468 |
| 19 | FGF19 | 9965 | ENSG00000162344 |
| 20 | FGF21 | 26291 | ENSG00000105550 |
| 21 | FNDC5 | 252995 | ENSG00000160097 |
| 22 | FOXC2 | 2303 | ENSG00000176692 |
| 23 | GDF3 | 9573 | ENSG00000184344 |
| 24 | HCRT (OREXIN) | 3060 | ENSG00000161610 |
| 25 | HOXC8 | 3224 | ENSG00000037965 |
| 26 | INSR | 3643 | ENSG00000171105 |
| 27 | IRS1 | 3667 | ENSG00000169047 |
| 28 | KDM3A (JMJD1A) | 55818 | ENSG00000115548 |
| 29 | KLF5 | 688 | ENSG00000102554 |
| 30 | KLF11 | 8462 | ENSG00000172059 |
| 31 | KLF15 | 28999 | ENSG00000163884 |
| 32 | LRP6 | 4040 | ENSG00000070018 |
| 33 | MAPK14 | 1432 | ENSG00000112062 |
| 34 | MED13 | 9969 | ENSG00000108510 |
| 35 | NCOA1 | 8648 | ENSG00000084676 |
| 36 | NCOA2 | 10499 | ENSG00000140396 |
| 37 | NCOA3 | 8202 | ENSG00000124151 |
| 38 | NR4A3 | 8013 | ENSG00000119508 |
| 39 | NRF1 | 4899 | ENSG00000106459 |
| 40 | PLAC8 | 51316 | ENSG00000145287 |
| 41 | PPARA | 5465 | ENSG00000186951 |
| 42 | PPARD | 5467 | ENSG00000112033 |
| 43 | PPARG | 5468 | ENSG00000132170 |
| 44 | PPARGC1A | 10891 | ENSG00000109819 |
| 45 | PPARGC1B | 133522 | ENSG00000155846 |
| 46 | PRDM16 | 63976 | ENSG00000142611 |
| 47 | PRDX3 | 10935 | ENSG00000165672 |
| 48 | PRKAA1 (AMPKA1) | 5562 | ENSG00000132356 |
| 49 | PRKAA2 (AMPKA2) | 5563 | ENSG00000162409 |
| 50 | PRKACA | 5566 | ENSG00000072062 |
| 51 | PRKACB | 5567 | ENSG00000142875 |
| 52 | PRKARIA | 5573 | ENSG00000108946 |
| 53 | SIRT1 | 23411 | ENSG00000096717 |
| 54 | SIRT3 | 23410 | ENSG00000142082 |
| 55 | SLC27A2 (FATP2) | 11001 | ENSG00000140284 |
| 56 | SREBF1 | 6720 | ENSG00000072310 |
| 58 | SREBF2 | 6721 | ENSG00000198911 |
| 58 | STAT5A | 6776 | ENSG00000126561 |
| 59 | TRPM8 | 79054 | ENSG00000144481 |
| 60 | UCP1 (SLC25A7) | 7350 | ENSG00000109424 |
| 61 | UCP2 (SLC25A8) | 7351 | ENSG00000175567 |
| 62 | UCP3 (SLC25A9) | 7352 | ENSG00000175564 |

| **Repressors** | | | |
|---|---|---|---|
| | **Name** | **Entrez Gene ID** | **Ensembl Gene ID** |
| 1 | ATG7 | 10533 | ENSG00000197548 |
| 2 | BMP2 | 650 | ENSG00000125845 |
| 3 | BMP4 | 652 | ENSG00000125378 |
| 4 | CIDEC | 63924 | ENSG00000187288 |
| 5 | CTNNB1 | 1499 | ENSG00000168036 |
| 6 | DLK1 (Pref-1) | 8788 | ENSG00000185559 |
| 7 | E2F4 (p107) | 1874 | ENSG00000205250 |
| 8 | EIF4EBP1 | 1978 | ENSG00000187840 |
| 9 | ESRRA (NR3B1) | 2101 | ENSG00000173153 |
| 10 | IKBKE | 9641 | ENSG00000143466 |
| 11 | NR1H3 (LXRA) | 10062 | ENSG00000025434 |
| 12 | NRIP1 (RIP140) | 8204 | ENSG00000180530 |
| 13 | RB1 (pRb) | 5925 | ENSG00000139687 |
| 14 | NR0B2 (SHP) | 8431 | ENSG00000131910 |
| 15 | RPS6KB1 | 6198 | ENSG00000108443 |
| 16 | RUNX1T1 | 862 | ENSG00000079102 |
| 17 | RUNX2 | 860 | ENSG00000124813 |
| 18 | TNFRSF1A | 7132 | ENSG00000067182 |
| 19 | TWIST1 | 7291 | ENSG00000122691 |
| 20 | WNT5A | 7474 | ENSG00000114251 |
| 21 | WNT10B | 7480 | ENSG00000169884 |

The STRING 9.0 database of known and predicted protein interactions (string-db.org/) was used to test these 83 candidate molecules. The interactions include direct (physical) and indirect (functional) associations; they are derived from four sources: genomic context; high-throughput experiments; co-expression; and previous knowledge. STRING quantitatively integrates interaction data from these sources for a large number of organisms, and transfers information between these organisms where applicable. The database currently covers 5,214,234 proteins from 1,133 organisms. As an example, the relationships between the 83 thermogenic regulator molecules were centered on UCP1, and molecules having direct and indirect connections with UCP1 could be distinguished using the highest confidence score of 0.90. This relationship is set forth in schematic form in Figure 1A. From this analysis, it was discovered that nine molecules (CEBPB, CIDEA, KDM3A, NRIP1, PRDM16, PPARG, PPARGC1A, PPKAA2, and UCP2) are directly linked to UCP1, whereas many more molecules are connected to UCP1 on a second or higher degree order.

When the degree of confidence was set to high with a score of 0.70, eight additional proteins were found to be directly linked to UCP1 (AZGP1, DIO2, KLF11, KLF15, NR1H3, PPARA, PPARD, and PPARGC1B), Figure 1B.

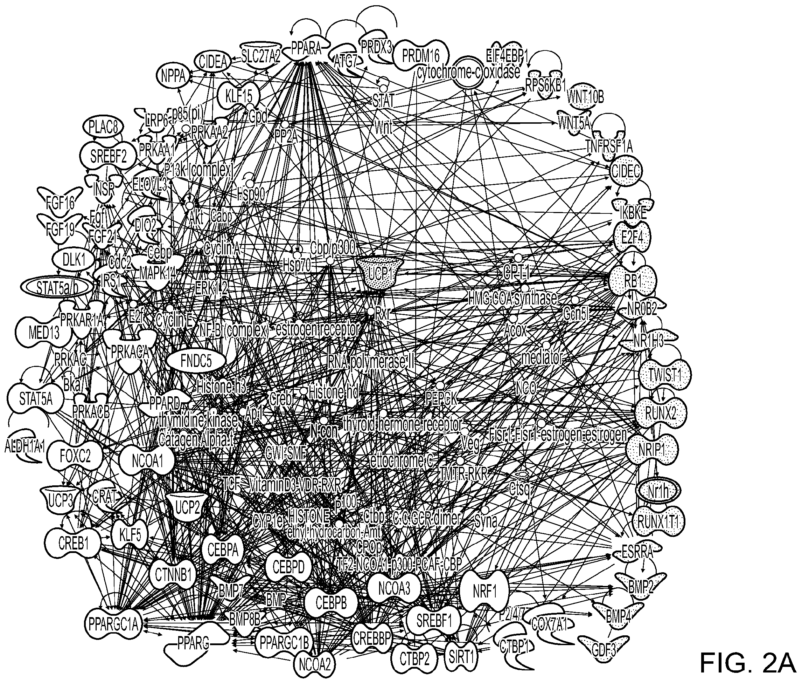

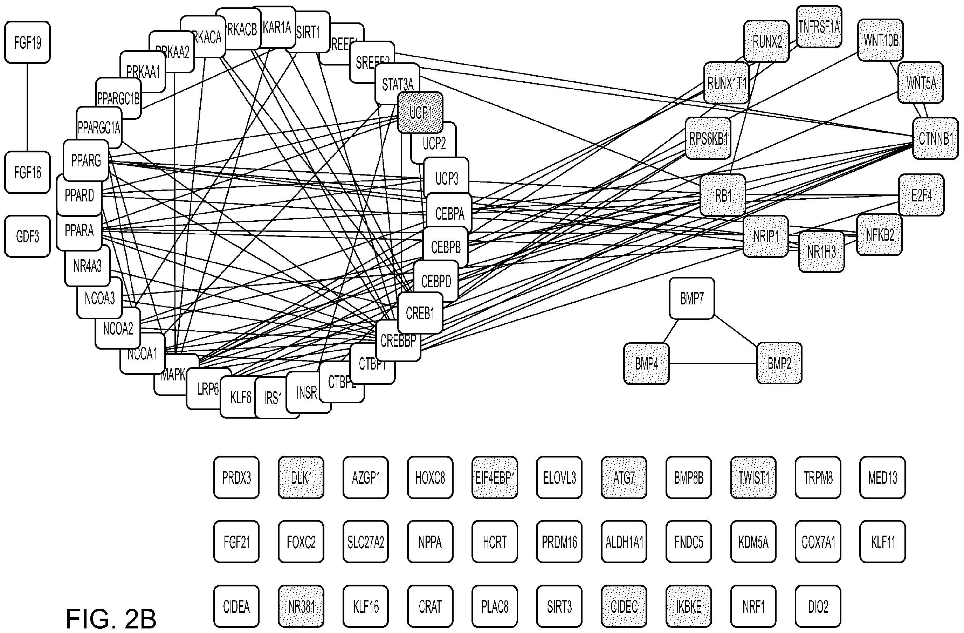

Similarly, the interactions among these 83 thermogenic regulator molecules were independently assessed using other software programs. The interactions predicted by the Ingenuity Pathway Analysis (IPA) Software program (www.ingenuity.com) are shown in Figure 2A (UCP1 in yellow, activators in green and repressors in purple). The interactions predicted by the Reactome Functional Interaction (Reactome IF) Software program (http://wiki.reactome.org) are shown in Figure 2B (UCP1 in yellow, activators in green and repressors in purple). The IPA and Reactome IF networks differ from the ones set forth in Figures 1A and 1B, obtained with the STRING program. It is not surprising that the results of these algorithms are different because they rely on different predefined parameters, sources of information and selection criteria.

### Example 2. In-silico Selection of Relevant miRNA Targets

To select thermogenic regulators suitable as targets for miRNA agents, several internet-based resources were employed to match miRNAs and their targets (the "micronome"). Exemplary tools are set forth in Table 3.

**Table 3.**

| **Exemplary bioinformatics tools used to select miRNAs and their targets:** | |
|---|---|
| **Field & Name** | **Web Address** |
| **Integrated Data Mining (8)** | |
| BioCarta | http://www.biocarta.com/ |
| Database for Annotation, Visualization and Integrated Discovery (DAVID) | http://david.abcc.ncifcrf.gov/home.jsp |
| GeneOntology | http://www.geneontology.org/ |
| Gene Set Enrichment Analysis (GSEA) | http://www.broadinstitute.org/gsea/index. j sp |
| KEGG | http: //www. genome.j p/kegg/ |
| PubGene | http://www.pubgene. org/ |
| Reactome | http://www.reactome. org/ReactomeGWT/entrypoint.ht ml |
| STRING | http://string-db. org/ |

| **miRNA Mining & Mapping (8)** | |
|---|---|
| deepBase | http://deepbase. sysu.edu.cn/ |
| Human microRNA disease database (HMDD) | http://202.38.126.151/hmdd/mirna/md/ |
| miRBase V19 | http://www.mirbase. org/ |
| miRGen 2.0 | http://diana. cslab.ece. ntua. gr/mirgen/ |
| miRNAMap | http://mirnamap.mbc.nctu. edu.tw/ |
| miRSel | http://services.bio.ifi.lmu.de/mirsel/ |
| miRStart | http://mirstart.mbc.nctu.edu.tw/home.php |
| miR2Disease | http://www.mir2disease. org |

| **miRNA Targets & Expression (21)** | |
|---|---|
| DIANA-microT 3.0 | http://diana. cslab.ece. ntua. gr/microT/ |
| DIANA-mirExTra | http://diana. cslab.ece. ntua. gr/hexamers/ |
| GSEA Molecular Signatures Database v3.0 | http://www.broadinstitute.org/gsea/index. j sp |
| MicroCosm Targets | http://www.ebi.ac.uk/enright-srv/microcosm/cgi-bin/targets/v5/download.pl |
| MicroInspector | http://bioinfo.uni-plovdiv.bg/microinspector/ |
| microRNA.org (ex. miRanda) | http://www.microrna. org/microrna/home. do |
| miRDB | http://mirdb. org/miRDB/ |
| miRTarBase | http://mirtarbase.mbc.nctu.edu.tw/index.html |
| miRTar.Human | http://mirtar.mbc.nctu.edu.tw/human/download.php |
| miRvestigator | http://mirvestigator. systemsbiology. net/ |
| mirZ | http: //www. mirz.unibas. ch/ElMMo2/ |
| MultiMiTar | http://www.isical.ac.in/∼bioinfo_miu/multimitar.htm |
| PhenomiR | http://mips.helmholtz-muenchen.de/phenomir/index. gsp |
| PicTar | http://pictar.mdc-berlin.de/ |
| PITA | http://genie.weizmann.ac.il/pubs/mir07/mir07_data.htm l |
| RepTar | http://bioinformatics.ekmd.huji.ac.il/reptar/ |
| RNAhybrid | http://bibiserv.techfak.uni-bielefeld.de/rnahybrid/ |
| RNA22 | http://cbcsrv.watson.ibm.com/rna22.html |
| Sylamer | http ://www.ebi.ac. uk/enright/sylamer/ |
| TarBase 6.0 | http://diana. cslab.ece. ntua. gr/DianaToolsNew/index. ph p?r=tarbase/index |
| TargetScanHuman 6.2 | http://www.targetscan. org/ |

| **Integrated miRNA Targets & Expression Tools (13)** | |
|---|---|
| GOmir | http://www. bioacademy.gr/bioinformatics/projects/GO mir/ |
| MAMI (MetaMiR: Target Inference) | http: //mami. med.harvard. edu/ |
| mimiRNA | http://mimirna.centenary.org.au/mep/formulaire. html |
| MMIA (microRNA and mRNA Integrated Analysis) | http://147.46.15.115/MMIA/index.html |
| mirDIP | http://ophid.utoronto. ca/mirDIP/ |
| miRGator V3.0 | http://mirgator.kobic.re.kr |
| miRecords | http://mirecords.biolead. org/ |
| MIRNA-DISTILLER | http://www.ikp-stuttgart. de/content/language 1/html/10415.asp |
| MiRon Top | http://www.microarray.fr: 8080/miRonTop/index |
| miRror | http://www.proto.cs.huji.ac.il/mirror |
| miRSystem | http://mirsystem.cgm.ntu.edu.tw/ |
| miRWalk | http://www.ma.uni-heidelberg.de/apps/zmf/mirwalk/index.html |
| StarBase | http://starbase. sysu.edu.cn/index. php |

| **miRNA Secondary Structure (5)** | |
|---|---|
| OligoWalk | http ://rna. urmc.rochester. edu/cgi-bin/server_exe/oligowalk/oligowalk_ form.cgi |
| PicTar RNA Studio | http://www.pictar. org/ |
| RNA2D | http://protein3 d.ncifcrf. gov/shuyun/rna2d. html |
| Vienna RNA Package | http://www.tbi.univie.ac.at/ivo/RNA/ |
| Whitehead siRNA algorithm | http://jura.wi.mit.edu/bioc/siRNAext/ |

| **Network Searches & Analyses (8**) | |
|---|---|
| ARIADNE Pathway Studio | http://www. ariadnegenomics.com/products/pathway-studio/ |
| Cytoscape | http://www. cytoscape.org/ |
| Database for Annotation, Visualization and Integrated Discovery (DAVID) | http://david.abcc.ncifcrf.gov/home.jsp |
| Genego MetaCore | http://www.genego.com/metacore.php |
| Ingenuity Systems IPA (Ingenuity Pathway Analysis) | http://www.ingenuity. com/products/IPA/microRNA. ht ml |
| MATISSE (Module Analysis via Topology of Interactions and Similarity Sets) | http://acgt. cs.tau.ac. il/matisse/ |
| MIR@NT@N | http://mironton.uni.lu |
| NAViGaTOR | http://ophid.utoronto. ca/navigator/index. html |

| **Molecular Visualization (4)** | |
|---|---|
| Foldit | http://fold.it/portal/info/science |
| PyMOL | http://www.pymol. org/ |
| Qlucore Omics Explorer | http://www. qlucore. com/ProdOverviewmiRNA. aspx |
| WebMol | http://www.cmpharm.ucsf.edu/cgi-bin/webmol.pl |

| **Information Integration (1)** | |
|---|---|
| TIBCO Spotfire | http://spotfire.tibco. com/ |

Specifically, these tools were used to perform: 1) Integrated Data Mining (8 tools); 2) miRNA Mining and Mapping (6 tools); 3) miRNA Target Targets and Expression (21 tools); 4) Integrated miRNA Targets and Expression (13 tools); 5) miRNA Secondary Structure Prediction and Comparison (5 tools); 6) Network Searches and Analyses (8 tools); 7) Molecular Visualization (4 tools); and 8) Information Integration and Exploitation (1 tool).

A single gene target can be controlled by several miRNAs whereas a single miRNA can control several gene targets. Sophisticated bioinformatics resources have been developed to select the most relevant miRNAs to target diseases (Gallagher IJ, et al. Genome medicine. 2010; Fujiki K, et al. BMC Biol. 2009; Okada Y, et al., J Androl. 2010; Hao T, et al., Mol Biosyst. 2012; Hao T, et al., Mol Biosyst. 2012). However, the results of these algorithms are acutely dependent on predefined parameters and the degree of convergence between these algorithms is rather limited. Therefore, there is a need to develop better performing bioinformatics tools with improved sensitivity, specificity and selectivity for the identification of miRNA/target relationships.

The interactions between miRNAs and their targets go beyond the original description of miRNAs as post-transcriptional regulators whose seed region of the driver strand (5' bases 2-7) bind to complementary sequences in the 3' UTR region of target mRNAs, usually resulting in translational repression or target degradation and gene silencing. The interactions can also involve various regions of the driver or passenger strands of the miRNAs as well as the 5'UTR, promoter, and coding regions of the mRNAs.

Upon analysis of the available data, it was decided to favor pathway-specific miRNAs which target multiple genes within one discrete signaling pathway, rather than universal miRNAs which are involved in many signaling pathways, functions or processes. Using 34 publicly available Internet tools predicting miRNA targets, specific human miRNAs were searched for that could potentially modulate several targets among the 83 thermogenic regulator molecules (which include 36 Transcription Factors) selected in Example 1.

Several paradigms were considered:

### A) A one microRNA-multiple mRNAs pathway-specific paradigm.

### A1. First example of one microRNA-multiple mRNAs pathway-specific paradigm

The methylation state of histones can be dynamically regulated by histone methyltransferases and demethylases. The human lysine (K)-specific demethylase 3A (KDM3A) is critically important in regulating the expression of metabolic genes. Its loss of function results in obesity and hyperlipidemia in mice. Beta-adrenergic stimulation of KDM3A binding to
the PPAR responsive element (PPRE) of the UCP1 gene not only decreases levels of H3K9me2 (dimethylation of lysine 9 of histone H3) at the PPRE, but also facilitates the recruitment of PPARG and RXRA and their co-activators PPARGC1A, CREBBP and NCOA1 to the PPRE. The interrogation of the TargetScan Human database (release 6.0) revealed that the human KDM3A 3' UTR 29-35 region is a conserved target for hsa-miR-22. Several other miRNA Targets Databases also confirmed this match between hsa-miR-22 and KDM3A. Therefore, increased production of the demethylase KDM3A by an hsa-miR-22 antagomir should lead to demethylation of the UCP1 gene promoter region, thus facilitating binding of several regulatory elements and increased UCP1 production.

In addition, we used the 34 miRNA Targets and Expression tools (Table 4) to identify the mRNA targets of a given miRNA.

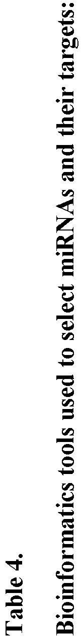

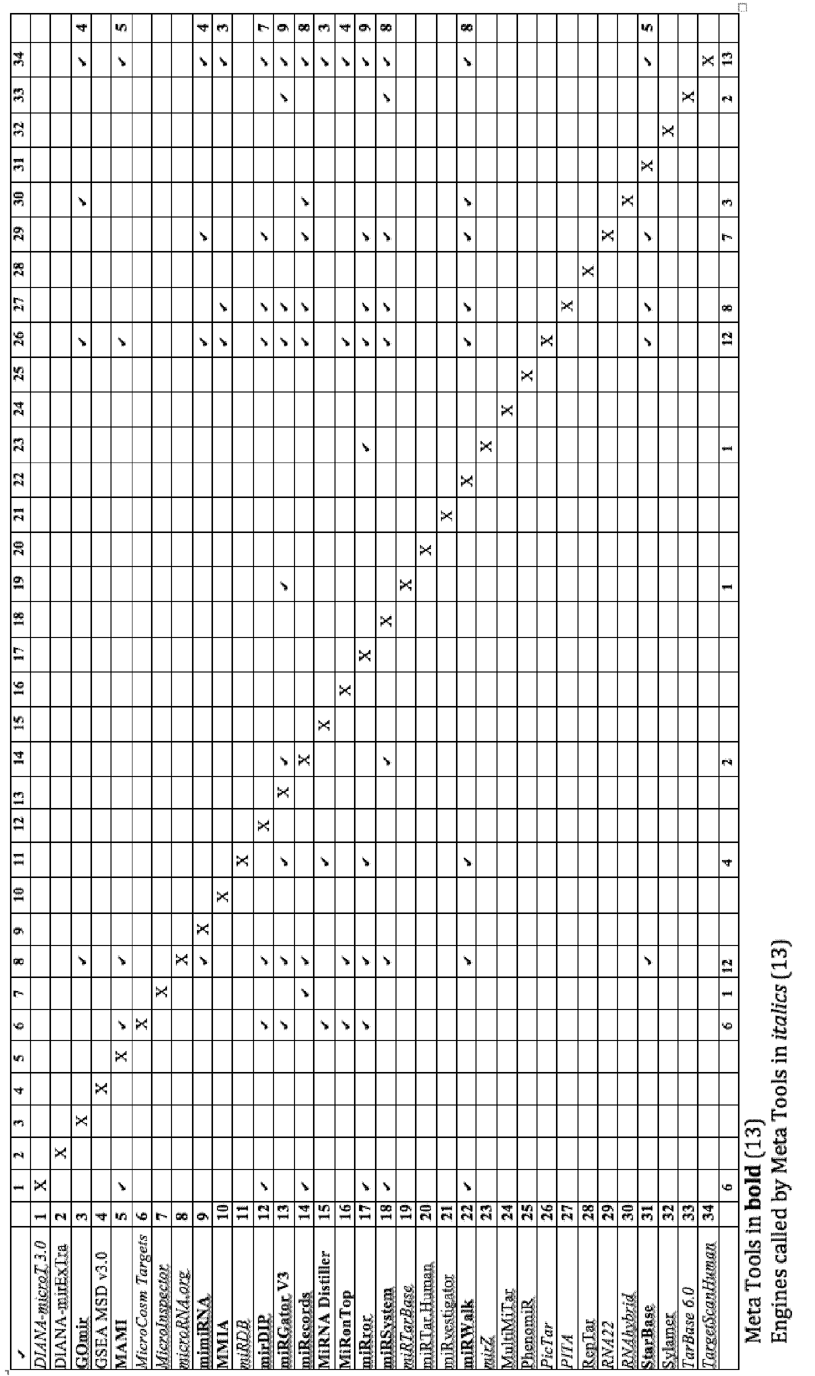

Applying the above in silico strategy, it was discovered that hsa-miR-22-3p and hsa-miR-22-5p interact respectively with a total of 42 and 8 of the chosen 83 thermogenic targets. This data is set forth in Table 5.

**Table 5.**

| **Thermogenic regulators identified as predicted and/or validated targets for hsa-miR-22:** | | | | | | | | | |
|---|---|---|---|---|---|---|---|---|---|
| **hsa-miR-22-3p** | | | | | | | | | |
| ALDH1A1 | DIO2 | | NCOA1 | | | PRKAA1 | | STAT5A | |
| BMP4 | FGF19 | | NPPA | | | PRKACA | | TNFRSF1A | |
| BMP7 | FGF21 | | NRF1 | | | PRKACB | | TRPM8 | |
| CEBPA | FOXC2 | | NRIP1 | | | PRKAR1A | | UCP2 | |
| CEBPD | INSR | | PPARA | | | RUNX1T1 | | WNT10B | |
| CIDEC | KDM3A | | PPARGC1A | | | RUNX2 | | WNT5A | |
| CREB1 | KLF11 | | PPARGC1B | | | SIRT1 | | | |
| CREBBP | LRP6 | | PRDM16 | | | SREBF1 | | | |
| CTNNB1 | MAPK14 | | PRDX3 | | | SREBF2 | | | |

| **hsa-miR-22-5p** | | | | | | | | | |
|---|---|---|---|---|---|---|---|---|---|
| BMP7 | DIO2 | FNDC5 | IKBKE | INSR | MAP | | NR1H3 | PPARA | |

### A2. Other examples of one microRNA-multiple mRNAs pathway-specific paradigm

We also utilized the 34 miRNA Targets and Expression tools (Table 4) to look for potential relations between any of the adipocyte 536 miRNAs (Table 1) and the 83 thermogenic targets (Table 2).

It appears that many adipocyte miRNAs interact (prediction and/or validation) with at least one of the 83 thermogenic targets. For example, miR-17-3p and hsa-miR-17-5p interact respectively with a total of 23 and 65 of the chosen thermogenic 83 targets. This data is set forth in Table 6.

**Table 6.**

| **Thermogenic regulators identified as predicted and/or validated targets for hsa-miR-17:** | | | | |
|---|---|---|---|---|
| **hsa-miR-17-3p** | | | | |
| ATG7 | CTBP2 | KLF11 | PPARD | TNFRSF1A |
| BMP2 | E2F4 | MAPK14 | PRDM16 | TWIST1 |
| BMP4 | FGF19 | NCOA3 | RB1 | WNT10B |
| CEBPB | IKBKE | PLAC8 | RUNX1T1 | |
| CREB1 | IRS1 | PPARA | STAT5A | |

| **hsa-miR-17-5p** | | | | |
|---|---|---|---|---|
| ALDH1A1 | CREB2 | IKBKE | NRIP1 | RB1 |
| ATG7 | CTNNB1 | INSR | PLAC8 | RPS6KB1 |
| BMP2 | CTBP1 | IRS1 | PPARA | RUNX1T1 |
| BMP4 | CTBP2 | KLF11 | PPARD | RUNX2 |
| BMP7 | DIO2 | MAPK14 | PPARG | SIRT1 |
| BMP8b | ELOVL3 | MED13 | PPARGC1A | SIRT3 |
| CEBPA | FGF19 | NCOA1 | PPARGC1B | SREBF1 |
| CEBPB | FGF21 | NCOA2 | PRDX3 | STAT5A |
| CEBPD | FNDC5 | NCOA3 | PRKAA1 | TNFRSF1A |
| CIDEC | FOXC2 | NPPA | PRKAA2 | TWIST1 |
| COX7A1 | GDF3 | NR1H3 | PRKACA | UCP1 |
| CRAT | HCRT | NR4A3 | PRKACB | UCP3 |
| CREB1 | HOXC8 | NRF1 | PRKAR1A | WNT5A |

Once the lists of miRNAs of interest and their mRNA targets were produced, the following filters were applied to refine the results:
Parameters
1 Expression of miRNAs in tissue/cell of interest
2 Number of algorithms predicting one miRNA for a given gene or set of genes
3 Score/percent from algorithms
4 Number of preferred genes targeted by one miRNA
5 Number of binding sites in a target gene for one miRNA
6 Number of binding sites in a target gene for several miRNAs
7 Over-representation of one miRNA seed complementary sequence among target genes (miRvestigator)
8 Validated miRNA-mRNA target couples
9 Genomic location of miRNA binding site (5'UTR-Promoter-CDS-3'UTR)
10 Intronic location of miRNA
11 Clustering of miRNAs
12 Abundance of miRNA in specific tissue/cell of interest

Applying the above parameters, it was discovered that 229 miRNAs met at least two of these criteria. This data is set forth in Table 7.

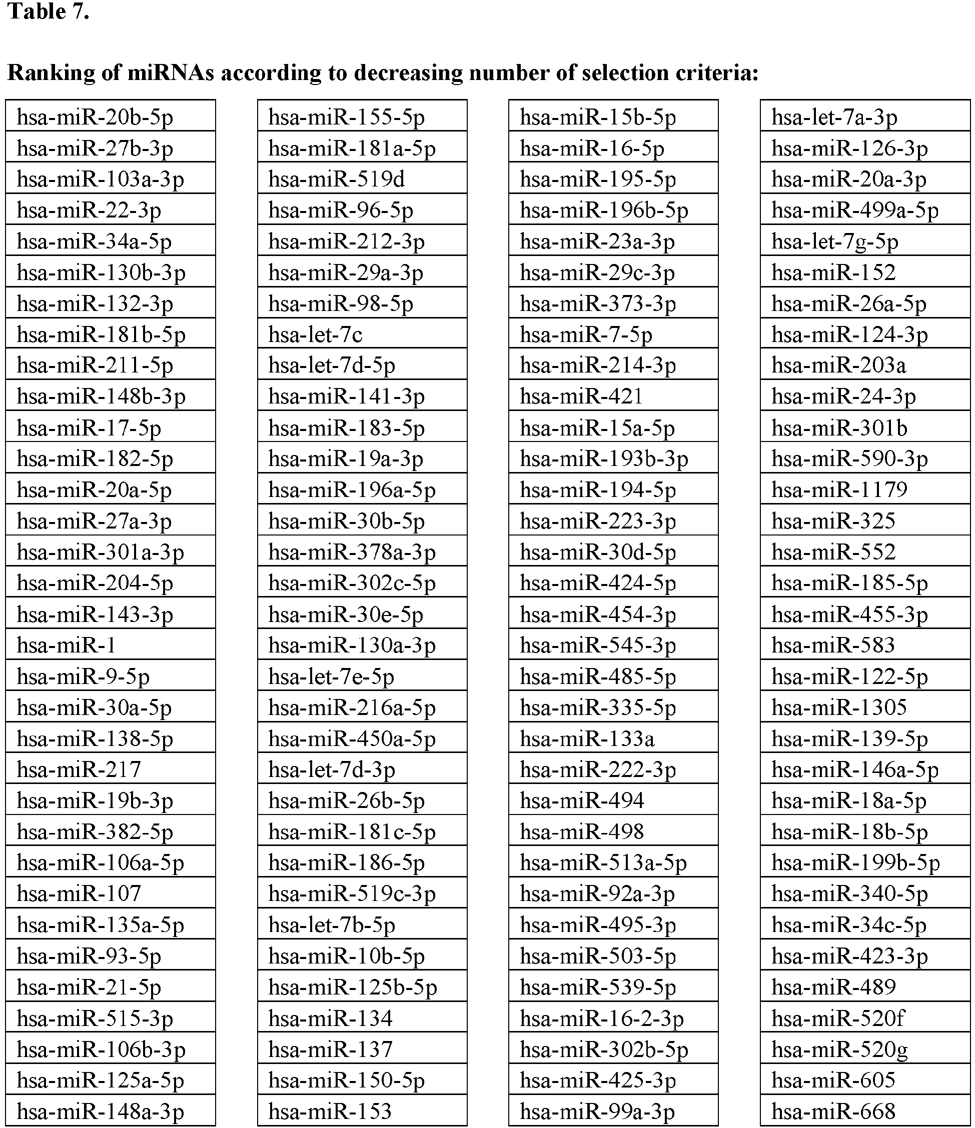

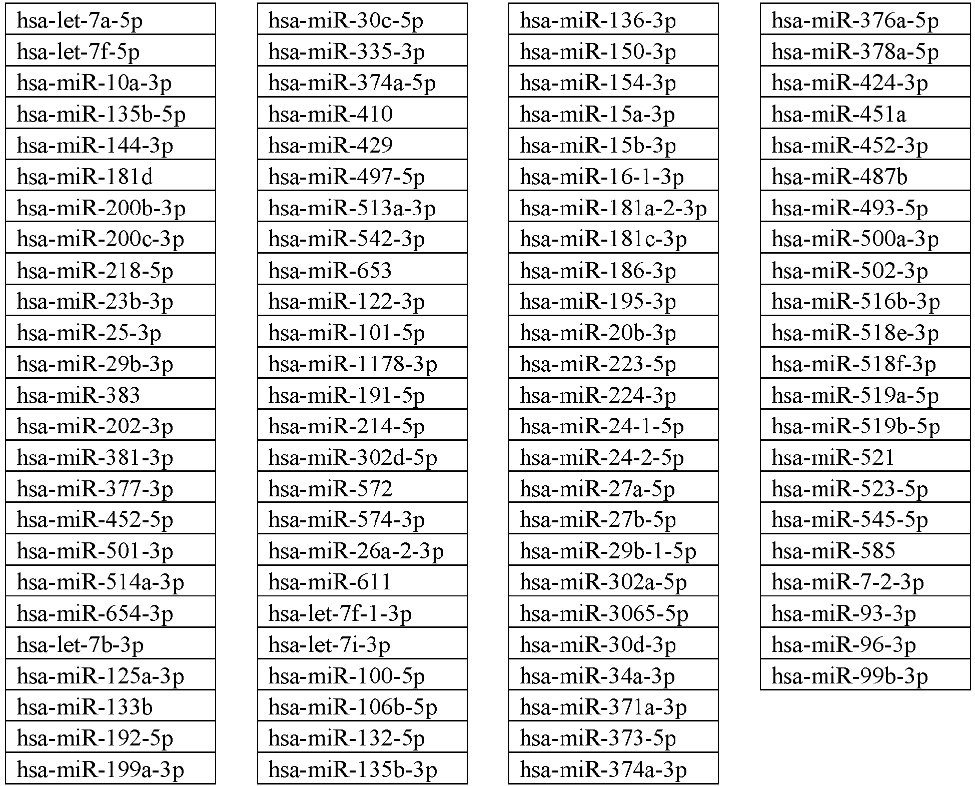

### B) A multiple microRNAs-one mRNA paradigm.

### B1. One exemplary multiple miRNAs-one mRNA paradigm involves UCP1.

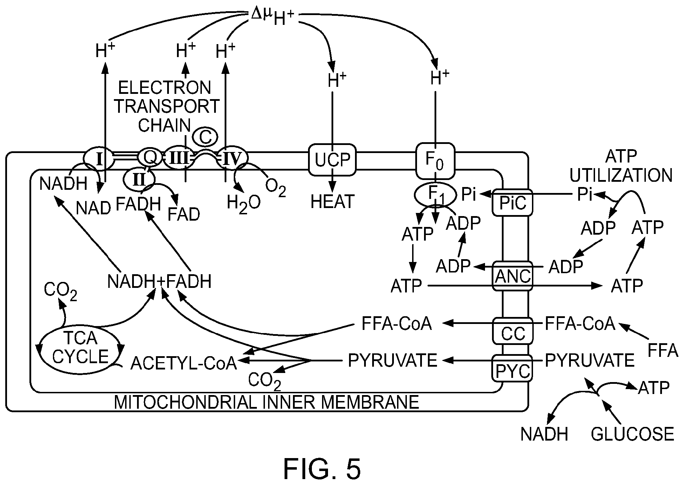

In adipocytes the key thermogenic regulator ultimately is UCP1 (also named thermogenin) and, thus, all thermogenic regulators must ultimately impact UCP1 activity. UCP1 is a mitochondrial transporter protein that creates proton leaks across the inner mitochondrial membrane, thus uncoupling oxidative phosphorylation from ATP synthesis. As a result, energy is dissipated in the form of heat (adaptive thermogenesis) (see Figure 5) Lowell et al., Nature (2000); Friedman et al., Bioinformatics (2010); Hsu et al., Nucleic acids research (2011); Rieger et al., Frontiers in Genetics (2011)).

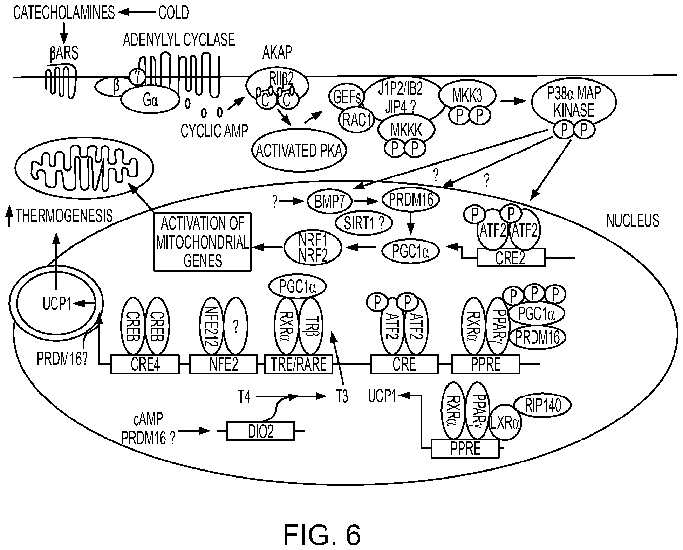

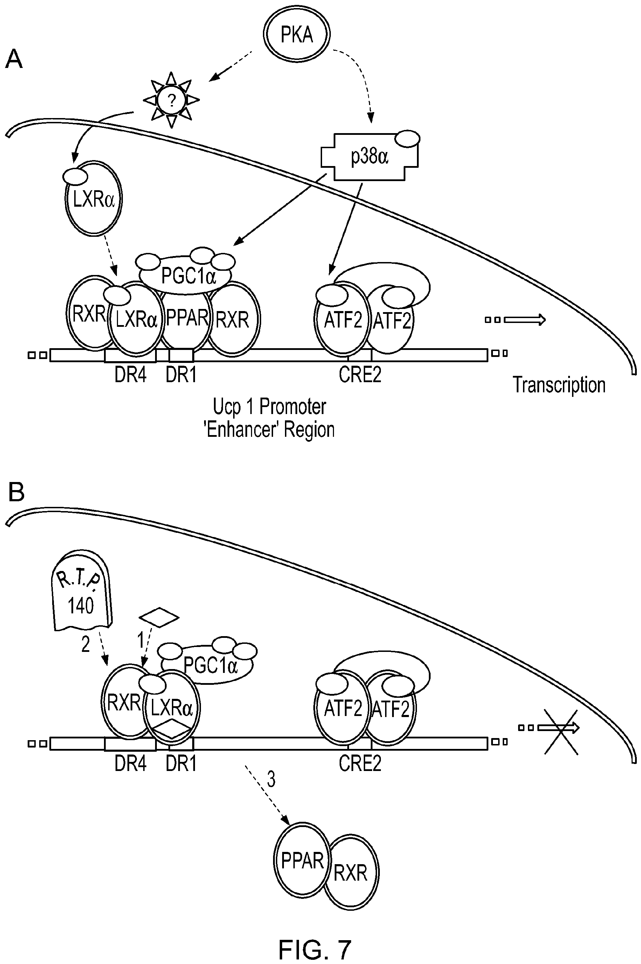

UCP1 biosynthesis is mainly controlled at the transcription level. Figure 6 depicts the transcriptional control of UCP1 by other exemplary thermogenic regulators. The promoter's region of the UCP1 gene contains many distinct regulatory sites, allowing a wide range of proteins to influence its transcription, both positively (see Figure 7a) and negatively (see Figure 7b).

Mendelian randomization is a method of using measured variation in genes of known function to examine the causal effect of a modifiable exposure on disease in non-experimental studies. Mendelian randomization can be thought of as a "natural" Randomized Clinical Trial.

Genetic polymorphism of the UCP1 gene, such as the -3826 A/G single nucleotide polymorphism in the promoter in exon 2 of UCP1, has been reported to be associated with reduced mRNA expression and obesity. Healthy children with the G/G genotype had a lower capacity for thermogenesis in response to a high-fat meal and acute cold exposure. The same - 3826 A/G UCP1 genetic polymorphism diminishes resting energy expenditure and thermoregulatory sympathetic nervous system activity in young females. In a study of 367 Korean women, the G allele of -3826A>G and the C allele of -412A>C were significantly associated with larger areas of abdominal subcutaneous fat in a dominant model (p<0.001 and p<0.0004, respectively); combining them together (ht2[GC]) enhanced this significance (p<0.00005). A study of 100 severe obese adults (BMI > 40 kg/m2) and 100 normal-weight control subjects (BMI range = 19-24.9 kg/m2) identified 7 variations in the promoter region, 4 in the intronic region and 4 in the exonic region of the UCP1 gene. These variations could contribute to the development of obesity, particularly, g.-451C>T, g.940G>A, and g.IVS4-208T>G could represent "thrifty" factors that promote energy storage. Finally, two polymorphisms (A-3826G and C-3740A), located in the upstream promoter region of the UCP1 gene affect gene expression and are correlated with human longevity.

All aforementioned information supports targeting UCP1 expression and activity as a meaningful way to alter adaptive thermogenesis and consequently treat human obesity. Many strategies could be implemented to achieve this goal, however, the one employed in the methods of the invention uses miRNA agents to modulate simultaneously several elements within the thermogenic pathways to increase UCP1 synthesis and activity. Both direct and indirect interactions between miRNAs and the UCP1 gene are considered. Direct interaction means the direct binding of miRNAs to the various regions of the UCP1 gene, resulting in alterations of the transcription, translation, stability and/or degradation of the UCP1 mRNA. Indirect interaction means that miRNAs alter the transcription, translation, stability and/or degradation of thermogenic mRNAs, whose expressed proteins alter the transcription of the UCP1 gene. Furthermore, indirect interaction means that miRNAs alter the transcription, translation, stability and/or degradation of other miRNAs that modify the transcription of the UCP1 gene.

The promoter region of the human UCP1 gene (gi|237858805|ref|NG_012139.1| Homo sapiens uncoupling protein 1 (mitochondrial, proton carrier) (UCP1), RefSeqGene on chromosome 4) is particularly rich is regulatory element motifs (Table 8).

**Table 8.**

| **UCP1 Gene Regulatory Elements:** | | | | | |
|---|---|---|---|---|---|
| | **Name of regulatory element** | **Sequence** | **SEQ ID NO:** | **Number** | **Nucleotide Location** |
| 1 | **BRE1** (Brown Fat Response Element 1) | CCTCTCTGCTTCTTCT | 1 | 1 | 1,129 to 1,144 |
| 2 | **BRE2** (Brown Fat Response Element 2) | CTCCTTGGAA | 2 | 1 | 1,269 to 1,278 |
| 3 **CRE2** | | ATTCTTTA | 3 | 4 | 1,121 to 1,128; |
| | | | | | 3,631 to 3,638; |
| | | | | | 10,982 to 10,989; |
| | | | | | 15,881 to 15,888 |
| 4 | **CREB** | ACGTCA | 4 | 5 | 1,082 to 1,087; |
| | | | | | 1,345 to 1,350; |
| | | | | | 1,348 to 1,343; |
| | | | | | 11,439 to 11,434; |
| | | | | | 13,831 to 13,836 |
| 5 | **DR1** | TTGCCCTTGCTCA | 5 | 1 | 1,099 to 1,111 |
| 6 | DR4 | ACGTCATAAAGGGTCA | 6 | 1 | 1,082 to 1,097 |
| 7 | **DR4 Type RARE** | RGKTCANNNNRGKTCA | 7 | 1 | 1,316 to 1,301 |
| 8 | **ERE** | GCTCATACTGACCT | 8 | 1 | 1,107 to 1,120 |
| 9 | **PRE** | GTTAATGTGTTCT | 9 | 1 | 1,009 to 1,021 |
| 10 | **RARE** | TGACCACAGTTTGATCA | 10 | 1 | 983 -> 999 |
| 11 | **RXR** | AGGTCA | 11 | 12 | 1,120 to 1,115; |
| | | | | | 1,316 to 1,311; |
| | | | | | 3,517 to 3,522; |
| | | | | | 3,560 to 3,555; |
| | | | | | 3,813 to 3,808; |
| | | | | | 5,318 to 5,313; |
| | | | | | 6,233 to 6,238; |
| | | | | | 6,831 to 6,836; |
| | | | | | 8,122 to 8,127; |
| | | | | | 9,966 to 9,971; |
| | | | | | 11,339 to 11,334; |
| | | | | | 11,412 to 11,407 |
| 12 | **GC Box 1** | CGCCC | 12 | 7 | 4,593 to 4,589; |
| | | | | | 4,615 to 4,619; |
| | | | | | 4,615 to 4,619; |
| | | | | | 4,747 to 4,751; |
| | | | | | 4,765 to 4,769; |
| | | | | | 5,914 to 5,910; |
| | | | | | 13,715 to 13,711 |
| 13 | **GC Box 2** | GCGGG | 13 | 9 | 4,463 to 4,459; |
| | | | | | 4,585 to 4,589; |
| | | | | | 4,593 to 4,597; |
| | | | | | 4,639 to 4,643; |
| | | | | | 4,883 to 4,887; |
| | | | | | 5,176 to 5,172; |
| | | | | | 5,929 to 5,933; |
| | | | | | 5,940 to 5,944; |
| | | | | | 14,994 to 14,990 |
| 14 | **GT Box 1** | CACCC | 14 | 25 | 194 to 190; |
| | | | | | 452 to 448; |
| | | | | | 1,184 to 1,188; |
| | | | | | 1,803 to 1,807; |
| | | | | | 2,428 to 2,424; |
| | | | | | 3,037 to 3,041; |
| | | | | | 3,330 to 3,334; |
| | | | | | 4,137 to 4,141; |
| | | | | | 4,566 to 4,562; |
| | | | | | 4,599 to 4,595; |
| | | | | | 4,869 to 4,865; |
| | | | | | 5,104 to 5,108; |
| | | | | | 5,461 to 5,457; |
| | | | | | 6,237 to 6,241; |
| | | | | | 6,293 to 6,289; |
| | | | | | 8,096 to 8,092; |
| | | | | | 8,198 to 8,194; |
| | | | | | 9,649 to 9,645; |
| | | | | | 9,912 to 9,908; |
| | | | | | 12,962 to 12,958; |
| | | | | | 13,136 to 13,132; |
| | | | | | 13,723 to 13,719; |
| | | | | | 14,404 to 14,400; |
| | | | | | 14,960 to 14,964; |
| | | | | | 15,576 to 15,572 |
| 15 | **GT Box 2** | GTGGG | 15 | 20 | 25 to 21; |
| | | | | | 1,805 to 1,801; |
| | | | | | 1,809 to 1,805; |
| | | | | | 2,119 to 2,123; |
| | | | | | 3,854 to 3,850; |
| | | | | | 4,310 to 4,314; |
| | | | | | 4,339 to 4,343; |
| | | | | | 4,765 to 4,761; |
| | | | | | 4,867 to 4,871; |
| | | | | | 6,291 to 6,295; |
| | | | | | 7,554 to 7,558; |
| | | | | | 8,280 to 8,284; |
| | | | | | 8,681 to 8,685; |
| | | | | | 9,615 to 9,619; |
| | | | | | 9,689 to 9,693; |
| | | | | | 9,906 to 9,910; |
| | | | | | 10,363 to 10,359; |
| | | | | | 13,074 to 13,070; |
| | | | | | 13,640 to 13,644; |
| | | | | | 13,941 to 13,945 |
| 16 | **CpG Methylation Island** | CG | 16 | 366 | 4,519 to 5,258; |
| | | | | | 5,639 to 6,694 |

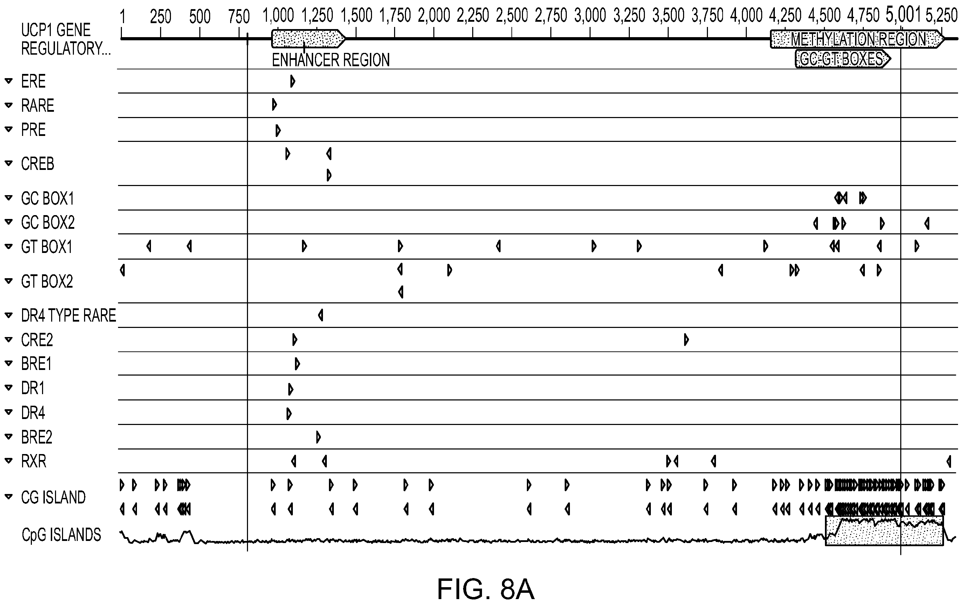

Figure 8A depicts the location of these various regulatory elements in reference to the UCP1 transcription start site at nucleotide position 5,001 of the 15,910 base pair human UCP-1 gene (FASTA accession number: >gi|237858805|ref|NG_012139.1| Homo sapiens uncoupling protein 1 (mitochondrial, proton carrier) (UCP1), RefSeqGene on chromosome 4; NCBI Reference Sequence: NG_012139.1).

Direct or indirect activation or repression of these regulatory elements by miRNAs will result in alterations of UCP1 gene expression and activity. Under normal conditions, the UCP1 gene expression and activity are repressed by a rich network of regulatory elements, in order to avoid energy wasting. Under stress, such as exposure to a cold environment, the expression of the UCP1 gene is upregulated, via various activators and repre1ssors which are under the control of several miRNAs.

An initial survey of miRNAs targeting the human UCP1 3'UTR with several programs, including microRNA.org, was negative. However, other programs, including MicroCosm Targets, using the UCP1 Ensembl 1,462 base pair transcript ENST00000262999 as a target revealed binding sites for 27 miRNAs at 28 locations in UCP1 3'UTR as shown in Table 9.

**Table 9.**

| **Binding sites for miRNAs in the 3'UTR of UCP1 (NCBI Reference Sequence NG_012139.1) determined using microCosm Targets:** | | | | | |
|---|---|---|---|---|---|
| **Name** | **Sequence** | **SEQ ID NO:** | **From bp** | **To bp** | **Length** |
| hsa-miR-21 | AATGTAATGCAGATAAGCTA | 17 | 14143 | 14162 | 20 |
| hsa-miR-219-2-3p | ACATGTTTTAATTACAATTC | 18 | 14217 | 14236 | 20 |
| hsa-miR-22 | GATTGGCAGCTT | 19 | 14857 | 14868 | 12 |
| hsa-miR-222a | GATTTTTAATGTTTAGAGTCCAG | 20 | 14500 | 14522 | 23 |
| hsa-miR-290-3p | TTTAGAGCTGGAGGGTACTT | 21 | 14621 | 14640 | 20 |
| hsa-miR-292-3p | TTTAGAGCTGGAGGGTACTT | 22 | 14621 | 14640 | 20 |
| hsa-miR-292-5p | GACAGAGGAACAGTTTGAG | 23 | 14648 | 14666 | 19 |
| hsa-miR-325 | ATTTTGGCAGGATTGCTACTAG | 24 | 14568 | 14589 | 22 |
| hsa-miR-331-5p | TTTTGAGATCTATACCTGG | 25 | 14383 | 14401 | 19 |
| hsa-miR-362-5p | ATTTTAAGCTAAATCCAAGGATT | 26 | 14838 | 14860 | 23 |
| hsa-miR-367 | TGACCATTTCTGGAGTGCAATT | 27 | 14170 | 14191 | 22 |
| hsa-miR-371-5p | ACAGTTTGAT | 28 | 988 | 997 | 10 |
| hsa-miR-371-5p | ACAGTTTGAG | 29 | 14657 | 14666 | 10 |
| hsa-miR-377 | CTGGAGTGCAATTGTGTGA | 30 | 14179 | 14197 | 19 |
| hsa-miR-378 | TTTTAATGTTTAGAGTCCAG | 31 | 14503 | 14522 | 20 |
| hsa-miR-382 | TGATGACATCTCTAACAACTTC | 32 | 14526 | 14547 | 22 |
| hsa-miR-460 | AGAAACTGAGTGAAATGCAG | 33 | 14250 | 14269 | 20 |
| hsa-miR-508-5p | TGACCATTTCTGGAGTG | 34 | 14170 | 14186 | 17 |
| hsa-miR-543 | TACTCTGAATGTT | 35 | 14478 | 14490 | 13 |
| hsa-miR-549 | TTAACCACAGTTGTCA | 36 | 14321 | 14336 | 16 |
| hsa-miR-643 | CAAGTTCACTAGAATACAAG | 37 | 14412 | 14431 | 20 |
| hsa-miR-654-3p | AAGGTTACAGGCTGCCAGACAT | 38 | 14880 | 14901 | 22 |
| hsa-miR-664 | GTGTGAATGAATG | 39 | 14192 | 14204 | 13 |
| hsa-miR-871 | TAGGCATGAACCTACTCTGAATG | 40 | 14466 | 14488 | 23 |
| hsa-miR -883a-3p | AAACTGAGTGAAATGCAGTT | 41 | 14252 | 14271 | 20 |
| hsa-miR -883b-3p | AAACTGAGTGAAATGCAGTT | 42 | 14252 | 14271 | 20 |
| hsa-miR-888-3p | TTTATTAACCACAGTTGTCAGTT | 43 | 14317 | 14339 | 23 |
| hsa-miR-92b | GAGTGCAAT | 44 | 14182 | 14190 | 9 |

Other programs, such as miRWalk, miRGen, miRGator-miRanda, and DIANA microT, using the UCP1 Ensembl 1,462 base pair transcript (ENST00000262999), the UCP1 Ensembl 9,371 base pair gene sequence (ENSG00000109424) or the 15,910 base pair UCP1 sequence (NCBI Reference Sequence: NG_012139.1) as targets, revealed binding sites for a total of 50 miRNAs at 69 locations in UCP1 3'UTR as shown in Table 10.

**Table 10.**

| **Binding sites for miRNAs in the 3'UTR of UCP1 (NCBI Reference Sequence. NG_12139.1) according to several programs:** | | | | | | |
|---|---|---|---|---|---|---|
| | **Name** | **Sequence** | **SEQ ID NO:** | **From bp** | **To bp** | **Length** |
| 1 | hsa-miR-1179 | AAGTATCCTTT | 45 | 15346 | 15356 | 11 |
| 2 | hsa-miR-1302 | ATGGGACACA | 46 | 15021 | 15030 | 10 |
| 3 | hsa-miR-130b | TTATTTTCCCT | 47 | 15161 | 15171 | 11 |
| 4 | hsa-miR-146a | TGACAACTGT | 48 | 14327 | 14336 | 10 |
| | hsa-miR-146a | AGGGAACTGA | 49 | 15231 | 15240 | 10 |
| | hsa-miR-146a | TGTGAACTGG | 50 | 15679 | 15688 | 10 |
| 5 | hsa-miR-181c | AACCATAGT | 51 | 15304 | 15312 | 9 |
| 6 | hsa-miR-19b-2 | ACTTTTGCGG | 52 | 14991 | 15000 | 10 |
| 7 | hsa-miR-203 | TTAAATGTT | 53 | 15584 | 15592 | 9 |
| 8 | hsa-miR-204-5p | TTCCTTTATC | 54 | 14006 | 14015 | 10 |
| | hsa-miR-204-5p | TTCCTCTGTC | 55 | 14648 | 14657 | 10 |
| 9 | hsa-miR-21-5p | TAGCTTATCT | 56 | 14153 | 14162 | 10 |
| 10 | hsa-miR-211-5p | TTCCCTATCTC | 57 | 14779 | 14789 | 11 |
| 11 | hsa-miR-214 | CAGCAAGCA | 58 | 15052 | 15060 | 9 |
| 12 | hsa-miR-22-3p | AAGCTGCCAA | 59 | 14859 | 14868 | 10 |
| | hsa-miR-22-5p | AGTTCTTCACA | 60 | 14203 | 14213 | 11 |
| 13 | hsa-miR-26a-2-3p | CATTTTCTTG | 61 | 13918 | 13927 | 10 |
| | hsa-miR-26a-2-3p | CCAATCCTTG | 62 | 14853 | 14862 | 10 |
| | hsa-miR-26a-2-3p | CCTTTTCATG | 63 | 15616 | 15625 | 10 |
| 14 | hsa-miR-30b | GTAACCTTCC | 64 | 14878 | 14887 | 10 |
| 15 | hsa-miR-325 | CAGAGTAGGT | 65 | 14475 | 14484 | 10 |
| | hsa-miR-325 | CCTTGTAGGC | 66 | 15378 | 15387 | 10 |
| 16 | hsa-miR-328 | CTGTTCCTCT | 67 | 14651 | 14660 | 10 |
| 17 | hsa-miR-362-5p | ATCCTTGGAT | 68 | 14850 | 14859 | 10 |
| 18 | hsa-miR-367-3p | AATTGCACTC | 69 | 14182 | 14191 | 10 |
| 19 | hsa-miR-371a-3p | AAGTGCCTGC | 70 | 15435 | 15444 | 10 |
| | hsa-miR-371a-5p | TCTCAAACTG | 71 | 14658 | 14667 | 10 |
| 20 | hsa-miR-378a-3p | ACTGGCCTTG | 72 | 15816 | 15825 | 10 |
| 21 | hsa-miR-382-3p | ATTCATTCAC | 73 | 14194 | 14203 | 10 |
| 22 | hsa-miR-382-5p | GAAGTTGTTAGAGAT | 74 | 14533 | 14547 | 15 |
| 23 | hsa-miR-383 | AGATTAGAA | 75 | 14545 | 14553 | 9 |
| 24 | hsa-miR-421 | ATTAACTGAC | 76 | 14333 | 14342 | 10 |
| | hsa-miR-421 | CTCAAAAGAC | 77 | 14380 | 14389 | 10 |
| 25 | hsa-miR-422a | ACTGGCCTT | 78 | 15817 | 15825 | 9 |
| 26 | hsa-miR-431 | TGTCTGGCA | 79 | 14892 | 14900 | 9 |
| 27 | hsa-miR-452 | TTATCTGC | 80 | 14151 | 14158 | 8 |
| | hsa-miR-452 | TCTTCTGC | 81 | 14773 | 14780 | 8 |
| | hsa-miR-452 | ACATCTGC | 82 | 15009 | 15016 | 8 |
| 28 | hsa-miR-455-3p | CAGTCCAT | 83 | 13893 | 13900 | 8 |
| | hsa-miR-455-5p | TGTGTGCCTT | 84 | 15641 | 15650 | 10 |
| 29 | hsa-miR-491-5p | AATGGGGAAG | 85 | 14975 | 14984 | 10 |
| 30 | hsa-miR-501-3p | ATGCATCAGG | 86 | 15547 | 15556 | 10 |
| 31 | hsa-miR-504 | AGACCCTGT | 87 | 15325 | 15333 | 9 |
| 32 | hsa-miR-508-5p | TATTCTAGTGAACTTG ACTCTTA | 88 | 14405 | 14427 | 23 |
| 33 | hsa-miR-512-5p | CACTCAG | 89 | 14255 | 14261 | 7 |
| 34 | hsa-miR-514a-3p | TTGACTCTT | 90 | 14406 | 14414 | 9 |
| 35 | hsa-miR-515-3p | GACTGCCTT | 91 | 15539 | 15547 | 9 |
| | hsa-miR-515-3p | GTGTGCCTT | 92 | 15641 | 15649 | 9 |
| 36 | hsa-miR-517a-3p | ATGGTGCATT | 93 | 15650 | 15659 | 10 |
| 37 | hsa-miR-545 | CAGCAAGCACT | 94 | 15050 | 15060 | 11 |
| 38 | hsa-miR-549 | TGACAACTGT | 95 | 14327 | 14336 | 10 |
| 39 | hsa-miR-552 | CACAGGTGA | 96 | 15130 | 15138 | 9 |
| 40 | hsa-miR-616-5p | ACTCTAAAC | 97 | 14510 | 14518 | 9 |
| 41 | hsa-miR-620 | ATGAATATAG | 98 | 14560 | 14569 | 10 |
| 42 | hsa-miR-643 | ACTGGTATGT | 99 | 13933 | 13942 | 10 |
| | hsa-miR-643 | TCTTGTATTC | 100 | 14423 | 14432 | 10 |
| | hsa-miR-643 | CCTTGTAGGC | 101 | 15378 | 15387 | 10 |
| | hsa-miR-643 | ACATGCATGC | 102 | 15553 | 15562 | 10 |
| 43 | hsa-miR-651 | TTAAAATAAG | 103 | 13988 | 13997 | 10 |
| | hsa-miR-651 | TTAGGTTAAA | 104 | 13993 | 14002 | 10 |
| | hsa-miR-651 | TCATGATAAG | 105 | 15700 | 15709 | 10 |
| 44 | hsa-miR-654-3p | TATCTCTTCT | 106 | 14775 | 14784 | 10 |
| | hsa-miR-654-3p | TATGTATACT | 107 | 15493 | 15502 | 10 |
| 45 | hsa-miR-655 | GTAATACAT | 108 | 15593 | 15601 | 9 |
| 46 | hsa-miR-767-3p | CCTGCTCAT | 109 | 14871 | 14879 | 9 |
| 47 | hsa-miR-888-3p | GACTGACTCC | 110 | 15772 | 15781 | 10 |
| 48 | hsa-miR-92b-3p | ATTGCACTCC | 111 | 14181 | 14190 | 10 |
| 49 | hsa-miR-941 | CACCCAGGT | 112 | 14396 | 14404 | 9 |
| 50 | hsa-miR-99a-3p | AAGCTGGCTC | 113 | 15117 | 15126 | 10 |

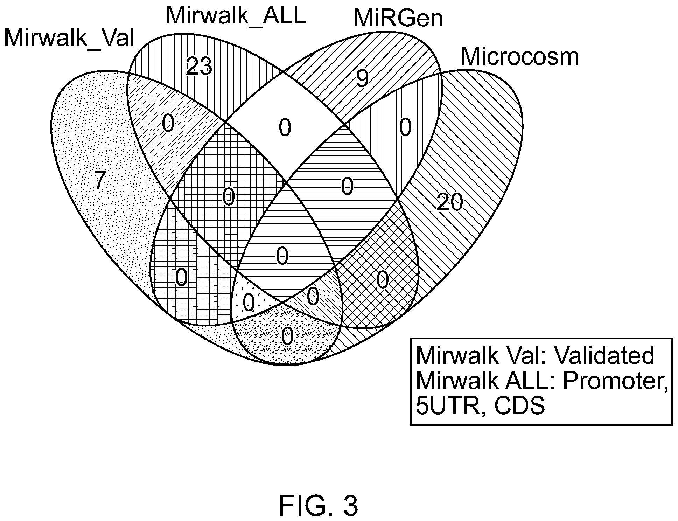

Alignment of the sequence of the human UCP1 gene with several miRNA sequences yielded matches in the 5'UTR, the promoter region and the coding regions of the UCP1 gene. Interrogation of the publicly available Internet tools predicting miRNAs targeting the various regions of the UCP1 gene elicited several hits. Surprisingly, the overlap between these prediction tools was zero, as shown in Figure 3.

Nevertheless, miRNA databases were screened using the alignment program Geneious. A total of 191 human microRNAs were found which have complementary 450 binding sites in the UCP1 gene sequence (Table 11). The length of the matches goes from 7 bases to 12 bases (e.g. hsa-miR-24-2-5p and hsa-miR-192-5p). The number of hits per miRNA varies from 1 to several (e.g. 9 for hsa-miR-19b2 (an abundant adipocyte miRNA), 14 for hsa-miR-26a-2-3p, 11 for hsa-miR-181c, and 12 for hsa-miR-620).

**Table 11.**

| **m iRNAs with predicted binding sites in the UCP1 gene sequence (NCBI Reference Sequence: NG_012139.1):** | | | | | |
|---|---|---|---|---|---|
| **miRNA** | **Sequence** | **SEQ ID NO:** | **From bp** | **To bp** | **Length** |
| hsa-let-7c | TAGAGTTTC | 114 | 5918 | 5926 | 9 |
| hsa-let-7e | GGAGGTAGG | 115 | 13283 | 13291 | 9 |
| hsa-let-7e | TGAAGTAGG | 116 | 7612 | 7620 | 9 |
| hsa-let-7e | AGAGGTAGG | 117 | 3306 | 3314 | 9 |
| hsa-let-7i-3p | CTGTGCAAG | 118 | 3588 | 3596 | 9 |
| hsa-miR-17 | CAAAGTGCT | 119 | 12200 | 12208 | 9 |
| hsa-miR-17 | CAAAGTGCT | 120 | 9931 | 9939 | 9 |
| hsa-miR-17 | CAAAGTGCT | 121 | 218 | 226 | 9 |
| hsa-miR-19a | TGTGCAAAT | 122 | 3916 | 3924 | 9 |
| hsa-miR-19a | TGTGCAAAT | 123 | 834 | 842 | 9 |
| hsa-miR-19b-2 | ACTTTTGCGG | 124 | 14991 | 15000 | 10 |
| hsa-miR-19b-2 | AGTTTTACAA | 125 | 11998 | 12007 | 10 |
| hsa-miR-19b-2 | AGTTTTGTAT | 126 | 10023 | 10032 | 10 |
| hsa-miR-19b-2 | AGTCTTGAAG | 127 | 9399 | 9408 | 10 |
| hsa-miR-19b-2 | AGGTTTGTAG | 128 | 7758 | 7767 | 10 |
| hsa-miR-19b-2 | AGTATTGAAG | 129 | 7159 | 7168 | 10 |
| hsa-miR-19b-2 | AGGCTTGCAG | 130 | 3546 | 3555 | 10 |
| hsa-miR-19b-2 | AATTTGGCAG | 131 | 529 | 538 | 10 |
| hsa-miR-19b-2 | AGTTTTGGAA | 132 | 312 | 321 | 10 |
| hsa-miR-20b | CAAAGTGCT | 133 | 12200 | 12208 | 9 |
| hsa-miR-20b | CAAAGTGCT | 134 | 9931 | 9939 | 9 |
| hsa-miR-20b | CAAAGTGCT | 135 | 218 | 226 | 9 |
| hsa-miR-21-5p | TAGCTTATCT | 136 | 14153 | 14162 | 10 |
| hsa-miR-22-3p | AAGCTGCCAA | 137 | 14859 | 14868 | 10 |
| hsa-miR-22-3p | AAGCTTCCAG | 138 | 1482 | 1491 | 10 |
| hsa-miR-22-5p | AGTTCTTCACA | 139 | 14203 | 14213 | 11 |
| hsa-miR-22-5p | AATTCTTCAGG | 140 | 8032 | 8042 | 11 |
| hsa-miR-22-5p | GGTTCTTCAGC | 141 | 5389 | 5399 | 11 |
| hsa-miR-24-2-5p | TGCCTACTGGCC | 142 | 8651 | 8662 | 12 |
| hsa-miR-25-3p | CATTGCAC | 143 | 11565 | 11572 | 8 |
| hsa-miR-25-5p | AGGCGGAG | 144 | 5963 | 5970 | 8 |
| hsa-miR-26a-2-3p | CCTTTTCATG | 145 | 15616 | 15625 | 10 |
| hsa-miR-26a-2-3p | CCAATCCTTG | 146 | 14853 | 14862 | 10 |
| hsa-miR-26a-2-3p | CATTTTCTTG | 147 | 13918 | 13927 | 10 |
| hsa-miR-26a-2-3p | CCTACTCTTC | 148 | 13505 | 13514 | 10 |
| hsa-miR-26a-2-3p | ACGATTCTTG | 149 | 13192 | 13201 | 10 |
| hsa-miR-26a-2-3p | TCTATTCTTT | 150 | 12883 | 12892 | 10 |
| hsa-miR-26a-2-3p | CATATTTTTG | 151 | 10197 | 10206 | 10 |
| hsa-miR-26a-2-3p | GCTAGTCTTG | 152 | 9978 | 9987 | 10 |
| hsa-miR-26a-2-3p | CATATTTTTG | 153 | 9890 | 9899 | 10 |
| hsa-miR-26a-2-3p | CCTTTTCTTT | 154 | 6631 | 6640 | 10 |
| hsa-miR-26a-2-3p | CCCATTCTCG | 155 | 4709 | 4718 | 10 |
| hsa-miR-26a-2-3p | TTTATTCTTG | 156 | 3893 | 3902 | 10 |
| hsa-miR-26a-2-3p | CCTTTACTTG | 157 | 1885 | 1894 | 10 |
| hsa-miR-26a-2-3p | GCGATTCTTG | 158 | 376 | 385 | 10 |
| hsa-miR-27-5p | AGAGCTTAGG | 159 | 2949 | 2958 | 10 |
| hsa-miR-30b | GTAACCTTCC | 160 | 14878 | 14887 | 10 |
| hsa-miR-30b | GTAACCATCA | 161 | 12991 | 13000 | 10 |
| hsa-miR-30b | GTAATCATAC | 162 | 12831 | 12840 | 10 |
| hsa-miR-30b | GTCAACATCA | 163 | 11401 | 11410 | 10 |
| hsa-miR-30b | GTAAACATAA | 164 | 9365 | 9374 | 10 |
| hsa-miR-30b | GTACTCATCC | 165 | 9016 | 9025 | 10 |
| hsa-miR-30b | CTATACATCC | 166 | 8586 | 8595 | 10 |
| hsa-miR-30b | CTAAACATCT | 167 | 7495 | 7504 | 10 |
| hsa-miR-31 | GGCTATGCC | 168 | 7712 | 7720 | 9 |
| hsa-miR-32 | ATTGCACA | 169 | 11564 | 11571 | 8 |
| hsa-miR-92b | ATTGCACTCC | 170 | 14181 | 14190 | 10 |
| hsa-miR-92b | ATTGCACTAG | 171 | 11282 | 11291 | 10 |
| hsa-miR-93 | CAAAGTGCTG | 172 | 12199 | 12208 | 10 |
| hsa-miR-93 | CAAAGTGCTG | 173 | 217 | 226 | 10 |
| hsa-miR-93-3p | ACTCCTGGGCT | 174 | 12356 | 12366 | 11 |
| hsa-miR-93-3p | ACTGATAAGCT | 175 | 11055 | 11065 | 11 |
| hsa-miR-93-3p | ACTCCTGACCT | 176 | 9966 | 9976 | 11 |
| hsa-miR-96-3p | AATCATGTGCC | 177 | 8659 | 8669 | 11 |
| hsa-miR-99a-3p | AAGCTGGCTC | 178 | 15117 | 15126 | 10 |
| hsa-miR-99a-3p | AAACTCTTTC | 179 | 13344 | 13353 | 10 |
| hsa-miR-99a-3p | AATCTTGTTC | 180 | 11952 | 11961 | 10 |
| hsa-miR-99a-3p | AAGCTCCTTT | 181 | 11050 | 11059 | 10 |
| hsa-miR-99a-3p | AAGCTCCTTT | 182 | 8099 | 8108 | 10 |
| hsa-miR-99a-3p | AAGCTCTGTC | 183 | 7523 | 7532 | 10 |
| hsa-miR-99b-3p | CAACCTCGAG | 184 | 13666 | 13675 | 10 |
| hsa-miR-99b-3p | CGAGCTCCTG | 185 | 13660 | 13669 | 10 |
| hsa-miR-99b-3p | GAAGCTTGTG | 186 | 6436 | 6445 | 10 |
| hsa-miR-99b-3p | CAAACTCCTG | 187 | 257 | 266 | 10 |
| hsa-miR-100 | TCCAGTAGAT | 188 | 11866 | 11875 | 10 |
| hsa-miR-100 | ACGCGCAGAT | 189 | 5634 | 5643 | 10 |
| hsa-miR-106b-5p | CAAAGTGCTG | 190 | 12199 | 12208 | 10 |
| hsa-miR-106b-5p | CAAAGTGCTG | 191 | 217 | 226 | 10 |
| hsa-miR-126-3P | TCATACAGT | 192 | 12828 | 12836 | 9 |
| hsa-miR-126-3P | TTGTACTGT | 193 | 11542 | 11550 | 9 |
| hsa-miR-126-3P | TGGTCCCGT | 194 | 7922 | 7930 | 9 |
| hsa-miR-126-3P | TCATACAGT | 195 | 932 | 940 | 9 |
| hsa-miR-130b | TTATTTTCCCT | 196 | 15161 | 15171 | 11 |
| hsa-miR-130b | CTCTTTTCAGT | 197 | 9670 | 9680 | 11 |
| hsa-miR-130b | CTCTCTTCACT | 198 | 8977 | 8987 | 11 |
| hsa-miR-130b | CTCTTTTTCCC | 199 | 8444 | 8454 | 11 |
| hsa-miR-130b | CTTTTTCCCCT | 200 | 6624 | 6634 | 11 |
| hsa-miR-130b | CTATTTTCCGT | 201 | 5742 | 5752 | 11 |
| hsa-miR-130b | TTCCTTTCCCT | 202 | 5007 | 5017 | 11 |
| hsa-miR-130b | CTCTTTGCCCC | 203 | 1845 | 1855 | 11 |
| hsa-miR-130b | CTCCTTTCCTT | 204 | 1033 | 1043 | 11 |
| hsa-miR-133a-1 | TTTGGTGCCC | 205 | 7393 | 7402 | 10 |
| hsa-miR-140-3p | TACCACAG | 206 | 5893 | 5900 | 8 |
| hsa-miR-141 | TAACACTG | 207 | 5852 | 5859 | 8 |
| hsa-miR-143 | GGTGCAGTG | 208 | 4132 | 4140 | 9 |
| hsa-miR-143-3p | TGAGATGAGG | 209 | 13727 | 13736 | 10 |
| hsa-miR-143-3p | TGAGATGGAG | 210 | 10172 | 10181 | 10 |
| hsa-miR-143-3p | TTAGATGAAG | 211 | 9572 | 9581 | 10 |
| hsa-miR-144-3p | TACAGTATT | 212 | 12825 | 12833 | 9 |
| hsa-miR-144-3p | TACAATATA | 213 | 8859 | 8867 | 9 |
| hsa-miR-144-3p | GACAGTATA | 214 | 1491 | 1499 | 9 |
| hsa-miR-146a | CCTCTGAAA | 215 | 3499 | 3507 | 9 |
| hsa-miR-146a | TGTGAACTGG | 216 | 15679 | 15688 | 10 |
| hsa-miR-146a | AGGGAACTGA | 217 | 15231 | 15240 | 10 |
| hsa-miR-146a | TGACAACTGT | 218 | 14327 | 14336 | 10 |
| hsa-miR-146a | TAAGAACTAA | 219 | 8935 | 8944 | 10 |
| hsa-miR-146a | TTAGAACAGA | 220 | 7908 | 7917 | 10 |
| hsa-miR-146a | TGAGAAGTGC | 221 | 6926 | 6935 | 10 |
| hsa-miR-146a | TGAAAACTTA | 222 | 3883 | 3892 | 10 |
| hsa-miR-146a | ACAGAACTGA | 223 | 2259 | 2268 | 10 |
| hsa-miR-146a | TGAGACCAGA | 224 | 2235 | 2244 | 10 |
| hsa-miR-146a | TGAGAAATAA | 225 | 1614 | 1623 | 10 |
| hsa-miR-147 | TGTGTGGATAA | 226 | 7223 | 7233 | 11 |
| hsa-miR-147 | TTTGTGCAAAT | 227 | 3916 | 3926 | 11 |
| hsa-miR-154 | AATCATACA | 228 | 12830 | 12838 | 9 |
| hsa-miR-154 | AATCATACA | 229 | 934 | 942 | 9 |
| hsa-miR-181c | AACCATAGT | 230 | 15304 | 15312 | 9 |
| hsa-miR-181c | AACCAAAGA | 231 | 13244 | 13252 | 9 |
| hsa-miR-181c | AACCATCAC | 232 | 12990 | 12998 | 9 |
| hsa-miR-181c | ATCCAGCGA | 233 | 11466 | 11474 | 9 |
| hsa-miR-181c | AAACATCTA | 234 | 7494 | 7502 | 9 |
| hsa-miR-181c | AAAAATCGA | 235 | 6201 | 6209 | 9 |
| hsa-miR-181c | AACCCCCGA | 236 | 5540 | 5548 | 9 |
| hsa-miR-181c | AACCCTCTA | 237 | 3614 | 3622 | 9 |
| hsa-miR-181c | AGCCAGCGA | 238 | 3471 | 3479 | 9 |
| hsa-miR-181c | AACCATAGG | 239 | 2801 | 2809 | 9 |
| hsa-miR-181c | AACCATCAC | 240 | 194 | 202 | 9 |
| hsa-miR-185 | TGGAGAGAA | 241 | 2979 | 2987 | 9 |
| hsa-miR-192-5p | CTAACATATGAA | 242 | 114 | 125 | 12 |
| hsa-miR-194-1 | TGTAACAGCA | 243 | 1895 | 1904 | 10 |
| hsa-miR-196a | AGGTAGTTT | 244 | 12139 | 12147 | 9 |
| hsa-miR-199a-5p | CCCTGTGTTC | 245 | 5753 | 5762 | 10 |
| hsa-miR-200a | TAACACTG | 246 | 5852 | 5859 | 8 |
| hsa-miR-200b | TAATAATGCC | 247 | 11184 | 11193 | 10 |
| hsa-miR-200b | GAATACTGCC | 248 | 10340 | 10349 | 10 |
| hsa-miR-200c-3p | TAATACTGT | 249 | 12466 | 12474 | 9 |
| hsa-miR-200c-3p | TAATAATGC | 250 | 11185 | 11193 | 9 |
| hsa-miR-200c-3p | GAATACTGC | 251 | 10341 | 10349 | 9 |
| hsa-miR-200c-3p | TAATACAGC | 252 | 7594 | 7602 | 9 |
| hsa-miR-203 | TTAAATGTT | 253 | 15584 | 15592 | 9 |
| hsa-miR-203 | TGAAATTTT | 254 | 9782 | 9790 | 9 |
| hsa-miR-203 | TGAAAGGTT | 255 | 4495 | 4503 | 9 |
| hsa-miR-204-5p | TTCCTCTGTC | 256 | 14648 | 14657 | 10 |
| hsa-miR-204-5p | TTCCTTTATC | 257 | 14006 | 14015 | 10 |
| hsa-miR-205 | TCCTTCATT | 258 | 10659 | 10667 | 9 |
| hsa-miR-208b | ATAAGAAGA | 259 | 9493 | 9501 | 9 |
| hsa-miR-208b | ATAAGAAGA | 260 | 1770 | 1778 | 9 |
| hsa-miR-211-5p | TTCCCTATCTC | 261 | 14779 | 14789 | 11 |
| hsa-miR-211-5p | TCCCCTCTGTC | 262 | 5238 | 5248 | 11 |
| hsa-miR-211-5p | TTCCCTTGCTC | 263 | 5002 | 5012 | 11 |
| hsa-miR-211-5p | TTCCCATTCTC | 264 | 4710 | 4720 | 11 |
| hsa-miR-214 | CAGCAAGCA | 265 | 15052 | 15060 | 9 |
| hsa-miR-214 | CAGAAGGCA | 266 | 6918 | 6926 | 9 |
| hsa-miR-214 | CCGCAGGCA | 267 | 5935 | 5943 | 9 |
| hsa-miR-214 | CACCAGGCA | 268 | 2087 | 2095 | 9 |
| hsa-miR-218 | TGTGCTTGA | 269 | 10385 | 10393 | 9 |
| hsa-miR-302c | TTTAACATG | 270 | 2932 | 2940 | 9 |
| hsa-miR-324-5p | CGCGTCCCCT | 271 | 4876 | 4885 | 10 |
| hsa-miR-325 | CCTTGTAGGC | 272 | 15378 | 15387 | 10 |
| hsa-miR-325 | CAGAGTAGGT | 273 | 14475 | 14484 | 10 |
| hsa-miR-325 | CCAAGTAGCT | 274 | 10066 | 10075 | 10 |
| hsa-miR-325 | CCAAGTAGCT | 275 | 354 | 363 | 10 |
| hsa-miR-328 | CTGTTCCTCT | 276 | 14651 | 14660 | 10 |
| hsa-miR-328 | CTGGCTCCCT | 277 | 8215 | 8224 | 10 |
| hsa-miR-328 | CTGGCCCTTC | 278 | 8062 | 8071 | 10 |
| hsa-miR-328 | CTGGCACTCA | 279 | 6653 | 6662 | 10 |
| hsa-miR-328 | CTGGCTTTCT | 280 | 6496 | 6505 | 10 |
| hsa-miR-328 | CTGCCCCTCC | 281 | 6048 | 6057 | 10 |
| hsa-miR-328 | CTGGGCCGCT | 282 | 4804 | 4813 | 10 |
| hsa-miR-328 | CTGGAGCTCT | 283 | 4477 | 4486 | 10 |
| hsa-miR-328 | CTGACCCTTT | 284 | 1089 | 1098 | 10 |
| hsa-miR-330 | CAAAGCACAC | 285 | 13845 | 13854 | 10 |
| hsa-miR-330 | CAAAGCACAC | 286 | 11657 | 11666 | 10 |
| hsa-miR-331-5p | CTAGGTGTGG | 287 | 7719 | 7728 | 10 |
| hsa-miR-361-3p | CCCCCAGG | 288 | 5112 | 5119 | 8 |
| hsa-miR-362-5p | ATCCTTGGAT | 289 | 14850 | 14859 | 10 |
| hsa-miR-367-3p | AATTGCACTC | 290 | 14182 | 14191 | 10 |
| hsa-miR-367-3p | AAATGCACTT | 291 | 999 | 1008 | 10 |
| hsa-miR-369 | AATAATACA | 292 | 2266 | 2274 | 9 |
| hsa-miR-371a-3p | AAGTGCCTGC | 293 | 15435 | 15444 | 10 |
| hsa-miR-371a-3p | AAGAGCCGAC | 294 | 11455 | 11464 | 10 |
| hsa-miR-371a-3p | ACGTGCCACC | 295 | 10044 | 10053 | 10 |
| hsa-miR-371a-3p | AAGTGCCTCT | 296 | 7047 | 7056 | 10 |
| hsa-miR-371a-3p | AAGTGCACCC | 297 | 5457 | 5466 | 10 |
| hsa-miR-371a-5p | TCTCAAACTG | 298 | 14658 | 14667 | 10 |
| hsa-miR-372 | AAAGTGCTG | 299 | 12199 | 12207 | 9 |
| hsa-miR-372 | AAAGTGCTG | 300 | 217 | 225 | 9 |
| hsa-miR-374a-3p | TCATCAGATT | 301 | 10606 | 10615 | 10 |
| hsa-miR-377-3p | AGCACACAAA | 302 | 13842 | 13851 | 10 |
| hsa-miR-378a-3p | ACTGGCCTTG | 303 | 15816 | 15825 | 10 |
| hsa-miR-378a-3p | ACTGGTCTTG | 304 | 11837 | 11846 | 10 |
| hsa-miR-378a-5p | CTCCTGCCTC | 305 | 12216 | 12225 | 10 |
| hsa-miR-378a-5p | CTCCTGCCTC | 306 | 10082 | 10091 | 10 |
| hsa-miR-378a-5p | CTCCTGTCTC | 307 | 8207 | 8216 | 10 |
| hsa-miR-378a-5p | CTCCTAACTC | 308 | 7650 | 7659 | 10 |
| hsa-miR-382-3p | ATTCATTCAC | 309 | 14194 | 14203 | 10 |
| hsa-miR-383 | AGATTAGAA | 310 | 14545 | 14553 | 9 |
| hsa-miR-383 | AGATTAGAA | 311 | 7912 | 7920 | 9 |
| hsa-miR-383 | AGAACAGAA | 312 | 5801 | 5809 | 9 |
| hsa-miR-412 | ACTTCACCT | 313 | 737 | 745 | 9 |
| hsa-miR-421 | CTCAAAAGAC | 314 | 14380 | 14389 | 10 |
| hsa-miR-421 | ATTAACTGAC | 315 | 14333 | 14342 | 10 |
| hsa-miR-421 | AACATCAGAC | 316 | 11398 | 11407 | 10 |
| hsa-miR-421 | ATCAACTGAG | 317 | 3427 | 3436 | 10 |
| hsa-miR-421 | ATCAACAGGT | 318 | 2443 | 2452 | 10 |
| hsa-miR-421 | ATCAAAAGAT | 319 | 2333 | 2342 | 10 |
| hsa-miR-422a | ACTGGCCTT | 320 | 15817 | 15825 | 9 |
| hsa-miR-422a | ACTGGTCTT | 321 | 11838 | 11846 | 9 |
| hsa-miR-422a | ACTGGACGT | 322 | 5847 | 5855 | 9 |
| hsa-miR-425 | AGCGGGAAGGT | 323 | 5167 | 5177 | 11 |
| hsa-miR-431 | TGTCTGGCA | 324 | 14892 | 14900 | 9 |
| hsa-miR-431 | TGTCTAGCA | 325 | 9218 | 9226 | 9 |
| hsa-miR-432-5p | TCCTGGAGT | 326 | 13624 | 13632 | 9 |
| hsa-miR-432-5p | TATTGGAGT | 327 | 10785 | 10793 | 9 |
| hsa-miR-432-5p | TCTTAGAGT | 328 | 9263 | 9271 | 9 |
| hsa-miR-432-5p | TCTTAGAGT | 329 | 6666 | 6674 | 9 |
| hsa-miR-432-5p | TCTTGGAGC | 330 | 2180 | 2188 | 9 |
| hsa-miR-452 | ACATCTGC | 331 | 15009 | 15016 | 8 |
| hsa-miR-452 | TCTTCTGC | 332 | 14773 | 14780 | 8 |
| hsa-miR-452 | TTATCTGC | 333 | 14151 | 14158 | 8 |
| hsa-miR-452 | TCCTCTGC | 334 | 13488 | 13495 | 8 |
| hsa-miR-452 | TCATGTGC | 335 | 8660 | 8667 | 8 |
| hsa-miR-452 | TCATCTGG | 336 | 8221 | 8228 | 8 |
| hsa-miR-452 | TCATGTGC | 337 | 7945 | 7952 | 8 |
| hsa-miR-452 | ACATCTGC | 338 | 7508 | 7515 | 8 |
| hsa-miR-452 | CCATCTGC | 339 | 6787 | 6794 | 8 |
| hsa-miR-452 | TCATCCGC | 340 | 5912 | 5919 | 8 |
| hsa-miR-452 | TCATCTGT | 341 | 4053 | 4060 | 8 |
| hsa-miR-452 | TCATCTCC | 342 | 3667 | 3674 | 8 |
| hsa-miR-452 | TCCTCTGC | 343 | 3457 | 3464 | 8 |
| hsa-miR-452 | TCTTCTGC | 344 | 2210 | 2217 | 8 |
| hsa-miR-455-3p | CAGTCCAT | 345 | 13893 | 13900 | 8 |
| hsa-miR-455-5p | TGTGTGCCTT | 346 | 15641 | 15650 | 10 |
| hsa-miR-455-5p | TCTGTGCCTT | 347 | 11203 | 11212 | 10 |
| hsa-miR-455-5p | TATGTGCTTT | 348 | 10522 | 10531 | 10 |
| hsa-miR-483-3p | CACTCCTC | 349 | 13536 | 13543 | 8 |
| hsa-miR-483-3p | CACTCCTC | 350 | 10333 | 10340 | 8 |
| hsa-miR-483-3p | CACTCCTC | 351 | 6101 | 6108 | 8 |
| hsa-miR-486-5p | TCATGTACT | 352 | 9835 | 9843 | 9 |
| hsa-miR-486-5p | TCCTGTCCT | 353 | 6526 | 6534 | 9 |
| hsa-miR-487a | AATCATACAG | 354 | 12829 | 12838 | 10 |
| hsa-miR-487a | AATCATACAG | 355 | 933 | 942 | 10 |
| hsa-miR-491-5p | AATGGGGAAG | 356 | 14975 | 14984 | 10 |
| hsa-miR-491-5p | AGAGGGGACC | 357 | 12315 | 12324 | 10 |
| hsa-miR-491-5p | AGTTGGGCAC | 358 | 11555 | 11564 | 10 |
| hsa-miR-491-5p | AGTAGAGAAC | 359 | 6909 | 6918 | 10 |
| hsa-miR-491-5p | GGTGAGGAAC | 360 | 6005 | 6014 | 10 |
| hsa-miR-491-5p | AGCGGGGCAC | 361 | 4455 | 4464 | 10 |
| hsa-miR-491-5p | AGTGGGAAAT | 362 | 3846 | 3855 | 10 |
| hsa-miR-496 | TTAGTATTA | 363 | 10948 | 10956 | 9 |
| hsa-miR-496 | TGAGTATAA | 364 | 10768 | 10776 | 9 |
| hsa-miR-496 | TCAGTATTA | 365 | 9666 | 9674 | 9 |
| hsa-miR-501-3p | ATGCATCAGG | 366 | 15547 | 15556 | 10 |
| hsa-miR-501-3p | ATCCACCGGG | 367 | 11497 | 11506 | 10 |
| hsa-miR-501-3p | AGGCACCAGG | 368 | 2089 | 2098 | 10 |
| hsa-miR-504 | AGACCCTGT | 369 | 15325 | 15333 | 9 |
| hsa-miR-504 | AGCCCCTGG | 370 | 12898 | 12906 | 9 |
| hsa-miR-504 | AGTCCCTGG | 371 | 10591 | 10599 | 9 |
| hsa-miR-504 | AGACCCGGG | 372 | 4767 | 4775 | 9 |
| hsa-miR-508-3p | TGATTATAGC | 373 | 13565 | 13574 | 10 |
| hsa-miR-508-3p | TGAGTGTAGC | 374 | 3231 | 3240 | 10 |
| hsa-miR-512-3p | CAGTGCTGTC | 375 | 13211 | 13220 | 10 |
| hsa-miR-512-3p | AAGTGCTCTC | 376 | 7688 | 7697 | 10 |
| hsa-miR-512-3p | AAGTGCTCTC | 377 | 3184 | 3193 | 10 |
| hsa-miR-512-5p | CACTCAG | 378 | 14255 | 14261 | 7 |
| hsa-miR-512-5p | CACTCAG | 379 | 13591 | 13597 | 7 |
| hsa-miR-512-5p | CACTCAG | 380 | 12291 | 12297 | 7 |
| hsa-miR-512-5p | CACTCAG | 381 | 6652 | 6658 | 7 |
| hsa-miR-512-5p | CACTCAG | 382 | 5067 | 5073 | 7 |
| hsa-miR-514a-3p | TTGACTCTT | 383 | 14406 | 14414 | 9 |
| hsa-miR-514a-3p | TTGACAGTT | 384 | 13870 | 13878 | 9 |
| hsa-miR-514a-3p | TTAACACTT | 385 | 11237 | 11245 | 9 |
| hsa-miR-514a-3p | ATGACACTT | 386 | 10617 | 10625 | 9 |
| hsa-miR-515-3p | GTGTGCCTT | 387 | 15641 | 15649 | 9 |
| hsa-miR-515-3p | GACTGCCTT | 388 | 15539 | 15547 | 9 |
| hsa-miR-515-3p | GAGTGACTT | 389 | 1371 | 1379 | 9 |
| hsa-miR-516a-3p | TGCTTCCT | 390 | 10301 | 10308 | 8 |
| hsa-miR-517a-3p | ATGGTGCATT | 391 | 15650 | 15659 | 10 |
| hsa-miR-517a-3p | ATCTTGCTTC | 392 | 10303 | 10312 | 10 |
| hsa-miR-519b-3p | AAAGTGCAT | 393 | 13782 | 13790 | 9 |
| hsa-miR-519e-3p | AAGTGCCTC | 394 | 7048 | 7056 | 9 |
| hsa-miR-520a-5p | CTCCAGATGG | 395 | 6274 | 6283 | 10 |
| hsa-miR-545 | CAGCAAGCACT | 396 | 15050 | 15060 | 11 |
| hsa-miR-545 | CAGAACACATT | 397 | 11639 | 11649 | 11 |
| hsa-miR-545 | CTGCAAACACT | 398 | 3450 | 3460 | 11 |
| hsa-miR-549 | TGACAACTGT | 399 | 14327 | 14336 | 10 |
| hsa-miR-551b-3p | GCTACCCAT | 400 | 2411 | 2419 | 9 |
| hsa-miR-552 | CACAGGTGA | 401 | 15130 | 15138 | 9 |
| hsa-miR-552 | AACAGGTCA | 402 | 11407 | 11415 | 9 |
| hsa-miR-552 | AACATGTGA | 403 | 9513 | 9521 | 9 |
| hsa-miR-552 | AACAGGTTA | 404 | 2441 | 2449 | 9 |
| hsa-miR-552 | AACAGGTAA | 405 | 1569 | 1577 | 9 |
| hsa-miR-583 | AAAAGAGGA | 406 | 2921 | 2929 | 9 |
| hsa-miR-583 | CAAATAGGA | 407 | 2833 | 2841 | 9 |
| hsa-miR-583 | CAACGAGGA | 408 | 1824 | 1832 | 9 |
| hsa-miR-583 | CAAAGAAGA | 409 | 1139 | 1147 | 9 |
| hsa-miR-593-3p | TGTCTCTGT | 410 | 8204 | 8212 | 9 |
| hsa-miR-593-3p | TGGCTCTGC | 411 | 6852 | 6860 | 9 |
| hsa-miR-593-3p | TGCCTCTGC | 412 | 231 | 239 | 9 |
| hsa-miR-593-5p | AGGCACCAG | 413 | 2090 | 2098 | 9 |
| hsa-miR-593-5p | AGGCACCAG | 414 | 2083 | 2091 | 9 |
| hsa-miR-598 | ACGTCATC | 415 | 11432 | 11439 | 8 |
| hsa-miR-611 | GCGAGGTCTC | 416 | 4779 | 4788 | 10 |
| hsa-miR-611 | GAGAGGCCCC | 417 | 2121 | 2130 | 10 |
| hsa-miR-611 | GAGAGGACCT | 418 | 1546 | 1555 | 10 |
| hsa-miR-616-5p | ACTCTAAAC | 419 | 14510 | 14518 | 9 |
| hsa-miR-619 | GACCTGGA | 420 | 5824 | 5831 | 8 |
| hsa-miR-620 | ATGAATATAG | 421 | 14560 | 14569 | 10 |
| hsa-miR-620 | ATGGAAATAT | 422 | 12111 | 12120 | 10 |
| hsa-miR-620 | TTGGATATAG | 423 | 11026 | 11035 | 10 |
| hsa-miR-620 | GTGGAGATGG | 424 | 10397 | 10406 | 10 |
| hsa-miR-620 | ATGGAGATCC | 425 | 6268 | 6277 | 10 |
| hsa-miR-620 | ATGGAGGGAG | 426 | 5626 | 5635 | 10 |
| hsa-miR-620 | CTGGAGAAAG | 427 | 3827 | 3836 | 10 |
| hsa-miR-620 | ATCCAGATAG | 428 | 2959 | 2968 | 10 |
| hsa-miR-620 | ATGGGGCTAG | 429 | 2843 | 2852 | 10 |
| hsa-miR-620 | AGGGAGAGAG | 430 | 1551 | 1560 | 10 |
| hsa-miR-620 | CAGGAGATAG | 431 | 1430 | 1439 | 10 |
| hsa-miR-620 | TTGGAGAGAG | 432 | 1201 | 1210 | 10 |
| hsa-miR-623 | TCCCTTGC | 433 | 8306 | 8313 | 8 |
| hsa-miR-623 | TCCCTTGC | 434 | 5004 | 5011 | 8 |
| hsa-miR-631 | CACCTGGCC | 435 | 9900 | 9908 | 9 |
| hsa-miR-631 | GACATGGCC | 436 | 8632 | 8640 | 9 |
| hsa-miR-634 | AACCAGCAC | 437 | 4520 | 4528 | 9 |
| hsa-miR-636 | TGTGCTTG | 438 | 10386 | 10393 | 8 |
| hsa-miR-638 | ACGGAGCGCG | 439 | 4905 | 4914 | 10 |
| hsa-miR-638 | AGGGAGGGCG | 440 | 4615 | 4624 | 10 |
| hsa-miR-642a-5p | ATCCCTCTC | 441 | 8983 | 8991 | 9 |
| hsa-miR-642a-5p | GTCCCTCCC | 442 | 4722 | 4730 | 9 |
| hsa-miR-643 | ACATGCATGC | 443 | 15553 | 15562 | 10 |
| hsa-miR-643 | CCTTGTAGGC | 444 | 15378 | 15387 | 10 |
| hsa-miR-643 | TCTTGTATTC | 445 | 14423 | 14432 | 10 |
| hsa-miR-643 | ACTGGTATGT | 446 | 13933 | 13942 | 10 |
| hsa-miR-643 | ACTTCTATTC | 447 | 12886 | 12895 | 10 |
| hsa-miR-643 | ACTTTTCTGC | 448 | 12044 | 12053 | 10 |
| hsa-miR-643 | GCTTGTAAGC | 449 | 11698 | 11707 | 10 |
| hsa-miR-643 | AGTTGTATGT | 450 | 10531 | 10540 | 10 |
| hsa-miR-643 | ACTTGGAAGC | 451 | 8105 | 8114 | 10 |
| hsa-miR-643 | ACTTGTGTGG | 452 | 7227 | 7236 | 10 |
| hsa-miR-643 | ACTTGTTTGA | 453 | 1880 | 1889 | 10 |
| hsa-miR-643 | ACATGTTTGC | 454 | 1695 | 1704 | 10 |
| hsa-miR-650 | AGGAGGCAC | 455 | 9647 | 9655 | 9 |
| hsa-miR-650 | AGAAGGCAG | 456 | 6917 | 6925 | 9 |
| hsa-miR-650 | AGGAGCCAG | 457 | 3474 | 3482 | 9 |
| hsa-miR-650 | ATGAGGCAG | 458 | 3052 | 3060 | 9 |
| hsa-miR-651 | TCATGATAAG | 459 | 15700 | 15709 | 10 |
| hsa-miR-651 | TTAGGTTAAA | 460 | 13993 | 14002 | 10 |
| hsa-miR-651 | TTAAAATAAG | 461 | 13988 | 13997 | 10 |
| hsa-miR-651 | TTAGCATAAC | 462 | 12788 | 12797 | 10 |
| hsa-miR-651 | TTATGATGAG | 463 | 12617 | 12626 | 10 |
| hsa-miR-651 | TTTGGATGAG | 464 | 11069 | 11078 | 10 |
| hsa-miR-651 | TGAGTATAAG | 465 | 10767 | 10776 | 10 |
| hsa-miR-651 | TTACAATAAG | 466 | 10546 | 10555 | 10 |
| hsa-miR-651 | TAAGGATAAA | 467 | 8265 | 8274 | 10 |
| hsa-miR-651 | TGTGGATAAG | 468 | 7222 | 7231 | 10 |
| hsa-miR-651 | GTAGGATAGG | 469 | 5553 | 5562 | 10 |
| hsa-miR-651 | CTAGGAAAAG | 470 | 2823 | 2832 | 10 |
| hsa-miR-651 | CTATGATAAG | 471 | 1635 | 1644 | 10 |
| hsa-miR-651 | TAAGGATAGG | 472 | 1562 | 1571 | 10 |
| hsa-miR-654-3p | TATGTATACT | 473 | 15493 | 15502 | 10 |
| hsa-miR-654-3p | TATCTCTTCT | 474 | 14775 | 14784 | 10 |
| hsa-miR-654-3p | TCTATCTGCT | 475 | 8354 | 8363 | 10 |
| hsa-miR-654-3p | AATGTCTGGT | 476 | 6720 | 6729 | 10 |
| hsa-miR-654-3p | TATGTTTCCT | 477 | 6638 | 6647 | 10 |
| hsa-miR-654-3p | TTTTTCTGCT | 478 | 6586 | 6595 | 10 |
| hsa-miR-654-3p | TATGTCTTTT | 479 | 6534 | 6543 | 10 |
| hsa-miR-654-3p | TATATCTGCA | 480 | 6214 | 6223 | 10 |
| hsa-miR-654-3p | TATGTAGGCT | 481 | 97 | 106 | 10 |
| hsa-miR-655 | GTAATACAT | 482 | 15593 | 15601 | 9 |
| hsa-miR-655 | ATAGTACAT | 483 | 4200 | 4208 | 9 |
| hsa-miR-655 | ATAAGACAT | 484 | 3642 | 3650 | 9 |
| hsa-miR-655 | ATAATACAG | 485 | 2265 | 2273 | 9 |
| hsa-miR-655 | ACAATACAT | 486 | 1757 | 1765 | 9 |
| hsa-miR-656 | AATATTATA | 487 | 657 | 665 | 9 |
| hsa-miR-664-3p | TATTCATTT | 488 | 9385 | 9393 | 9 |
| hsa-miR-765 | TGGAGGA | 489 | 5020 | 5026 | 7 |
| hsa-miR-766 | CTCCAGCCCC | 490 | 12901 | 12910 | 10 |
| hsa-miR-766 | CTCCAGCCCC | 491 | 5032 | 5041 | 10 |
| hsa-miR-767-3p | CCTGCTCAT | 492 | 14871 | 14879 | 9 |
| hsa-miR-767-3p | TCTTCTCAT | 493 | 9155 | 9163 | 9 |
| hsa-miR-875 | CCTGGAAATA | 494 | 5820 | 5829 | 10 |
| hsa-miR-875 | CCTAGAAACA | 495 | 5294 | 5303 | 10 |
| hsa-miR-876 | TGGATTTCT | 496 | 6366 | 6374 | 9 |
| hsa-miR-876 | TGGATTTCT | 497 | 142 | 150 | 9 |
| hsa-miR-888-3p | GACTGACTCC | 498 | 15772 | 15781 | 10 |
| hsa-miR-888-3p | GACTGACAGC | 499 | 9119 | 9128 | 10 |
| hsa-miR-890 | TACTTGGAAG | 500 | 8106 | 8115 | 10 |
| hsa-miR-940 | AAGGCAGTG | 501 | 1807 | 1815 | 9 |
| hsa-miR-941 | CACCCAGGT | 502 | 14396 | 14404 | 9 |
| hsa-miR-941 | CACCCTGCC | 503 | 13715 | 13723 | 9 |
| hsa-miR-941 | CACCCCTCT | 504 | 13128 | 13136 | 9 |
| hsa-miR-941 | CACTCAGCT | 505 | 12289 | 12297 | 9 |
| hsa-miR-941 | CTCCCGGGT | 506 | 10102 | 10110 | 9 |
| hsa-miR-941 | CAGCCTGCT | 507 | 10034 | 10042 | 9 |
| hsa-miR-941 | CACCCACCT | 508 | 9904 | 9912 | 9 |
| hsa-miR-941 | CACCTGGCC | 509 | 9900 | 9908 | 9 |
| hsa-miR-941 | CATCTGGCT | 510 | 8219 | 8227 | 9 |
| hsa-miR-941 | CACTCGACT | 511 | 8148 | 8156 | 9 |
| hsa-miR-941 | CTCCCAGCT | 512 | 6840 | 6848 | 9 |
| hsa-miR-941 | CTCACGGCT | 513 | 6031 | 6039 | 9 |
| hsa-miR-941 | CAGCCCGCT | 514 | 5928 | 5936 | 9 |
| hsa-miR-941 | CACCTGACT | 515 | 5510 | 5518 | 9 |
| hsa-miR-941 | CACGCCGCT | 516 | 5142 | 5150 | 9 |
| hsa-miR-941 | CTCCCTGCT | 517 | 3983 | 3991 | 9 |
| hsa-miR-941 | CACCAGGCA | 518 | 2087 | 2095 | 9 |
| hsa-miR-941 | CTCCCGGGT | 519 | 390 | 398 | 9 |
| hsa-miR-941 | CACCCAGCC | 520 | 186 | 194 | 9 |
| hsa-miR-941-2 | ATCCGACTGT | 521 | 9657 | 9666 | 10 |
| hsa-miR-941-2 | TCCCTGCTGT | 522 | 8726 | 8735 | 10 |
| hsa-miR-941-2 | TCCCAGCTGT | 523 | 6838 | 6847 | 10 |
| hsa-miR-941-2 | AGCCCGCTGT | 524 | 5926 | 5935 | 10 |
| hsa-miR-941-2 | ACCCGGGCGT | 525 | 4764 | 4773 | 10 |
| hsa-miR-1179 | AAGTATCCTTT | 526 | 15346 | 15356 | 11 |
| hsa-miR-1179 | ATGCATTCTGT | 527 | 3357 | 3367 | 11 |
| hsa-miR-1179 | ATGCATTCTCT | 528 | 1854 | 1864 | 11 |
| hsa-miR-1207-5p | TGGCAGGG | 529 | 11441 | 11448 | 8 |
| hsa-miR-1224-3p | CTCCACCTCC | 530 | 399 | 408 | 10 |
| hsa-miR-1228-3p | TCCCACCTG | 531 | 13637 | 13645 | 9 |
| hsa-miR-1228-3p | TCACGCCTG | 532 | 4992 | 5000 | 9 |
| hsa-miR-1231 | GTGTCTGGC | 533 | 12807 | 12815 | 9 |
| hsa-miR-1231 | GTGTCCGGG | 534 | 4739 | 4747 | 9 |
| hsa-miR-1245 | AAGTGATCT | 535 | 8341 | 8349 | 9 |
| hsa-miR-1245 | AAGTGATCT | 536 | 2020 | 2028 | 9 |
| hsa-miR-1249 | CGCCCTTC | 537 | 5907 | 5914 | 8 |
| hsa-miR-1251 | ACTCTAGGT | 538 | 12854 | 12862 | 9 |
| hsa-miR-1251 | ACTCTATCT | 539 | 8357 | 8365 | 9 |
| hsa-miR-1251 | ACTCCAGCT | 540 | 4044 | 4052 | 9 |
| hsa-miR-1251 | AGTCTAGCT | 541 | 457 | 465 | 9 |
| hsa-miR-1252 | AGAGGGAAAT | 542 | 3819 | 3828 | 10 |
| hsa-miR-1252 | GGAAGGAAAT | 543 | 1625 | 1634 | 10 |
| hsa-miR-1268 | CGGGCGTGG | 544 | 4762 | 4770 | 9 |
| hsa-miR-1270 | CTGGAAATA | 545 | 5820 | 5828 | 9 |
| hsa-miR-1270 | CTGGAGATG | 546 | 5055 | 5063 | 9 |
| hsa-miR-1270 | CTGGAGAAA | 547 | 3828 | 3836 | 9 |
| hsa-miR-1270 | CAGGAGATA | 548 | 1431 | 1439 | 9 |
| hsa-miR-1272 | GATGATGA | 549 | 10622 | 10629 | 8 |
| hsa-miR-1275 | GTAGGGGAGA | 550 | 1189 | 1198 | 10 |
| hsa-miR-1302 | ATGGGACACA | 551 | 15021 | 15030 | 10 |
| hsa-miR-1302 | TTTGGATATA | 552 | 11027 | 11036 | 10 |
| hsa-miR-1302 | TTAGGGCATA | 553 | 8421 | 8430 | 10 |
| hsa-miR-1302 | TTGGAACAGA | 554 | 6076 | 6085 | 10 |
| hsa-miR-1302 | CTGGGACTTA | 555 | 4819 | 4828 | 10 |
| hsa-miR-1302 | GTGGGAAATA | 556 | 3845 | 3854 | 10 |
| hsa-miR-1302 | TTGTGAGATA | 557 | 1944 | 1953 | 10 |
| hsa-miR-1302 | CTGGGAAATA | 558 | 867 | 876 | 10 |
| hsa-miR-1324 | TCAAGACAGA | 559 | 9426 | 9435 | 10 |
| hsa-miR-1827 | TGAGGCAGT | 560 | 3051 | 3059 | 9 |
| hsa-miR-1911-3p | CACCAGGCA | 561 | 2087 | 2095 | 9 |
| hsa-miR-1915 | CCCCAGGG | 562 | 5111 | 5118 | 8 |
| hsa-miR-2909 | TTTAGGGCC | 563 | 3728 | 3736 | 9 |

### B2. Another exemplary multiple miRNAs-one mRNA paradigm involves UCP2.

UCP2 is a mitochondrial transporter protein expressed in WAT, skeletal muscle, pancreatic islets and the central nervous system. Like UCP1, it creates proton leaks across the inner mitochondrial membrane, thus uncoupling oxidative phosphorylation from ATP synthesis (adaptive thermogenesis, see Figure 5) (Lowell et al., Nature (2000)).

Two recent meta-analyses report an association between polymorphisms in the promoter region of UCP2 and obesity (Liu et al., Gene (2013); Andersen et al., Int. J. Obes. (2013)). The first meta-analysis included 14 studies (7,647 cases and 11,322 controls) and concluded that there is a significant association of the A allele of the UCP2 -866G/A polymorphism with reduced risk of obesity, especially in European populations. In the second meta-analysis including 12,984 subjects, the common UCP2 -866G allele is associated with obesity. The same UCP2 -866G allele is associated with decreased insulin sensitivity in 17,636 Danish subjects. In a study, UCP2 mRNA levels in visceral fat were decreased in subjects with the GG phenotype (Esterbauer et al., Nat. Genet. (2001)). A trend toward a negative correlation between subcutaneous adipocyte UCP2 mRNA and percent body fat was found in another study (Wang et al., American Journal of Physiol. (2004)). This information supports targeting UCP2 expression and activity as a meaningful way to alter adaptive thermogenesis and consequently treat human obesity. Many strategies could be implemented to achieve this goal, however, the one employed in the methods of the invention uses miRNA agents to modulate simultaneously several elements within the thermogenic pathways to increase UCP2 synthesis and activity. Both direct and indirect interactions between miRNAs and the UCP2 gene are considered. Direct interaction means the direct binding of miRNAs to the various regions of the UCP2 gene, resulting in alterations of the transcription, translation, stability and/or degradation of the UCP1 mRNA. Indirect interaction means that miRNAs alter the transcription, translation, stability and/or degradation of thermogenic mRNAs, whose expressed proteins alter the transcription of the UCP2 gene. Furthermore, indirect interaction means that miRNAs alter the transcription, translation, stability and/or degradation of other miRNAs that modify the transcription of the UCP2 gene.

The promoter region of the human UCP2 gene (ENSG00000175567, Homo sapiens uncoupling protein 2 (mitochondrial, proton carrier) (UCP2), RefSeqGene on chromosome 11) is rich is regulatory element motifs (Table 12).

**Table 12.**

| **UCP2 Gene Regulatory Elements:** | | | | |
|---|---|---|---|---|
| | **Name of regulatory element** | **Sequence** | **Number** | **Nucleotide Location** |
| 1 | **RXR/T3RE** | AGGTCA | 8 | 1,074 to 1,079; 3,083 to 3,088; |
| | | | | 3,239 to 3,244; 4,304 to 4,309; |
| | | | | 6,965 to 6,970; 7,420 to 7,425; |
| | | | | 7,677 to 7,682; 13,319 to 13,324 |
| 2 | **GC Box 1** | CGCCC | 16 | 2,605 to 2,609; 4,323 to 4,327; |
| | | | | 4,523 to 4,527; 4,933 to 4,937; |
| | | | | 4,959 to 4,963; 5,048 to 5,052; |
| | | | | 5,066 to 5,070; 5,146 to 5,150; |
| | | | | 5,155 to 5,159; 5,387 to 5,391; |
| | | | | 5,483 to 5,487; 6,067 to 6,071; |
| | | | | 8,523 to 8,527; 9,790 to 9,794; |
| | | | | 10,819 to 10,823; 11,754 to 11,758 |
| 3 | **GC Box 2** | GCGGG | 5 | 4,263 to 4,267; 4,757 to 4,761; |
| | | | | 4,860 to 4,864; 7,619 to 7,623; |
| | | | | 11,262 to 11,266 |
| 4 | **GT Box 1** | CACCC | 30 | 1,421 to 1,425; 1,677 to 1,681; |
| | | | | 1,761 to 1,765; 1,825 to 1,829; |
| | | | | 1,833 to 1,837; 2,036 to 2,040; |
| | | | | 3,003 to 3,007; 4,903 to 4,907; |
| | | | | 4,947 to 4,951; 5,210 to 5,214; |
| | | | | 6,204 to 6,208; 6,247 to 6,251; 6,469 |
| | | | | to 6,473; 6,828 to 6,832; |
| | | | | 7,681 to 7,685; 8,048 to 8,052; |
| | | | | 8,437 to 8,441; 8,572 to 8,576; |
| | | | | 8,599 to 8,603; 8,702 to 8,706; |
| | | | | 11,077 to 11,081; 11,235 to 11,239; |
| | | | | 12,006 to 12,010; 12,374 to 12,378; |
| | | | | 13,475 to 13,479; 13,666 to 13,670; |
| | | | | 13,687 to 13,691; 13,838 to 13,842; |
| | | | | 14,410 to 14,414; 14,545 to 14,549 |
| 5 | **GT Box 2** | GTGGG | 26 | 123 to 127; 1,006 to 1,010; |
| | | | | 2,105 to 2,109; 4,562 to 4,566; |
| | | | | 5,793 to 5,797; 6,029 to 6,033; |
| | | | | 6,034 to 6,038; 6,040 to 6,044; |
| | | | | 6,150 to 6,154; 7,271 to 7,275; |
| | | | | 7,392 to 7,396; 9,040 to 9,044; |
| | | | | 9,697 to 9,701; 10,227 to 10,231; |
| | | | | 10,238 to 10,242; 10,247 to 10,251; |
| | | | | 11,817 to 11,821; 12,410 to 12,414; |
| | | | | 12,414 to 12,418; 12,678 to 12,682; |
| | | | | 13,047 to 13,051; 13,238 to 13,742; |
| | | | | 13,743 to 13,747; 14,252 to 14,256; |
| | | | | 14,969 to 14,973; 15,104 to 15,108 |
| 6 | **CpG Methylation Island** | CG | 295 | 4,071 to 5,212 |

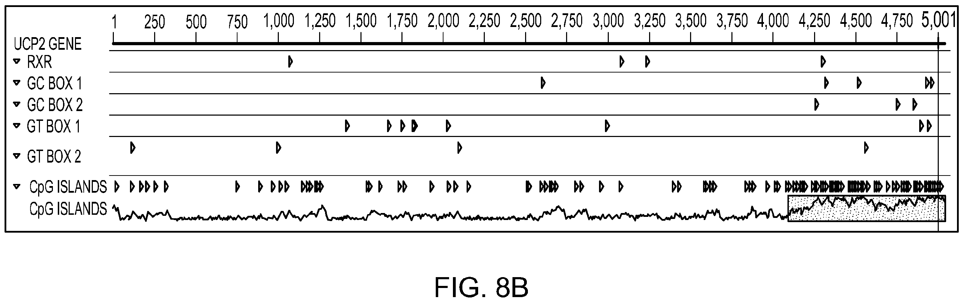

Figure 8B depicts the location of these various regulatory elements in reference to the UCP2 transcription start site at nucleotide position 5,001 of the 15,174 base pair human UCP2 gene. Direct or indirect activation or repression of these regulatory elements by miRNAs will result in alterations of UCP2 gene expression and activity.

A survey of miRNAs targeting the human UCP2 3'UTR with several prediction programs, using the UCP2 Ensembl 2,113 base pair transcript ENST00000310473 as a target revealed binding sites for 161 miRNAs as shown in Table 13.

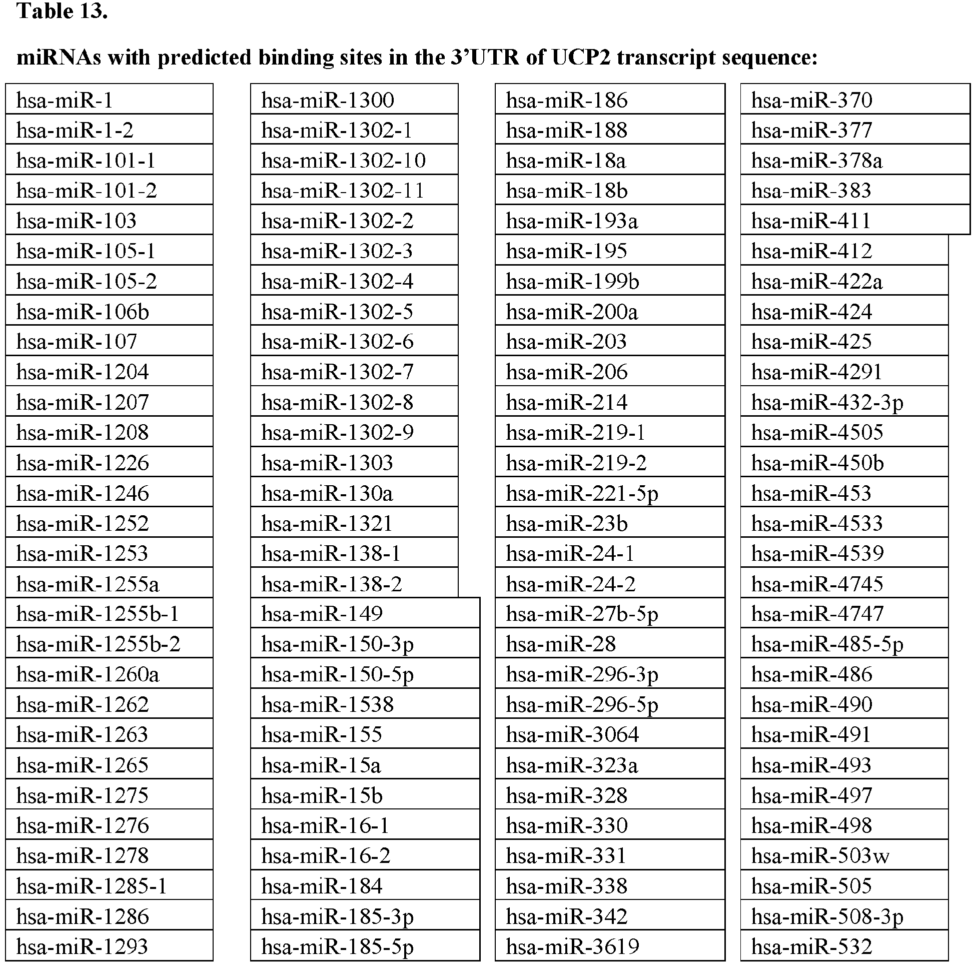

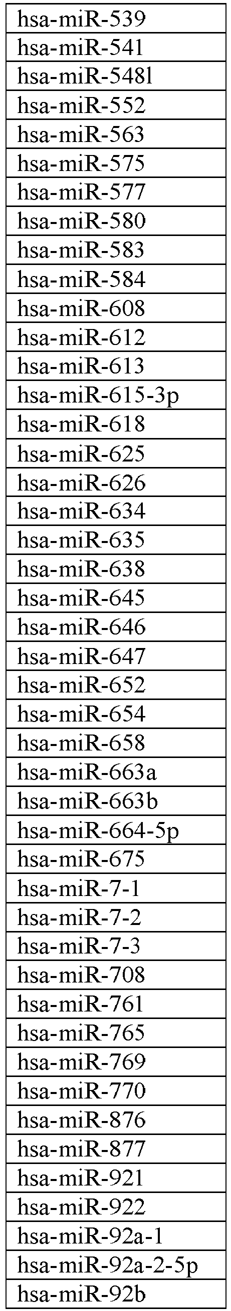

Moreover, a survey of miRNAs targeting the human UCP2 5'UTR with several prediction programs, using the human UCP2 gene (ENSG00000175567, 15,174 base pair (bp), including 5,000 bp 5'UTR as a target revealed binding sites for 54 miRNAs in UCP2 5'UTR as shown in Table 14.

**Table 14.**

| **miRNAs with predicted binding sites in the 5'UTR of UCP2 gene sequence:** | | | | | | |
|---|---|---|---|---|---|---|
| **MicroRNA** | **Seed Length** | **Start** | **Sequence** | **SEQ ID NO:** | **End** | **P value** |
| hsa-let-7c | 9 | 3052 | UAGAGUUAC | 564 | 3044 | 0.0374 |
| hsa-let-7i-3p | 9 | 3051 | CUGCGCAAG | 565 | 3043 | 0.0374 |
| hsa-miR-1228-5p | 9 | 3419 | UGGGCGGGG | 566 | 3411 | 0.0374 |
| hsa-miR-1229-3p | 9 | 3419 | UCUCACCAC | 567 | 3411 | 0.0374 |
| hsa-miR-129-1-3p | 10 | 2784 | AGCCCUUACC | 568 | 2775 | 0.0095 |
| hsa-miR-1302 | 9 | 4219 | UGGGACAUA | 569 | 4211 | 0.0374 |
| hsa-miR-1303 | 9 | 2159 | UUAGAGACG | 570 | 2151 | 0.0374 |
| hsa-miR-136 | 9 | 4486 | CUCCAUUUG | 571 | 4478 | 0.0374 |
| hsa-miR-155 | 9 | 2160 | UUAAUGCUA | 572 | 2152 | 0.0374 |
| hsa-miR-16 | 10 | 3603 | UAGCAGCACG | 573 | 3594 | 0.0095 |
| hsa-miR-18a-3p | 10 | 3603 | ACUGCCCUAA | 574 | 3594 | 0.0095 |
| hsa-miR-190 | 9 | 2428 | UGAUAUGUU | 575 | 2420 | 0.0374 |
| hsa-miR-191 | 9 | 3052 | CAACGGAAU | 576 | 3044 | 0.0374 |
| hsa-miR-192 | 9 | 4390 | CUGACCUAU | 577 | 4382 | 0.0374 |
| hsa-miR-194 | 9 | 1643 | UGUAACAGC | 578 | 1635 | 0.0374 |
| hsa-miR-197 | 9 | 5001 | UCACCACCU | 579 | 4993 | 0.0374 |
| hsa-miR-19b-2-5p | 10 | 3052 | AGUUUUGCAG | 580 | 3043 | 0.0095 |
| hsa-miR-203 | 9 | 3051 | UGAAAUGUU | 581 | 3043 | 0.0374 |
| hsa-miR-218 | 10 | 3603 | UUGUGCUUGA | 582 | 3594 | 0.0095 |
| hsa-miR-218-1-3p | 9 | 5001 | UGGUUCCGU | 583 | 4993 | 0.0374 |
| hsa-miR-219-1-3p | 9 | 3614 | AGAGUUGAG | 584 | 3606 | 0.0374 |
| hsa-miR-26a-2-3p | 9 | 2163 | CCUAUUCUU | 585 | 2155 | 0.0374 |
| hsa-miR-27a-3p | 10 | 3603 | UUCACAGUGG | 586 | 3594 | 0.0095 |
| hsa-miR-27a-5p | 11 | 3336 | AGGGCUUAGCU | 587 | 3326 | 0.0024 |
| hsa-miR-28-5p | 10 | 3603 | AAGGAGCUCA | 588 | 3594 | 0.0095 |
| hsa-miR-331-3p | 9 | 4134 | GCCCCUGGG | 589 | 4126 | 0.0374 |
| hsa-miR-337-5p | 10 | 115 | GAACGGCUUC | 590 | 106 | 0.0095 |
| hsa-miR-340-3p | 9 | 1872 | CCGUCUCAG | 591 | 1864 | 0.0374 |
| hsa-miR-34c-3p | 11 | 2162 | AAUCACUAACC | 592 | 2152 | 0.0024 |
| hsa-miR-373-5p | 11 | 530 | ACUCAAAAUGG | 593 | 520 | 0.0024 |
| hsa-miR-425 | 9 | 1013 | AAUGACACG | 594 | 1005 | 0.0374 |
| hsa-miR-497 | 9 | 3661 | AGCAGCACA | 595 | 3653 | 0.0374 |
| hsa-miR-501-5p | 9 | 4164 | AUCCUUUGU | 596 | 4156 | 0.0374 |
| hsa-miR-505 | 9 | 1015 | GUCAACACU | 597 | 1007 | 0.0374 |
| hsa-miR-508-3p | 9 | 1274 | GAUUGUAGC | 598 | 1266 | 0.0374 |
| hsa-miR-509-3p | 12 | 2554 | UGAUUGGUACGU | 599 | 2543 | 0.0006 |
| hsa-miR-512-5p | 10 | 987 | ACUCAGCCUU | 600 | 978 | 0.0095 |
| hsa-miR-514 | 9 | 5001 | UUGACACUU | 601 | 4993 | 0.0374 |
| hsa-miR-515-5p | 9 | 59 | UUCUCCAAA | 602 | 51 | 0.0374 |
| hsa-miR-518a-3p | 9 | 19 | GAAAGCGCU | 603 | 11 | 0.0374 |
| hsa-miR-519e-5p | 11 | 2525 | UCUCCAAAAGG | 604 | 2515 | 0.0024 |
| hsa-miR-548a-3p | 10 | 680 | CAAAACUGGC | 605 | 671 | 0.0095 |
| hsa-miR-550a-3p | 9 | 4312 | GUCUUACUC | 606 | 4304 | 0.0374 |
| hsa-miR-571 | 9 | 739 | UGAGUUGGC | 607 | 731 | 0.0374 |
| hsa-miR-578 | 9 | 1377 | CUUCUUGUG | 608 | 1369 | 0.0374 |
| hsa-miR-606 | 9 | 4420 | AACUACUGA | 609 | 4412 | 0.0374 |
| hsa-miR-615-5p | 10 | 1140 | GGGGGUCCCC | 610 | 1131 | 0.0095 |
| hsa-miR-638 | 9 | 2710 | GGGAUCGCG | 611 | 2702 | 0.0374 |
| hsa-miR-657 | 12 | 1316 | GCAGGUUCUCAC | 612 | 1305 | 0.0006 |
| hsa-miR-658 | 9 | 3673 | GGCGGAGGG | 613 | 3665 | 0.0374 |
| hsa-miR-877-3p | 9 | 4349 | UCCUCUUCU | 614 | 4341 | 0.0374 |
| hsa-miR-93-3p | 9 | 799 | ACUGCUGAG | 615 | 791 | 0.0374 |
| hsa-miR-96-3p | 9 | 799 | AAUCAUGUG | 616 | 791 | 0.0374 |
| hsa-miR-99b-3p | 9 | 2163 | CAAGCUCGU | 617 | 2155 | 0.0374 |

### C) A multiple microRNAs-multiple mRNAs paradigm.

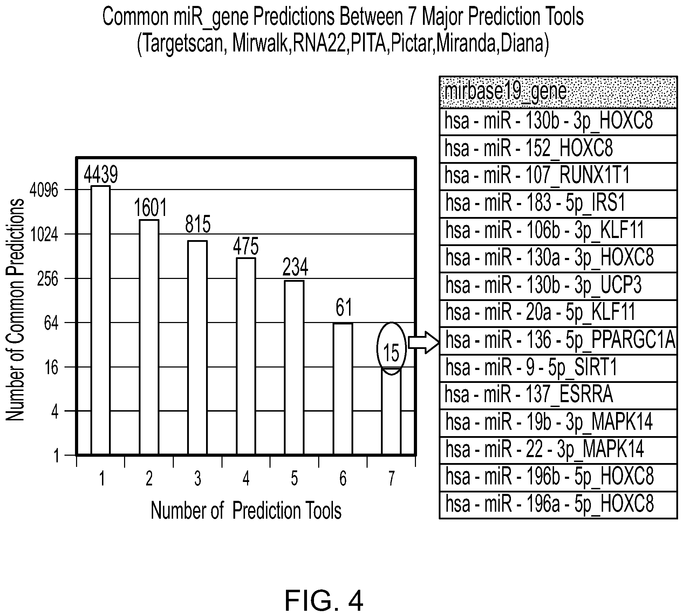

The 83 thermogenic regulator molecules selected in Table 2 were screened for high stringency Multiple miRNAs-Multiple mRNAs associations. The results of these analyses with 7 major prediction tools are shown in Figure 4. The union of these 7 tools produces 4439 miRNA-gene couples. Overlap between these tools decreases as the number of tools increases, reaching only 15 miRNA-gene couples when 7 tools are considered.

### D) An over-representation of one microRNA seed sequence motif among co-regulated mRNA targets paradigm.

Several approaches can be used to identify pathway-specific miRNAs. For example, searching the 3'-UTRs of putatively co-regulated genes for an over-represented sequence from a miRNA seed region could identify a common regulatory miRNA. To determine if particular miRNA seed sequences were overrepresented among the 3' UTR of the chosen 83 thermogenesis targets, the miRvestigator web application (miRvestigator. systemsbiology.net/) was employed. Using the following parameters (motif size of 8bp, default Weeder model, seed model of 8mer, 100% complementarity homology and 0.25 wobble base-pairing allowed), it was determined that that the motif 5'-UUUGUACA-3' recognized by hsa-miR-19a/19b is overrepresented among 15 of the 83 thermogenesis targets with a complementarity p value of 1.7 x 10⁻⁰⁴ as shown in Table 15. Of note is that hsa-miR-19 has been reported as an abundant human adipocyte miRNA.

**Table 15.**

| **Complementarity between the common motif UUUGUACA and hsa-miR-19a/19b:** | | | | | |
|---|---|---|---|---|---|
| miRNA Name | miRNA Seed | Seed Model | Length of Complementarity | Complementary Base-Palring | Complementarity P-Value |
| hsa-miR-19a | UGUGCAAA | 8mer | 8 | Motif 5' UUUGUACA 3' ;;;;:;;; 3' AAACGUGU 5' miRNA Seed | 1.7e-04 |
| hsa-miR-19b | | | | | |

The Minimum Free Energy levels of the hsa-miR-19 mRNA/miRNA duplexes identified by miRvestigator were quite low, favoring tight binding. Accordingly, the miRvestigator analysis was repeated with less stringent levels of complementarity. This analysis identified a further 10 additional targets (CEBPD, PRKAA1, TWIST1, IRS1, NCOA1, NCOA2, NCOA3, KLF5, RPS6KB1, NRIP1) with 95% similarity to the consensus hsa-miR-19 motif. Interestingly, hsa-miR-19 is among the most abundant miRNAs in adipose tissue. The genes identified as containing a sequence complementary to hsa-miR-19 seed region are set forth in Table 16.

**Table 16.**

| **Thermogenic regulators identified as targets for hsa-miR-19:** | | | | | |
|---|---|---|---|---|---|
| **Gene** | **Gene symbol** | **Sequence of Site** | **Start Relative to Stop Codon (bp)** | **% Similarity to Consensus Motif (Quality = \|Medium\|Fair)** | **Minimum Free Energy (MFE) of mRNA-miRNA Duplex** |
| 650 | BMP2 | UUUGUACA | 386 | 100.00 | -6.80 |
| 1052 | CEBPD | UUUGUAAA | 263 | 95.44 | -3.40 |
| 7132 | TNFRSF1A | UUUGUACA | 510 | 100.00 | -6.80 |
| 5569 | PRKAA1 | UUUGUAAA | 2400 | 95.44 | -3.40 |
| 5563 | PRKAA2 | UUUGUACA | 542 | 1043.00 | -6.80 |
| 655 | BMP7 | UUUGUACA | 1927 | 1040.00 | -6.80 |
| 652 | BMP4 | UUUGUACA | 770 | 100.00 | -6.80 |
| 133592 | PPARGC1B | UUUGUACA | 7199 | 100.00 | -6.80 |
| 7474 | WNT5A | UUUGUACA | 1414 | 100.00 | -6.80 |
| 6720 | SREBF1 | UUUGUACA | 510 | 100.00 | -6.80 |
| 7291 | TWIST1 | UUUGUAAA | 649 | 95.44 | -3.40 |
| 3697 | IRS1 | UUUGUAAA | 992 | 93.44 | -3.40 |
| 10499 | NCOA2 | UUUGUAAA | 1381 | 95.44 | -3.40 |
| 8204 | NRIP1 | UUUGUACA | 1718 | 100.00 | -6.80 |
| 8204 | NRIP 1 | UUUGUAAA | 1935 | 95.44 | -3.40 |
| 8202 | NCOA3 | UUUGUAAA | 965 | 95.44 | -3.40 |
| 1385 | CREB1 | UUUGUAAA | 1973 | 95.44 | -3.40 |
| 1385 | CREB1 | UUUGUACA | 2822 | 100.00 | -6.80 |
| | | | | | |
| 1385 | CREB1 | UUUGUACA | 2822 | 100.00 | -6.80 |
| 1385 | CREB1 | UUUGUAAA | 4175 | 95.44 | -3.40 |
| 3643 | INSR | UUUGUAAA | 2105 | 95.44 | -3.40 |
| 8013 | NR4A3 | UUUGUACA | 2347 | 100.00 | -6.80 |
| 860 | RUNX2 | UUUGUACA | 2425 | 100.00 | -6.80 |
| 6776 | STAT5A | UUUGUACA | 1214 | 100.00 | -6.80 |
| 1874 | E2F4 | UUUGUACA | 755 | 100.00 | -6.80 |
| 668 | KLF5 | UUUGUAAA | 549 | 95.44 | -3.40 |
| 8648 | NCOA1 | UUUGUAAA | 381 | 95.44 | -3.40 |
| 6198 | RPS6KB1 | UUUGUAAA | 2531 | 95.44 | -3.40 |

Accordingly, the miRvestigator analysis was repeated with less stringent levels of complementarity (motif size of 8bp, default Weeder model, seed model of 8mer, 95% complementarity homology and 0.25 wobble base-pairing allowed). This analysis identified a further 10-12 additional targets (CEBPD, CREB1, PRKAA1, TWIST1, INSR, IRS1, NCOA1, NCOA2, NCOA3, KLF5, RPS6KB1, NRIP1) with 95% similarity to the consensus hsa-miR-19 motif. Interestingly, hsa-miR-19 is among the most abundant miRNAs in adipose tissue. The genes identified as containing a sequence complementary to hsa-miR-19 seed region are set forth in Table 17.

**Table 17.**

| **Thermogenic regulators identified as targets for hsa-miR-19a/b with 95% to 100% similarity to consensus motif:** | | | | | |
|---|---|---|---|---|---|
| **Gene** | **Gene symbol** | **Sequence of Site** | **Start Relative to Stop Codon (bp)** | **% Similarity to Consensus Motif (Quality = High \|Medium\|Fair)** | **Minimum Free Energ (MFE) of mRNA-miRNA Duplex** |
| 650 | BMP2 | UUUGUACA | 386 | | -6.80 |
| 1052 | CEBPD | UUUGUAAA | 263 | 95.42 | -3.40 |
| 7132 | TNFRSF1A | UUUGUACA | 510 | 100.00 | -6.80 |
| 4040 | LRP6 | UUUGUACA | 151 | 100.00 | -6.80 |
| 4040 | LRP6 | UUUGUAAA | 4965 | 95.42 | -3.40 |
| 5562 | PRKAA1 | UUUGUAAA | 2400 | 95.42 | -3.40 |
| 5563 | PRKAA2 | UUUGUACA | 542 | 100.00 | -6.80 |
| 655 | BMP7 | UUUGUACA | 1927 | 100.00 | -6.80 |
| 652 | BMP4 | UUUGUACA | 770 | 100.00 | -6.80 |
| 133522 | PPARGC1B | UUUGUACA | 7199 | 100.00 | -6.80 |
| 1814 | E2F4 | UUUGUACA | 755 | 100.00 | -6.80 |
| 7474 | WNT5A | UUUGUACA | 1414 | 100.00 | -6.80 |
| 6720 | SREBF1 | UUUGUACA | 510 | 100.00 | -6.80 |
| 7291 | TWIST1 | UUUGUAAA | 649 | 95.42 | -3.40 |
| 3667 | IRS1 | UUUGUAAA | 992 | 95.42 | -3.40 |
| 10499 | NCOA2 | UUUGUAAA | 1381 | 95.42 | -3.40 |
| 8204 | NRIP1 | UUUGUACA | 1718 | 100.00 | -6.80 |
| 8204 | NRIP1 | UUUGUAAA | 1935 | 95.42 | -3.40 |
| 8202 | NCOA3 | UUUGUAAA | 965 | 95.42 | -3.40 |
| 3643 | INSR | UUUGUAAA | 2105 | 95.42 | -3.40 |
| 8013 | NR4A3 | UUUGUACA | 2347 | 100.00 | -6.80 |
| 6776 | STAT5A | UUUGUACA | 1214 | 100.00 | -6.80 |
| 688 | KLF5 | UUUGUAAA | 549 | 95.42 | -3.40 |
| 8648 | NCOA1 | UUUGUAAA | 381 | 95.42 | -3.40 |
| 860 | RUNX2 | UUUGUACA | 2425 | 100.00 | -6.80 |
| 1385 | CREB1 | UUUGUAAA | 1973 | 95.42 | -3.40 |
| 1385 | CREB1 | UUUGUACA | 2822 | 100.00 | -6.80 |
| 1385 | CREB1 | UUUGUAAA | 4175 | 95.42 | -3.40 |
| 6198 | RPS6KB1 | UUUGUAAA | 2531 | 95.42 | -3.40 |

Without wobbling, the same motif 5'-UUUGUACA-3' is overrepresented among targets of hsa-miR-1283 with a complementarity p value of 1.4 x10⁻⁴. Furthermore, hsa-miR-1283 binds to other mRNAs of interest like ABCA1 (cholesterol transporter), the adiponectin receptor and the transcription factor TCF7L2 that is implicated in genetic human obesity.

Similarly, other miRNA over-represented seed sequences were identified for miRNAs expressed in adipocytes. They include the universal hsa-let-7 family (sequence CUAUACAA, p value = 7.5e-04) and the adipocyte-rich hsa-miR-30 family (sequence UGUAAACA, p value = 1.9 x 10⁻³) to name a few.

With respect to PRDM16, CIDEA, NRIP1, KDM3A, CEPPB, PPARG, PPARGC1A, and PPKAA2, which according to the STRING software package are directly linked to UCP1, it appears that all of them share (at motif size 8bp, default Weeder model, seed model 8mer, 95% complementarity homology and 0.25 wobble base-pairing allowed) a consensus sequence with several miRNAs, including hsa-miR-3658 (p value = 1.9e-003) and the hsa-miR-30 family (p value = 6.3e-003) as follows:
**hsa-miR-3658:** 
Motif 5' UUUUUUAC- 3'
||:||||
3' -AAGAAUUU 5' miRNA Seed
**hsa-miR-30a/b/c/d/e:**

Motif 5' UUUUUUAC— 3'
|||||||
3' —ACAAAUGU 5' miRNA Seed

### E) An intronic miRNA-multiple mRNAs pathway-specific paradigm.

Many mammalian miRNAs are located within introns of protein-coding genes rather than in their own unique transcription units. Intronic miRNAs are typically expressed and processed with the precursor mRNA in which they reside. Although the intronic miRNAs and their host genes can be regulated independently, an intronic miRNA can down-regulate its own host protein-coding gene by targeting the host gene's UTR. Feedback regulation on host protein-coding genes could be achieved by selecting the transcription factors that are miRNA targets or by protein-protein interactions between intronic miRNA host gene product and miRNA target gene products. As an example, miR-33 acts in concert with the SREBP host genes to control cholesterol homeostasis and the pharmacological inhibition of miR-33a and miR-33b is a promising therapeutic strategy to raise plasma HDL and lower VLDL triglyceride levels for the treatment of dyslipidemias.

Examination of the 83 thermogenic target genes reveals two intronic miRNAs: miR-378 located in the PPARGC1B gene and miR-4251 located in the PRDM16 gene.

Mining of the Internet tools predicting miRNA targets indicates that miR-378 targets include BMP2, PPARA, PPARGC1A, PRDM16, STAT5 and WNT10A as well as ADIPOQ and IGFR1; and that miR-4251 targets include BMP2, CTBP1, CTBP2, MAPK14, NCOA3, PLAC8, PPARA, PPARD, TRPM8, as well as ABCA5, ABCA13, ADIPOQR2, KDM5B, KLF-12, KLF-14 and TCF7L2.

### Example 3. High-Content Cellular Phenotypic Screening

High-content screening methods are used to screen for novel miRNA agents that modulate the activity of thermogenic regulators (e.g., UCP1 and UCP2). High-content screening is a drug discovery method that uses images of living cells to facilitate molecule discovery. Such automated image based screening methods are particularly suitable for identifying agents which alter cellular phenotypes.

WAT cells contain large lipid droplets, whereas, in contrast, BAT cells contain numerous smaller droplets and a much higher number of mitochondria, which contain iron and make them appear brown. The large number of mitochondria in BAT leads to an increased oxygen consumption, when compared to WAT. Accordingly, it is possible to distinguish between BAT and WAT cells visually based on their cellular phenotype.

Accordingly, high-content screening methods were used to screen for novel miRNA agents that modulate the activity of thermogenic regulators. Specifically, the phenotypic appearance of cultured human adipocytes and adipose tissue derived mesenchymal stem cells grown in the presence and absence of miRNA agonists or antagonists was assessed over two weeks by phase contrast microscopy of the cultured cells, measurement of the cellular lipid content (using Oil Red O Staining or Nile Red fluorescence); mitochondrial content (e.g., using Life Technologies Mito-Tracker Red FM), and/or oxygen consumption *in vitro* (e.g., using the Seahorse Bioscience Extra-Cellular Flux Instrument). mRNA expression is measured by targeted q-RT-PCR, NanoString and universal RNA-Sequencing. Protein expression is measured by targeted Western Blotting and universal proteomic profiling.

### A. Differentiation of human pre-adipocytes into adipocytes.

### 1. Differentiation Protocol.

In order to assess the effect of miRNA analogs on human pre-adipocytes differentiation into mature adipocytes, human subcutaneous pre-adipocytes (SuperLot 0048 from 8 female donors, ZenBio, NC) were plated on Day 0 into 96-well plates and allowed to attach overnight in preadipocyte medium (DMEM/Ham's F-12 (1:1, v/v), HEPES buffer, Fetal bovine serum and Antibiotics). The next day (Day 1), the medium was removed and replaced with differentiation medium (DMEM/Ham's F-12 (1:1, v/v), 100 µM Ascorbic Acid, 0.85 µM insulin , 20 nM sodium selenite , 0.2 nM, tri-iodothyronine , 1 µM dexamethasone , 100 µM isobutyl-methylxanthine , 100 nM Rosiglitazone and Antibiotics. The cells were allowed to incubate for 2 days at 37°, 5% CO₂. After 2 days (Day 3), the medium was removed and replaced with fresh maintenance medium (DMEM/Ham's F-12 (1:1, v/v), 100 µM Ascorbic Acid, 0.85 µM insulin, 20 nM sodium selenite , 0.2 nM tri-iodothyronine, and Antibiotics). On Day 3, the cells were transfected with miRNA analogs (Dharmacon specific miRIDIAN Mimics and Hairpin Inhibitors) using the transfecting agent Dharmafect1. All treatments were in triplicate. Post transfection, the negative control was maintenance medium only and the positive control was maintenance medium with 100 nM of the PPARG agonist rosiglitazone. After 2 days, medium was removed and replaced with fresh maintenance medium. The maintenance medium then changed every two days until the end of the treatment period (Day 15). At the end of the treatment (total of 15 days in culture) cells were processed for Phenotyping and Genotyping Screening.

### 2. Transfection of pre-adipocytes.

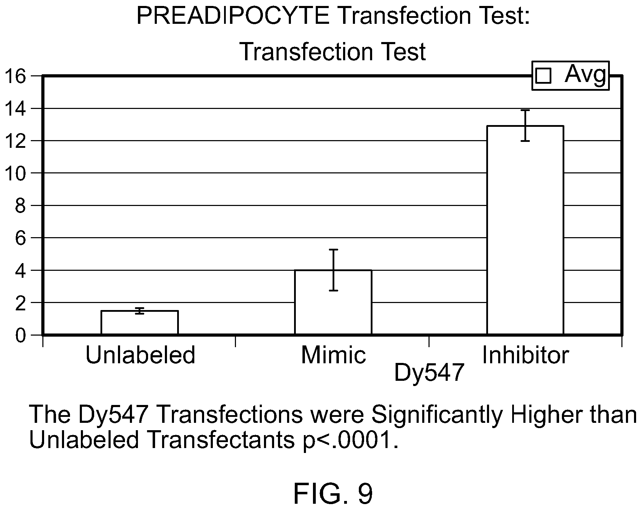

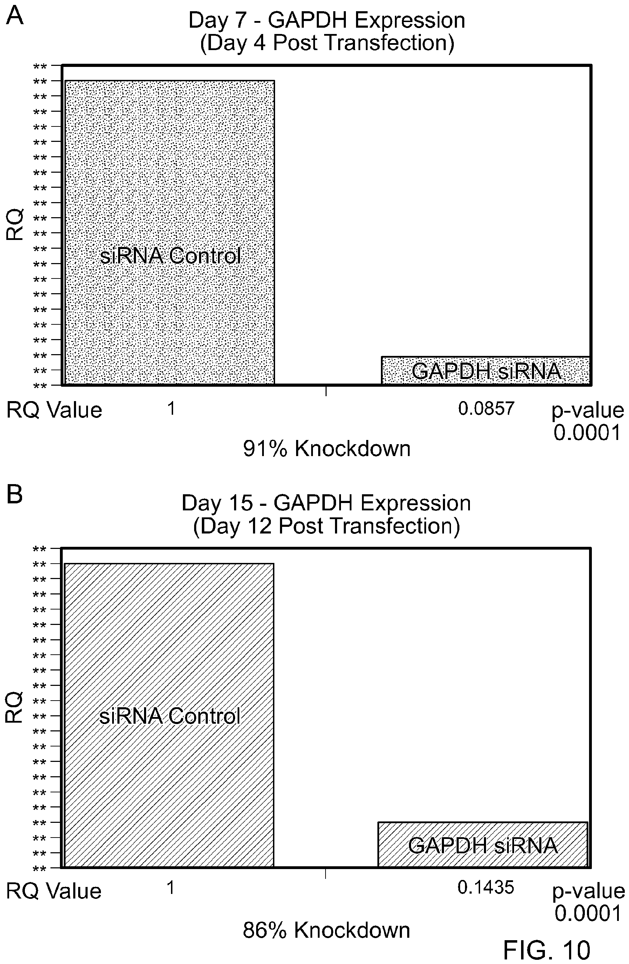

Transfection reagents are used to facilitate the penetration of miRNA analogs into target cells. As an example, the extent of transfection efficiency we achieved in pre-adipocytes with the transfecting agent Dharmafect 1 (Dharmacon, CO) is depicted herein. Transfection efficiency was assessed in two ways:
a. Measurement of cellular epifluorescence after transfection with fluorescent miRNA analogs.
   Fluorescence was measured on Day 15 (540 excitation/590 emission) in cells transfected on Day 3 with the Dy547-labeled non-targeting miRIDIAN Mimic and Hairpin Inhibitor (100 nM). As shown in Figure 9, there was a significantly greater fluorescence of cells transfected with the fluorescent miRNA analogs, even 12 days after transfection:
b. Reduction of control gene expression.
   To confirm successful transfection of pre-adipocytes, the reduction of expression of the control gene GAPDH ("housekeeping gene") was measured 4 days (Day 7) (Figure 10A) and 12 days (Day 15) (Figure 10B) after transfection of pre-adipocytes with a GAPDH-specific siRNA. Cell lysates were obtained and RT-PCR was conducted using pure RNA obtained by Cells-to-Ct reagents. 91% and 86% knockdowns of the GAPDH mRNA expression were observed at Day 4 and Day 12 post transfection, both highly significant, as shown in Figures 10A and 10B.

### 3. Phenotypic changes during human pre-adipocytes differentiation into adipocytes.

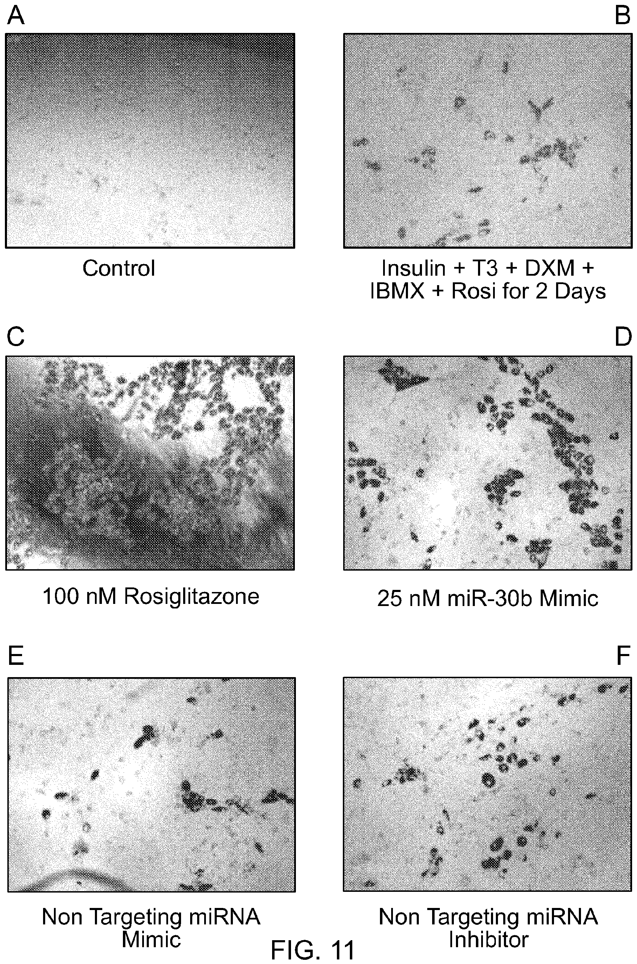

At the end of treatment (15 days in culture) cells were stained with Oil Red O for assessment of lipid content. As shown in Figures 11A to F, in the presence of medium without rosiglitazone, the pre-adipocytes show little differentiation into lipid-loaded mature adipocytes. In the presence of differentiation medium including 100 nM Rosiglitazone for 2 days followed by maintenance medium for 12 days (negative control), some differentiation into lipid-loaded mature adipocytes is noted. In the presence of 100 nM rosiglitazone throughout the experiment (positive control), most of the cells became lipid-loaded mature adipocytes. As an example, in the presence of 25 nM hsa-miR-30b mimic, about half of the cells became lipid-loaded mature adipocytes. The non-targeting miRNA mimic and inhibitor showed patterns similar to the negative control.

### 4. Genotypic changes during human pre-adipocyte differentiation into adipocytes.

Profiling of mRNA changes occurring during the differentiation of human pre-adipocyte into mature adipocyte induced by rosiglitazone or miRNA analogs was performed by RNA-Seq technology. Small RNA sequencing (RNA-Seq) is a high-throughput next-generation sequencing platform which now allows transcriptome-wide profiling of all small RNAs, known and unknown, with no need for prior sequence or secondary structure information.

RNA samples were extracted from pre-adipocytes (pre-adipocyte negative control) and from pre-adipocytes cultured in the presence of 100 nM rosiglitazone (differentiation positive control) or 25 nM miRNA mimics or inhibitors for 12 days. RNA sequencing was performed on the Illumina Hi-Seq 2000 equipment. The results were mapped against Human Genome 19 (http://genome.ucsc.edu/). It appears that in the presence of a miRNA analog, between 313 and 449 mRNA are significantly differentially expressed in reference to pre-adipocytes. In reference to Rosiglitazone, the number of significantly differentially expressed genes is reduced between 111 and 216, thus suggesting common pathways of activation of adipocyte differentiation between miRNAs and the PPARG analog.

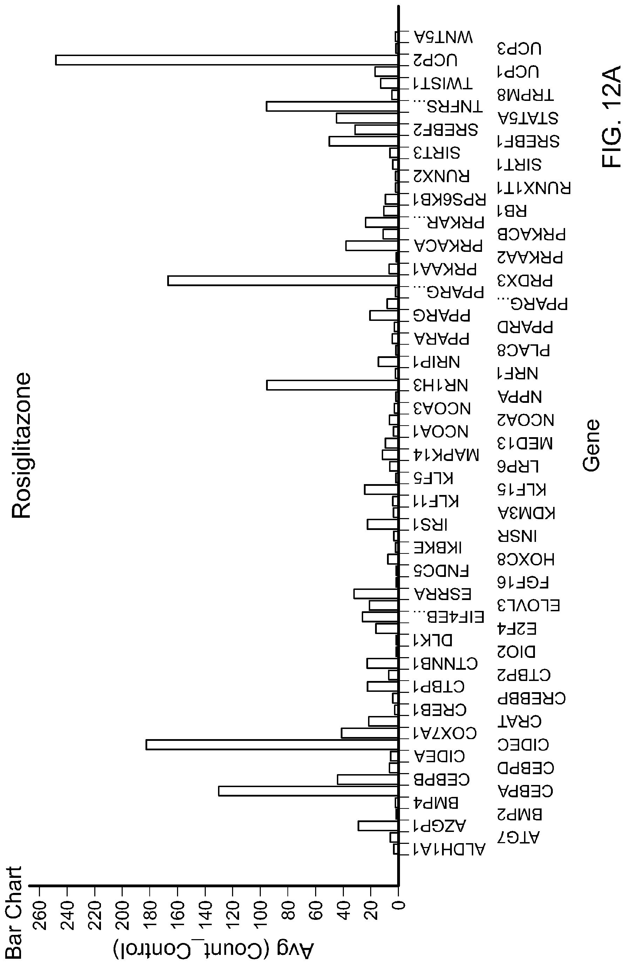

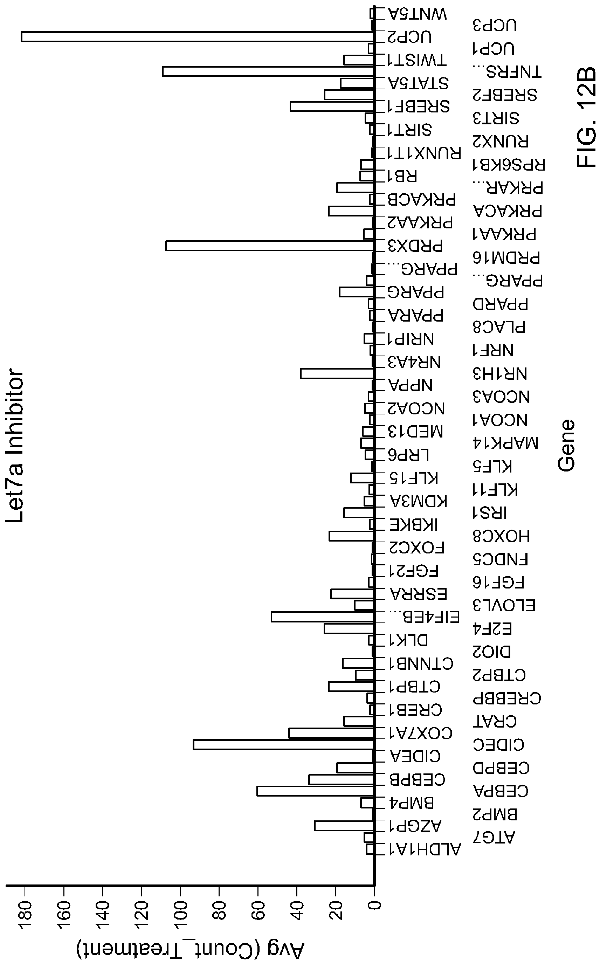

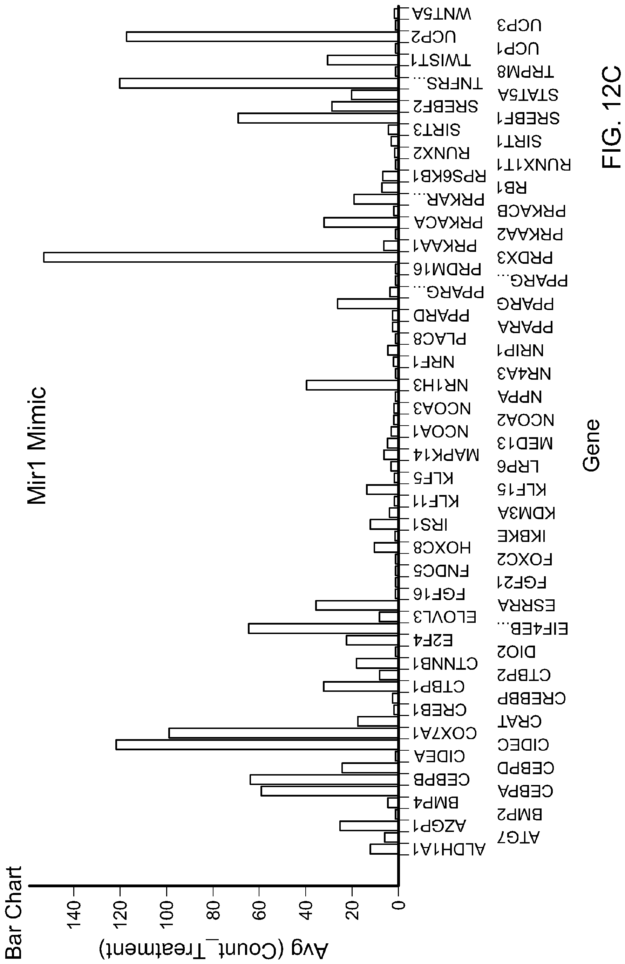

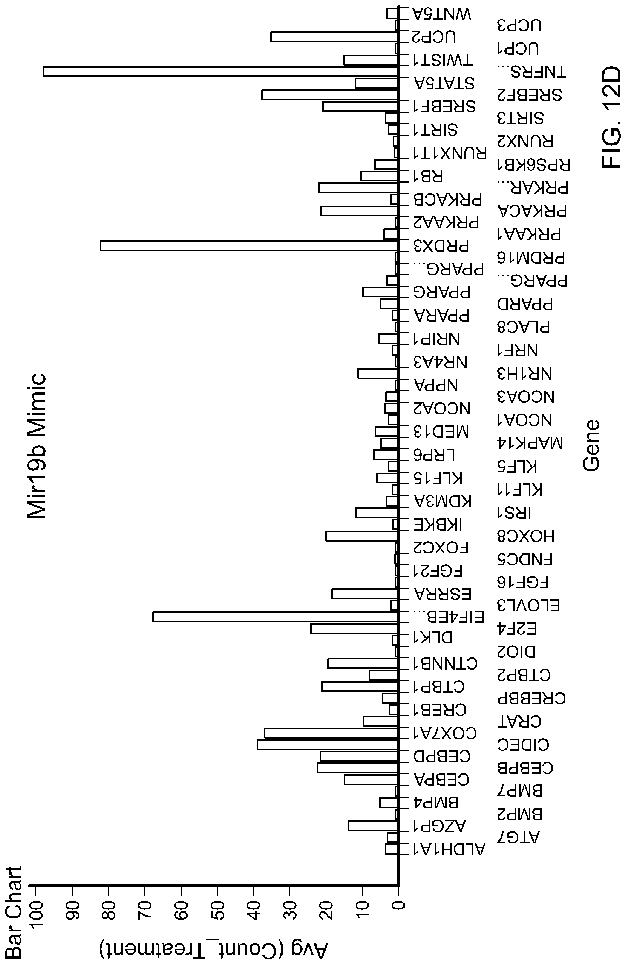

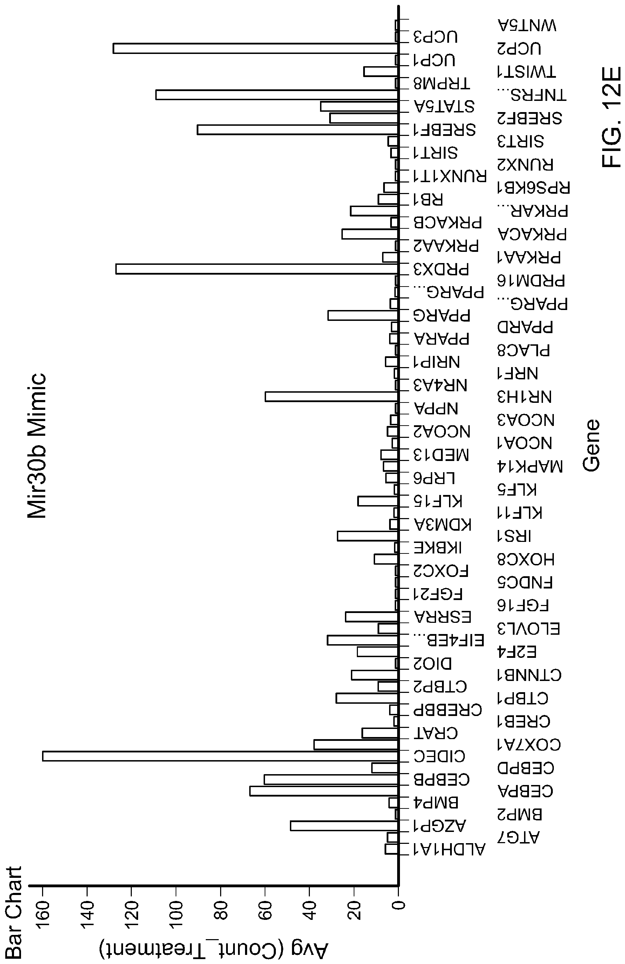

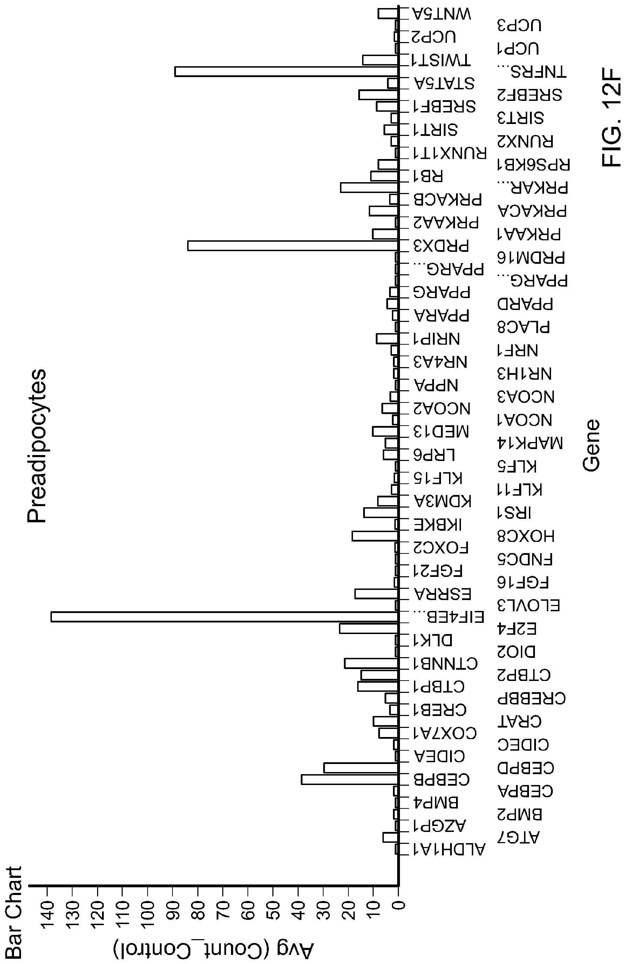

Regarding our 83 thermogenic activators and inhibitors, the expression of 73 of them is altered in the presence of rosiglitazone or miRNA analogs. The changes of mRNA expression of the thermogenesis targets in the presence of rosiglitazone (Figure 12A) or miRNA analogs hsa-let-7a inhibitor, hsa-miR-1 mimic, hsa-miR-19b mimic, hsa-miR-30b mimic or control adipocytes are shown on Figures 12B-F, respectively).

Changes in mRNA expression of UCP1, 2 and 3 were also measured in the presence of rosiglitazone or miRNA analogs, as shown below in Table 18.

**Table 18.**

| **Changes in thermogenic mRNA expression:** | | | |
|---|---|---|---|
| | **mRNA Expression changes (log ratios)** | | |
| **Agent** | **UCP1** | **UCP2** | **UCP3** |
| Rosiglitazone | 15.70 | 263 | 0.26 |
| hsa-let-7a inhibitor | 2.23 | 173 | 0.65 |
| hsa-miR-1 mimic | 0.41 | 110 | 0.40 |
| hsa-miR-19b mimic | 0.18 | 33 | 0.26 |
| hsa-miR-30b mimic | 0.76 | 119 | 0.28 |
| *Baseline level in pre-adipocytes* | 0.02 | 1.35 | 0.30 |

The expression levels of the three Uncoupling Proteins were low in pre-adipocytes. The expression of UCP1 was significantly increased in the presence of rosiglitazone 100 nM which was renewed with the culture medium every other day. The magnitude of UCP1 mRNA rise with the miRNA analogs was lower than with rosiglitazone, but one has to keep in mind the miRNA analogs concentration used (25 nM) and the fact that only one transfection was performed 12 days before RNA extraction. A major finding is the dramatic increase of UCP2 expression in the presence of rosiglitazone as well as the miRNA analogs. The expression of UCP3 did not change in any condition, as expected for a gene that is mainly expressed in myocytes. This increase in UCP1 and UCP2 expression suggests that administration of these miRNA produces a cellular differentiation into adipocytes with greater potential for thermogenesis and thus are likely effective pharmaceuticals for the treatment of obesity and other metabolic diseases and disorders.

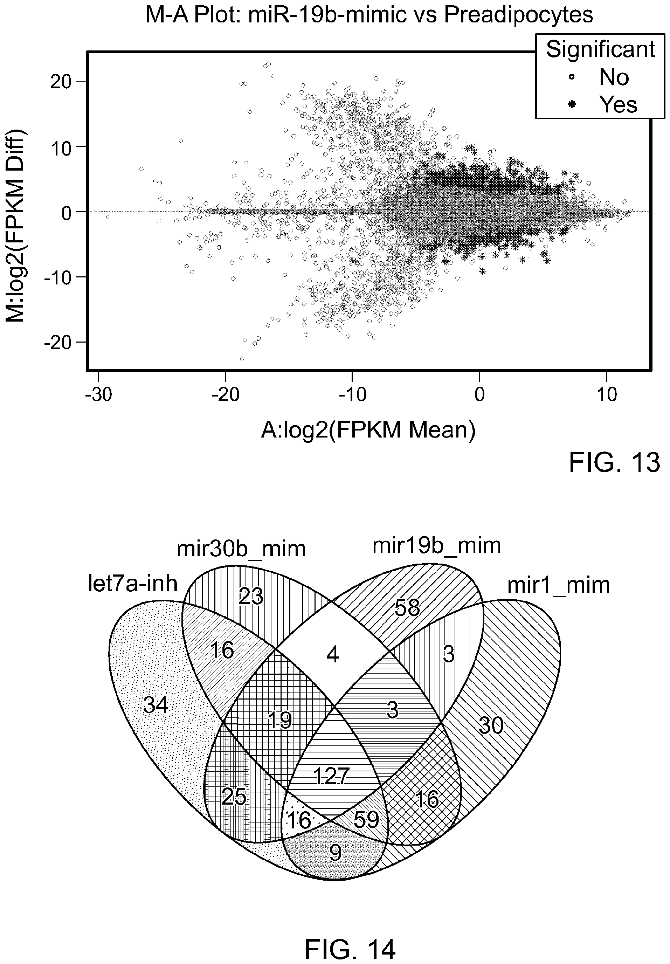

Furthermore, we looked at genes differentially expressed during pre-adipocyte culture in the presence of miRNA analogs. As an example shown on Figure 13, an M-A plot was created to visualize the differences of mRNA expression between pre-adipocytes grown in maintenance medium and pre-adipocytes grown in the presence of hsa-miR-19b mimic. The x-axis is the mean gene expression and the y-axis is the difference between pairs in logarithmic scale. The red dots are the differentially expressed genes (up regulated above zero and down regulated below zero). The gray dots are the genes not differentially expressed between control and hsa-miR-19b mimic (up regulated above zero and down regulated below zero).

As an example shown on Figure 14, in reference to pre-adipocytes cultured in maintenance medium only, the numbers of significantly differentially expressed genes in the presence of the miRNA analogs hsa-let-7a inhibitor, hsa-miR-1 mimic, hsa-miR-19b mimic and hsa-miR-30b mimic were respectively 406, 382, 370 and 433. A set of 127 genes was commonly upregulated by these 4 miRNA analogs (Venn Diagram, Figure 14).

They include not only some of our 83 thermogenic targets like ALDH1A1, AZGP1, CEBPA, PPARGC1A, UCP1 and UCP2, but also numerous genes involved in lipid metabolism and adipocyte differentiation (Table 19).

**Table 19.**

| **Set of 127 genes commonly upregulated by 4 miRNA analogs:** | | | |
|---|---|---|---|
| ABCC6 | CHI3L2 | KCNE3 | PPL |
| ABCD2 | CILP | KCNK3 | PPP1R1A |
| ACACB | CKB | KIT | PRKAR2B |
| ACHE | CKMT1B | KLB | PTGDS |
| ACSF2 | CLCA2 | LBP | QPRT |
| ACSM5 | CLMN | LEP | RASL12 |
| ACSS2 | COL14A1 | LGALS12 | RNF157 |
| ADH1B | COL21A1 | LIPE | S100B |
| AIF1L | CPB1 | LPL | SDPR |
| AKR1C3 | CYB5A | LRRC4C | SELENBP1 |
| ALDH1A1 | CYP4F12 | LRRN4CL | SEMA3G |
| AOC3 | CYP4F22 | MAN1C1 | SEPP1 |
| AOC4 | DARC | MAOA | SLC2A4 |
| APCDD1 | DGAT2 | MAOB | SLC2A5 |
| APOC1 | DHCR24 | MARCO | SLC40A1 |
| AQP3 | DPT | MCAM | SLCO4C1 |
| AQP7 | DTX4 | METTL7A | SMOC2 |
| AQP9 | EPHB6 | MGP | SNCG |
| AZGP1 | FABP4 | MLXIPL | SPARCL1 |
| BBOX1 | FADS2 | MOBKL2B | SPRY1 |
| BHLHE22 | FAM65C | MOSC1 | SVEP1 |
| C11orf87 | FMOl | MVD | TF |
| C14orf180 | FMO2 | NAT8L | TM7SF2 |
| C1orf115 | G0S2 | NKD2 | TMEM132C |
| C1orf95 | GPD1 | PCSK9 | TMEM176B |
| C3 | GPR109A | PFKFB1 | TMEM37 |
| CA2 | GPR109B | PKD1L2 | TNMD |
| CADM3 | HAVCR2 | PLA2G2A | TPRG1 |
| CDO1 | HRASLS5 | PLIN1 | TRIL |
| CEBPA | IGSF10 | PLIN4 | UCP1 |
| CFD | ITIH1 | PLXDC1 | UCP2 |
| CFHR1 | ITIH5 | PPARGC1A | |

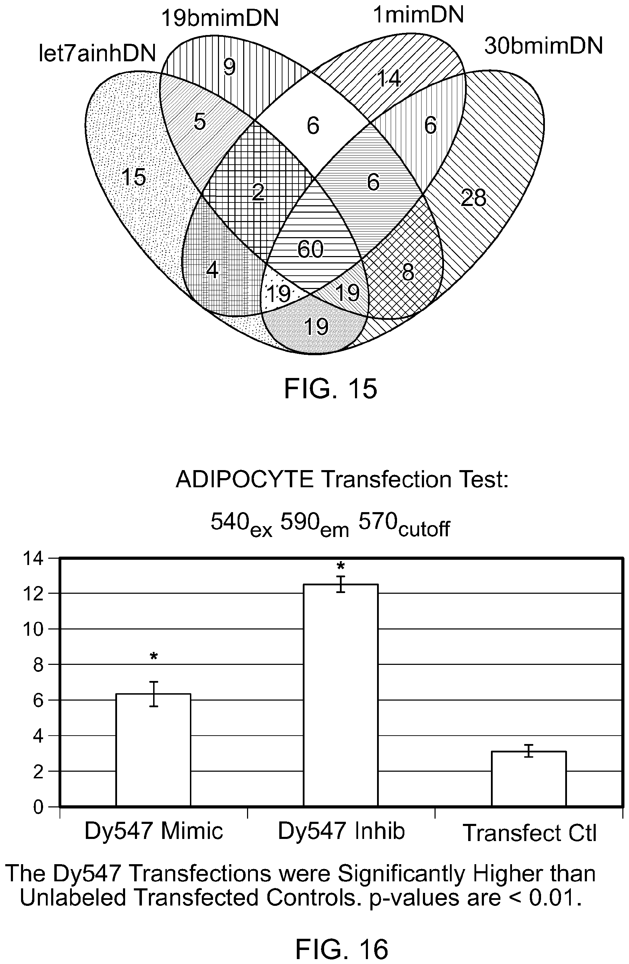

A set of 60 genes was commonly downregulated by these 4 miRNA analogs (Venn Diagram, Figure 15).

They include numerous chemokines genes and genes involved in cell proliferation and (Table 20).

**Table 20.**

| **Set of 60 genes commonly downregulated by 4 miRNA analogs:** | | | |
|---|---|---|---|
| ACTC1 | CENPF | ID1 | KRTAP2-1 |
| ANLN | CKAP2L | ID3 | MALL |
| ARSI | CXCL1 | IER3 | MMP3 |
| ATOH8 | CXCL2 | IL13RA2 | NCAPH |
| AURKB | CXCL3 | IL6 | PHLDA1 |
| BLM | CXCL5 | IL8 | PLK1 |
| BRCA2 | CXCL6 | INHBA | PPAPDC1A |
| BUB1 | E2F7 | IQGAP3 | PTGS2 |
| BUB1B | ESCO2 | KIAA1244 | RELN |
| CASC5 | FAM83D | KIF11 | SHCBP1 |
| CCL26 | GABBR2 | KIF14 | SLC17A9 |
| CDC6 | GREM2 | KIF18B | SLC6A17 |
| CDCA5 | GTSE1 | KIF2C | THBD |
| CDCA8 | HAS1 | KIFC1 | TMSL3 |
| CDH15 | HJURP | KRT34 | TOP2A |

### B. Differentiation of human white adipocytes into brown adipocytes.

### 1. Differentiation Protocol.

In order to assess the effect of miRNA analogs on human white adipocytes differentiation into brown adipocytes, human subcutaneous pre-adipocytes (SuperLot 0048 from 8 female donors, ZenBio, NC) were plated on Day 0 into 96-well plates and allowed to attach overnight in preadipocyte medium (DMEM/Ham's F-12 (1:1, v/v), HEPES buffer, Fetal bovine serum and Antibiotics). The next day (Day 1), the medium was removed and replaced with differentiation medium-2 (DMEM/Ham's F-12 (1:1, v/v), HEPES buffer, Fetal bovine serum, Biotin, Pantothenate, Human insulin, Dexamethasone, Isobutyl-methylxanthine, Proprietary PPARG agonist and Antibiotics. The cells were allowed to incubate for 7 days at 37° C, 5% CO₂. After 7 days (Day 7), a partial medium exchange was performed with AM-1 adipocyte maintenance medium (DMEM/Ham's F-12 (1:1, v/v), HEPES buffer, Fetal bovine serum, Biotin, Pantothenate, Human insulin, Dexamethasone and Antibiotics). The cells were allowed to incubate for an additional 7 days at 37° C, 5% CO₂. On Day 17, the cells were transfected with miRNA analogs (Dharmacon specific miRIDIAN Mimics and Hairpin Inhibitors) using the transfecting agent Dharmafect 3. All treatments were in triplicate. Post transfection, the negative control was maintenance medium only and the positive control was maintenance medium with 100 nM of the PPARG agonist rosiglitazone. After 2 days, medium was removed and replaced with fresh maintenance medium. The maintenance medium then changed every two to three days until the end of the treatment period (Day 30). At the end of the treatment (total of 30 days in culture) cells were processed for Phenotyping and Genotyping Screening.

### 2. Transfection of adipocytes.

Transfection reagents are used to facilitate the penetration of miRNA analogs into target cells.

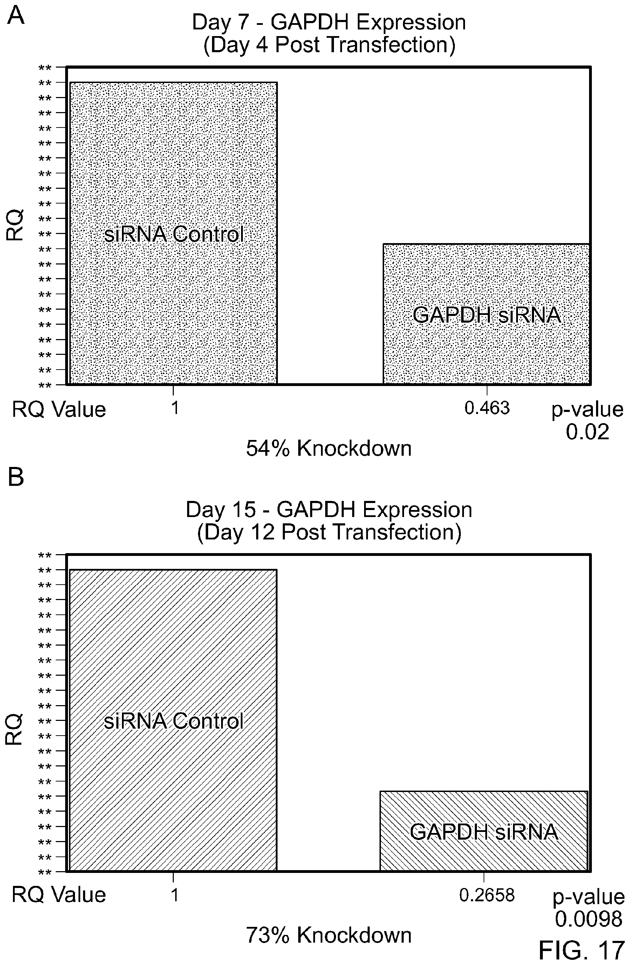

As an example, the extent of transfection efficiency we achieved in adipocytes with the transfecting agent Dharmafect 3 (Dharmacon, CO) is depicted herein. Transfection efficiency was assessed in two ways:
a. Measurement of cellular epifluorescence after transfection with fluorescent miRNA analogs.
   Fluorescence was measured on Day 30 (540 excitation/590 emission) in cells transfected on Day 17 with the Dy547-labeled non-targeting miRIDIAN Mimic and Hairpin Inhibitor (100 nM). As shown in Figure 16, there was a significantly greater fluorescence of cells transfected with the fluorescent miRNA analogs, even 12 days after transfection.
b. Reduction of control gene expression.
   To confirm successful transfection of adipocytes, the reduction of expression of the control gene GAPDH ("housekeeping gene") was measured 4 days (Day 22) and 12 days (Day 30) after Dharmafect 3 (Dharmacon, CO) mediated transfection of adipocytes with a GAPDH-specific siRNA. Cell lysates were obtained and RT-PCR was conducted using pure RNA obtained by Cells-to-Ct reagents. Efficient transfection of mature adipocytes (a cell type known to be difficult to transfect) was achieved with the transfecting agent Dharmafect 3.54% and 73% knockdowns of the GAPDH mRNA expression were observed at Day 4 and Day 12 post transfection, both highly significant, as shown in Figure 17.

### 3. Phenotypic changes during maintenance of human adipocytes in culture for thirty days.

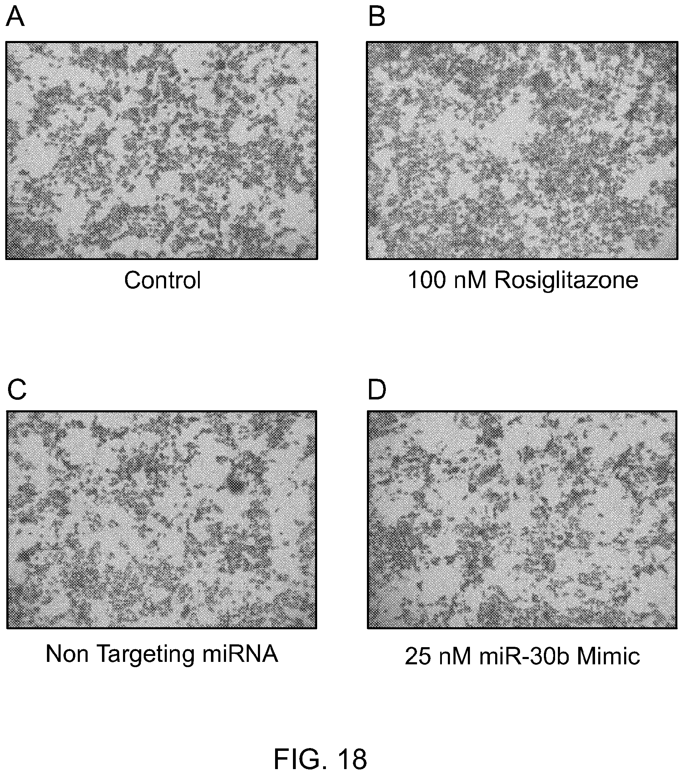

At the end of treatment (total of 30 days in culture) cells were stained with Oil Red O for assessment of lipid content (Figure 18). In the presence of maintenance medium only from Day 16 to Day 30 (control), the adipocytes appear loaded with large lipid droplets. In the presence of 100 nM rosiglitazone throughout the experiment (positive control), the intensity of the red staining seems reduced and the lipid droplets appear smaller. As an example, in the presence of 25 nM hsa-miR-30b mimic, the intensity of the red staining seems also reduced and the lipid droplets appear smaller. No such change was notice in the presence of a non targeting miRNA analog.

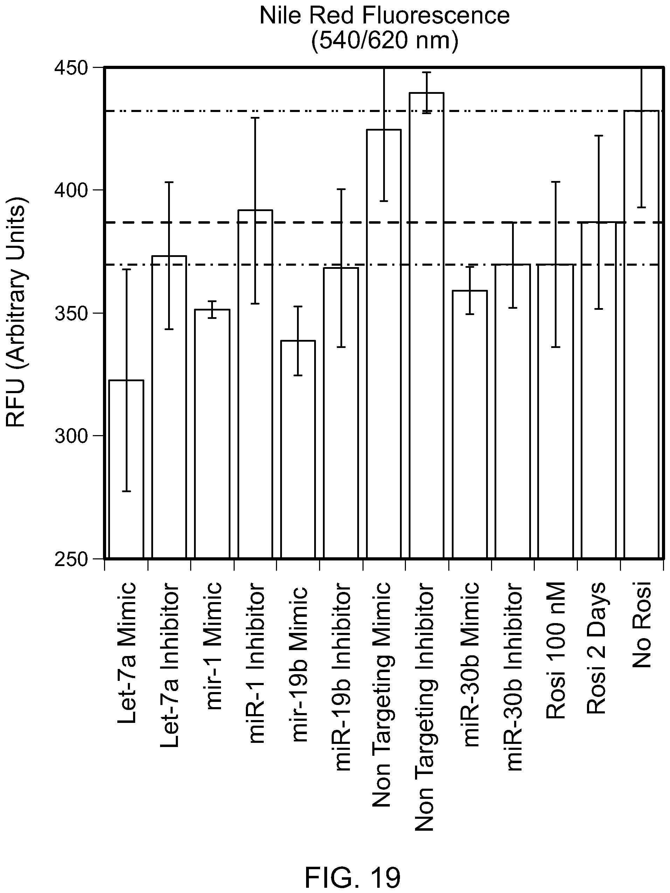

The amount of lipids present in the mature adipocytes at Day 30 was measured with the fluorescent Nile Red Dye. As shown in Figure 19, the highest fluorescence was noted in the adipocytes which were not exposed to rosiglitazone from Day 15 to day 30. A similar fluorescence level was noted in the cells which were transfected with the non-targeting miRNA mimic and inhibitor. When the cells were exposed to rosiglitazone for two days, the fluorescence dropped significantly and was further reduced in the presence of rosiglitazone from Day 15 to Day 30. It appears that in the presence of the miRNA inhibitors tested, the level of fluorescence is within the range observed with rosiglitazone 2 day to throughout. In the presence of miRNA mimics, the level of fluorescence appears lower, an indication of lower lipid content.

### 4. Optimization of human mature adipocyte transfection.

As efficient transfection of mature adipocytes is known to be difficult to achieve, we tested eleven different transfecting agents and assessed the degree of reduction of mRNA expression of the control gene GAPDH. Human subcutaneous pre-adipocytes were plated in 6-well plates and differentiated for two weeks following the protocol described above. Subsequently, a miRNA mimic (50 nM) targeting GAPDH was introduced into the differentiated adipocytes using transfecting agents following their manufacters' protocol. The transfected cells were incubated for 72 hours with reagents and miRNA mimic, then switched to maintenance medium. Fourteen days post-transfection, RNA was isolated using RNeasy Mini kit and RT-PCR reactions for the control gene GAPDH and the reference gene 18S were performed in triplicate using 100 ng of cDNA per well.

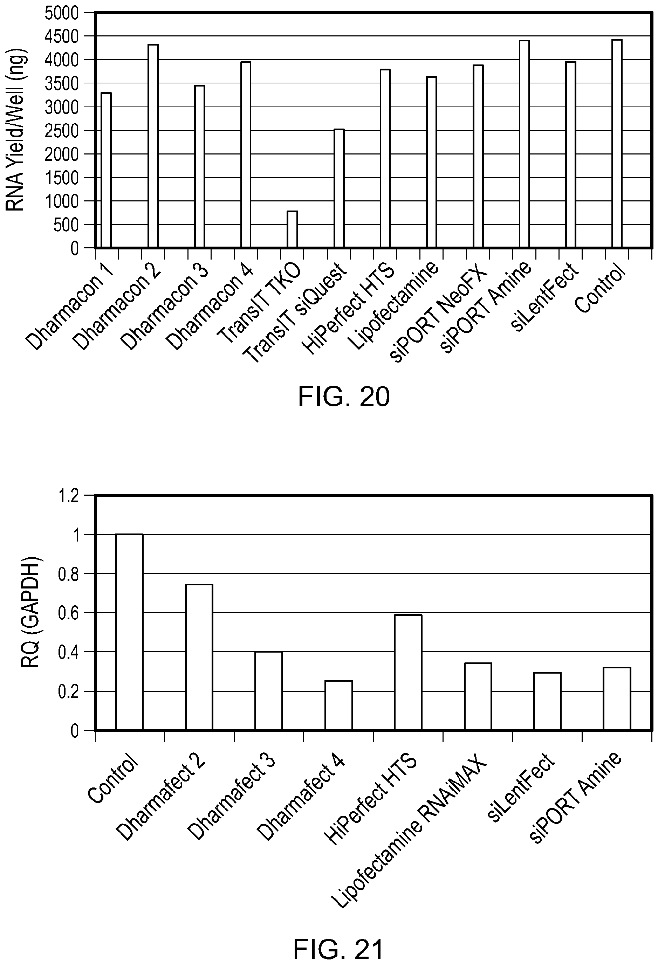

The amounts of RNA extracted per well were very similar, except for the transfecting agents TransIT TKO and TransIT siQuest which may produce potential cellular toxicity in the conditions of the experiment (Figure 20).

The cells transfected with Dharmafect 1 and siPORT NeoFX had significantly reduced levels of 18S expression and were excluded from the RT-PCR experiment analysis.
Among the remaining 7 transfecting agents analyzed, the often-used transfecting agent Lipofectamine RNAiMAX led to a 66% reduction of GAPDH expression at day 14 post-transfection, Dharmafect 3 and Dharmafect 4 respectively produced 60% and 75% reduction of GAPDH expression (Figure 21).

### 5. Phenotypic changes of human mature adipocytes cultured for two weeks in the presence of miRNA analogs or known activators of adipogenesis and/or thermogenesis.

Human subcutaneous adipocytes were plated in 6-well plates at a density of 391,000 cells per well as described above. Using Dharmafect 4, these adipocytes were transfected at Day 14 with:
1. One of the following miRNA analogs (50 nM):
   - hsa-let-7a inhibitor (hsa-let-7a is a universal miRNA reported to modulate adipogenesis)
   - hsa-miR-1 mimic (hsa-miR-1 has been reported to modulate PRDM16 and UCP1)
   - hsa-miR-19b mimic (hsa-miR-19b is an abundant adipocyte miRNA which according to our in silico work is predicted to interact with many of our 83 mRNA targets) or
   - hsa-miR-30b mimic (hsa-miR-30b is a miRNA which according to our in silico work is predicted to interact with many of our 83 mRNA targets and whose over-expression stimulates adipogenesis)
2. A negative control (mock transfection)
3. Three positive controls (the PPARG agonist rosiglitazone (100 nM), the beta 3 adrenergic receptor agonist CL316,243 (10µM) or the thyroid hormone tri-iodothyronine (10 nM) known to alter adipogenesis and/or adaptive thermogenesis).

At day 17, the cells were switched to maintenance medium, which was then changed every two-three days until day 28 when bright field microscopy pictures of the cells were taken.

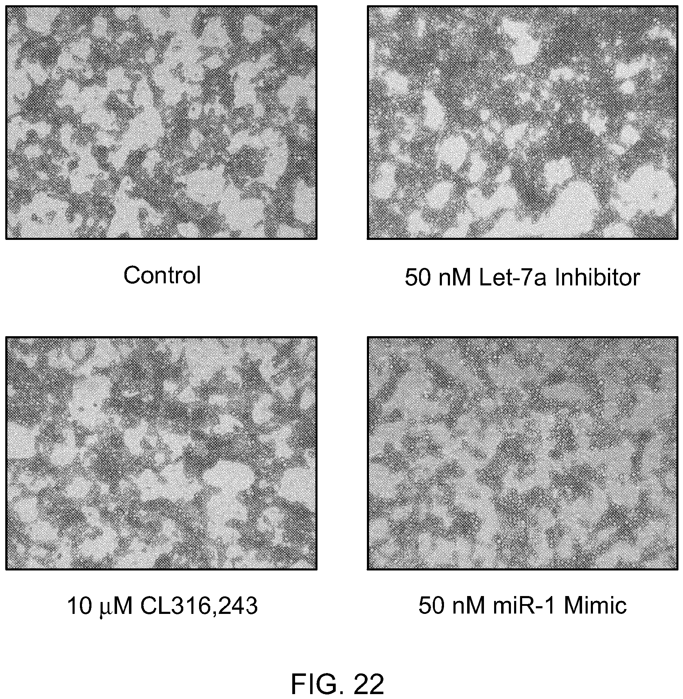

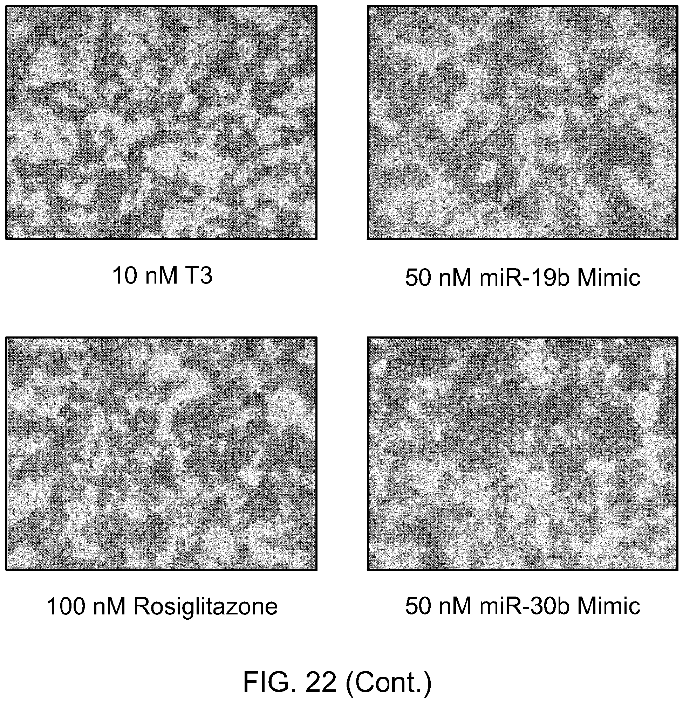

As shown on Figure 22, and in reference to the control condition, there is an increase in cell density and "browning" appearance in the presence of the positive controls CL316,243 and Rosiglitazone as well as in the presence of hsa-let-7a inhibitor, has-miR-19b and hsa-miR-30b mimic. The effects on cell density, lipid content, number and size of lipid droplets of the different agents are summarized in Table 21.

**Table 21.**

| **Agent** | **Picture field covered by adipocytes (% of control)** | **Cell area occupied by lipid droplets (%)** | **Number of lipid droplet per cell** | **Average size of lipid droplet** |
|---|---|---|---|---|
| Control | Ref | 42% | 68 | Ref |
| 50 nM hsa-let-7a inhibitor | + 147% | 56% | 103 | -13% |
| 10 µM CL316,243 | + 24% | 44% | 124 | -44% |
| 50 nM hsa-miR-1 mimic | + 15% | 36% | 69 | -16% |
| 10 nM T3 | + 13% | 42% | 94 | -27% |
| 50 nM hsa-miR-19b mimic | + 145% | 58% | 91 | +3% |
| 100 nM Rosiglitazone | + 198% | 57% | 113 | -19% |
| 50 nM hsa-miR-30b mimic | + 246% | 53% | 140 | -63% |

### Example 4. High-Throughput miRNA Target Screening by Luciferase Activity and qRT-PCR

High-throughput screening using luciferase reporter assay constructs are used to identify novel miRNA targets involved in thermogenesis.

Luciferase is commonly used as a reporter to assess the transcriptional activity in cells that are transfected with a genetic construct containing the luciferase gene under the control of a promoter of interest. SwitchGear Genomics has created a genome-wide library of over 18,000 human promoters and 12,000 human 3' UTR regions cloned into an optimized luciferase reporter vector system containing SwitchGear's RenSP reporter cassette (GoClone™) as a component of the LightSwitch™ Luciferase Assay System. This modified form of luciferase greatly facilitates detailed kinetic studies, especially those focusing on repression, which might otherwise be obscured by reporter protein accumulation.

The multiple microRNAs-one mRNA paradigm was tested with the SwitchGear Genomic GoClone system, using UCP1 as the single thermogenic target gene. In order to explore the possible interactions between various human miRNAs and the 3'UTR region, the 5'UTR region and the promoter/enhancer region of the human UCP1 gene in Hela and HepG2 cells, three reporter constructs were made:
1. A human UCP1 3'UTR construct containing a reporter gene driven by a strong constitutive promoter (RPL10-prom) with a 2,218 bp 3'UTR fragment of the human UCP1 sequence cloned in the 3'UTR region of the reporter gene. The effects of a specific miRNA mimic, inhibitor, or non-targeting control on this reporter's activity are compared to those of an empty-3'UTR and an Actin Beta-3'UTR to identify effects that are specific to the putative UCP1 3'UTR construct.
2. A human UCP1 Promoter construct containing a reporter gene driven by a 4,147 bp 5'UTR fragment of the human UCP1 sequence that spans the Transcription Start Site and upstream region covering the methylation region and the enhancer region of the human UCP1 gene sequence. The effects of a specific miRNA mimic, inhibitor, or non-targeting control on this reporter's activity are compared to those of an Actin Beta-Promoter to identify effects that are specific to the putative UCP1 5'UTR construct.
3. A human UCP1 Enhancer Region construct containing a reporter gene driven by a short minimal promoter from the HSV-TK locus with a 601 bp 5'UTR fragment of the human UCP1 sequence that spans the Enhancer Region of the human UCP1 gene sequence. The effects of a specific miRNA mimic, inhibitor, or non-targeting control on this reporter's activity are compared to those of an empty 5'Enhancer Region to identify effects that are specific to the putative UCP1 5'Enhancer construct.

In addition, miRNAxxx_3'UTR constructs were made. They contain the reporter gene driven by a strong promoter (RPL10_prom) with a perfect match to the target sequence of miRNAxxx cloned into the 3'UTR region of the reporter gene. The effect of a miRNA mimic, inhibitor, or non-targeting control on this reporter's activity can be compared to EMPTY_3'UTR and Actin B_3'UTR to determine whether a miRNA mimic's or inhibitor's activity can be reasonably detected in the experimental cell type. If the cell type has no endogenous expression of the miRNA in question, the addition of a mimic should knock down the activity of this reporter, and the addition of an inhibitor should have no significant effect. If the cell type has high endogenous expression of the miRNA in question, the addition of an inhibitor should increase the activity of this reporter, and the addition of a mimic should have no significant effect. The range of endogenous miRNA expression in Hela and HepG2 cell types is broad, so the synthetic target activity changes are likely to reflect this variability.

For each miRNA candidate (38 in total), the following conditions were tested:
- miRNA mimic (specific) ^{∗} 8 reporter constructs in Hela cells
- miRNA mimic (specific) ^{∗} 8 reporter constructs in HepG2 cells
- miRNA mimic non-targeting control ^{∗} 8 reporter constructs in Hela cells
- miRNA mimic non-targeting control ^{∗} 8 reporter constructs in HepG2 cells
- miRNA inhibitor (specific) ^{∗} 8 reporter constructs in Hela cells
- miRNA inhibitor (specific) ^{∗} 8 reporter constructs in HepG2 cells
- miRNA inhibitor non-targeting control ^{∗} 8 reporter constructs in Hela cells
- miRNA inhibitor non-targeting control ^{∗} 8 reporter constructs in HepG2 cells

To the extensive list of miRNAs that may bind to the UCP1 sequence, 10 filters were applied (in addition to required binding to UCP1 3'UTR region) to reduce the number of miRNA candidates to be tested. These filters were length of binding sites, number of binding sites, binding to the 5'UTR region, chromosomal clustering with other miRNAs, intronic location, wobbling, expression across species, binding to the Enhancer Region, binding to the Methylation Region and proof of experimental evidence of a relation to UCP1. 38 miRNAs that met at least 3 of these criteria were tested (Table 22).

**Table 22.**

| **miRNA with putative binding sites in the UCP1 gene sequence:** | | | | | | | | | | | | | |
|---|---|---|---|---|---|---|---|---|---|---|---|---|---|
| | **miRNA** | **# of criteria** | **Binding length** | **# of sites** | **3'UTR** | **5'UTR** | **Chr. Clusters** | **Intronic** | **Wobbling** | **Inter-species** | **Enhancer Region** | **Methylation Region** | **Exp. Evidence** |
| 1 | hsa-miR-130b-5p | 7 | 11 | 3 | + | + | 22 | | | | + | + | + |
| 2 | hsa-miR-328 | 6 | 10 | 4 | + | + | | | | | + | + | + |
| 3 | hsa-miR-655 | 6 | 10 | 5 | + | + | 14 | | | | | + | + |
| 4 | hsa-miR-19b-2-5p | 5 | 10 | 4 | + | + | X | | + | | | | + |
| 5 | hsa-miR-26a-2-3p | 5 | 10 | 7 | + | + | | | | | | + | + |
| 6 | hsa-miR-367-3p | 5 | 10 to 18 | 3 | + | + | 4 | | + | + | + | | |
| 7 | hsa-miR-371a-5p | 5 | 10 to 12 | 9 | + | + | 19 | | | + | | | + |
| 8 | hsa-miR-377-3p | 5 | 10 to 14 | 5 | + | + | 14 | | | + | | | + |
| 9 | hsa-miR-378a-3p | 5 | 7 to 13 | 19 | + | + | | + | + | + | | | + |
| 10 | hsa-miR-382-3p/5p | 5 | 15 | 2 | + | + | 14 | | + | + | | | |
| 11 | hsa-miR-421 | 5 | 10 | 5 | + | + | X | | | | | | + |
| 12 | hsa-miR-515-3p | 5 | 9 | 3 | + | + | 19 | | | | + | | + |
| 13 | hsa-miR-620 | 5 | 10 | 7 | + | + | | | | | + | | + |
| 14 | hsa-miR -941/2 | 5 | 9 | 5 | + | + | 20 | | | | | + | |
| 15 | hsa-miR-1179 | 4 | 11 | 3 | + | + | 15 | | + | | | | |
| 16 | hsa-miR-1302 | 4 | 10 | 5 | + | + | | | | | | + | |
| 17 | hsa-miR -146a | 4 | 9 to 10 | 8 | + | + | | | | | | | + |
| 18 | hsa-miR-181c | 4 | 9 | 5 | + | + | 19 | | | | | | + |
| 19 | hsa-miR-203 | 4 | 9 | 1 | + | | 14 | | | | | + | + |
| 20 | hsa-miR-331-5p | 4 | 8 to 15 | 6 | + | + | 12 | | + | + | | | |
| 21 | hsa-miR-422a | 4 | 7 to 14 | 6 | + | + | | | + | + | | | + |
| 22 | hsa-miR-452 | 4 | 8 | 7 | + | + | X | | | | | | + |
| 23 | hsa-miR-491-5p | 4 | 10 | 3 | + | + | | | | | | | |
| 24 | hsa-miR-501-3p | 4 | 10 | 2 | + | + | X | | | | | | + |
| 25 | hsa-miR-543 | 4 | 10 to 14 | 4 | + | + | 14 | | + | + | | | |
| 26 | hsa-miR-545 | 4 | 11 | 2 | + | + | X | | | | | | + |
| 27 | hsa-miR-549 | 4 | 13 to 14 | 3 | + | + | | | | + | | | + |
| 28 | hsa-miR-643 | 4 | 10 to 14 | 9 | + | + | | | + | | | | + |
| 29 | hsa-miR -651 | 4 | 10 | 6 | + | + | | | | | | | + |
| 30 | hsa-miR-654-3p | 4 | 8 to 10 | 11 | + | + | 14 | | + | | | | |
| 31 | hsa-miR-21-5p | 3 | 10 to 14 | 2 | + | + | | | + | + | | | + |
| 32 | hsa-miR-211-5p | 3 | 11 | 1 | + | | | | | | | + | + |
| 33 | hsa-miR-22-3 | 3 | 9 | 5 | + | + | | | + | | | | + |
| 34 | hsa-miR-30b-5p | 3 | 10 | 1 | + | | 8 | | | | | | + |
| 35 | hsa-miR-325 | 3 | 7 to 8 | 11 | + | + | | | | | | | + |
| 36 | hsa-miR-362-5p | 3 | 10 | 1 | + | | X | | | | | | + |
| 37 | hsa-miR-504 | 3 | 9 | 2 | + | + | | | | | | + | + |
| 38 | hsa-miR-552 | 3 | 9 | 3 | + | + | | | | | | | + |

In these Luciferase reporter gene assay experiments, a miRNA candidate was considered to interact with UCP1 if both the specific miRNA inhibitor increases the luciferase signal and the specific miRNA mimic decreases the luciferase signal with an Inhibitor/Mimic Ratio ≥ 1.5 and or/a p value < 0.05. These selection criteria identify 9 miRNAs (hsa-miR-19b-2-5p, hsa-miR-21-5p, hsa-miR-130b-5p, hsa-miR-211, hsa-miR-325, hsa-miR-382-3p/5p, hsa-miR-543, hsa-miR-515-3p, and hsa-miR-545) (Table 23). A few more barely missed these selection criteria; they are hsa-miR-331-5p, hsa-miR-552, hsa-miR-620, and hsa-miR-1179.

**Table 23.**

| **miRNA identified as regulators of UCP1 gene expression by luciferase reporter assay in Hela and/or HepG2 cells:** | |
|---|---|
| **Cell Line(s)** | **miRNA** |
| Hela | hsa-miR-130b-5p |
| Hela + HepG2 | hsa-miR-19b-2-5p |
| HepG2 | hsa-miR-382-3p/5p |
| Hela | hsa-miR-515-3p |
| Hela | hsa-miR-543 |
| HepG2 | hsa-miR-545 |
| Hela + HepG2 | hsa-miR-21-5p |
| Hela | hsa-miR-211-5p |
| Hela + HepG2 | hsa-miR-325 |

Out of these 9 selected miRNAs, 3 appear to bind to the 3 regions of UCP1 which were studied (hsa-miR-21-5p, hsa-miR-211, and hsa-miR-515-3p); 3 appear to bind to 2 regions of UCP1 (hsa-miR-19b-2-5p, hsa-miR-130b-5p, and hsa-miR-325), and 3 bind to a single region of UCP1 (hsa-miR-331-5p, hsa-miR-543, and hsa-miR-545). All but hsa-miR-331-5p appear to bind to the 3'UTR region of UCP1 (Table 24).

**Table 24.**

| **miRNA identified as regulators of UCP1 gene expression by luciferase reporter assay:** | | | | |
|---|---|---|---|---|
| | **miRNA** | **UCP1 3' UTR** | **UCP1 Enhancer** | **UCP1 Promoter** |
| 1 | hsa-miR-21-5p | X | X | X |
| 2 | hsa-miR-211 | X | X | X |
| 3 | hsa-miR-515-3p | X | X | X |
| 4 | hsa-miR-19b-2-5p | X | | X |
| 5 | hsa-miR-130b-5p | X | X | |
| 6 | hsa-miR-325 | X | X | |
| 7 | hsa-miR-331-5p | | X | |
| 8 | hsa-miR-543 | X | | |
| 9 | hsa-miR-545 | X | | |

Further screening is performed by transfection of the promoter/3'UTR library into human adipocytes or adipose-derived mesenchymal stem cells in cell culture, followed by addition of miRNA agents (e.g., agomirs or antagomirs) to the cell culture. Measurement of luciferase activity and identification of mRNAs is performed 24 hours after transfection and addition of miRNA agents.

In order to confirm the results of the transfection experiments set forth above over a longer time frame, lentiviral transduction experiments are performed using lentiviral vectors containing the miRNA agents of interest (from System Biosciences (SBI) collection of miRNA precursors expressed in the pMIRNA1 SBI vectors allowing the expression of the copGFP fluorescent marker). Specifically, cells containing the promoter/3'UTR library are transduced with lentiviral particles at an MOI of 1:10 and GFP-positive cells are sorted by FACS, according to the supplier's instructions. The level of expression of the mature miRNAs and their targeted mRNAs is assessed at several time points (0, 3, and 6 hr.; 1, 4, and 7 days) by *Taqman Quantitative Real-time PCR* in control cells (HEK293 cells), Human Adipose-Derived Mesenchymal Stem Cells, Human Subcutaneous Pre-adipocytes, and Human Proliferating Subcutaneous Adipocytes. Pooling of RNAs from 5 different time points after transduction is optionally employed to reduce the complexity of the qRT-PCR based screening approach while preserving the detection sensitivity.

### Example 5. Proteomic Profiling

Proteomic Profiling is also used to identify novel miRNA targets involved in thermogenesis.

Shotgun proteomics is a method of identifying proteins in complex mixtures using high performance liquid chromatography (HPLC) combined with mass spectrometry (MS). Transfected and transduced cells with miRNA agents and promoter/3'UTR library (as described in Example 4) are harvested and lysed to produce crude soluble (cytosolic) and insoluble (nuclear) fractions. Peptides are from these fractions are then separated by HPLC and analyzed using nanoelectrospray-ionization tandem MS using the isotopic labeling technique SILAC to quantify protein abundance. Spectra are searched against the Ensembl release 54 human protein-coding sequence database using Sequest (Bioworks version 3.3.1, Thermo Scientific).

To avoid missing low abundance proteins, a targeted proteomics approach is also employed to accurately quantify a set of proteins that are known regulators of adipogenesis, adipocyte differentiation and BAT function. Some examples include UCP1, KDM3A, PRDM16, PPARA, PPARGC1A, CEBPB, CIDEA, BMP7, COX7A1, SIRT1, SIRT3, DIO2, FABP4, and ADIPOQ. These proteins are analyzed via ELISA based or Luminex based immunoassays using commercially available antibodies.

Optionally, the protein fractions are analyzed using Multiple Reaction Monitoring-Mass Spectrometry on a proteomics platform, whereby only one protein (e.g. UCP1) of the thermogenic pathway is accurately quantified using LC-MS-MS.

### Example 6. Development and characterization of clonal DNA aptamers specifically targeting human adipocytes

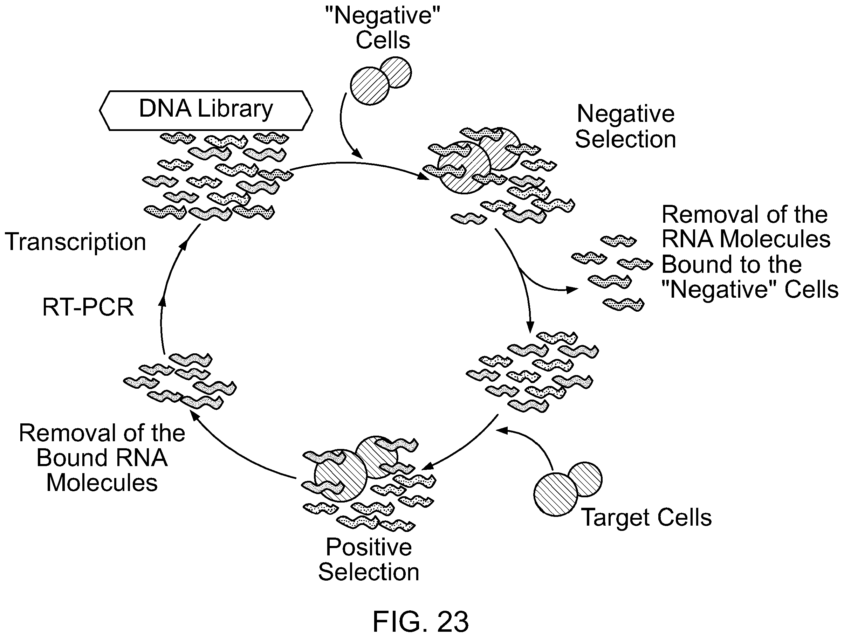

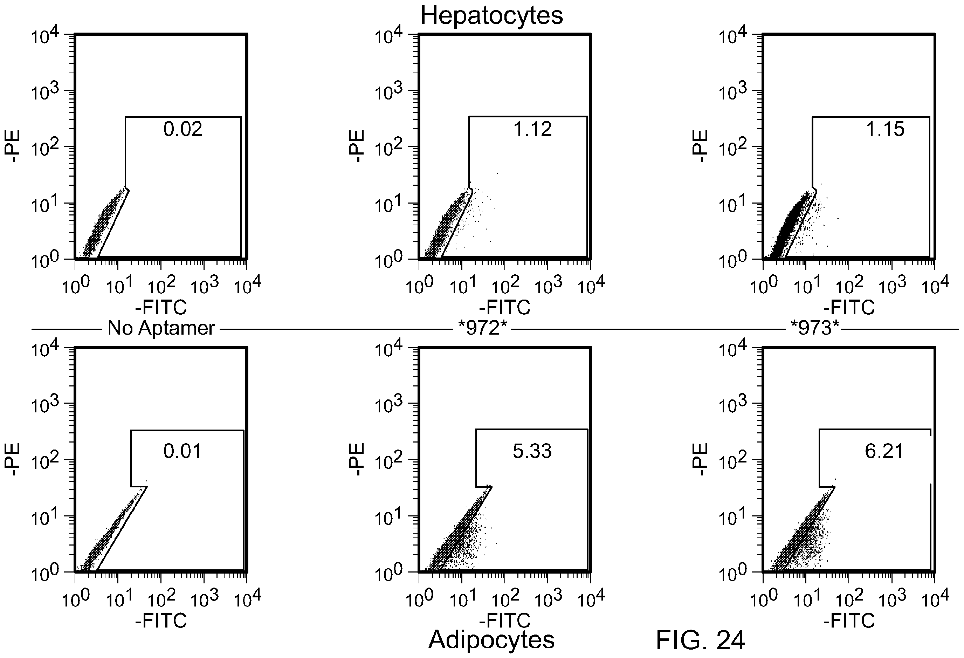

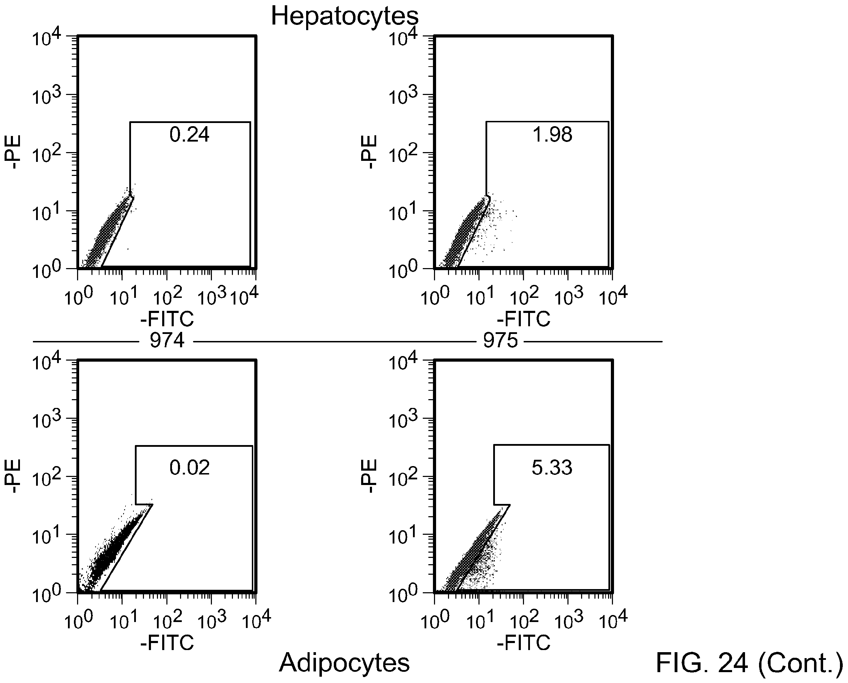

We used the Cell-SELEX technology to develop and characterize DNA aptamers that specifically recognize mature human subcutaneous adipocytes. With Cell-SELEX, aptamers recognizing specific molecules in their native conformation in their natural environment on the surface of intact cells are selected by repeated amplification and binding to living cells. In this cell-based selection illustrated in Figure 23, specific known and unknown cell surface markers or membrane receptors can be directly targeted within their native environment, allowing a straightforward enrichment of cell-specific aptamers. Cell-SELEX consists of a combination of positive selection with the target cells and negative selection with non-targeted cells. In the present case, negative selection was performed with freshly isolated human hepatocytes and positive selection utilized primary cultures of human subcutaneous adipocytes. Two rounds of negative selection and five rounds of positive selection from a 32 mer library were completed. Isolated aptamers were sequenced, synthesized and labeled with 6-fluorescein amidite (FAM) for binding studies. Human hepatocytes (negative cells) and adipocytes (positive cells) were labeled for 15 minutes at room temperature with a saturating concentration (1 µM) of FAM conjugated aptamers and analyzed by fluorescence-activated cell sorting (FACS). As shown on Figure 24, some aptamers (e.g. aptamer 974) do not bind to adipocytes nor hepatocytes, some aptamers (e.g. aptamer 975 bind to both adipocytes and hepatocytes, ratio: 2.69) and other aptamers bind preferentially to adipocytes (e.g. aptamers 972 and 973, ratio: 4.76 and 5.40, respectively). Further characterization of these adipocyte-specific aptamers is in progress.

### Example 7. Reconciliation of the Phenotypic, Genotyping, and Proteomic datasets

The results of the *in vitro* experiments set forth in Examples 3-5, herein, are reconciled. Specifically, to narrow further the initial set of microRNAs, mRNAs and target proteins and pathways to a relevant yet manageable number of targets, the experimental data is integrated with Network Searches and Analyses Packages (DAVID, Ingenuity Systems IPA and ARIADNE Pathway Studio.
Global analysis of the results of the *in vitro* experiments set forth in Examples 3-5, herein, is performed the Business Intelligence tool TIBCO Spotfire. This allows for a visualization of the relationships between the miRNA agents and target gene.

### Example 8. Animal Models of Obesity

Several animal models of obesity have been developed and validated (Kanasaki K et al., J. Biomed. Biotechnol., 2011:197636 (2011); Speakman J et al., Obesity reviews : an official journal of the International Association for the Study of Obesity, 8 Suppl 1:55-61 (2007)). The most commonly used are the Leptin Signaling Defects Lep^{ob/ob} and Lepr^{db/db} Mouse Models as well as the High-Fat Diet model in C57BL/6J mice (Wang CY et al., Methods in molecular biology, 821:421-433 (2012). This diet-induced obesity (DIO) model closely mimics the increased availability of the high-fat/high-density foods in modern society.

A DIO mouse model is used for *in vivo* validation of the effectiveness of the miRNA analogs described herein for the increase in thermogenesis and/or the treatment of obesity and other metabolic disorders (Yin H et al., Cell Metab.,17(2):210-224 (2013))..

DIO mice are administered one or more of an hsa-let-7a agomir, hsa-let-7a antagomir, hsa-miR-1 agomir, hsa-miR-1 antagomir, hsa-miR-19b agomir, hsa-miR-19b antagomir, hsa-miR-30b agomir, and hsa-miR-30b antagomir. Rosiglitazone is used as a positive control. Food intake, blood metabolic parameters, body composition (body weight,, body fat, bone mineral and lean mass, body fat distribution, body temperature, O₂ consumption and CO₂ production, exercise induced thermogenesis, cold induced thermogenesis and resting thermogenesis are measured in the mice prior to and after treatment. A reduction in body mass or body fat or an increase in body temperature or any kind of thermogenesis indicate the in vivo effectiveness of the administered composition.

### Example 9. Nucleic acid sequences of human UCP1 and UCP2 genes and transcripts

**Table 25.**

| **Nucleic acid sequence of the 1,462 base pair (bp) transcript ENST00000262999 of the human UCP1 gene (Six Exons are in capital letters):** | | | | | | |
|---|---|---|---|---|---|---|
| **No.** | **Exon / Intron** | **Start** | **End** | **Length** | **Sequence** | **SEQ ID NO** |
| | 5' upstream sequence | | | | ..........gtcggttcaaaaaacagaaatcgggtt tgctgcccggcggacaggcgtga | 618 |
| 1 | ENSE00001081761 | 141,489,959 | 141,489,758 | 202 | AGAGCAAGGGAAAGGAACTT CCTCCACCTTCGGGGCTGGA GCCCTTTTCCTCTGCATCTCC AGTCTCTGAGTGAAGATGGG GGGCCTGACAGCCTCGGACG TACACCCGACCCTGGGGGTC CAGCTCTTCTCAGCTGGAAT AGCGGCGTGCTTGGCGGACG TGATCACCTTCCCGCTGGAC ACGGCCAAAGTCCGGCTCCA G | 619 |
| | Intron 1-2 | 141,489,757 | 141,489,132 | 626 | gtagctaggcagaggggtaagacaa.......... tgttctgcacctttcttatttccag | 620 |
| | | | | | | 621 |
| 2 | ENSE00001009006 | 141,489,131 | 141,488,933 | 199 | GTCCAAGGTGAATGCCCGAC GTCCAGTGTTATTAGGTATA AAGGTGTCCTGGGAACAATC ACCGCTGTGGTAAAAACAGA AGGGCGGATGAAACTCTACA GCGGGCTGCCTGCGGGGCTT CAGCGGCAAATCAGCTCCGC CTCTCTCAGGATCGGCCTCTA CGACACGGTCCAGGAGTTCC TCACCGCAGGGAAAGAAA | 622 |
| | Intron 2-3 | 141,488,932 | 141,484,673 | 4,260 | gtaagccgtgagcgttcctgggagg.......... aataatttttttctctctggatag | 623 |
| | | | | | | 624 |
| 3 | ENSE00001081759 | 141,484,672 | 141,484,472 | 201 | CAGCACCTAGTTTAGGAAGC AAGATTTTAGCTGGTCTAAC GACTGGAGGAGTGGCAGTAT TCATTGGGCAACCCACAGAG GTCGTGAAAGTCAGACTTCA AGCACAGAGCCATCTCCACG GAATCAAACCTCGCTACACG GGGACTTATAATGCGTACAG AATAATAGCAACAACCGAAG GCTTGACGGGTCTTTGGAAA G | 625 |
| | Intron 3-4 | 141,484,471 | 141,484,366 | 106 | gtaactaacttcaaaatgggtttta.......... acattttcttttttttttccccag | 626 |
| | | | | | | 627 |
| 4 | ENSE00001081762 | 141,484,365 | 141,484,264 | 102 | GGACTACTCCCAATCTGATG AGAAGTGTCATCATCAATTG | 628 |
| | | | | | TACAGAGCTAGTAACATATG ATCTAATGAAGGAGGCCTTT GTGAAAAACAACATATTAGC AG | |
| | Intron 4-5 | 141,484,263 | 141,483,528 | 736 | gtaacttcccatttcatataacaaa.......... gacctgtttcatcgatccattttag | 629 |
| | | | | | | 630 |
| 5 | ENSE00001081763 | 141,483,527 | 141,483,347 | 181 | ATGACGTCCCCTGCCACTTG GTGTCGGCTCTTATCGCTGGA TTTTGCGCAACAGCTATGTCC TCCCCGGTGGATGTAGTAAA AACCAGATTTATTAATTCTCC ACCAGGACAGTACAAAAGTG TGCCCAACTGTGCAATGAAA GTGTTCACTAACGAAGGACC AACGGCTTTCTTCAAGGG | 631 |
| | Intron 5-6 | 141,483,346 | 141,481,165 | 2,182 | gtaagatatgatcttgtgtatctgt.......... cgaacgatgacatgcacttttctag | 632 |
| | | | | | | 633 |
| 6 | ENSE00001081760 | 141,481,164 | 141,480,588 | 577 | GTTGGTACCTTCCTTCTTGCG ACTTGGATCCTGGAACGTCA TTATGTTTGTGTGCTTTGAAC AACTGAAACGAGAACTGTCA AAGTCAAGGCAGACTATGGA CTGTGCCACATAATCAGCTTC AAGAAAATGATGTAACATAC CAGTGGGAATCTTGCTGACT GGATCATAAAAACAAACAAA ACTTATTCACTTATTTTAACC TAAAAAGATAAAGGAATTTT GGCAGAGAATTTTGGACTTT TTTATATAAAAAAGAGGAAA ATTAATGCCTATTTCATATAA CTTTTTTTTTTCTCAGTGTCT TAAGAAGGGGAAAGCAAAA CATTCAGCATATACCCTGGC AAATGTAATGCAGATAAGCT ACTGCATTTGACCATTTCTGG AGTGCAATTGTGTGAATGAA TGTGAAGAACTTTAACATGT TTTAATTACAATTCCAACTGG TGGAAAAGAAACTGAGTGAA ATGCAGTTTATATTTATAAAT ACTTAAAAATGAAGTTATTA AAAATATTAGTTTTTATTAAC CACAGTTGTCAGTTAATATAT TCAATAAAGTATTGCTAATA CCTTTT | 634 |
| | 3' downstream sequence | | | | aaagtttgtcttttgagatctatacctgggtgtaa gagtcaagttcacta.......... | 635 |

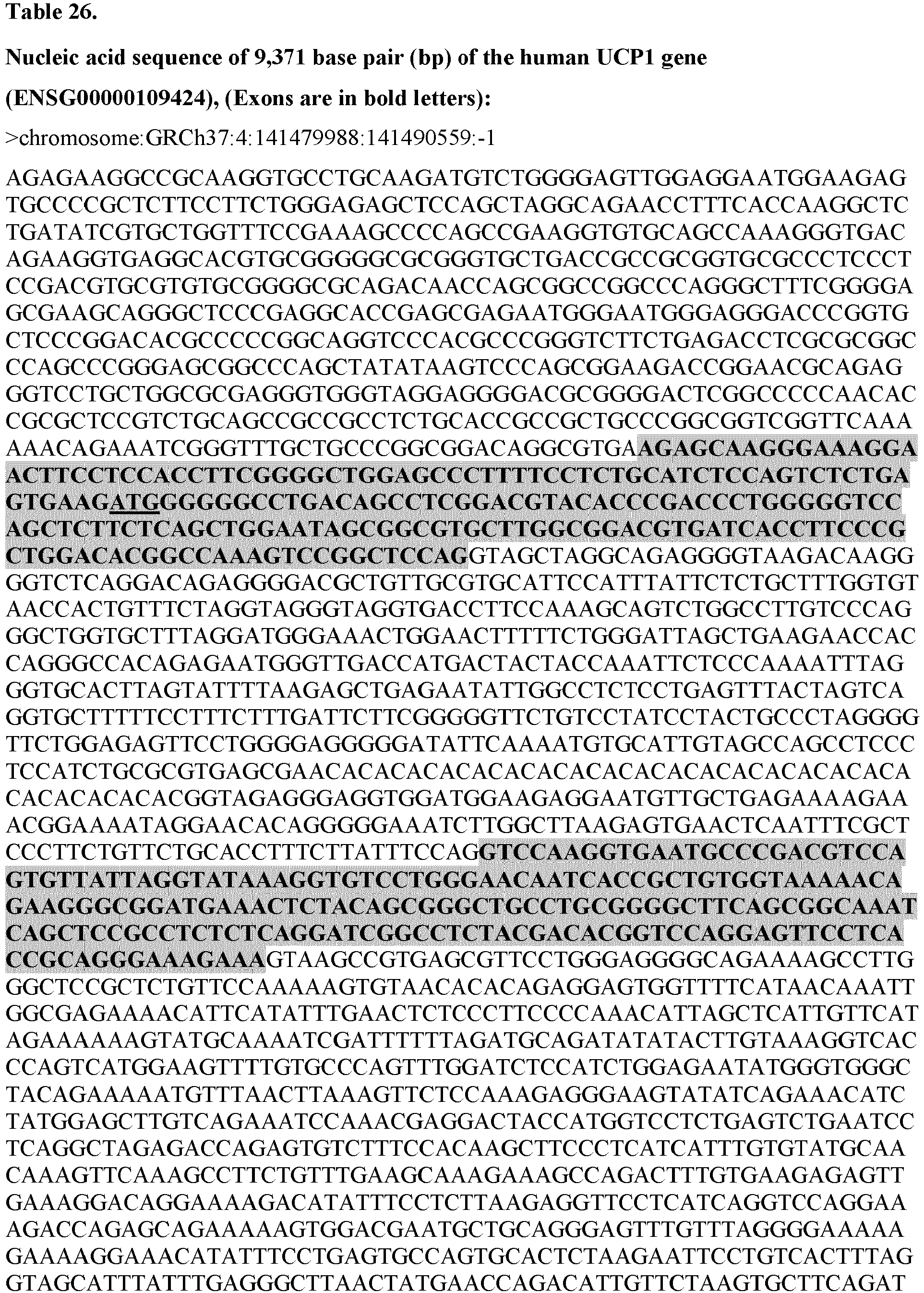

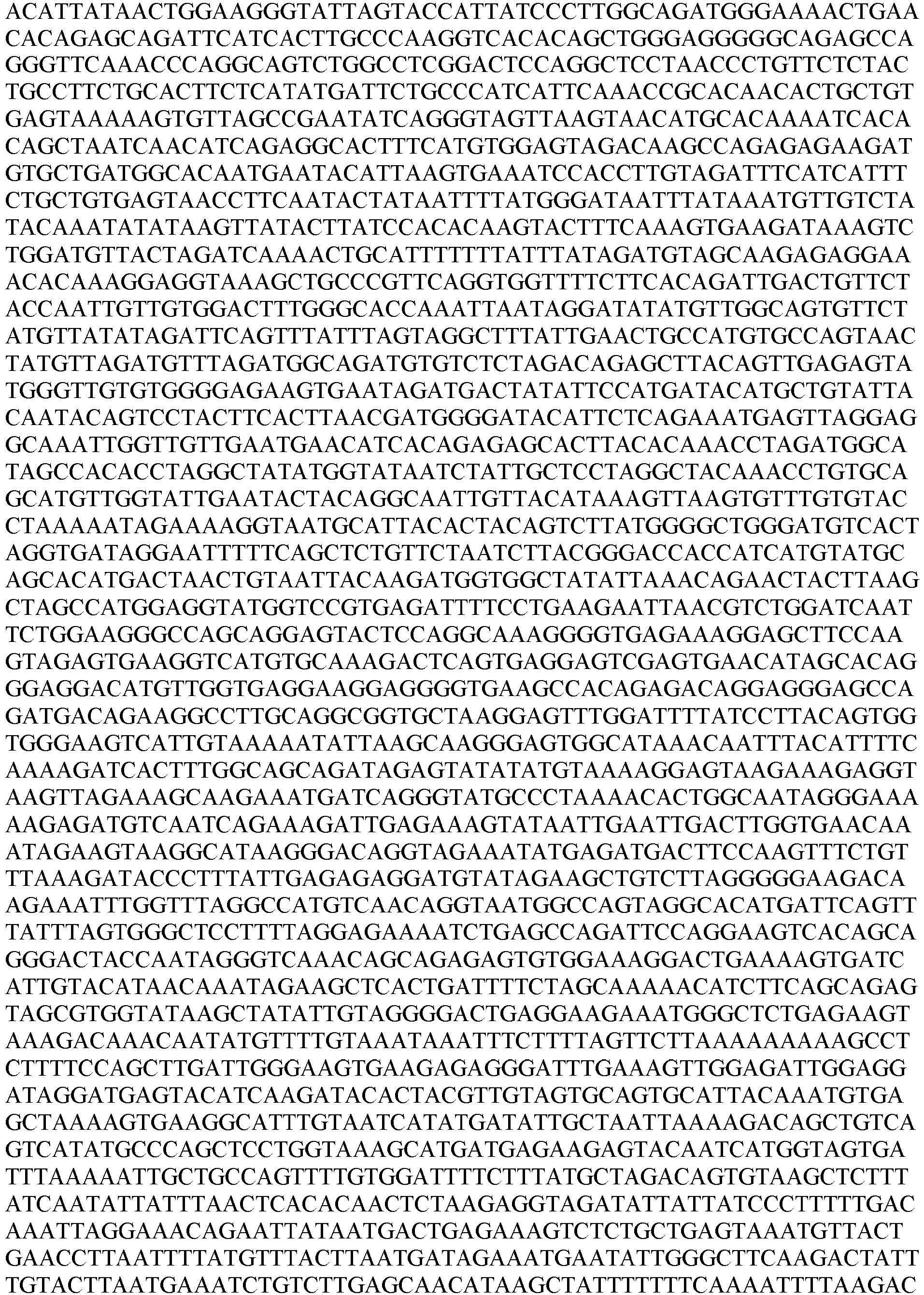

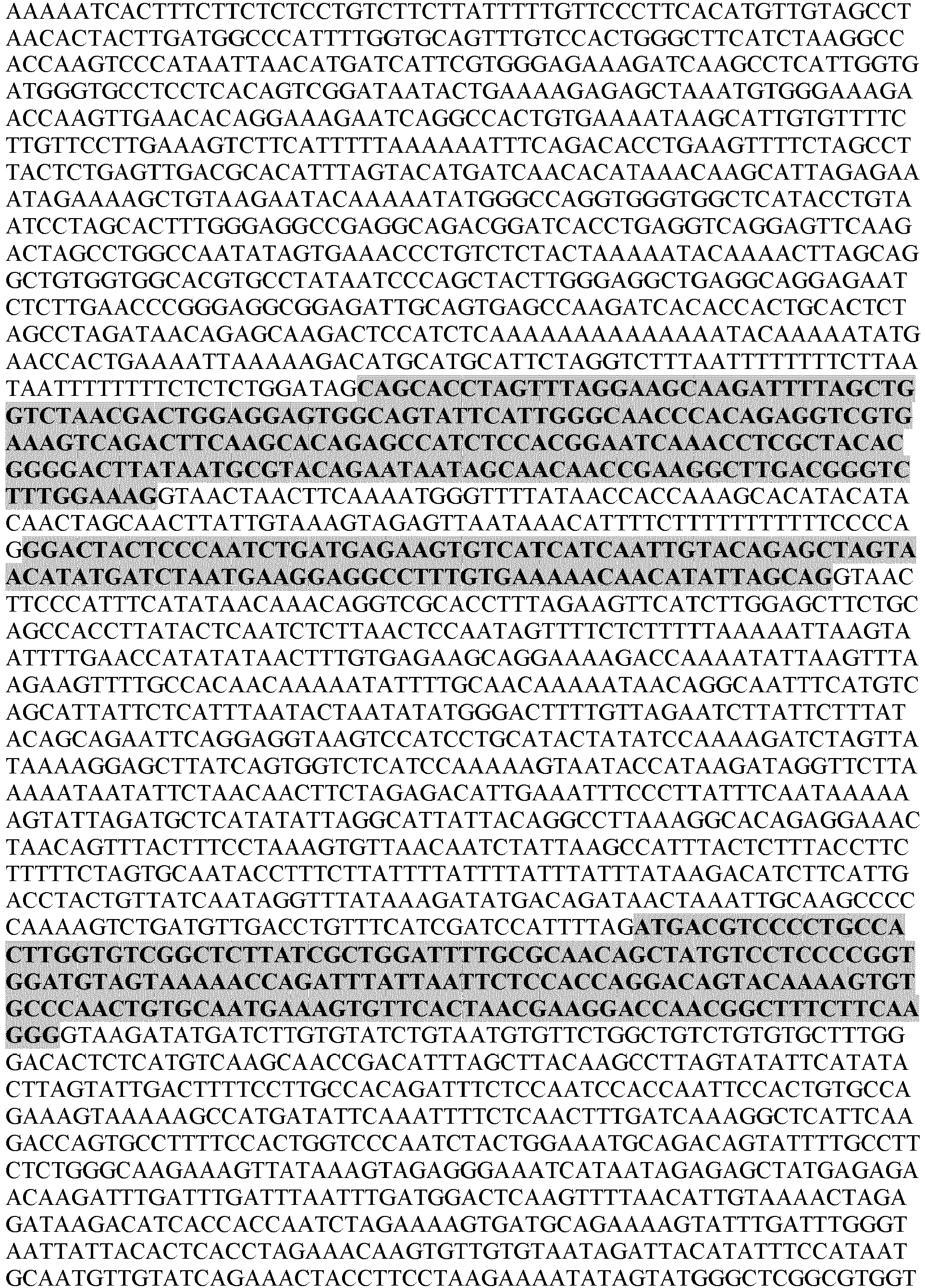

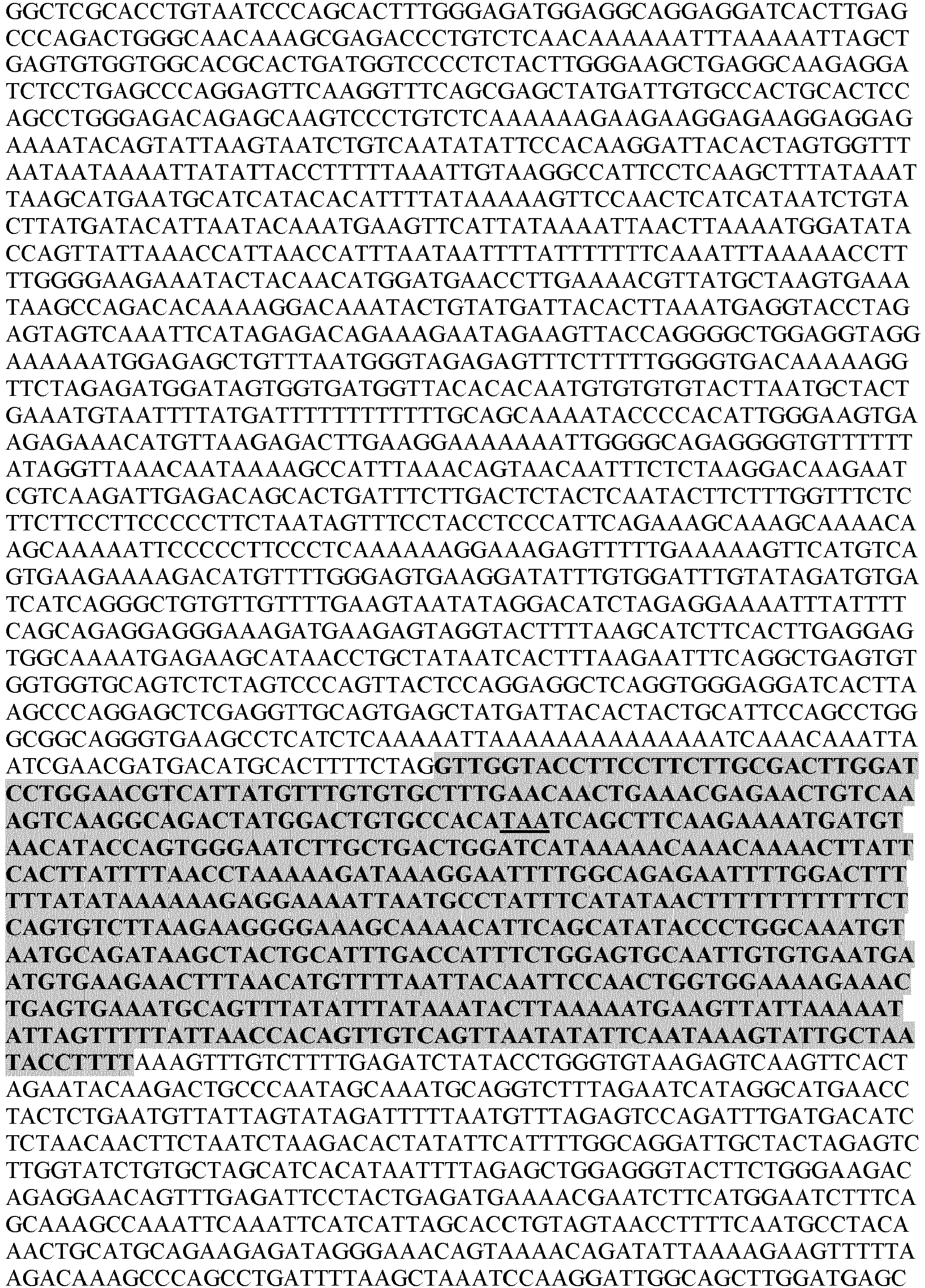

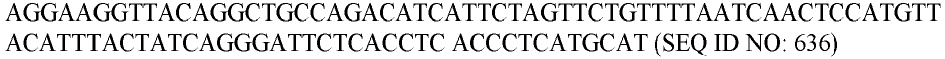

**Table 27.**

| **Nucleic acid sequence of 15,910 base pair (bp) of the human UCP1 gene (NCBI Reference Sequence: NG_012139.1, RefSeqGene on chromosome 4): (SEQ ID NO: 637)** |
|---|
| CTGTACAGCT CTCCGACAAT CCCACATCTA GATGCCAAGC TGAGGTTGGC ATTCTCACTA 61 ATTTGCTGTT ATAAATATTA AGCTATCATA AGCGTTAGCC TACATATGAC TCTTTCATAT 121 GTTAGTTAAT TATTTTAGGG TAGAAATCCA AAAGTGGAGT TACCAGAAGT GGATATAGAC 181 ATTCTGGCTG GGTGTGATGG TTCATGCCTG TAATCCCAGC ACTTTGGGAG GCAGAGGCAG 241 GCGGATCACT TGAGGCCAGG AGTTTGAGAT CAGCCTGGGC CAACACAGCG AAACCCCATC 301 TCTACTAAAA ATTCCAAAAC TAGCCAGGCA TAGTGGCACA TGCCTGTACT CCCAGCTACT 361 TGGGAGGCTA AGACACAAGA ATCGCTTGAA CCCGGGAGGG AGGTGGAGGT TGCGGTGAGC 421 TGAGATTGTG CCACCGTACT CCAGCCTGGG TGACACAGCT AGACTCTGTT TCAAAAAAAA 481 AAAGAAAAAG AAAAGAAAAA AATAGACTTT CTCTTGGCTC AGTGTATACT GCCAAATTGT 541 TTTCCAAAAA AATTGTGTCA ATGTATAACA CCATCACTAA TATAGTATTG ATATTATGGT 601 TATTACATTT TAAAATTCAT AATTTGTAAT TATAACATTC ATAATTTATT ACTATTTATA 661 ATATTAATGT AAATGTATAT TATATATAAA TGTTATAGTA ATTATAACTT TGGTAGTGAC 721 AAAGTATTAA TTTATTAGGT GAAGTATATG CTTTTTTATT AGTGATAATA AATATATCCT 781 CTCTCCCATT ATAAAAGTTT GTATTTCTTC TTTTAGAAAT TGATTCTTCT GTCATTTGCA 841 CATTTATCTG TATAATTATA ACAGGGTATT TCCCAGTGGT GGCTAATGAG AGAATTATGG 901 GAAAGTATAG AACACTATTC AAATGCAAAG CACTGTATGA TTTTTATTTA ATAGGAAGAC 961 ATTTTGTGCA GCGATTTCTG ATTGACCACA GTTTGATCAA GTGCATTTGT TAATGTGTTC 1021 TACATTTTCA AAAAGGAAAG GAGAATTTGT TACATTCAGA ACTTGCTGCC ACTCCTTTGC 1081 TACGTCATAA AGGGTCAGTT GCCCTTGCTC ATACTGACCT ATTCTTTACC TCTCTGCTTC 1141 TTCTTTGTGC CAGAAGAGTA GAAATCTGAC CCTTTGGGGA TACCACCCTC TCCCCTACTG 1201 CTCTCTCCAA CCTGAGGCAA ACTTTCTCCT ACTTCCCAGA GCCTGTCAGA AGTGGTGAAG 1261 CCAGCCTGCT CCTTGGAATC CAGAACTACT TTCAGAATCT TGAACTTCTG TGACCTCTCA 1321 GGGTCCCCTT GTGTGAAGTT TTTGACGTCA GCTTCTCCTG TGACCCTTAG AAGTCACTCT 1381 TGTGTCTAGC ACATCCCAGG TGCTCAGTCA CCATTGAACT ACAGTCATAC TATCTCCTGG 1441 CAAAGGCTCT TAACTGTCCA TGTTAGCCTG ATATTAATAT CCTGGAAGCT TATACTGTCG 1501 TTCTTCCTTC CAGGTTTAAA TAAGGCAGCC CCTTTATCCT GTCACAGGTC CTCTCTCCCT 1561 ACCTATCCTT ACCTGTTTTG GATAACAACC TTTCTTATTT CTAATAGATT TATTTATTTC 1621 TCACATTTCC TTCCCTTATC ATAGTTTTCC TCTCACTTTC TCCTCTAGTT TGTCATACTC 1681 TGGCTTTAAA ACATGCAAAC ATGTGCCTTA TGGGGAAAAA AAGACAATTT TAATTTACCT 1741 TGCTTCTTCT TTACAAATGT ATTGTGGCTT CTTCTTATAG TCCAAATCTA AAACTCTTTA 1801 CCCACCCACT GCCTTGAACT CCTTCCTCGT TGTGAAAGTA GGATGGGGCA AAGAGAGAAT 1861 GCATGCCCCT CCCAACTGCT CAAACAAGTA AAGGTGCTGT TACAGTTATC TTTTGCTACC 1921 TTAATACAAT AATTATTTTA TTATATCTCA CAATTTTATG GATCAGGAAT TTAGACTGGG 1981 CTCAGCTAGG CGATTCTTCT GCTTTACTGA CATCATAGGA GATCACTTGG TGGTATTCAA 2041 CTGTCAGGTA GGCTTATCTG GAGGGTCCAA GATAGCTGTA CTCTGGTGCC TGGTGCCTTG 2101 GTAAAGAGGG ATGATGATGT GGGGCCTCTC CAGCATGAAC AGCCTCAGAG AAGTTTGCTT 2161 TCTTACATGC TGGCCCAGGG CTCCAAGAGC AAATGTTGCA GTGAGTAAAG CAGAAGATAC 2221 AAGGACTTTT ATAATCTGGT CTCAGAAGCC ACATGGCATC AGTTCTGTAT TATTCTATTG 2281 GTCAAAACAT TCATAAGCCT GCCAGATGCA AGGGGAAGGC ATATGTACCC TCATCTTTTG 2341 ATGGGAGGAA TGTGATGGAT TTGCAATTAT GTTTTAAAAC TACTACAGAC AGAACCACTG 2401 AGAAAGATTC ATGGGTAGCT TTGGGGTGAG GACTGGGAAT TAACCTGTTG ATAGCAGAGG 2461 TTCACTAGAG TCAACAAGGA ATAAGGTCTC CTCTTGTACA CTTTAGTCAT ACTATACCAA 2521 CATTCTTAAC CACTGCTTAG CCATCAGCCT CACAACATAA CAACTCCATC ATAGTTGTAC 2581 TCCCTAAGAT CACCAACAAT GTTAGAGTCA AATCCGGTAG GTTTTTCTTT GTTTTTGTCC 2641 TCCTGACATT TTTTCTAAAC TTGACACTGG TCAGACCCAA TCTTTCTTTA ATCATATTCT 2701 TAAATACCAG TTCTATCACT GGATATGTTA CTGTTTCTTG TTCTCACTCT ACCTTTGACA 2761 AAGCCATTCT TTCCAGACTA TAACTCTGGG TCTGGGTCCC CCTATGGTTT GGCCCTTGAA 2821 TTCTTTTCCT AGTCCTATTT GACTAGCCCC ATTTTCCCGT GAAAAGCATG CCCCTTTCAT 2881 TGCATCCATA TCATGACTAC CAAATACCTC CTCTATTTCT TCCTCTTTTA GCATGTTAAA 2941 |
| TGCAGCTTCC TAAGCTCTCT ATCTGGATAT CAACAGTATT CTCTCCAAAT AATTCTAAGA 3001 CTTTAAAAAT TGGTTTAATC TTCTTACCCC TAAAATCACC CCCCTTACCA ACTGCCTCAT 3061 GACAATCATT GGTACTGTCA CTGAGCTTGC AACCCATGTT CTTAAACATA GAGTAATCTT 3121 TGACTCCACA TCTAATCATT CATAAAGCTG TATTGTCTAT CAAATTAAAT CTGACATTTA 3181 TGTGAGAGCA CTTCATAGTC TGTAAAGCAC TACACAGGTG ATAACATGAA GCTACACTCA 3241 TAATGGATTT GCAGGCTCTG CTTCTCATTT GGCTTCTACA GCCTCATCCC TCACCAACTT 3301 CTTGCCCTAC CTCTCTCTTT CTTCCCCATC ACCCAATTTC CCAGTCAGTC AGGCCAACAG 3361 AATGCATTCT ATATACGCGA CTTGCTTTCC CCAACATCTT TGCCTGTATG CATGCCACTT 3421 ATTTGCCTCA GTTGATCTTT ATTTCAACAA GTGTTTGCAG AGGAGAAACC TCGCTGGCTC 3481 CTTCTCCTTT CTATTTTTTT TCAGAGGCTA CCCGTCAGGT CAACATTGCC TTTTTCAGGG 3541 AAGCTCTGCA AGCCTGACCT CCCTTGGAAG TGCCTTAGGA CTGGCTTCTT GCACAGTACA 3601 CAACCTTTAC TTATAGAGGG TTTGGAGATT ATTCTTTATT CATGTCTTAT TTCTCCTGCT 3661 CCTGGAGGAG ATGACTCTGA CTTCCACTGA CTCTTTTGGG GGGCTTAAGT CAGGGTTGAG 3721 TACCAGAGGC CCTAAATAGC TGGACGTGGA TTCTGGTAAT ATCAAATCCA TCTTTGGCTT 3781 AACTGAGAGG TTCTGAAAGC TGGGACCTGA CCTTGTCCAT TTCCCTCTTT CTCCAGTTTC 3841 CTATTATTTC CCACTGTTTT TTTTAAAAGT TTTTTGTTTT CTTAAGTTTT CACAAGAATA 3901 AACATTGAAA ATAAAATTTG CACAAAGATC GAACTAGGAA AGGCCACACA ACCAACACAT 3961 ATTACATCAT TATAGGTAAG TTAGCAGGGA GATTTCAGAC CTGGGCTAGC TCTGGAACCA 4021 CATTTTACAC TGTTGAAAAT AAAAGCTGGA GTACAGATGA CTTTCCCAGG TTCACAGAGT 4081 TGGTAAGCTG GAGAGCTGCA CCTGGAGCCA AGCAACCTGC CCTGTCCTTT CCACTGCACC 4141 CTCTAAGAAA TCTAATTAGA AGGAACAGGT GGTATCTCAT TTTGTACGGT GCTTTAGCAA 4201 TGTACTATTT GCTTTCTAGT GTGTCTATTG TCTCGTTTGA CATCTTCTCT CAAAAAGTGA 4261 TGAAACGAAA CGCTCTTTTT GACAAGTTCA GAGTGCTCTT GGTTCCTGTG TGGGATTCTT 4321 CCAAGTCTGA ATTTGGTAGT GGGAAGAGAA GGAATCCGGA GGAAGGAGGA TGAGAAGTTT 4381 AAAGGAGAGG AAAGGGAAGC AGAGAAGGCC GCAAGGTGCC TGCAAGATGT CTGGGGAGTT 4441 GGAGGAATGG AAGAGTGCCC CGCTCTTCCT TCTGGGAGAG CTCCAGCTAG GCAGAACCTT 4501 TCACCAAGGC TCTGATATCG TGCTGGTTTC CGAAAGCCCC AGCCGAAGGT GTGCAGCCAA 4561 AGGGTGACAG AAGGTGAGGC ACGTGCGGGG GCGCGGGTGC TGACCGCCGC GGTGCGCCCT 4621 CCCTCCGACG TGCGGTGTGC GGGGCGCAGA CAACCAGCGG CCGGCCCAGG GCTTTCGGGG 4681 AGCGAAGCAG GGCTCCCGAG GCACCGAGCG AGAATGGGAA TGGGAGGGAC CCGGTGCTCC 4741 CGGACACGCC CCCGGCAGGT CCCACGCCCG GGTCTTCTGA GACCTCGCGC GGCCCAGCCC 4801 GGGAGCGGCC CAGCTATATA AGTCCCAGCG GAAGACCGGA ACGCAGAGGG TCCTGCTGGC 4861 GCGAGGGTGG GTAGGAGGGG ACGCGGGGAC TCGGCCCCCA ACACCGCGCT CCGTCTGCAG 4921 CCGCCGCCTC TGCACCGCCG CTGCCCGGCG GTCGGTTCAA AAAACAGAAA TCGGGTTTGC 4981 TGCCCGGCGG ACAGGCGTGA AGAGCAAGGG AAAGGAACTT CCTCCACCTT CGGGGCTGGA 5041 GCCCTTTTCC TCTGCATCTC CAGTCTCTGA GTGAAGATGG GGGGCCTGAC AGCCTCGGAC 5101 GTACACCCGA CCCTGGGGGT CCAGCTCTTC TCAGCTGGAA TAGCGGCGTG CTTGGCGGAC 5161 GTGATCACCT TCCCGCTGGA CACGGCCAAA GTCCGGCTCC AGGTAGCTAG GCAGAGGGGT 5221 AAGACAAGGG GTCTCAGGAC AGAGGGGACG CTGTTGCGTG CATTCCATTT ATTCTCTGCT 5281 TTGGTGTAAC CACTGTTTCT AGGTAGGGTA GGTGACCTTC CAAAGCAGTC TGGCCTTGTC 5341 CCAGGGCTGG TGCTTTAGGA TGGGAAACTG GAACTTTTTC TGGGATTAGC TGAAGAACCA 5401 CCAGGGCCAC AGAGAATGGG TTGACCATGA CTACTACCAA ATTCTCCCAA AATTTAGGGT 5461 GCACTTAGTA TTTTAAGAGC TGAGAATATT GGCCTCTCCT GAGTTTACTA GTCAGGTGCT 5521 TTTTCCTTTC TTTGATTCTT CGGGGGTTCT GTCCTATCCT ACTGCCCTAG GGGTTCTGGA 5581 GAGTTCCTGG GGAGGGGGAT ATTCAAAATG TGCATTGTAG CCAGCCTCCC TCCATCTGCG 5641 CGTGAGCGAA CACACACACA CACACACACA CACACACACA CACACACACA CACACACGGT 5701 AGAGGGAGGT GGATGGAAGA GGAATGTTGC TGAGAAAAGA AACGGAAAAT AGGAACACAG 5761 GGGGAAATCT TGGCTTAAGA GTGAACTCAA TTTCGCTCCC TTCTGTTCTG CACCTTTCTT 5821 ATTTCCAGGT CCAAGGTGAA TGCCCGACGT CCAGTGTTAT TAGGTATAAA GGTGTCCTGG 5881 GAACAATCAC CGCTGTGGTA AAAACAGAAG GGCGGATGAA ACTCTACAGC GGGCTGCCTG 5941 CGGGGCTTCA GCGGCAAATC AGCTCCGCCT CTCTCAGGAT CGGCCTCTAC GACACGGTCC 6001 AGGAGTTCCT CACCGCAGGG AAAGAAAGTA AGCCGTGAGC GTTCCTGGGA GGGGCAGAAA 6061 AGCCTTGGGC TCCGCTCTGT TCCAAAAAGT GTAACACACA GAGGAGTGGT TTTCATAACA 6121 AATTGGCGAG AAAACATTCA TATTTGAACT CTCCCTTCCC CAAACATTAG CTCATTGTTC 6181 ATAGAAAAAA GTATGCAAAA TCGATTTTTT AGATGCAGAT ATATACTTGT AAAGGTCACC 6241 CAGTCATGGA AGTTTTGTGC CCAGTTTGGA TCTCCATCTG GAGAATATGG GTGGGCTACA 6301 GAAAAATGTT TAACTTAAAG TTCTCCAAAG AGGGAAGTAT ATCAGAAACA TCTATGGAGC 6361 TTGTCAGAAA TCCAAACGAG GACTACCATG GTCCTCTGAG TCTGAATCCT CAGGCTAGAG 6421 |
| ACCAGAGTGT CTTTCCACAA GCTTCCCTCA TCATTTGTGT ATGCAACAAA GTTCAAAGCC 6481 TTCTGTTTGA AGCAAAGAAA GCCAGACTTT GTGAAGAGAG TTGAAAGGAC AGGAAAAGAC 6541 ATATTTCCTC TTAAGAGGTT CCTCATCAGG TCCAGGAAAG ACCAGAGCAG AAAAAGTGGA 6601 CGAATGCTGC AGGGAGTTTG TTTAGGGGAA AAAGAAAAGG AAACATATTT CCTGAGTGCC 6661 AGTGCACTCT AAGAATTCCT GTCACTTTAG GTAGCATTTA TTTGAGGGCT TAACTATGAA 6721 CCAGACATTG TTCTAAGTGC TTCAGATACA TTATAACTGG AAGGGTATTA GTACCATTAT 6781 CCCTTGGCAG ATGGGAAAAC TGAACACAGA GCAGATTCAT CACTTGCCCA AGGTCACACA 6841 GCTGGGAGGG GGCAGAGCCA GGGTTCAAAC CCAGGCAGTC TGGCCTCGGA CTCCAGGCTC 6901 CTAACCCTGT TCTCTACTGC CTTCTGCACT TCTCATATGA TTCTGCCCAT CATTCAAACC 6961 GCACAACACT GCTGTGAGTA AAAAGTGTTA GCCGAATATC AGGGTAGTTA AGTAACATGC 7021 ACAAAATCAC ACAGCTAATC AACATCAGAG GCACTTTCAT GTGGAGTAGA CAAGCCAGAG 7081 AGAAGATGTG CTGATGGCAC AATGAATACA TTAAGTGAAA TCCACCTTGT AGATTTCATC 7141 ATTTCTGCTG TGAGTAACCT TCAATACTAT AATTTTATGG GATAATTTAT AAATGTTGTC 7201 TATACAAATA TATAAGTTAT ACTTATCCAC ACAAGTACTT TCAAAGTGAA GATAAAGTCT 7261 GGATGTTACT AGATCAAAAC TGCATTTTTT TATTTATAGA TGTAGCAAGA GAGGAAACAC 7321 AAAGGAGGTA AAGCTGCCCG TTCAGGTGGT TTTCTTCACA GATTGACTGT TCTACCAATT 7381 GTTGTGGACT TTGGGCACCA AATTAATAGG ATATATGTTG GCAGTGTTCT ATGTTATATA 7441 GATTCAGTTT ATTTAGTAGG CTTTATTGAA CTGCCATGTG CCAGTAACTA TGTTAGATGT 7501 TTAGATGGCA GATGTGTCTC TAGACAGAGC TTACAGTTGA GAGTATGGGT TGTGTGGGGA 7561 GAAGTGAATA GATGACTATA TTCCATGATA CATGCTGTAT TACAATACAG TCCTACTTCA 7621 CTTAACGATG GGGATACATT CTCAGAAATG AGTTAGGAGG CAAATTGGTT GTTGAATGAA 7681 CATCACAGAG AGCACTTACA CAAACCTAGA TGGCATAGCC ACACCTAGGC TATATGGTAT 7741 AATCTATTGC TCCTAGGCTA CAAACCTGTG CAGCATGTTG GTATTGAATA CTACAGGCAA 7801 TTGTTACATA AAGTTAAGTG TTTGTGTACC TAAAAATAGA AAAGGTAATG CATTACACTA 7861 CAGTCTTATG GGGCTGGGAT GTCACTAGGT GATAGGAATT TTTCAGCTCT GTTCTAATCT 7921 TACGGGACCA CCATCATGTA TGCAGCACAT GACTAACTGT AATTACAAGA TGGTGGCTAT 7981 ATTAAACAGA ACTACTTAAG CTAGCCATGG AGGTATGGTC CGTGAGATTT TCCTGAAGAA 8041 TTAACGTCTG GATCAATTCT GGAAGGGCCA GCAGGAGTAC TCCAGGCAAA GGGGTGAGAA 8101 AGGAGCTTCC AAGTAGAGTG AAGGTCATGT GCAAAGACTC AGTGAGGAGT CGAGTGAACA 8161 TAGCACAGGG AGGACATGTT GGTGAGGAAG GAGGGGTGAA GCCACAGAGA CAGGAGGGAG 8221 CCAGATGACA GAAGGCCTTG CAGGCGGTGC TAAGGAGTTT GGATTTTATC CTTACAGTGG 8281 TGGGAAGTCA TTGTAAAAAT ATTAAGCAAG GGAGTGGCAT AAACAATTTA CATTTTCAAA 8341 AGATCACTTT GGCAGCAGAT AGAGTATATA TGTAAAAGGA GTAAGAAAGA GGTAAGTTAG 8401 AAAGCAAGAA ATGATCAGGG TATGCCCTAA AACACTGGCA ATAGGGAAAA AGAGATGTCA 8461 ATCAGAAAGA TTGAGAAAGT ATAATTGAAT TGACTTGGTG AACAAATAGA AGTAAGGCAT 8521 AAGGGACAGG TAGAAATATG AGATGACTTC CAAGTTTCTG TTTAAAGATA CCCTTTATTG 8581 AGAGAGGATG TATAGAAGCT GTCTTAGGGG GAAGACAAGA AATTTGGTTT AGGCCATGTC 8641 AACAGGTAAT GGCCAGTAGG CACATGATTC AGTTTATTTA GTGGGCTCCT TTTAGGAGAA 8701 AATCTGAGCC AGATTCCAGG AAGTCACAGC AGGGACTACC AATAGGGTCA AACAGCAGAG 8761 AGTGTGGAAA GGACTGAAAA GTGATCATTG TACATAACAA ATAGAAGCTC ACTGATTTTC 8821 TAGCAAAAAC ATCTTCAGCA GAGTAGCGTG GTATAAGCTA TATTGTAGGG GACTGAGGAA 8881 GAAATGGGCT CTGAGAAGTA AAGACAAACA ATATGTTTTG TAAATAAATT TCTTTTAGTT 8941 CTTAAAAAAA AAGCCTCTTT TCCAGCTTGA TTGGGAAGTG AAGAGAGGGA TTTGAAAGTT 9001 GGAGATTGGA GGATAGGATG AGTACATCAA GATACACTAC GTTGTAGTGC AGTGCATTAC 9061 AAATGTGAGC TAAAAGTGAA GGCATTTGTA ATCATATGAT ATTGCTAATT AAAAGACAGC 9121 TGTCAGTCAT ATGCCCAGCT CCTGGTAAAG CATGATGAGA AGAGTACAAT CATGGTAGTG 9181 ATTTAAAAAT TGCTGCCAGT TTTGTGGATT TTCTTTATGC TAGACAGTGT AAGCTCTTTA 9241 TCAATATTAT TTAACTCACA CAACTCTAAG AGGTAGATAT TATTATCCCT TTTTGACAAA 9301 TTAGGAAACA GAATTATAAT GACTGAGAAA GTCTCTGCTG AGTAAATGTT ACTGAACCTT 9361 AATTTTATGT TTACTTAATG ATAGAAATGA ATATTGGGCT TCAAGACTAT TTGTACTTAA 9421 TGAAATCTGT CTTGAGCAAC ATAAGCTATT TTTTTCAAAA TTTTAAGACA AAAATCACTT 9481 TCTTCTCTCC TGTCTTCTTA TTTTTGTTCC CTTCACATGT TGTAGCCTAA CACTACTTGA 9541 TGGCCCATTT TGGTGCAGTT TGTCCACTGG GCTTCATCTA AGGCCACCAA GTCCCATAAT 9601 TAACATGATC ATTCGTGGGA GAAAGATCAA GCCTCATTGG TGATGGGTGC CTCCTCACAG 9661 TCGGATAATA CTGAAAAGAG AGCTAAATGT GGGAAAGAAC CAAGTTGAAC ACAGGAAAGA 9721 ATCAGGCCAC TGTGAAAATA AGCATTGTGT TTTCTTGTTC CTTGAAAGTC TTCATTTTTA 9781 AAAAATTTCA GACACCTGAA GTTTTCTAGC CTTACTCTGA GTTGACGCAC ATTTAGTACA 9841 TGATCAACAC ATAAACAAGC ATTAGAGAAA TAGAAAAGCT GTAAGAATAC AAAAATATGG 9901 |
| GCCAGGTGGG TGGCTCATAC CTGTAATCCT AGCACTTTGG GAGGCCGAGG CAGACGGATC 9961 ACCTGAGGTC AGGAGTTCAA GACTAGCCTG GCCAATATAG TGAAACCCTG TCTCTACTAA 10021 AAATACAAAA CTTAGCAGGC TGTGGTGGCA CGTGCCTATA ATCCCAGCTA CTTGGGAGGC 10081 TGAGGCAGGA GAATCTCTTG AACCCGGGAG GCGGAGATTG CAGTGAGCCA AGATCACACC 10141 ACTGCACTCT AGCCTAGATA ACAGAGCAAG ACTCCATCTC AAAAAAAAAA AAAATACAAA 10201 AATATGAACC ACTGAAAATT AAAAAGACAT GCATGCATTC TAGGTCTTTA ATTTTTTTTC 10261 TTAATAATTT TTTTTCTCTC TGGATAGCAG CACCTAGTTT AGGAAGCAAG ATTTTAGCTG 10321 GTCTAACGAC TGGAGGAGTG GCAGTATTCA TTGGGCAACC CACAGAGGTC GTGAAAGTCA 10381 GACTTCAAGC ACAGAGCCAT CTCCACGGAA TCAAACCTCG CTACACGGGG ACTTATAATG 10441 CGTACAGAAT AATAGCAACA ACCGAAGGCT TGACGGGTCT TTGGAAAGGT AACTAACTTC 10501 AAAATGGGTT TTATAACCAC CAAAGCACAT ACATACAACT AGCAACTTAT TGTAAAGTAG 10561 AGTTAATAAA CATTTTCTTT TTTTTTTTCC CCAGGGACTA CTCCCAATCT GATGAGAAGT 10621 GTCATCATCA ATTGTACAGA GCTAGTAACA TATGATCTAA TGAAGGAGGC CTTTGTGAAA 10681 AACAACATAT TAGCAGGTAA CTTCCCATTT CATATAACAA ACAGGTCTGC ACCTTTAGAA 10741 GTTCATCTTG GAGCTTCTGC AGCCACCTTA TACTCAATCT CTTAACTCCA ATAGTTTTCT 10801 CTTTTTAAAA ATTAAGTAAT TTTGAACCAT ATATAACTTT GTGAGAAGCA GGAAAAGACC 10861 AAAATATTAA GTTTAAGAAG TTTTGCCACA ACAAAAATAT TTTGCAACAA AAATAACAGG 10921 CAATTTCATG TCAGCATTAT TCTCATTTAA TACTAATATA TGGGACTTTT GTTAGAATCT 10981 TATTCTTTAT ACAGCAGAAT TCAGGAGGTA AGTCCATCCT GCATACTATA TCCAAAAGAT 11041 CTAGTTATAA AAGGAGCTTA TCAGTGGTCT CATCCAAAAA GTAATACCAT AAGATAGGTT 11101 CTTAAAAATA ATATTCTAAC AACTTCTAGA GACATTGAAA TTTCCCTTAT TTCAATAAAA 11161 AAGTATTAGA TGCTCATATA TTAGGCATTA TTACAGGCCT TAAAGGCACA GAGGAAACTA 11221 ACAGTTTACT TTCCTAAAGT GTTAACAATC TATTAAGCCA TTTACTCTTT ACCTTCTTTT 11281 TCTAGTGCAA TACCTTTCTT ATTTTATTTT ATTTATTTAT AAGACATCTT CATTGACCTA 11341 CTGTTATCAA TAGGTTTATA AAGATATGAC AGATAACTAA ATTGCAAGCC CCCAAAAGTC 11401 TGATGTTGAC CTGTTTCATC GATCCATTTT AGATGACGTC CCCTGCCACT TGGTGTCGGC 11461 TCTTATCGCT GGATTTTGCG CAACAGCTAT GTCCTCCCCG GTGGATGTAG TAAAAACCAG 11521 ATTTATTAAT TCTCCACCAG GACAGTACAA AAGTGTGCCC AACTGTGCAA TGAAAGTGTT 11581 CACTAACGAA GGACCAACGG CTTTCTTCAA GGGGTAAGAT ATGATCTTGT GTATCTGTAA 11641 TGTGTTCTGG CTGTCTGTGT GCTTTGGGAC ACTCTCATGT CAAGCAACCG ACATTTAGCT 11701 TACAAGCCTT AGTATATTCA TATACTTAGT ATTGACTTTT CCTTGCCACA GATTTCTCCA 11761 ATCCACCAAT TCCACTGTGC CAGAAAGTAA AAAGCCATGA TATTCAAATT TTCTCAACTT 11821 TGATCAAAGG CTCATTCAAG ACCAGTGCCT TTTCCACTGG TCCCAATCTA CTGGAAATGC 11881 AGACAGTATT TTGCCTTCTC TGGGCAAGAA AGTTATAAAG TAGAGGGAAA TCATAATAGA 11941 GAGCTATGAG AGAACAAGAT TTGATTTGAT TTAATTTGAT GGACTCAAGT TTTAACATTG 12001 TAAAACTAGA GATAAGACAT CACCACCAAT CTAGAAAAGT GATGCAGAAA AGTATTTGAT 12061 TTGGGTAATT ATTACACTCA CCTAGAAACA AGTGTTGTGT AATAGATTAC ATATTTCCAT 12121 AATGCAATGT TGTATCAGAA ACTACCTTCC TAAGAAAATA TAGTATGGGC TCGGCGTGGT 12181 GGCTCGCACC TGTAATCCCA GCACTTTGGG AGATGGAGGC AGGAGGATCA CTTGAGCCCA 12241 GACTGGGCAA CAAAGCGAGA CCCTGTCTCA ACAAAAAATT TAAAAATTAG CTGAGTGTGG 12301 TGGCACGCAC TGATGGTCCC CTCTACTTGG GAAGCTGAGG CAAGAGGATC TCCTGAGCCC 12361 AGGAGTTCAA GGTTTCAGCG AGCTATGATT GTGCCACTGC ACTCCAGCCT GGGAGACAGA 12421 GCAAGTCCCT GTCTCAAAAA AGAAGAAGGA GAAGGAGGAG AAAATACAGT ATTAAGTAAT 12481 CTGTCAATAT ATTCCACAAG GATTACACTA GTGGTTTAAT AATAAAATTA TATTACCTTT 12541 TTAAATTGTA AGGCCATTCC TCAAGCTTTA TAAATTAAGC ATGAATGCAT CATACACATT 12601 TTATAAAAAG TTCCAACTCA TCATAATCTG TACTTATGAT ACATTAATAC AAATGAAGTT 12661 CATTATAAAA TTAACTTAAA ATGGATATAC CAGTTATTAA ACCATTAACC ATTTAATAAT 12721 TTTATTTTTT TCAAATTTAA AAACCTTTTG GGGAAGAAAT ACTACAACAT GGATGAACCT 12781 TGAAAACGTT ATGCTAAGTG AAATAAGCCA GACACAAAAG GACAAATACT GTATGATTAC 12841 ACTTAAATGA GGTACCTAGA GTAGTCAAAT TCATAGAGAC AGAAAGAATA GAAGTTACCA 12901 GGGGCTGGAG GTAGGAAAAA ATGGAGAGCT GTTTAATGGG TAGAGAGTTT CTTTTTGGGG 12961 TGACAAAAAG GTTCTAGAGA TGGATAGTGG TGATGGTTAC ACACAATGTG TGTGTACTTA 13021 ATGCTACTGA AATGTAATTT TATGATTTTT TTTTTTTGCA GCAAAATACC CCACATTGGG 13081 AAGTGAAGAG AAACATGTTA AGAGACTTGA AGGAAAAAAA TTGGGGCAGA GGGGTGTTTT 13141 TTATAGGTTA AACAATAAAA GCCATTTAAA CAGTAACAAT TTCTCTAAGG ACAAGAATCG 13201 TCAAGATTGA GACAGCACTG ATTTCTTGAC TCTACTCAAT ACTTCTTTGG TTTCTCTTCT 13261 TCCTTCCCCC TTCTAATAGT TTCCTACCTC CCATTCAGAA AGCAAAGCAA AACAAGCAAA 13321 AATTCCCCCT TCCCTCAAAA AAGGAAAGAG TTTTTGAAAA AGTTCATGTC AGTGAAGAAA 13381 |
| AGACATGTTT TGGGAGTGAA GGATATTTGT GGATTTGTAT AGATGTGATC ATCAGGGCTG 13441 TGTTGTTTTG AAGTAATATA GGACATCTAG AGGAAAATTT ATTTTCAGCA GAGGAGGGAA 13501 AGATGAAGAG TAGGTACTTT TAAGCATCTT CACTTGAGGA GTGGCAAAAT GAGAAGCATA 13561 ACCTGCTATA ATCACTTTAA GAATTTCAGG CTGAGTGTGG TGGTGCAGTC TCTAGTCCCA 13621 GTTACTCCAG GAGGCTCAGG TGGGAGGATC ACTTAAGCCC AGGAGCTCGA GGTTGCAGTG 13681 AGCTATGATT ACACTACTGC ATTCCAGCCT GGGCGGCAGG GTGAAGCCTC ATCTCAAAAA 13741 TTAAAAAAAA AAAAAATCAA ACAAATTAAT CGAACGATGA CATGCACTTT TCTAGGTTGG 13801 TACCTTCCTT CTTGCGACTT GGATCCTGGA ACGTCATTAT GTTTGTGTGC TTTGAACAAC 13861 TGAAACGAGA ACTGTCAAAG TCAAGGCAGA CTATGGACTG TGCCACATAA TCAGCTTCAA 13921 GAAAATGATG TAACATACCA GTGGGAATCT TGCTGACTGG ATCATAAAAA CAAACAAAAC 13981 TTATTCACTT ATTTTAACCT AAAAAGATAA AGGAATTTTG GCAGAGAATT TTGGACTTTT 14041 TTATATAAAA AAGAGGAAAA TTAATGCCTA TTTCATATAA CTTTTTTTTT TTCTCAGTGT 14101 CTTAAGAAGG GGAAAGCAAA ACATTCAGCA TATACCCTGG CAAATGTAAT GCAGATAAGC 14161 TACTGCATTT GACCATTTCT GGAGTGCAAT TGTGTGAATG AATGTGAAGA ACTTTAACAT 14221 GTTTTAATTA CAATTCCAAC TGGTGGAAAA GAAACTGAGT GAAATGCAGT TTATATTTAT 14281 AAATACTTAA AAATGAAGTT ATTAAAAATA TTAGTTTTTA TTAACCACAG TTGTCAGTTA 14341 ATATATTCAA TAAAGTATTG CTAATACCTT TTAAAGTTTG TCTTTTGAGA TCTATACCTG 14401 GGTGTAAGAG TCAAGTTCAC TAGAATACAA GACTGCCCAA TAGCAAATGC AGGTCTTTAG 14461 AATCATAGGC ATGAACCTAC TCTGAATGTT ATTAGTATAG ATTTTTAATG TTTAGAGTCC 14521 AGATTTGATG ACATCTCTAA CAACTTCTAA TCTAAGACAC TATATTCATT TTGGCAGGAT 14581 TGCTACTAGA GTCTTGGTAT CTGTGCTAGC ATCACATAAT TTTAGAGCTG GAGGGTACTT 14641 CTGGGAAGAC AGAGGAACAG TTTGAGATTC CTACTGAGAT GAAAACGAAT CTTCATGGAA 14701 TCTTTCAGCA AAGCCAAATT CAAATTCATC ATTAGCACCT GTAGTAACCT TTTCAATGCC 14761 TACAAACTGC ATGCAGAAGA GATAGGGAAA CAGTAAAACA GATATTAAAA GAAGTTTTTA 14821 AGACAAAGCC CAGCCTGATT TTAAGCTAAA TCCAAGGATT GGCAGCTTGG ATGAGCAGGA 14881 AGGTTACAGG CTGCCAGACA TCATTCTAGT TCTGTTTTAA TCAACTCCAT GTTACATTTA 14941 CTATCAGGGA TTCTCACCTC ACCCTCATGC ATGTCTTCCC CATTCATTAC CCGCAAAAGT 15001 GTCTTGTAGC AGATGTCTTC TGTGTCCCAT ACATACCATT TTGCTCTTTA GTGCTTGCTG 15061 GCCTGACTTC CTATTGTCAT GTCAGCATCT GCCCTTTTTA GGGTCTCTGG CCACCAGAGC 15121 CAGCTTTACT CACCTGTGCA TGGCATTCTA GAAGAGCAGC AGGGAAAATA ACACAGCCCC 15181 AGTGCAGCCC TTAACCACCA ATAACTGGTA GTAGTTGGTG TACAAATATC TCAGTTCCCT 15241 CAACTGTCAG GTGGAATACC GCTGAGGGAT CAAACTCTAG TAACACACAG TAGTGTTTTG 15301 CTTACTATGG TTAACTAAAA AATCACAGGG TCTTCATGCA TTTGGAAAGG ATACTTTATT 15361 TCTTACAAAG GGTTACAGCC TACAAGGTGG TCATTCTGCA GGCTAGAAAG CGTAACCTCC 15421 AGCAAAGACC GGAGGCAGGC ACTTCTAGGG AAGGAAGAGT AAGACAGAAA TTTAAATTGA 15481 ATGGGTTGGC CAAGTATACA TATTCAACAG GCTACAGGTG GATTCATGAA TATTCATGAA 15541 GGCAGTCCTG ATGCATGCAT GTTACACCTT GGGGTGGAGG CTTAACATTT AAATGTATTA 15601 CAGTTAGGCC CTATACATGA AAAGGTGAAG CAGTAACACG AAGGCACACA ATGCACCATT 15661 TCTGTAAACA GGCCAGAGCC AGTTCACAGT GGTTGGTCTC TTATCATGAG AAAGCTACTA 15721 AAATCCTCTT GTCCAGTTAA AACTGTAGTT ATGGCTGGTG GAAAATGGGC TGGAGTCAGT 15781 CAACACTTGG TGAAGCTGCA GTTGCTTCAG ACACTCAAGG CCAGTGTTTG TTTAGCTGCT 15841 CGAGAAAAAG AAAAATCTTG TGGCAGTTAG AACATAGTTT ATTCTTTAAG TGTAGGAGTG 15901 TGTGACTTAA // |

**Table 28.**

| **Nucleic acid sequence of the 2,113 base pair (bp) transcript ENST00000310473 of the human UCP2 gene (Eight Coding Exons are in capital letters):** | | | | | | |
|---|---|---|---|---|---|---|
| **No.** | **Exon / Intron** | **Start** | **End** | **Length** | **Sequence** | **SEQ ID NO** |
| | 5' upstream sequence | | | | ..........aatcgacagcgaggccggtcgcgag gccccagtcccgccctgcaggagcc | 638 |
| 1 | ENSE00002287650 | 73,694,352 | 73,693,766 | 587 | AGCCGCGCGCTCGCTCGCAG GAGGGTGGGTAGTTTGCCCA | 639 |
| | | | | | GCGTAGGGGGCTGGGCCCA TAAAAGAGGAAGTGCACTTA AGACACGGCCCCGCTGGACG CTGTTAGAAACCGTCCTGGC TGGGAAGGCAAGAGGTGTGT GACTGGACAAGACTTGTTTC TGGCGGTCAGTCTTGCCATCC TCACAGAGGTTGGCGGCCCG AGAGAGTGTGAGGCAGAGGC GGGGAGTGGCAAGGGAGTG ACCATCTCGGGGAACGAAGG AGTAAACGCGGTGATGGGAC GCACGGAAACGGGAGTGGA GAAAGTCATGGAGAGAACCC TAGGCGGGGCGGTCCCCGCG GAAAGGCGGCTGCTCCAGGG T CTCCGCACCCAAGTAGGAGC TGGCAGGCCCGGCCCCGCCC CGCAGGCCCCACCCCGGGCC CCGCCCCCGAGGCTTAAGCC GCGCCGCCGCCTGCGCGGAG CCCCACTGCGAAGCCCAGCT GCGCGCGCCTTGGGATTGAC TGTCCACGCTCGCCCGGCTC GTCCGACGCGCCCTCCGCCA GCCGACAGACACAGCCGCAC GCACTGCCGTGTTCTCCCTGC GGCTCG | |
| | Intron 1-2 | 73,693,765 | 73,692,678 | 1,088 | gtgagcctggccccagccctgcgcc.......... actctctgcctttgctcacccacag | 640 |
| | | | | | | 641 |
| 2 | ENSE00001184362 | 73,692,677 | 73,692,521 | 157 | GACACATAGTATGACCATTA GGTGTTTCGTCTCCCACCCAT TTTCTATGGAAAACCAAGGG GATCGGGCCATGATAGCCAC TGGCAGCTTTGAAGAACGGG ACACCTTTAGAGAAGCTTGA TCTTGGAGGCCTCACCGTGA GACCTTACAAAGCCGG | 642 |
| | Intron 2-3 | 73,692,520 | 73,689,523 | 2,998 | gtaagagtccagtccaaggaagagg.......... tggggctttctcctcttggcttag | 643 |
| | | | | | | 644 |
| 3 | ENSE00001184370 | 73,689,522 | 73,689,298 | 225 | ATTCCGGCAGAGTTCCTCTAT CTCGTCTTGTTGCTGATTAAA GGTGCCCCTGTCTCCAGTTTT TCTCCATCTCCTGGGACGTAG CAGGAAATCAGCATCATGGT TGGGTTCAAGGCCACAGATG TGCCCCCTACTGCCACTGTGA AGTTTCTTGGGGCTGGCACA GCTGCCTGCATCGCAGATCT CATCACCTTTCCTCTGGATAC TGCTAAAGTCCGGTTACAG | 645 |
| | Intron 3-4 | 73,689,297 | 73,689,142 | 156 | gtgaggggatgaagcctgggagtct.......... tagctaccctgtcttggccttgcag | 646 |
| | | | | | | 647 |
| 4 | ENSE00001252503 | 73,689,141 | 73,688,931 | 211 | ATCCAAGGAGAAAGTCAGGG GCCAGTGCGCGCTACAGCCA GCGCCCAGTACCGCGGTGTG ATGGGCACCATTCTGACCAT GGTGCGTACTGAGGGCCCCC GAAGCCTCTACAATGGGCTG GTTGCCGGCCTGCAGCGCCA AATGAGCTTTGCCTCTGTCCG CATCGGCCTGTATGATTCTGT CAAACAGTTCTACACCAAGG GCTCTGAGC | 648 |
| | Intron 4-5 | 73,688,930 | 73,688,063 | 868 | gtgagtatggagcaagggtgtaggc.......... cactgaccccatggctcgcccacag | 649 |
| | | | | | | 650 |
| 5 | ENSE00001184355 | 73,688,062 | 73,687,868 | 195 | ATGCCAGCATTGGGAGCCGC CTCCTAGCAGGCAGCACCAC AGGTGCCCTGGCTGTGGCTG TGGCCCAGCCCACGGATGTG GTAAAGGTCCGATTCCAAGC TCAGGCCCGGGCTGGAGGTG GTCGGAGATACCAAAGCACC GTCAATGCCTACAAGACCAT TGCCCGAGAGGAAGGGTTCC GGGGCCTCTGGAAAG | 651 |
| | Intron 5-6 | 73,687,867 | 73,687,788 | 80 | gtgtgtaccagttgttttcccttcc.......... acccaggatcttcctcctcctacag | 652 |
| | | | | | | 653 |
| 6 | ENSE00003361285 | 73,687,787 | 73,687,686 | 102 | GGACCTCTCCCAATGTTGCTC GTAATGCCATTGTCAACTGT GCTGAGCTGGTGACCTATGA CCTCATCAAGGATGCCCTCCT GAAAGCCAACCTCATGACAG | 654 |
| | Intron 6-7 | 73,687,685 | 73,686,717 | 969 | gtgagtcatgaggtagacggtgctg.......... tgccttgcctgctcctccttggcag | 655 |
| | | | | | | 656 |
| 7 | ENSE00001184349 | 73,686,716 | 73,686,536 | 181 | ATGACCTCCCTTGCCACTTCA CTTCTGCCTTTGGGGCAGGCT TCTGCACCACTGTCATCGCCT CCCCTGTAGACGTGGTCAAG ACGAGATACATGAACTCTGC CCTGGGCCAGTACAGTAGCG CTGGCCACTGTGCCCTTACCA TGCTCCAGAAGGAGGGGCCC CGAGCCTTCTACAAAGG | 657 |
| | Intron 7-8 | 73,686,535 | 73,686,167 | 369 | gtgagcctctggtcctccccaccca.......... atgacctgtgatttttctcctctag | 658 |
| | | | | | | 659 |
| 8 | ENSE00001184368 | 73,686,166 | 73,685,712 | 455 | GTTCATGCCCTCCTTTCTCCG CTTGGGTTCCTGGAACGTGG TGATGTTCGTCACCTATGAGC AGCTGAAACGAGCCCTCATG GCTGCCTGCACTTCCCGAGA GGCTCCCTTCTGAGCCTCTCC TGCTGCTGACCTGATCACCTC | 660 |
| | | | | | TGGCTTTGTCTCTAGCCGGGC CATGCTTTCCTTTTCTTCCTTC TTTCTCTTCCCTCCTTCCCTTC TCTCCTTCCCTCTTTCCCCAC CTCTTCCTTCCGCTCCTTTAC CTACCACCTTCCCTCTTTCTA CATTCTCATCTACTCATTGTC TCAGTGCTGGTGGAGTTGAC ATTTGACAGTGTGGGAGGCC TCGTACCAGCCAGGATCCCA AGCGTCCCGTCCCTTGGAAA GTTCAGCCAGAATCTTCGTCC TGCCCCCGACAGCCCAGCCT AGCCCA CTTGTCATCCATAAAGCAAG CTCAACCTTGGCGTC | |
| | 3' downstream sequence | | | | tcctccctctcttgtagctcttaccagaggtcttg gtccaatggcctttt.......... | 661 |

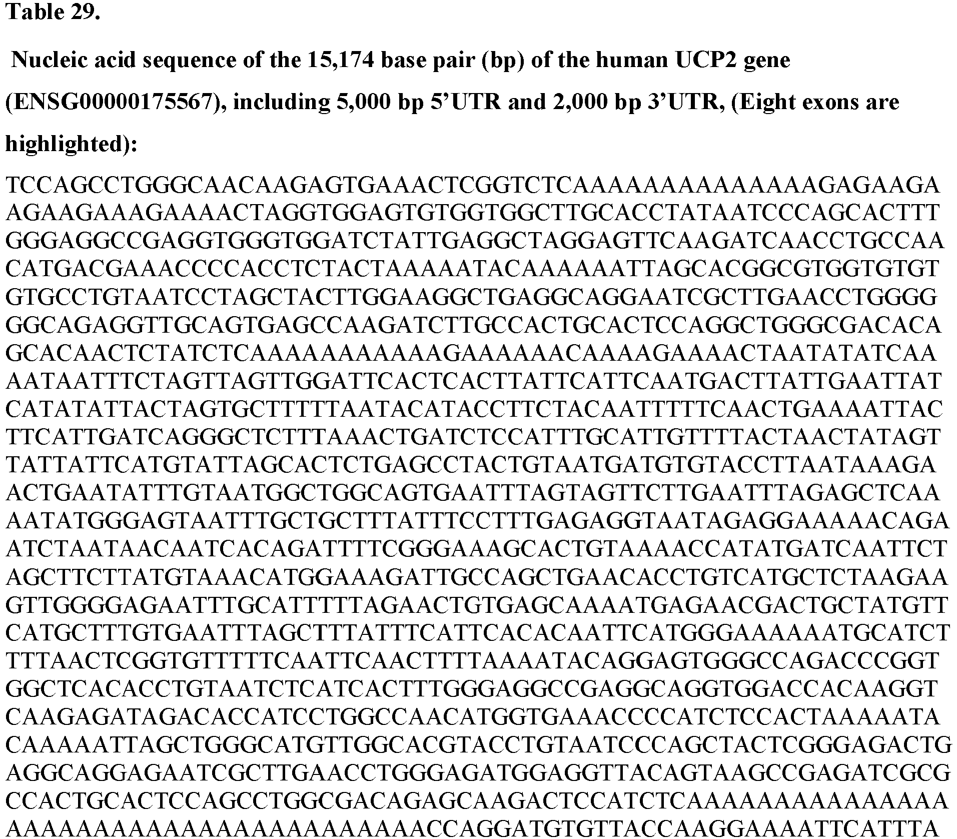

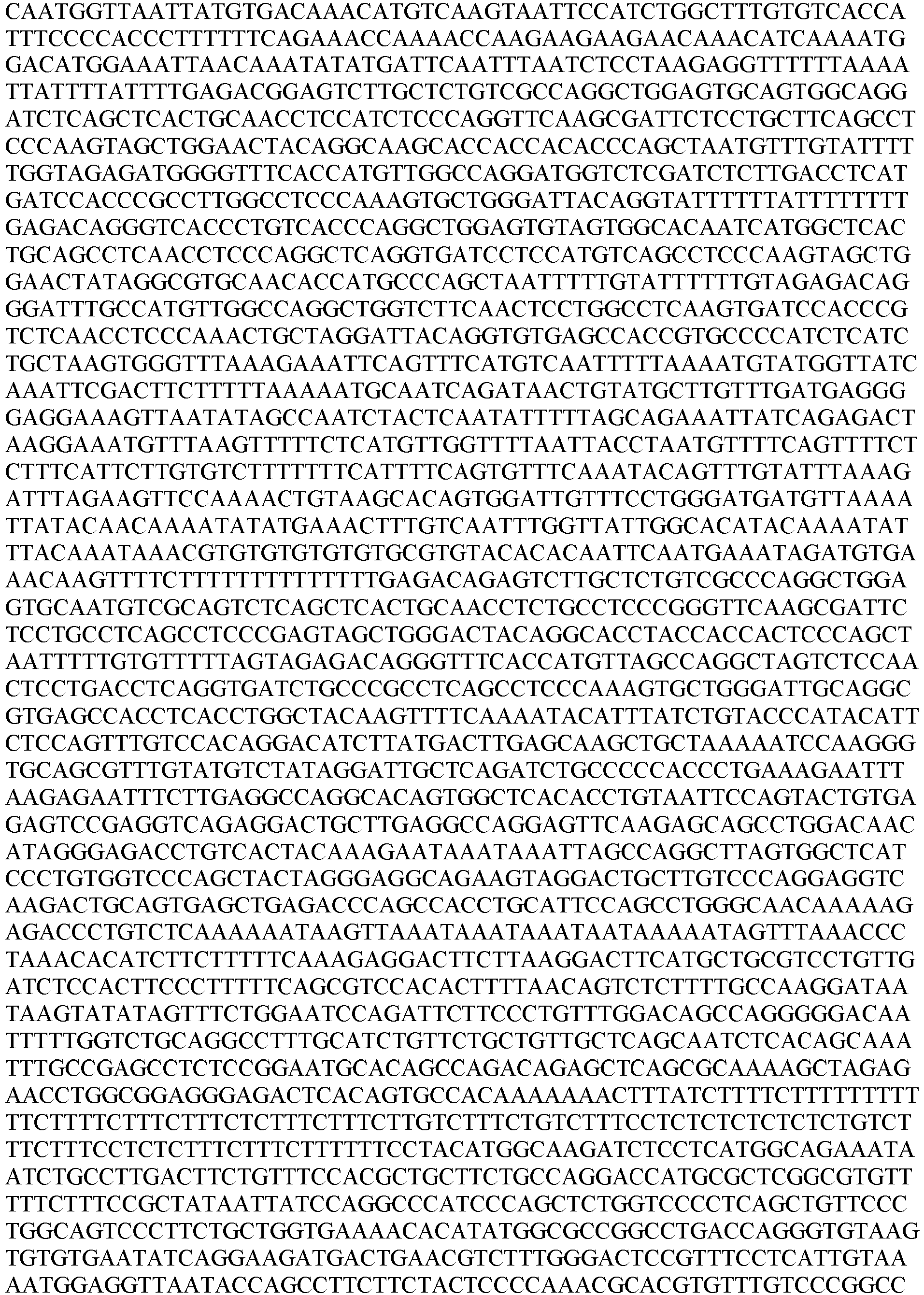

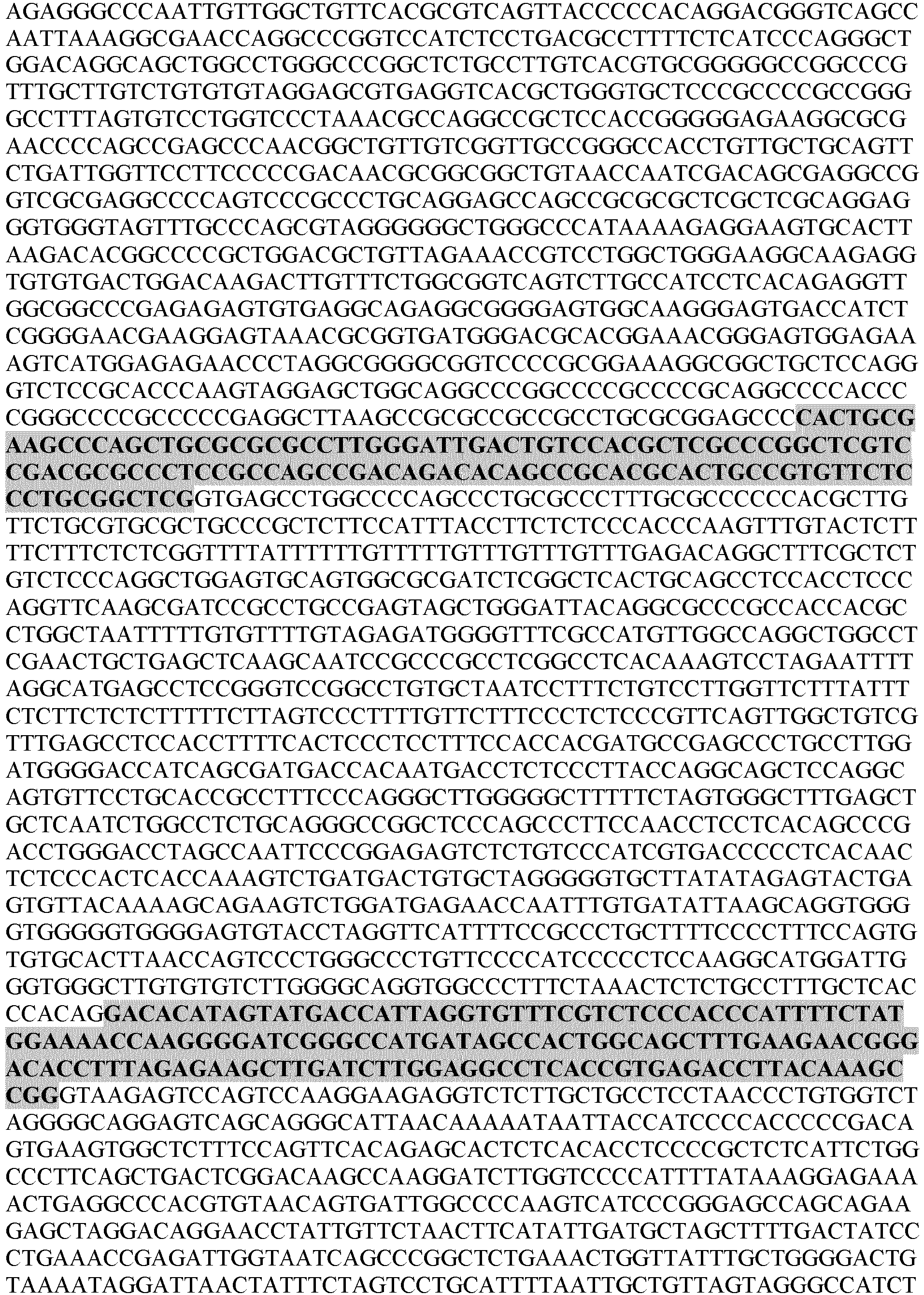

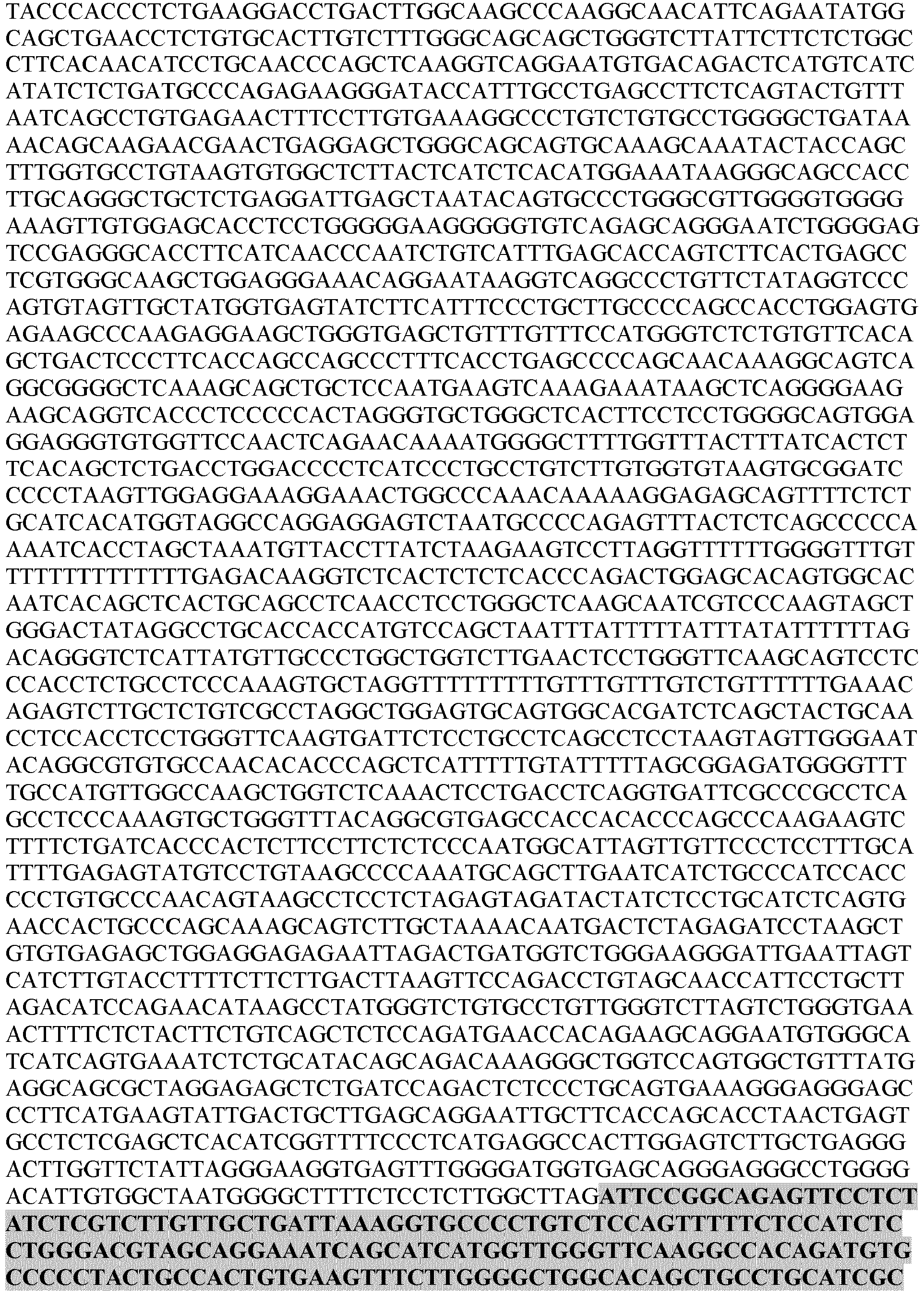

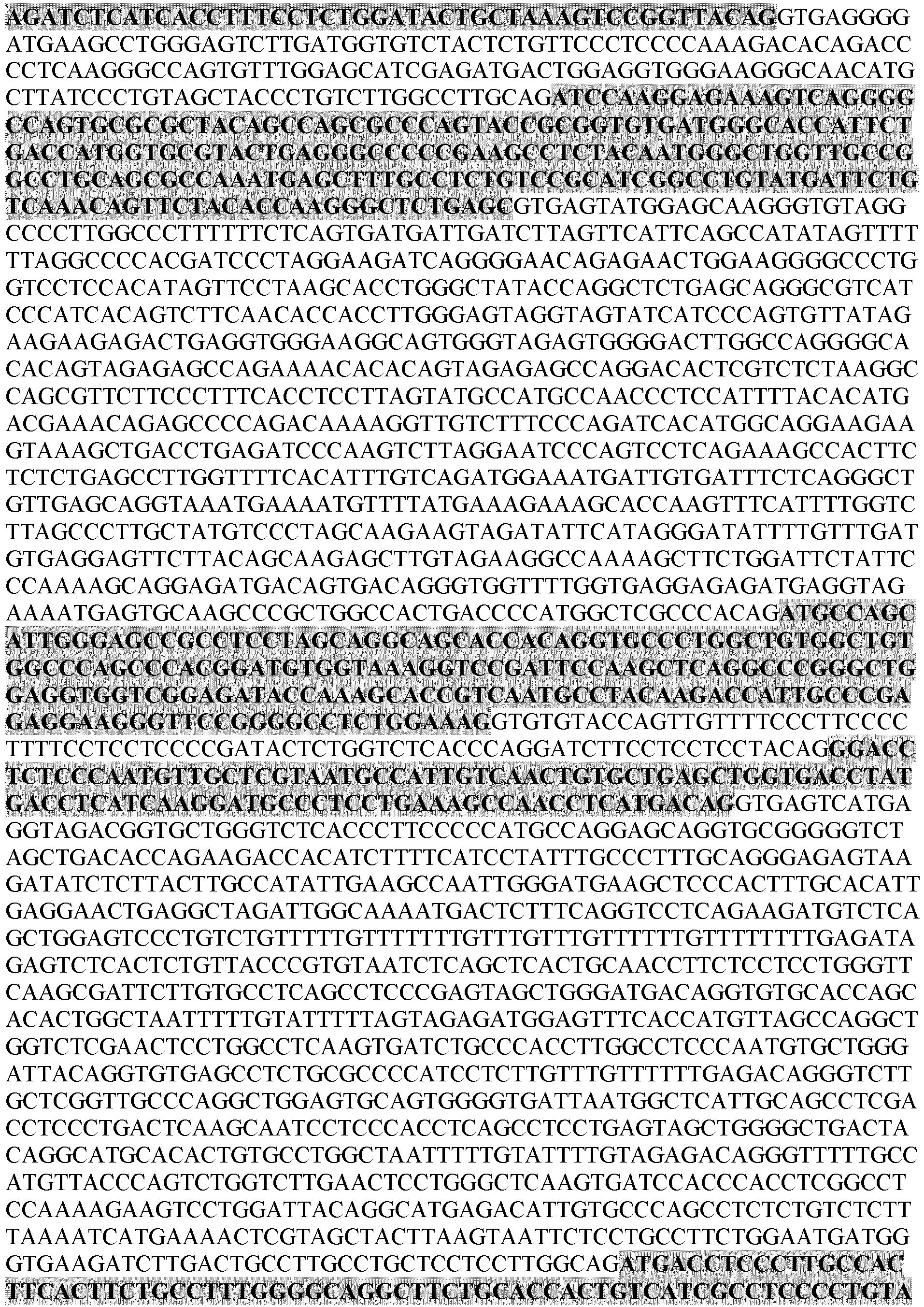

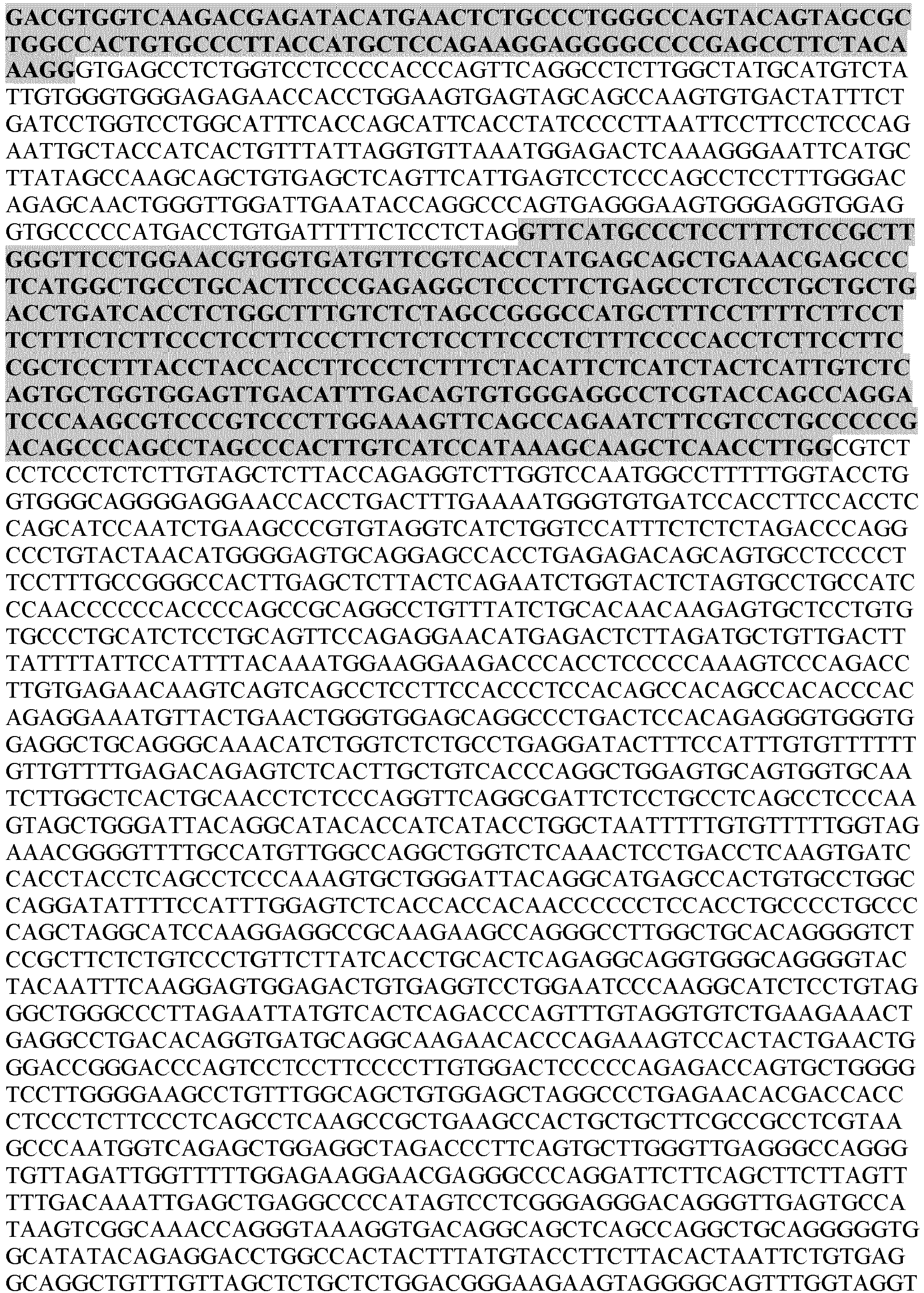

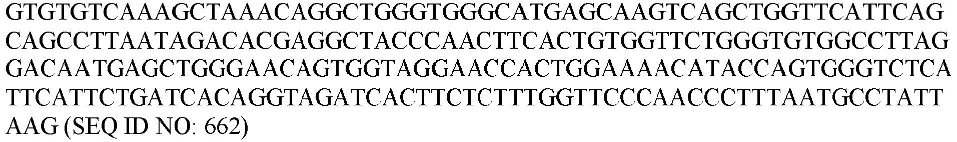

## Claims

1. An antagomir of hsa-miR-22-3p for use in a method of treating diabetes or obesity in a human subject in need of treatment thereof.

2. The antagomir for use according to claim **1,** wherein said diabetes is type 2 diabetes.

3. The antagomir for use according to claim **1,** wherein the human subject selected for treatment of obesity has a genetic or epigenetic predisposition to obesity.

4. The antagomir for use according to any one of claims **1** to **3,** wherein said antagomir modulates respiratory chain uncoupling in a cell, preferably said cell being a preadipocyte, adipocyte, adipose tissue-derived mesenchymal stem cell, hepatocyte, myocyte or a precursor thereof.

5. The antagomir for use according to any one of claims **1** to **4,** wherein said antagomir modulates thermogenesis in a tissue, preferably said tissue being brown fat, white fat, subcutaneous adipose tissue, liver or muscle.

6. The antagomir for use according to any one of claims **1** to **5,** wherein said antagomir causes fat loss in the subject.

7. The antagomir for use according to any one of claims **1** to **6,** wherein said antagomir is linked to a targeting moiety, which preferably delivers said antagomir to a specific cell type or tissue.

8. The antagomir for use according to any one of claims **1** to **7,** wherein said antagomir causes the mRNA or protein expression of at least one mitochondrial uncoupler to be upregulated, or wherein said antagomir causes the mitochondrial uncoupling activity of at least one mitochondrial uncoupler to be upregulated.

9. The antagomir for use according to any one of claims **1** to **8,** wherein said antagomir increases expression or activity of UCP1 in a cell.

10. An *in vitro* or *ex vivo* method of:
- modulating respiratory chain uncoupling in a cell;
- modulating thermogenesis in a tissue; or
- increasing expression or activity of at least one thermogenic regulator in a cell, preferably of UCP1;
comprising contacting said cell or tissue with an antagomir of hsa-miR-22-3p.

11. The *in vitro* or *ex vivo* method according to claim **10,** wherein said antagomir is linked to a targeting moiety, which preferably delivers said antagomir to a specific cell type or tissue.

12. The *in vitro* or *ex vivo* method according to claim **10** or **11,** wherein said cell is a preadipocyte, adipocyte, adipose tissue-derived mesenchymal stem cell, hepatocyte, myocyte or a precursor thereof; or said tissue is brown fat, white fat, subcutaneous adipose tissue, liver or muscle, and is preferably contacted with said antagomir *ex vivo.*

## Patentansprüche

1. Antagomir von hsa-miR-22-3p zur Verwendung in einem Verfahren zur Behandlung von Diabetes oder Adipositas bei einem menschlichen Patienten, der einer Behandlung davon bedarf.

2. Antagomir zur Verwendung nach Anspruch 1, wobei es sich bei dem Diabetes um Typ-2-Diabetes handelt.

3. Antagomir zur Verwendung nach Anspruch 1, wobei der menschliche Patient, der für die Behandlung von Adipositas ausgewählt wurde, eine genetische oder epigenetische Prädisposition für Adipositas hat.

4. Antagomir zur Verwendung nach einem der Ansprüche 1 bis 3, wobei das Antagomir die Entkopplung der Atmungskette in einer Zelle moduliert, wobei die Zelle vorzugweise ein Präadipozyt, ein Adipozyt, eine aus Fettgewebe abgeleitete mesenchymale Stammzelle, ein Hepatozyt, ein Myozyt oder eine Vorläuferzelle davon ist.

5. Antagomir zur Verwendung nach einem der Ansprüche 1 bis 4, wobei das Antagomir die Thermogenese in einem Gewebe moduliert, wobei das Gewebe vorzugsweise braunes Fettgewebe, weißes Fettgewebe, Unterhautfettgewebe, Lebergewebe oder Muskelgewebe ist.

6. Antagomir zur Verwendung nach einem der Ansprüche 1 bis 5, wobei das Antagomir Fettabbau bei dem Patienten auslöst.

7. Antagomir zur Verwendung nach einem der Ansprüche 1 bis 6, wobei das Antagomir mit einer Zieleinheit verbunden ist, die das Antagomir vorzugsweise zu einem spezifischen Zelltyp oder Gewebe bringt.

8. Antagomir zur Verwendung nach einem der Ansprüche 1 bis 7, wobei das Antagomir bewirkt, dass die mRNA- oder Proteinexpression von wenigstens einem mitochondrialen Entkoppler heraufreguliert wird, oder wobei das Antagomir bewirkt, dass die mitochondriale Entkopplungsaktivität von wenigstens einem mitochondrialen Entkoppler heraufreguliert wird.

9. Antagomir zur Verwendung nach einem der Ansprüche 1 bis 8, wobei das Antagomir die Expression oder Aktivität von UCP1 in einer Zelle erhöht.

10. *In-vitro-* oder *Ex-vivo*-Verfahren zum:
- Modulieren der Entkopplung der Atmungskette in einer Zelle,
- Modulieren der Thermogenese in einem Gewebe oder
- Erhöhen der Expression oder Aktivität von wenigstens einem thermogenen Regler in einer Zelle, vorzugsweise von UCP1,
umfassend das Inkontaktbringen der Zelle oder des Gewebes mit einem Antagomir von hsa-miR-22-3p.

11. *In-vitro-* oder *Ex-vivo*-Verfahren nach Anspruch 10, wobei das Antagomir mit einer Zieleinheit verbunden ist, die das Antagomir vorzugsweise zu einem spezifischen Zelltyp oder Gewebe bringt.

12. *In-vitro-* oder *Ex-vivo*-Verfahren nach Anspruch 10 oder 11, wobei die Zelle ein Präadipozyt, ein Adipozyt, eine aus Fettgewebe abgeleitete mesenchymale Stammzelle, ein Hepatozyt, ein Myozyt oder eine Vorläuferzelle davon ist, oder wobei das Gewebe braunes Fettgewebe, weißes Fettgewebe, Unterhautfettgewebe, Lebergewebe oder Muskelgewebe ist und vorzugsweise *ex vivo* mit dem Antagomir in Kontakt gebracht wird.

## Revendications

1. Antagomir de hsa-miR-22-3p destiné à être utilisé dans un procédé de traitement du diabète ou de l'obésité chez un sujet humain nécessitant son traitement.

2. Antagomir destiné à être utilisé selon la revendication 1, où ledit diabète est le diabète de type 2.

3. Antagomir destiné à être utilisé selon la revendication 1, où le sujet humain choisi pour le traitement de l'obésité a une prédisposition génétique ou épigénétique à l'obésité.

4. Antagomir destiné à être utilisé selon l'une quelconque des revendications 1 à 3, où ledit antagomir module le découplage de la chaîne respiratoire dans une cellule, de préférence ladite cellule étant un préadipocyte, un adipocyte, une cellule souche mésenchymateuse dérivée de tissu adipeux, un hépatocyte, un myocyte ou un précurseur de ceux-ci.

5. Antagomir destiné à être utilisé selon l'une quelconque des revendications 1 à 4, où ledit antagomir module la thermogenèse dans un tissu, de préférence ledit tissu étant de la graisse brune, de la graisse blanche, du tissu adipeux sous-cutané, du foie ou du muscle.

6. Antagomir destiné à être utilisé selon l'une quelconque des revendications 1 à 5, où ledit antagomir provoque une perte de graisse chez le sujet.

7. Antagomir destiné à être utilisé selon l'une quelconque des revendications 1 à 6, où ledit antagomir est lié à un groupe de ciblage, qui de préférence délivre ledit antagomir à un type de cellule ou tissu spécifique.

8. Antagomir destiné à être utilisé selon l'une quelconque des revendications 1 à 7, où ledit antagomir régule positivement l'expression d'ARNm ou de protéine d'au moins un découpleur mitochondrial, ou bien où ledit antagomir régule positivement l'activité de découplage mitochondrial d'au moins un découpleur mitochondrial.

9. Antagomir destiné à être utilisé selon l'une quelconque des revendications 1 à 8, où ledit antagomir augmente l'expression ou l'activité de UCP1 dans une cellule.

10. Procédé *in vitro* ou *ex vivo* de:
- modulation du découplage de la chaîne respiratoire dans une cellule;
- modulation de la thermogenèse dans un tissu; ou
- augmentation de l'expression ou de l'activité d'au moins un régulateur thermogène dans une cellule, de préférence de UCP1;
comprenant la mise en contact de ladite cellule ou dudit tissu avec un antagomir de hsa-miR-22-3p.

11. Procédé *in vitro* ou *ex vivo* selon la revendication 10, où ledit antagomir est lié à un groupe de ciblage, qui de préférence délivre ledit antagomir à un type de cellule ou tissu spécifique.

12. Procédé *in vitro* ou *ex vivo* selon la revendication 10 ou 11, où ladite cellule est un préadipocyte, un adipocyte, une cellule souche mésenchymateuse dérivée de tissu adipeux, un hépatocyte, un myocyte ou un précurseur de ceux-ci; ou ledit tissu est de la graisse brune, de la graisse blanche, du tissu adipeux sous-cutané, du foie ou du muscle, et est de préférence mis en contact avec ledit antagomir *ex vivo.*
